# EUROPEAN PATENT APPLICATION

(11) **EP 4 488 274 A2**
(43) Date of publication of application: **08.01.2025**
(21) Application number: 24214832.8
(22) Date of filing: 30.10.2020
(51) Int. Cl.: C07D 413/12

(54) **HERBICIDE AND PRODUCTION METHOD FOR INTERMEDIATE THEREOF**

(30) Priority: 31.10.2019 JP 2019198600
(62) Divisional of application: 20835297.1
(71) Applicant: Kumiai Chemical Industry Co., Ltd., Taito-ku Tokyo 110-8782 (JP)
(72) Inventor: The designation of the inventor has not yet been filed
(74) Representative: Kraus & Lederer PartGmbB

(57) **Abstract**

The present invention provides a sulfone derivative that is useful as a herbicide, and an industrially preferable production method for an intermediate of said derivative. Provided is a production method for a compound of formula (4), the method including step ii, wherein step ii is a step for producing a compound of formula (4) by reacting a compound of formula (2) with a compound of formula (3) in the presence of a base. Also provided is a production method for a compound of formula (5), the method including step iii, wherein step iii is a step for producing a compound of formula (5) by reacting a compound of formula (4) with hydrogen peroxide in the presence of a metal catalyst.

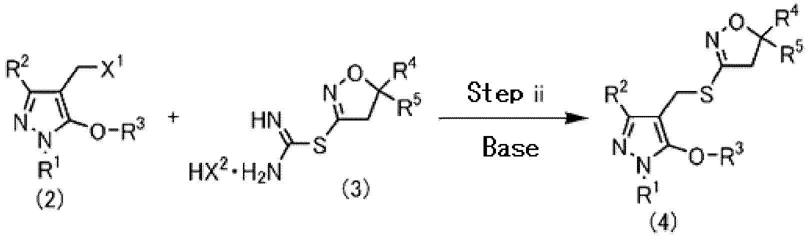

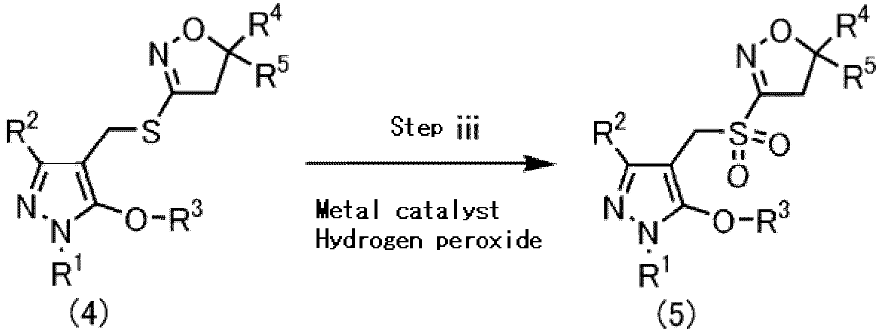

## Description

### Technical Field

The present invention relates to a process for producing a sulfone derivative useful as a herbicide, that is, a compound of the following formula (5): 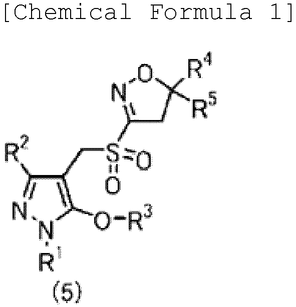 wherein R¹, R², R³, R⁴ and R⁵ are as described herein.

### Background Art

It is known that sulfone derivatives of the above formula (5) have a herbicidal activity as disclosed in WO 2002/062770 A1 (Patent Document 1). Among them, pyroxasulfone is well known as a superior herbicide.

As a process for producing the compound of the formula (5), a process by the oxidation of a sulfide derivative, i.e., a compound of the following formula (4) has been known, which is shown below.

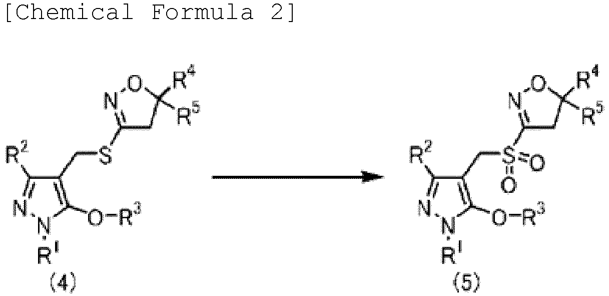

As shown in the following scheme, in Reference Example 3 in WO 2004/013106 A1 (Patent Document 2) is disclosed a process for producing 3-(5-difluoromethoxy-1-methyl-3-trifluoromethyl-1H-pyrazol-4-ylmethanesulfonyl)-5,5-dimethyl-2-isoxazoline (5-a) (pyroxasulfone) by oxidizing 3-(5-difluoromethoxy-1-methyl-3-trifluoromethyl-1H-pyrazol-4-ylmethylthio)-5,5-dimethyl-2-isoxazoline (4-a) (ISFP) with m-chloroperoxybenzoic acid (mCPBA).

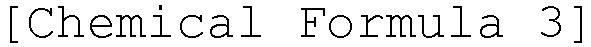

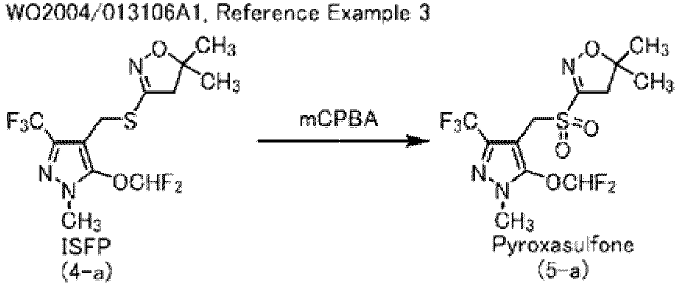

In addition, WO 2004/013106 A1 (Patent Document 2) and JP 2008-544966 A (corresponding to US 2007/015805 A1) (Patent Document 9) describe a process for producing the compound of the formula (4-a). However, the process described in WO 2004/013106 A1 (Patent Document 2) and JP 2008-544966 A (corresponding to US 2007/015805 A1) (Patent Document 9) has a plurality of problems, e.g., the yield is poor and the preparation of starting materials is inefficient.

On the other hand, the preparation of the compound of the formula (4-a) is also disclosed in WO 2005/095352 A1 (Patent Document 3) and WO 2005/105755 A1 (Patent Document 4), and these are shown below.

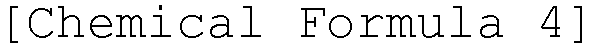

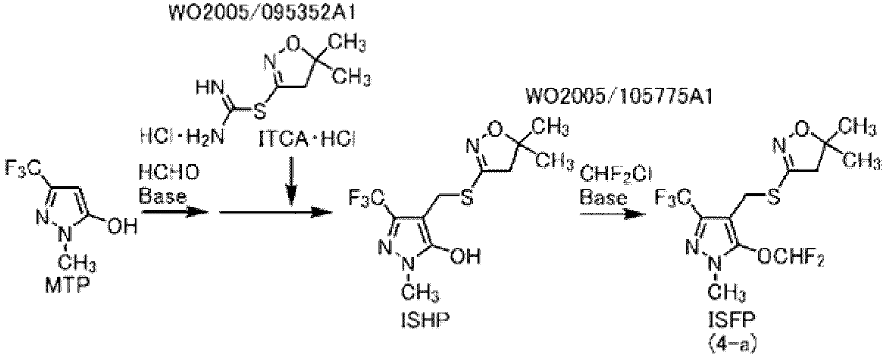

The process described in WO 2005/095352 A1 (Patent Document 3) and WO 2005/105755 A1 (Patent Document 4) is a superior process that has solved the problems in the preparation of the compound of the formula (4-a) described in WO 2004/013106 A1 (Patent Document 2) and JP 2008-544966 A (corresponding to US 2007/015805 A1) (Patent Document 9). On the other hand, there is still room for improvement in this process because a special manufacturing equipment (hermetically closed equipment) is required for the sensitization of the intermediate (ISHP in the above scheme).

As a further problem, in the process for producing the compound of the formula (5) from the compound of the formula (4), m-chloroperoxybenzoic acid (mCPBA) disclosed in WO 2004/013106 A1 (Patent Document 2) is expensive for industrial use and has problems in handling and waste. Therefore, the process for producing described in WO 2004/013106 A1 (Patent Document 2) is not practical for production on an industrial scale.

In addition, in the process for producing the compound of the formula (5) (sulfone derivative: SO₂ derivative) from the compound of the formula (4) (sulfide derivative: S derivative), there is a possibility that the reaction stops at a sulfoxide derivative (SO derivative) that is an intermediate of the oxidation reaction, i.e., a compound of the following formula (6): 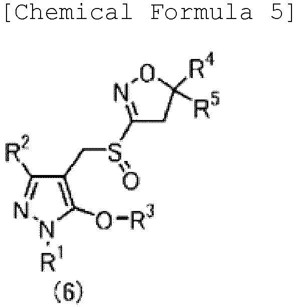 wherein R¹, R², R³, R⁴ and R⁵ are as described herein. Therefore, the compound of the formula (6) sometimes remains in the product as a by-product. The compound of the formula (6) that has contaminated a product such as a herbicide leads to the possibility of reduced quality and crop injury. However, the physical and chemical properties of the compound of the formula (6) are very similar to those of the compound of the formula (5), so that it is difficult to separate the compound of the formula (6) to purify the compound of the formula (5). Therefore, regarding the process for producing the compound of the formula (5) from the compound of the formula (4), there has been desired a process in which the oxidation reaction sufficiently proceeds and substantially no compound of the formula (6) remains in the product.

Patent Document 7 (JP 2013-512201 A) corresponds to Patent Document 10 (US 2012/264947 A1).

Patent Document 9 (JP 2008-544996 A) corresponds to Patent Document 11 (US 2007/015805 A1).

### Citation List

### Patent Document

Patent Document 1: WO 2002/062770 A1
Patent Document 2: WO 2004/013106 A1
Patent Document 3: WO 2005/095352 A1
Patent Document 4: WO 2005/105755 A1
Patent Document 5: WO 2007/094225 A1
Patent Document 6: WO 2006/068092 A1
Patent Document 7: JP 2013-512201 A
Patent Document 8: WO 2019/131715 A1
Patent Document 9: JP 2008-544996 A
Patent Document 10: US 2012/264947 A1
Patent Document 11: US 2007/015805 A1

### Summary of Invention

### Technical Problem

It is an object of the present invention to provide a process for producing the compound of the formula (4) that can safely and simply produce the compound of the formula (4), is superior in yield and is novel and industrially advantageous.

It is a further object of the invention to provide a process for producing the compound of the formula (5) from the compound of the formula (4) that affords a product containing the compound of the formula (6) in a sufficiently low percentage, is superior in yield, advantageous for the production on an industrial scale, and industrially advantageous.

It is still further object of the present invention is to provide an improved agrochemical formulation comprising pyroxasulfone (the compound of the formula (5-a)). That is, the present invention aims to provide a solid formulation comprising pyroxasulfone which does not form a lump when diluted in water, and also aims to provide a pyroxasulfone-containing liquid formulation, a sprinkling liquid of which does not form a hard cake.

### Solution to Problem

As a result of earnest study, the present inventors have found that the compound of the formula (4) can be efficiently produced by reacting the compound of the formula (2) with the compound of the formula (3) in the presence of a base as shown in the step ii below. Based on this finding, the present inventors have accomplished the present invention. 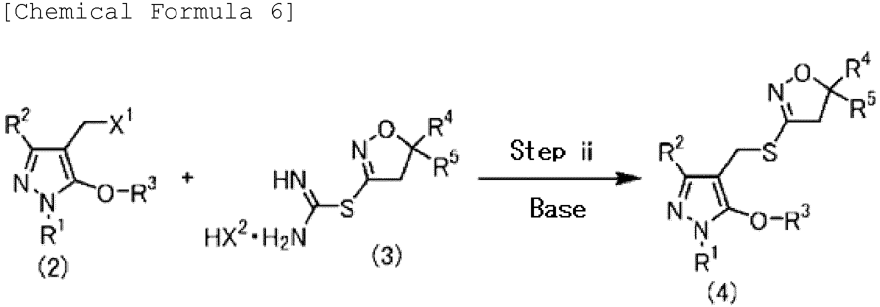 wherein R¹, R², R³, R⁴, R⁵, X¹ and X² are as described herein.

Furthermore, the present inventors have found that, in the process for producing the compound of the formula (5) from the compound of the formula (4), even when an excessive amount of hydrogen peroxide is used in a reaction system of a hydrogen peroxide-tungsten catalyst, the compound of the formula (6), which is a reaction intermediate that is not completely oxidized, surprisingly remains in a product (see Reference Examples). On the other hand, it has been found that the oxidation reaction can be sufficiently advanced by performing the reaction with hydrogen peroxide in the presence of a metal catalyst in a mixed solvent composed of water and a certain organic solvent. Based on these findings, the present inventors have accomplished a production process in which substantially no compound of the formula (6) remains in the target product.

One aspect of the present invention further provides a novel crystal of pyroxasulfone (the compound of the formula (5-a)) and a process for producing the same. This crystal exhibits a powder X-ray diffraction spectrum having characteristics different from conventional ones, and has good wettability and redispersibility.

Conventional agrochemical formulations containing pyroxasulfone have sometimes shown physicochemical defects.

Specifically, for example, when a solid formulation such as a wettable powder is diluted in water in order to prepare a sprinkling liquid, the powder is slow to blend with water, and thus, there was a case where lumps are formed. Sprinkling cannot be performed unless a homogeneous sprinkling liquid is obtained. Therefore, in such a case, it is necessary to continue stirring the sprinkling liquid until the lumps are loosened. As a result, the operation time for sprinkling agrochemicals is prolonged, which is inconvenient.

Also as for a liquid formulation such as an aqueous suspension concentrate (SC) or an oil dispersion (OD), a diluted liquid is prepared by dispersing such an agrochemical formulation in water in order to sprinkle the formulation. Once stirring is stopped after the preparation and a deposit layer of solid matter is formed, the solid matter is not easily redispersed even if stirring is resumed and there was a case where a homogeneous suspension state cannot be recovered. In order to prevent such a situation, it is necessary to continue stirring the prepared sprinkling liquid until sprinkling is actually completed, which is disadvantageous in terms of operation cost.

The agrochemical formulation prepared using the crystal of pyroxasulfone of the present invention does not cause the above-mentioned problems and is industrially advantageous.

### Advantageous Effects of Invention

The present invention provides a novel process for producing the compound of the formula (4), that can safely and simply produce the compound of the formula (4), is superior in yield and is novel.

Furthermore, the present invention provides a process for producing the compound of the formula (5) (sulfone derivative: SO₂ derivative) from the compound of the formula (4) (sulfide derivative: S derivative), wherein the proportion of the compound of the formula (6) (sulfoxide derivative: SO derivative) in the product is sufficiently low, the yield is superior, and the process is advantageous for production on an industrial scale. The compound of the formula (5) produced by the process of the present invention contains substantially no compound of the formula (6) that can cause the loss of quality as a herbicide and crop injury, and therefore it is useful as a herbicide.

The process of the present invention can be implemented on a large scale using low-cost materials, and is superior in economic efficiency, and is suitable for production on an industrial scale.

One aspect of the present invention further provides a crystal of pyroxasulfone suitable as a raw material of an agrochemical formulation. The solid formulation containing pyroxasulfone formulated using this crystal does not form lumps when diluted in water, and is practically convenient. In addition, a liquid formulation containing pyroxasulfone formulated using this crystal has an improved hard cake tendency of a sprinkling liquid and is practically convenient.

### Brief Description of Drawings

FIG. 1 is a powder X-ray diffraction spectrum of a crystal of pyroxasulfone produced by a process according to one embodiment of the present invention. Of the two upper and lower charts shown in the figure, the upper one is a powder X-ray diffraction spectrum measured by a transmission method, and the lower one is a powder X-ray diffraction spectrum measured by a reflection method.
FIG. 2 is a powder X-ray diffraction spectrum of a crystal of pyroxasulfone produced by the process disclosed in Patent Document 2. Of the two upper and lower charts shown in the figure, the upper one is a powder X-ray diffraction spectrum measured by a transmission method, and the lower one is a powder X-ray diffraction spectrum measured by a reflection method.
FIG. 3 is a powder X-ray diffraction spectrum of a crystal of pyroxasulfone produced by the process disclosed in Patent Document 7. Of the two upper and lower charts shown in the figure, the upper one is a powder X-ray diffraction spectrum measured by a transmission method, and the lower one is a powder X-ray diffraction spectrum measured by a reflection method.
FIG. 4 is a microscopic photograph of a crystal of pyroxasulfone of one embodiment of the present invention.
FIG. 5 is a microscopic photograph of a crystal of pyroxasulfone produced by the process disclosed in Patent Document 2.
FIG. 6 is a microscopic photograph of a crystal of pyroxasulfone produced by the process disclosed in Patent Document 7.

### Description of Embodiments

In one aspect, the present invention is as follows:
[I-1] A process for producing a compound of the formula (4), the process comprising the following step ii:
   (step ii) a step of reacting a compound of the formula (2) with a compound of the formula (3) in the presence of a base to produce the compound of the formula (4); 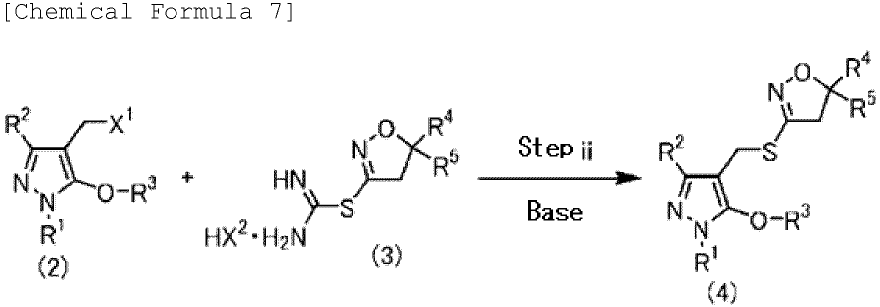
   wherein in the formula (2),
      R¹, R² and R³ are each independently a (C1-C6)alkyl optionally substituted with one or more substituents, a (C3-C6)cycloalkyl optionally substituted with one or more substituents, a (C2-C6)alkenyl optionally substituted with one or more substituents, a (C2-C6)alkynyl optionally substituted with one or more substituents, or a (C6-C10)aryl optionally substituted with one or more substituents, and
      X¹ is a leaving group,
      in the formula (3),
      R⁴ and R⁵ are each independently a (C1-C6)alkyl optionally substituted with one or more substituents, a (C3-C6)cycloalkyl optionally substituted with one or more substituents, a (C2-C6)alkenyl optionally substituted with one or more substituents, a (C2-C6)alkynyl optionally substituted with one or more substituents, a (C1-C6)alkoxy optionally substituted with one or more substituents; or a (C6-C10)aryl optionally substituted with one or more substituents, or
      R⁴ and R⁵, together with the carbon atom to which they are attached, form a 4- to 12-membered carbocyclic ring, wherein the formed ring is optionally substituted with one or more substituents,
      X² is an atom or an atomic group that forms an acid,
      in the formula (4), R¹, R², R³, R⁴ and R⁵ are as defined above.
[I-2] The process according to [I-1], further comprising the following step i before the step ii:
   (step i) a step of reacting a compound of the formula (1) with a halogenating agent to produce the compound of the formula (2): 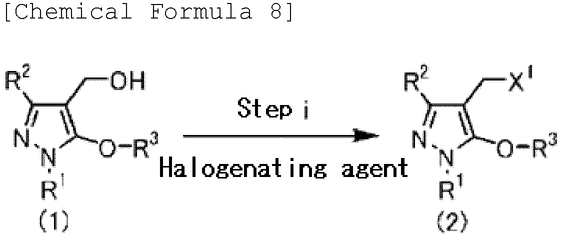
   wherein in the formula (1), R¹, R² and R³ are as defined in [1-1],
   in the formula (2), R¹, R² and R³ are as defined in [I-1], and X¹ is a halogen atom.
[I-3] The process according to [I-1], wherein the compound of the formula (2) is produced by a process comprising the following step i:
   (step i) a step of reacting a compound of the formula (1) with a halogenating agent to produce the compound of the formula (2): 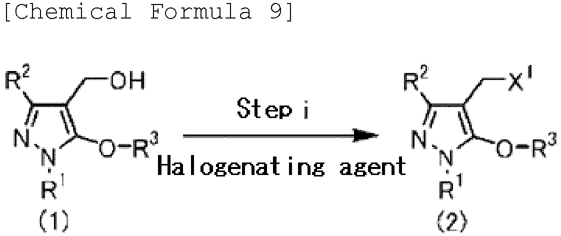
   wherein in the formula (1), R¹, R² and R³ are as defined in [I-1],
   in the formula (2), R¹, R² and R³ are as defined in [I-1], and X¹ is a halogen atom.
[I-4] The process according to [I-1], wherein as the compound of the formula (2), a compound of the formula (2) produced by a process comprising the following step i is used:
   (step i) a step of reacting a compound of the formula (1) with a halogenating agent to produce the compound of the formula (2): 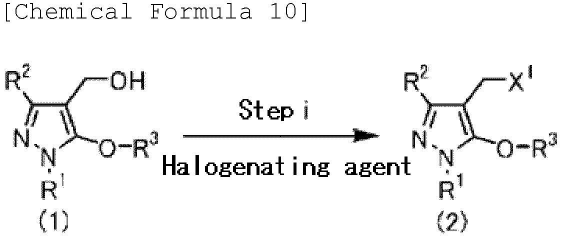
   wherein in the formula (1), R¹, R² and R³ are as defined in [1-1],
   in the formula (2), R¹, R² and R³ are as defined in [I-1], and X¹ is a halogen atom.
[I-5] The process according to [I-1], further comprising the following step iii after the step ii:
   (step iii) a step of reacting the compound of the formula (4) with hydrogen peroxide in the presence of a metal catalyst to produce a compound of the formula (5): 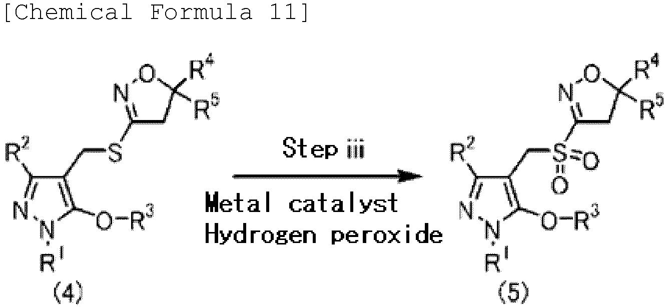
   wherein R¹, R², R³, R⁴ and R⁵ in the formula (4) and the formula (5) are as defined in [I-1].
[I-6] A process for producing a compound of the formula (4) : 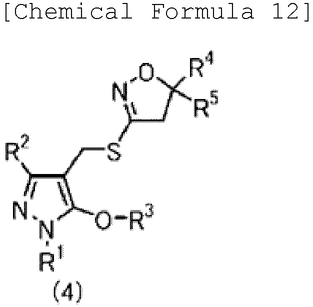
   wherein in the formula (4),
   R¹, R² and R³ are each independently a (C1-C6)alkyl optionally substituted with one or more substituents, a (C3-C6)cycloalkyl optionally substituted with one or more substituents, a (C2-C6)alkenyl optionally substituted with one or more substituents, a (C2-C6)alkynyl optionally substituted with one or more substituents, or a (C6-C10)aryl optionally substituted with one or more substituents,
   R⁴ and R⁵ are each independently a (C1-C6)alkyl optionally substituted with one or more substituents, a (C3-C6)cycloalkyl optionally substituted with one or more substituents, a (C2-C6)alkenyl optionally substituted with one or more substituents, a (C2-C6)alkynyl optionally substituted with one or more substituents, a (C1-C6)alkoxy optionally substituted with one or more substituents; or a (C6-C10)aryl optionally substituted with one or more substituents, or
   R⁴ and R⁵, together with the carbon atom to which they are attached, form a 4- to 12-membered carbocyclic ring, wherein the formed ring is optionally substituted with one or more substituents,
   the process comprising the following step i and step ii:
      (step i) a step of reacting a compound of the formula (1) with a halogenating agent to produce the compound of the formula (2): 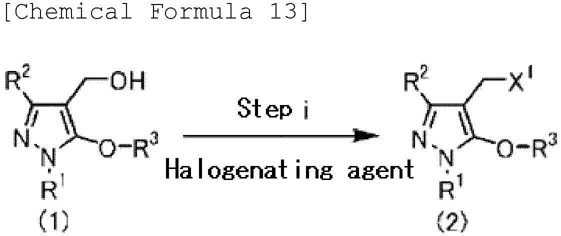
      wherein in the formula (1), R¹, R² and R³ are as defined above,
      in the formula (2), R¹, R² and R³ are as defined above, and X¹ is a halogen atom,
      (step ii) a step of reacting a compound of the formula (2) with a compound of the formula (3) in the presence of a base to produce the compound of the formula (4); 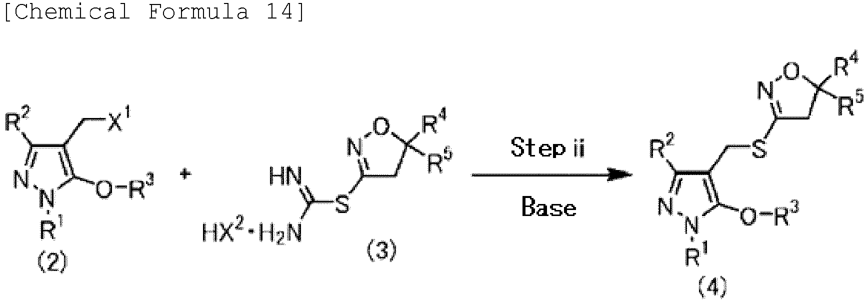
      wherein in the formula (2), R¹, R², R³ and X¹ are as defined above,
      in the formula (3), R⁴ and R⁵ are as defined above, and X² is an atom or an atomic group that forms an acid,
      in the formula (4), R¹, R², R³, R⁴ and R⁵ are as defined above.
[I-7] A process for producing a compound of the formula (5) : 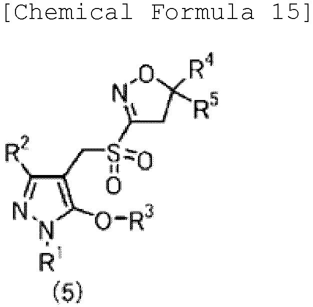
wherein in the formula (5),
   R¹, R² and R³ are each independently a (C1-C6)alkyl optionally substituted with one or more substituents, a (C3-C6)cycloalkyl optionally substituted with one or more substituents, a (C2-C6)alkenyl optionally substituted with one or more substituents, a (C2-C6)alkynyl optionally substituted with one or more substituents, or a (C6-C10)aryl optionally substituted with one or more substituents,
   R⁴ and R⁵ are each independently a (C1-C6)alkyl optionally substituted with one or more substituents, a (C3-C6)cycloalkyl optionally substituted with one or more substituents, a (C2-C6)alkenyl optionally substituted with one or more substituents, a (C2-C6)alkynyl optionally substituted with one or more substituents, a (C1-C6)alkoxy optionally substituted with one or more substituents; or a (C6-C10)aryl optionally substituted with one or more substituents, or
   R⁴ and R⁵, together with the carbon atom to which they are attached, form a 4- to 12-membered carbocyclic ring, wherein the formed ring is optionally substituted with one or more substituents,
   the process comprising the following step ii and step iii:
      (step ii) a step of reacting a compound of the formula (2) with a compound of the formula (3) in the presence of a base to produce the compound of the formula (4); 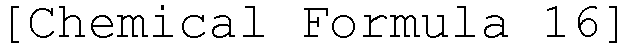 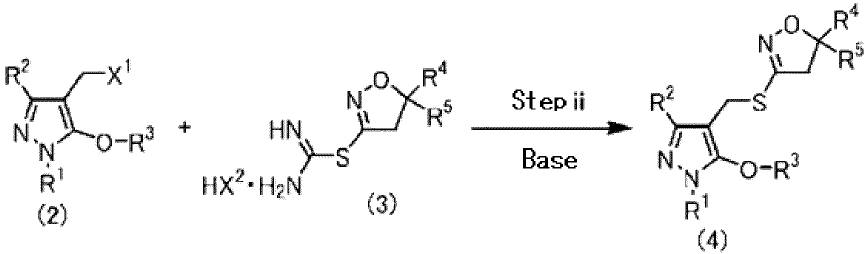
      wherein in the formula (2), R¹, R² and R³ are as defined above, and X¹ is a leaving group,
      in the formula (3), R⁴ and R⁵ are as defined above, and X² is an atom or an atomic group that forms an acid,
      in the formula (4), R¹, R², R³, R⁴ and R⁵ are as defined above,
      (step iii) a step of reacting the compound of the formula (4) with hydrogen peroxide in the presence of a metal catalyst to produce a compound of the formula (5): 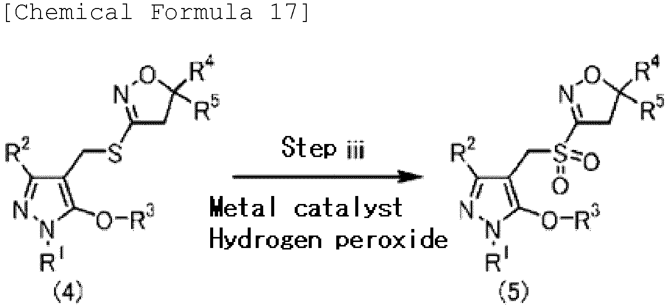
      wherein in the formula (4) and the formula (5), R¹, R², R3, R⁴ and R⁵ are as defined above.
[I-8] A process for producing a compound of the formula (5) : 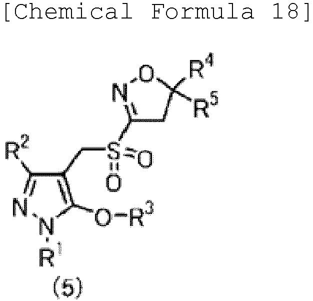
wherein in the formula (5),
   R¹, R² and R³ are each independently a (C1-C6)alkyl optionally substituted with one or more substituents, a (C3-C6)cycloalkyl optionally substituted with one or more substituents, a (C2-C6)alkenyl optionally substituted with one or more substituents, a (C2-C6)alkynyl optionally substituted with one or more substituents, or a (C6-C10)aryl optionally substituted with one or more substituents,
   R⁴ and R⁵ are each independently a (C1-C6)alkyl optionally substituted with one or more substituents, a (C3-C6)cycloalkyl optionally substituted with one or more substituents, a (C2-C6)alkenyl optionally substituted with one or more substituents, a (C2-C6)alkynyl optionally substituted with one or more substituents, a (C1-C6)alkoxy optionally substituted with one or more substituents; or a (C6-C10)aryl optionally substituted with one or more substituents, or
   R⁴ and R⁵, together with the carbon atom to which they are attached, form a 4- to 12-membered carbocyclic ring, wherein the formed ring is optionally substituted with one or more substituents,
   the process comprising the following step i, step ii and step iii:
      (step i) a step of reacting a compound of the formula (1) with a halogenating agent to produce the compound of the formula (2): 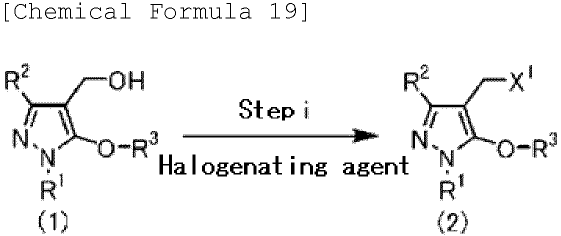
      wherein in the formula (1), R¹, R² and R³ are as defined above,
      in the formula (2), R¹, R² and R³ are as defined above, and X¹ is a halogen atom,
      (step ii) a step of reacting a compound of the formula (2) with a compound of the formula (3) in the presence of a base to produce the compound of the formula (4); 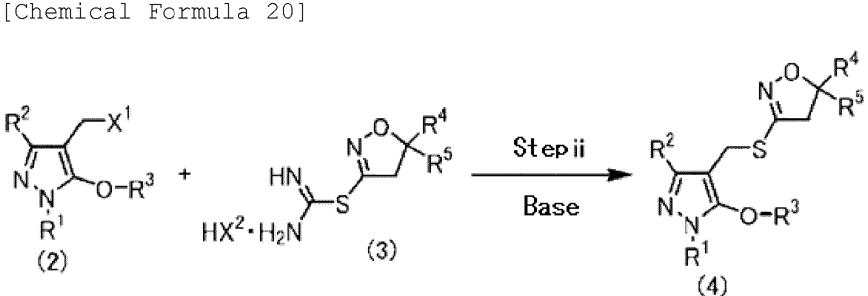
      wherein in the formula (2), R¹, R² and R³ are as defined above, and X¹ is a halogen atom,
      in the formula (3), R⁴ and R⁵ are as defined above, and X² is an atom or an atomic group that forms an acid,
      in the formula (4), R¹, R², R³, R⁴ and R⁵ are as defined above,
      (step iii) a step of reacting the compound of the formula (4) with hydrogen peroxide in the presence of a metal catalyst to produce a compound of the formula (5): 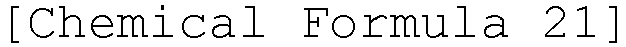 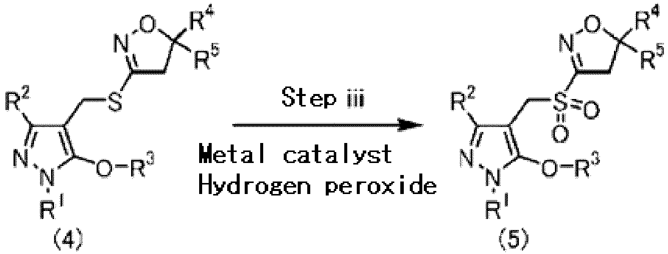
      wherein in the formula (4) and the formula (5), R¹, R², R3, R⁴ and R⁵ are as defined above.
[I-9] A process for producing a compound of the formula (5), the process comprising the following step iii:
   (step iii) a step of reacting the compound of the formula (4) with hydrogen peroxide in the presence of a metal catalyst to produce a compound of the formula (5): 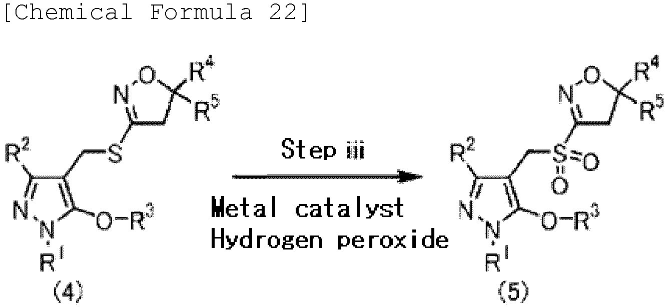
   wherein
      R¹, R² and R³ in the formula (4) and the formula (5) are each independently a (C1-C6)alkyl optionally substituted with one or more substituents, a (C3-C6)cycloalkyl optionally substituted with one or more substituents, a (C2-C6)alkenyl optionally substituted with one or more substituents, a (C2-C6)alkynyl optionally substituted with one or more substituents, or a (C6-C10)aryl optionally substituted with one or more substituents,
      R⁴ and R⁵ are each independently a (C1-C6)alkyl optionally substituted with one or more substituents, a (C3-C6)cycloalkyl optionally substituted with one or more substituents, a (C2-C6)alkenyl optionally substituted with one or more substituents, a (C2-C6)alkynyl optionally substituted with one or more substituents, a (C1-C6)alkoxy optionally substituted with one or more substituents; or a (C6-C10)aryl optionally substituted with one or more substituents, or
      R⁴ and R⁵, together with the carbon atom to which they are attached, form a 4- to 12-membered carbocyclic ring, wherein the formed ring is optionally substituted with one or more substituents.
[I-10] The process according to [I-9], wherein the compound of the formula (4) is produced by a process comprising the following step ii:
   (step ii) a step of reacting a compound of the formula (2) with a compound of the formula (3) in the presence of a base to produce the compound of the formula (4); 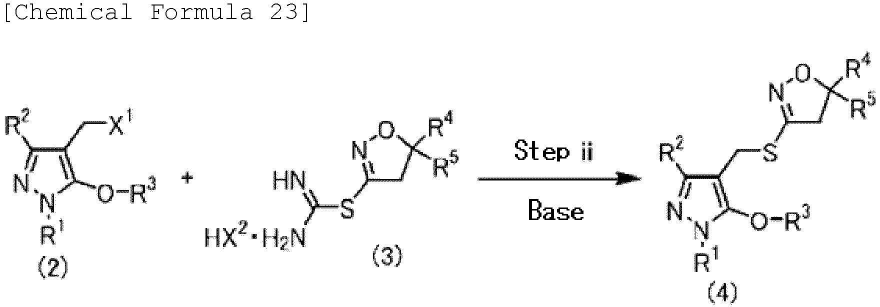
   wherein in the formula (2), R¹, R² and R³ are as defined above, and X¹ is a leaving group,
   in the formula (3), R⁴ and R⁵ are as defined above, and X² is an atom or an atomic group that forms an acid,
   in the formula (4), R¹, R², R3, R⁴ and R⁵ are as defined above.
[I-11] The process according to [I-9], wherein as the compound of the formula (4), a compound of the formula (4) produced by a process comprising the following step ii is used:
   (step ii) a step of reacting a compound of the formula (2) with a compound of the formula (3) in the presence of a base to produce the compound of the formula (4); 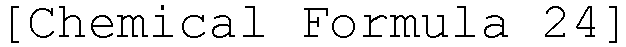 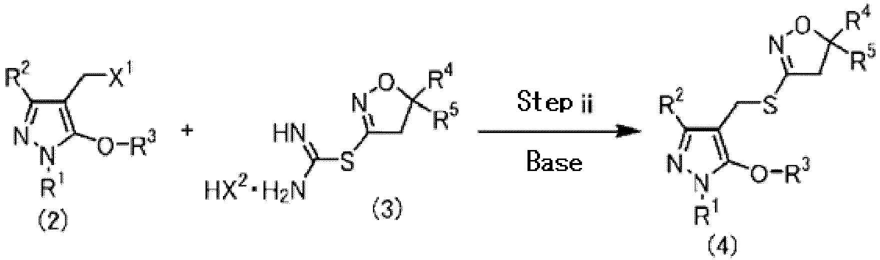
   wherein in the formula (2), R¹, R² and R³ are as defined above, and X¹ is a leaving group,
   in the formula (3), R⁴ and R⁵ are as defined above, and X² is an atom or an atomic group that forms an acid,
   in the formula (4), R¹, R², R³, R⁴ and R⁵ are as defined above.
[I-12] The process according to any one of [I-2] to [I-11], wherein the halogenating agent in the step i is a chlorinating agent, with the proviso that any process not comprising the step i is excluded.
[I-13] The process according to any one of [I-2] to [I-11], wherein the halogenating agent in the step i is chlorine, thionyl chloride, sulfuryl chloride, phosphorus trichloride, phosphorus pentachloride, phosphorus oxychloride, sulfonyl chloride, phosgene, or benzoyl chloride, with the proviso that any process not comprising the step i is excluded.
[I-14] The process according to any one of [1-2] to [I-11], wherein the halogenating agent in the step i is chlorine, thionyl chloride, sulfuryl chloride, phosphorus trichloride, phosphorus pentachloride, or phosphorus oxychloride, with the proviso that any process not comprising the step i is excluded.
[I-15] The process according to any one of [I-2] to [I-11], wherein the halogenating agent is thionyl chloride, with the proviso that any process not comprising the step i is excluded.
[I-16] The process according to any one of [I-2] to [I-15], wherein the amount of the halogenating agent used in the step i is 1.0 to 2.0 mol based on 1 mol of the compound of the formula (1), with the proviso that any process not comprising the step i is excluded.
[I-17] The process according to any one of [I-2] to [I-15], wherein the amount of the halogenating agent used in the step i is 1.0 to 1.1 mol based on 1 mol of the compound of the formula (1), with the proviso that any process not comprising the step i is excluded.
[I-18] The process according to any one of [I-2] to [1-17], wherein the reaction in the step i is performed in the absence of a catalyst, with the proviso that any process not comprising the step i is excluded.
[I-19] The process according to any one of [I-2] to [I-17], wherein the reaction in the step i is performed in the presence of a catalyst, with the proviso that any process not comprising the step i is excluded.
[I-20] The process according to [I-19], wherein the catalyst is an amide.
[I-21] The process according to [I-19], wherein the catalyst is N,N-dimethylformamide.
[I-22] The process according to any one of [I-19] to [I-21], wherein the amount of the catalyst used is 0 to 0.1 mol based on 1 mol of the compound of the formula (4).
[I-23] The process according to any one of [I-19] to [I-21], wherein the amount of the catalyst used is 0.001 to 0.1 mol based on 1 mol of the compound of the formula (4).
[I-24] The process according to any one of [I-2] to [I-23], wherein the reaction in the step i is performed in the absence of a solvent, with the proviso that any process not comprising the step i is excluded.
[I-25] The process according to any one of [I-2] to [I-23], wherein the reaction in the step i is performed in the presence of a solvent, with the proviso that any process not comprising the step i is excluded.
[I-26] The process according to [I-25], wherein the solvent in the reaction in the step i is
   one or more organic solvents selected from aromatic hydrocarbon derivatives, halogenated aliphatic hydrocarbons and nitriles.
[I-27] The process according to [I-25], wherein the solvent in the reaction in the step i is
   one or two organic solvents selected from aromatic hydrocarbon derivatives, halogenated aliphatic hydrocarbons and nitriles.
[I-28] The process according to [I-25], wherein the solvent in the reaction in the step i is
   one organic solvent selected from aromatic hydrocarbon derivatives, halogenated aliphatic hydrocarbons and nitriles.
[I-29] The process according to [1-25], wherein the solvent in the reaction in the step i is
   one or more organic solvents selected from halogenated aromatic hydrocarbons, halogenated aliphatic hydrocarbons and nitriles.
[I-30] The process according to [I-25], wherein the solvent in the reaction in the step i is
   one or two organic solvents selected from halogenated aromatic hydrocarbons, halogenated aliphatic hydrocarbons and nitriles.
[I-31] The process according to [I-25], wherein the solvent in the reaction in the step i is
   one organic solvent selected from halogenated aromatic hydrocarbons, halogenated aliphatic hydrocarbons and nitriles.
[I-32] The process according to [I-25], wherein the solvent in the reaction in the step i is
   one or more organic solvents selected from chlorobenzene, dichlorobenzene, trichlorobenzene, dichloromethane, 1,2-dichloroethane and acetonitrile.
[I-33] The process according to [1-25], wherein the solvent in the reaction in the step i is
   one or two organic solvents selected from chlorobenzene, dichlorobenzene, trichlorobenzene, dichloromethane, 1,2-dichloroethane and acetonitrile.
[I-34] The process according to [I-25], wherein the solvent in the reaction in the step i is
   one organic solvent selected from chlorobenzene, dichlorobenzene, trichlorobenzene, dichloromethane, 1,2-dichloroethane and acetonitrile.
[I-35] The process according to [I-25], wherein the solvent in the reaction in the step i is
   dichloromethane or acetonitrile.
[I-36] The process according to [I-25], wherein the solvent in the reaction in the step i is
   acetonitrile.
[I-37] The process according to any one of [I-25] to [I-36], wherein the amount of the organic solvent used in the reaction in the step i is 0 to 2 liters based on 1 mol of the compound of the formula (1).
[I-38] The process according to any one of [1-25] to [I-36], wherein the amount of the organic solvent used in the reaction in the step i is 0 to 1.5 liters based on 1 mol of the compound of the formula (1).
[I-39] The process according to any one of [I-25] to [I-36], wherein the amount of the organic solvent used in the reaction in the step i is 0.4 to 2 liters based on 1 mol of the compound of the formula (1).
[I-40] The process according to any one of [I-25] to [I-36], wherein the amount of the organic solvent used in the reaction in the step i is 0.6 to 1.2 liters based on 1 mol of the compound of the formula (1).
[I-41] The process according to any one of [I-2] to [I-40], wherein the reaction in the step i is performed at -5°C to 80°C, with the proviso that any process not comprising the step i is excluded.
[I-42] The process according to any one of [I-2] to [I-40], wherein the reaction in the step i is performed at 0°C to 30°C, with the proviso that any process not comprising the step i is excluded.
[I-43] The process according to any one of [I-2] to [1-42], wherein the reaction in the step i is performed in 1 hour to 48 hours, with the proviso that any process not comprising the step i is excluded.
[I-44] The process according to any one of [I-2] to [I-42], wherein the reaction in the step i is performed in 1 hour to 24 hours, with the proviso that any process not comprising the step i is excluded.
[I-45] The process according to any one of [I-2] to [I-42], wherein the reaction in the step i is performed in 2 hours to 12 hours, with the proviso that any process not comprising the step i is excluded.
[I-46] The process according to any one of [I-1] to [I-45], wherein the amount of the compound of the formula (3) used in the step ii is 0.8 to 1.5 mol based on 1 mol of the compound of the formula (2), with the proviso that any process not comprising the step ii is excluded.
[I-47] The process according to any one of [I-1] to [I-45], wherein the amount of the compound of the formula (3) used in the step ii is 1.0 to 1.5 mol based on 1 mol of the compound of the formula (2), with the proviso that any process not comprising the step ii is excluded.
[I-48] The process according to any one of [I-1] to [I-45], wherein the amount of the compound of the formula (3) used in the step ii is 1.0 to 1.1 mol based on 1 mol of the compound of the formula (2), with the proviso that any process not comprising the step ii is excluded.
[I-49] The process according to any one of [I-1] to [I-45], wherein the base in the step ii is an alkali metal hydroxide, an alkali metal carbonate, or a mixture thereof, with the proviso that any process not comprising the step ii is excluded.
[I-50] The process according to any one of [I-1] to [I-45], wherein the base in the step ii is an alkali metal hydroxide, with the proviso that any process not comprising the step ii is excluded.
[I-51] The process according to any one of [I-1] to [I-45], wherein the base in the step ii is lithium hydroxide, sodium hydroxide, potassium hydroxide, lithium carbonate, sodium carbonate, potassium carbonate, or a mixture thereof, with the proviso that any process not comprising the step ii is excluded.
[I-52] The process according to any one of [I-1] to [I-45], wherein the base in the step ii is sodium hydroxide, potassium hydroxide, sodium carbonate, potassium carbonate, or a mixture thereof, with the proviso that any process not comprising the step ii is excluded.
[I-53] The process according to any one of [I-1] to [I-45], wherein the base in the step ii is sodium hydroxide, potassium hydroxide, or a mixture thereof, with the proviso that any process not comprising the step ii is excluded.
[I-54] The process according to any one of [I-1] to [I-45], wherein the base in the step ii is sodium hydroxide, with the proviso that any process not comprising the step ii is excluded.
[I-55] The process according to any one of [I-1] to [I-45], wherein the amount of the base used in the step ii is 1 to 10 equivalents based on 1 equivalent of the compound of the formula (2), with the proviso that any process not comprising the step ii is excluded.
[I-56] The process according to any one of [I-1] to [I-45], wherein the amount of the base used in the step ii is 5 to 10 equivalents based on 1 equivalent of the compound of the formula (2), with the proviso that any process not comprising the step ii is excluded.
[I-57] The process according to any one of [I-1] to [I-45], wherein the amount of the base used in the step ii is 5 to 8 equivalents based on 1 equivalent of the compound of the formula (2), with the proviso that any process not comprising the step ii is excluded.
[I-58] The process according to any one of [I-1] to [I-45], wherein the reaction in the step ii is 5 to 6 equivalents based on 1 equivalent of the compound of the formula (2), with the proviso that any process not comprising the step ii is excluded.
[I-59] The process according to any one of [I-1] to [I-58], wherein the reaction in the step ii is performed in the presence of an organic solvent and a water solvent, with the proviso that any process not comprising the step ii is excluded.
[I-60] The process according to [I-59], wherein the organic solvent in the reaction in the step ii is an organic solvent having an acceptor number of 0 to 50.
[I-61] The process according to [I-59], wherein the organic solvent in the reaction in the step ii is an organic solvent having an acceptor number of 3 to 45.
[I-62] The process according to [I-59], wherein the organic solvent in the reaction in the step ii is an organic solvent having an acceptor number of 5 to 45.
[I-63] The process according to [I-59], wherein the organic solvent in the reaction in the step ii is an organic solvent having an acceptor number of 5 to 35.
[I-64] The process according to [I-59], wherein the organic solvent in the reaction in the step ii is an organic solvent having an acceptor number of 5 to 30.
[I-65] The process according to [I-59], wherein the organic solvent in the reaction in the step ii is an organic solvent having an acceptor number of 5 to 20.
[I-66] The process according to [I-59], wherein the organic solvent in the reaction in the step ii is an organic solvent having an acceptor number of 8 to 20.
[I-67] The process according to [1-59], wherein the organic solvent in the reaction in the step ii is selected from aromatic hydrocarbon derivatives, halogenated aliphatic hydrocarbons, alcohols, nitriles, carboxylic acid esters, ethers, ketones, amides, ureas, sulfoxides and sulfones.
[I-68] The process according to [I-59], wherein the organic solvent in the reaction in the step ii is one or more (preferably one or two, more preferably one) organic solvents selected from aromatic hydrocarbon derivatives, halogenated aliphatic hydrocarbons, alcohols, nitriles, carboxylic acid esters, ethers, ketones, amides, ureas, sulfoxides and sulfones.
[I-69] The process according to [I-59], wherein the organic solvent in the reaction in the step ii is one or more (preferably one or two, more preferably one) organic solvents selected from alcohols, nitriles, carboxylic acid esters, ethers, amides and sulfones.
[I-70] The process according to [I-59], wherein the organic solvent in the reaction in the step ii is one or more (preferably one or two, more preferably one) organic solvents selected from alcohols, nitriles, carboxylic acid esters, ethers and amides.
[I-71] The process according to [I-59], wherein the organic solvent in the reaction in the step ii is selected from alcohols, nitriles, carboxylic acid esters and amides.
[I-72] The process according to [I-59], wherein the organic solvent in the reaction in the step ii is one or more organic solvents selected from alcohols, nitriles, carboxylic acid esters and amides.
[I-73] The process according to [I-59], wherein the organic solvent in the reaction in the step ii is one or two organic solvents selected from alcohols, nitriles, carboxylic acid esters and amides.
[I-74] The process according to [I-59], wherein the organic solvent in the reaction in the step ii is one organic solvent selected from alcohols, nitriles, carboxylic acid esters and amides.
[I-75] The process according to [I-59], wherein the organic solvent in the reaction in the step ii is selected from alcohols, nitriles and carboxylic acid esters.
[I-76] The process according to [1-59], wherein the organic solvent in the reaction in the step ii is one or more organic solvents selected from alcohols, nitriles and carboxylic acid esters.
[I-77] The process according to [I-59], wherein the organic solvent in the reaction in the step ii is one or two organic solvents selected from alcohols, nitriles and carboxylic acid esters.
[I-78] The process according to [I-59], wherein the organic solvent in the reaction in the step ii is one organic solvent selected from alcohols, nitriles and carboxylic acid esters.
[I-79] The process according to [I-59], wherein the organic solvent in the reaction in the step ii is selected from nitriles and carboxylic acid esters.
[I-80] The process according to [I-59], wherein the organic solvent in the reaction in the step ii is one or more organic solvents selected from nitriles and carboxylic acid esters.
[I-81] The process according to [I-59], wherein the organic solvent in the reaction in the step ii is one or two organic solvents selected from nitriles and carboxylic acid esters.
[I-82] The process according to [I-59], wherein the organic solvent in the reaction in the step ii is one organic solvent selected from nitriles and carboxylic acid esters.
[I-83] The process according to [I-59], wherein the organic solvent in the reaction in the step ii is a nitrile.
[I-84] The process according to [I-59], wherein the organic solvent in the reaction in the step ii is selected from nitriles.
[I-85] The process according to [I-59], wherein the organic solvent in the reaction in the step ii is one or more organic solvents selected from nitriles.
[I-86] The process according to [I-59], wherein the organic solvent in the reaction in the step ii is one or two organic solvents selected from nitriles.
[I-87] The process according to [I-59], wherein the organic solvent in the reaction in the step ii is one organic solvent selected from nitriles.
[I-88] The process according to [I-59], wherein the organic solvent in the reaction in the step ii is a carboxylic acid ester.
[I-89] The process according to [I-59], wherein the organic solvent in the reaction in the step ii is selected from carboxylic acid esters.
[I-90] The process according to [I-59], wherein the organic solvent in the reaction in the step ii is one or more organic solvents selected from carboxylic acid esters.
[I-91] The process according to [I-59], wherein the organic solvent in the reaction in the step ii is preferably one to five organic solvents selected from carboxylic acid esters.
[I-92] The process according to [I-59], wherein the organic solvent in the reaction in the step ii is preferably one or two organic solvents selected from carboxylic acid esters.
[I-93] The process according to [I-59], wherein the organic solvent in the reaction in the step ii is one organic solvent selected from carboxylic acid esters.
[I-94] The process according to [1-59], wherein the organic solvent in the reaction in the step ii is selected from (C1-C6)alcohols, (C2-C5)alkane nitriles, (C1-C4)alkyl (C1-C4)carboxylates and N,N-di ((C1-C4) alkyl) (C1-C4)alkaneamides.
[I-95] The process according to [I-59], wherein the organic solvent in the reaction in the step ii is one or more organic solvents selected from (C1-C6)alcohols, (C2-C5)alkane nitriles, (C1-C4)alkyl (C1-C4)carboxylates and N,N-di((C1-C4)alkyl)(C1-C4)alkaneamides.
[I-96] The process according to [I-59], wherein the organic solvent in the reaction in the step ii is one or two organic solvents selected from (C1-C6)alcohols, (C2-C5)alkane nitriles, (C1-C4)alkyl (C1-C4)carboxylates and N,N-di((C1-C4)alkyl)(C1-C4)alkaneamides.
[I-97] The process according to [I-59], wherein the organic solvent in the reaction in the step ii is one organic solvent selected from among (C1-C6)alcohols, (C2-C5)alkane nitriles, (C1-C4)alkyl (C1-C4)carboxylates and N,N-di((C1-C4) alkyl) (C1-C4) alkaneamides .
[I-98] The process according to [1-59], wherein the organic solvent in the reaction in the step ii is one or more (preferably one or two, more preferably one) organic solvents selected from (C2-C6)alcohols, (C2-C5)alkane nitriles, (C1-C4)alkyl (C1-C4)carboxylates and N,N-di((C1-C4) alkyl) (C1-C4) alkaneamides .
[I-99] The process according to [I-59], wherein the organic solvent in the reaction in the step ii is one or more (preferably one or two, more preferably one) organic solvents selected from (C1-C4)alcohols, (C2-C5)alkane nitriles, (C1-C4)alkyl (C1-C4)carboxylates and N,N-di((C1-C4)alkyl) (C1-C4)alkaneamides.
[I-100] The process according to [I-59], wherein the organic solvent in the reaction in the step ii is one or more (preferably one or two, more preferably one) organic solvents selected from (C2-C4)alcohols, (C2-C5)alkane nitriles, (C1-C4)alkyl (C1-C4)carboxylates and N,N-di((C1-C4) alkyl) (C1-C4) alkaneamides .
[I-101] The process according to [I-59], wherein the organic solvent in the reaction in the step ii is selected from (C1-C6)alcohols, (C2-C5)alkane nitriles and (C1-C4)alkyl (C1-C4)carboxylates.
[I-102] The process according to [I-59], wherein the organic solvent in the reaction in the step ii is one or more organic solvents selected from (C1-C6)alcohols, (C2-C5)alkane nitriles and (C1-C4)alkyl (C1-C4)carboxylates.
[I-103] The process according to [I-59], wherein the organic solvent in the reaction in the step ii is one or two organic solvents selected from (C1-C6)alcohols, (C2-C5)alkane nitriles and (C1-C4)alkyl (C1-C4)carboxylates.
[I-104] The process according to [I-59], wherein the organic solvent in the reaction in the step ii is one organic solvent selected from (C1-C6)alcohols, (C2-C5)alkane nitriles and (C1-C4)alkyl (C1-C4)carboxylates.
[I-105] The process according to [I-59], wherein the organic solvent in the reaction in the step ii is one or more (preferably one or two, more preferably one) organic solvents selected from (C2-C6)alcohols, (C2-C5)alkane nitriles and (C1-C4)alkyl (C1-C4)carboxylates.
[I-106] The process according to [I-59], wherein the organic solvent in the reaction in the step ii is one or more (preferably one or two, more preferably one) organic solvents selected from (C1-C4)alcohols, (C2-C5)alkane nitriles and (C1-C4)alkyl (C1-C4)carboxylates.
[I-107] The process according to [I-59], wherein the organic solvent in the reaction in the step ii is one or more (preferably one or two, more preferably one) organic solvents selected from (C2-C4)alcohols, (C2-C5)alkane nitriles and (C1-C4)alkyl (C1-C4)carboxylates.
[I-108] The process according to [I-59], wherein the organic solvent in the reaction in the step ii is selected from (C2-C5)alkane nitriles and (C1-C4)alkyl (C1-C4)carboxylates.
[I-109] The process according to [I-59], wherein the organic solvent in the reaction in the step ii is one or more organic solvents selected from (C2-C5)alkane nitriles and (C1-C4)alkyl (C1-C4)carboxylates.
[1-110] The process according to [I-59], wherein the organic solvent in the reaction in the step ii is one or two organic solvents selected from (C2-C5)alkane nitriles and (C1-C4)alkyl (C1-C4)carboxylates.
[1-111] The process according to [I-59], wherein the organic solvent in the reaction in the step ii is one organic solvent selected from (C2-C5)alkane nitriles and (C1-C4)alkyl (C1-C4)carboxylates.
[I-112] The process according to [I-59], wherein the organic solvent in the reaction in the step ii is one or more (preferably one or two, more preferably one) organic solvents selected from toluene, xylene, chlorobenzene, dichlorobenzene, dichloromethane, 1,2-dichloroethane, methanol, ethanol, 2-propanol, butanol, tert-butanol, acetonitrile, ethyl acetate, propyl acetate, isopropyl acetate, butyl acetate and isomers thereof, tetrahydrofuran, 1,4-dioxane, diisopropyl ether, dibutyl ether, di-tert-butyl ether, cyclopentyl methyl ether, methyl-tert-butyl ether, 1,2-dimethoxyethane, diglyme, acetone, methyl ethyl ketone, methyl isopropyl ketone, methyl isobutyl ketone, N,N-dimethylformamide, N,N-dimethylacetamide, N-methylpyrrolidone, N,N'-dimethylimidazolidinone, tetramethylurea, dimethyl sulfoxide and sulfolane.
[I-113] The process according to [I-59], wherein the organic solvent in the reaction in the step ii is one or more (preferably one or two, more preferably one) organic solvents selected from methanol, ethanol, 2-propanol, butanol, tert-butanol, acetonitrile, ethyl acetate, propyl acetate, isopropyl acetate, butyl acetate and isomers thereof, tetrahydrofuran, 1,4-dioxane, diisopropyl ether, dibutyl ether, di-tert-butyl ether, cyclopentyl methyl ether, methyl-tert-butyl ether, 1,2-dimethoxyethane, diglyme, N,N-dimethylformamide, N,N-dimethylacetamide, N-methylpyrrolidone and sulfolane.
[I-114] The process according to [I-59], wherein the organic solvent in the reaction in the step ii is one or more (preferably one or two, more preferably one) organic solvents selected from methanol, ethanol, 2-propanol, butanol, tert-butanol, acetonitrile, ethyl acetate, propyl acetate, isopropyl acetate, butyl acetate and isomers thereof, tetrahydrofuran, 1,4-dioxane, diisopropyl ether, dibutyl ether, di-tert-butyl ether, cyclopentyl methyl ether, methyl-tert-butyl ether, 1,2-dimethoxyethane, diglyme, N,N-dimethylformamide, N,N-dimethylacetamide and N-methylpyrrolidone.
[I-115] The process according to [I-59], wherein the organic solvent in the reaction in the step ii is one or more (preferably one or two, more preferably one) organic solvents selected from methanol, ethanol, 2-propanol, butanol, tert-butanol, acetonitrile, ethyl acetate, propyl acetate, isopropyl acetate, butyl acetate and isomers thereof, N,N-dimethylformamide, N,N-dimethylacetamide and N-methylpyrrolidone.
[I-116] The process according to [I-59], wherein the organic solvent in the reaction in the step ii is one organic solvent selected from methanol, ethanol, 2-propanol, butanol, tert-butanol, acetonitrile, ethyl acetate, propyl acetate, isopropyl acetate, butyl acetate and N,N-dimethylformamide.
[I-117] The process according to [I-59], wherein the organic solvent in the reaction in the step ii is one or more (preferably one or two, more preferably one) organic solvents selected from methanol, ethanol, 2-propanol, butanol, tert-butanol, acetonitrile, ethyl acetate, propyl acetate, isopropyl acetate, butyl acetate and isomers thereof.
[I-118] The process according to [I-59], wherein the organic solvent in the reaction in the step ii is one organic solvent selected from methanol, ethanol, 2-propanol, butanol, tert-butanol, acetonitrile, ethyl acetate, propyl acetate, isopropyl acetate and butyl acetate.
[I-119] The process according to [I-59], wherein the organic solvent in the reaction in the step ii is one or more (preferably one or two, more preferably one) organic solvents selected from butanol, acetonitrile, ethyl acetate, propyl acetate, isopropyl acetate and butyl acetate.
[I-120] The process according to [I-59], wherein the organic solvent in the reaction in the step ii is one or more (preferably one or two, more preferably one) organic solvents selected from acetonitrile, ethyl acetate, isopropyl acetate and butyl acetate.
[I-121] The process according to [I-59], wherein the organic solvent in the reaction in the step ii is one organic solvent selected from acetonitrile, ethyl acetate, isopropyl acetate and butyl acetate.
[I-122] The process according to [I-59], wherein the organic solvent in the reaction in the step ii is one or two (preferably one) organic solvents selected from acetonitrile and butyl acetate.
[I-123] The process according to [I-59], wherein the organic solvent in the reaction in the step ii is acetonitrile or butyl acetate.
[I-124] The process according to [I-59], wherein the organic solvent in the reaction in the step ii is acetonitrile.
[I-125] The process according to [I-59], wherein the organic solvent in the reaction in the step ii is butyl acetate.
[I-126] The process according to any one of [I-59] to [I-125], wherein the total amount of the solvent used in the reaction in the step ii is 1 to 3 liters based on 1 mol of the compound of the formula (2).
[I-127] The process according to any one of [I-59] to [I-125], wherein the total amount of the solvent used in the reaction in the step ii is 1.5 to 3.0 liters based on 1 mol of the compound of the formula (2).
[I-128] The process according to any one of [I-59] to [I-125], wherein the total amount of the solvent used in the reaction in the step ii is 1.5 to 2.5 liters based on 1 mol of the compound of the formula (2).
[I-129] The process according to any one of [1-59] to [I-125], wherein the total amount of the solvent used in the reaction in the step ii is 1.7 to 2.0 liters based on 1 mol of the compound of the formula (2).
[I-130] The process according to any one of [I-59] to [I-129], wherein the amount of the organic solvent used in the reaction in the step ii is 0.5 to 1.5 liters based on 1 mol of the compound of the formula (2).
[1-131] The process according to any one of [I-59] to [I-129], wherein the amount of the organic solvent used in the reaction in the step ii is 0.7 to 0.9 liters based on 1 mol of the compound of the formula (2).
[I-132] The process according to any one of [I-59] to [I-129], wherein the amount of the organic solvent used in the reaction in the step ii is 0.3 to 2.0 liters based on 1 mol of the compound of the formula (2).
[I-133] The process according to any one of [I-59] to [I-129], wherein the amount of the organic solvent used in the reaction in the step ii is 0.6 to 0.8 liters based on 1 mol of the compound of the formula (2).
[I-134] The process according to any one of [1-59] to [I-133], wherein the amount of the water solvent used in the reaction in the step ii is 0.5 to 2.0 liters based on 1 mol of the compound of the formula (2).
[I-135] The process according to any one of [I-59] to [I-133], wherein the amount of the water solvent used in the reaction in the step ii is 0.5 to 1.5 liters based on 1 mol of the compound of the formula (2).
[I-136] The process according to any one of [I-59] to [I-133], wherein the amount of the water solvent used in the reaction in the step ii is 0.7 to 1.4 liters based on 1 mol of the compound of the formula (2).
[I-137] The process according to any one of [I-59] to [I-133], wherein the amount of the water solvent used in the reaction in the step ii is 0.9 to 1.2 liters based on 1 mol of the compound of the formula (2).
[I-138] The process according to any one of [I-59] to [I-137], wherein the ratio of the organic solvent to the water solvent used in the reaction in the step ii is 90 : 10 to 0 : 100.
[I-139] The process according to any one of [1-59] to [I-137], wherein the ratio of the organic solvent to the water solvent used in the reaction in the step ii is 70 : 30 to 30 : 70.
[I-140] The process according to any one of [I-59] to [I-137], wherein the ratio of the organic solvent to the water solvent used in the reaction in the step ii is 50 : 50 to 35 : 65.
[I-141] The process according to any one of [I-59] to [I-137], wherein the ratio of the organic solvent to the water solvent used in the reaction in the step ii is 50 : 50 to 40 : 60.
[I-142] The process according to any one of [I-59] to [I-137], wherein in the reaction in the step ii, the amount of the water solvent in the whole solvent composed of the organic solvent and the water solvent is 10 vol% to 100 vol% based on the amount of the whole solvent.
[I-143] The process according to any one of [I-59] to [I-137], wherein in the reaction in the step ii, the amount of the water solvent in the whole solvent composed of the organic solvent and the water solvent is 30 vol% to 70 vol% based on the amount of the whole solvent.
[I-144] The process according to any one of [I-59] to [I-137], wherein in the reaction in the step ii, the amount of the water solvent in the whole solvent composed of the organic solvent and the water solvent is 50 vol% to 65 vol% based on the amount of the whole solvent.
[I-145] The process according to any one of [I-59] to [I-72], wherein in the reaction in the step ii, the amount of the water solvent in the whole solvent composed of the organic solvent and the water solvent is 50 vol% to 60 vol% based on the amount of the whole solvent.
[I-146] The process according to any one of [I-1] to [I-145], wherein the reaction in the step ii is performed at - 10°C to 100°C, with the proviso that any process not comprising the step ii is excluded.
[I-147] The process according to any one of [I-1] to [I-145], wherein the reaction in the step ii is performed at - 10°C to 70°C, with the proviso that any process not comprising the step ii is excluded.
[I-148] The process according to any one of [I-1] to [I-145], wherein the reaction in the step ii is performed at - 10°C to 50°C, with the proviso that any process not comprising the step ii is excluded.
[I-149] The process according to any one of [I-1] to [I-145], wherein the reaction in the step ii is performed at 0°C to 40°C, with the proviso that any process not comprising the step ii is excluded.
[I-150] The process according to any one of [I-1] to [I-145], wherein the reaction in the step ii is performed at 0°C to 30°C, with the proviso that any process not comprising the step ii is excluded.
[I-151] The process according to any one of [I-1] to [I-150], wherein the reaction in the step ii is performed in 1 hour to 48 hours, with the proviso that any process not comprising the step ii is excluded.
[I-152] The process according to any one of [I-1] to [I-150], wherein the reaction in the step ii is performed in 4 hours to 24 hours, with the proviso that any process not comprising the step ii is excluded.
[I-153] The process according to any one of [I-1] to [I-152], wherein the reaction in the step ii is performed by adding dropwise the base to a mixture of the compound of the formula (2), the compound of the formula (3) and a solvent, with the proviso that any process not comprising the step ii is excluded.
[I-154] The process according to any one of [I-1] to [I-152], wherein the reaction in the step ii is performed by adding dropwise the compound of the formula (2) to a mixture of the compound of the formula (3), the base and a solvent, with the proviso that any process not comprising the step ii is excluded.
[I-155] The process according to any one of [I-1] to [I-152], wherein the reaction in the step ii is performed by adding dropwise successively the compound of the formula (2) and the compound of the formula (3) to a mixture of the base and a solvent, with the proviso that any process not comprising the step ii is excluded.
[I-156] The process according to any one of [I-1] to [I-152], wherein the reaction in the step ii is performed by adding dropwise the compound of the formula (3) to a mixture of the base and a solvent and then adding the compound of the formula (2), with the proviso that any process not comprising the step ii is excluded.
[I-157] The process according to any one of [I-1] to [I-156], wherein the hydrogen peroxide in the step iii is a 10 to 70 wt% aqueous hydrogen peroxide solution, with the proviso that any process not comprising the step iii is excluded.
[I-158] The process according to any one of [I-1] to [I-156], wherein the hydrogen peroxide in the step iii is a 25 to 65 wt% aqueous hydrogen peroxide solution, with the proviso that any process not comprising the step iii is excluded.
[I-159] The process according to any one of [I-1] to [I-156], wherein the amount of the hydrogen peroxide used in the step iii is 2 to 8 mol based on 1 mol of the compound of the formula (4), with the proviso that any process not comprising the step iii is excluded.
[I-160] The process according to any one of [I-1] to [I-156], wherein the amount of the hydrogen peroxide used in the step iii is 2 to 6 mol based on 1 mol of the compound of the formula (4), with the proviso that any process not comprising the step iii is excluded.
[I-161] The process according to any one of [I-1] to [I-156], wherein the amount of the hydrogen peroxide used in the step iii is 2 to 5 mol based on 1 mol of the compound of the formula (4), with the proviso that any process not comprising the step iii is excluded.
[I-162] The process according to any one of [I-1] to [I-156], wherein the amount of the hydrogen peroxide used in the step iii is 2 to 4 mol based on 1 mol of the compound of the formula (4), with the proviso that any process not comprising the step iii is excluded.
[I-163] The process according to any one of [I-1] to [I-156], wherein the amount of the hydrogen peroxide used in the step iii is 2 to 3 mol based on 1 mol of the compound of the formula (4), with the proviso that any process not comprising the step iii is excluded.
[I-164] The process according to any one of [I-1] to [I-156], wherein the metal catalyst in the step iii is a tungsten catalyst, with the proviso that any process not comprising the step iii is excluded.
[I-165] The process according to any one of [I-1] to [I-156], wherein the metal catalyst in the step iii is tungstic acid, a tungstic acid salt, metal tungsten, tungsten oxide, tungsten carbide, tungsten chloride, tungsten bromide, tungsten sulfide, phosphotungstic acid or a salt thereof, silicotungstic acid or a salt thereof, or a mixture of them, with the proviso that any process not comprising the step iii is excluded.
[I-166] The process according to any one of [I-1] to [I-156], wherein the metal catalyst in the step iii is tungstic acid, sodium tungstate, potassium tungstate, calcium tungstate, ammonium tungstate, metal tungsten, tungsten oxide, tungsten carbide, or a mixture thereof, with the proviso that any process not comprising the step iii is excluded.
[I-167] The process according to any one of [I-1] to [I-156], wherein the metal catalyst in the step iii is sodium tungstate, with the proviso that any process not comprising the step iii is excluded.
[I-168] The process according to any one of [I-1] to [I-156], wherein the metal catalyst in the step iii is sodium tungstate dihydrate, with the proviso that any process not comprising the step iii is excluded.
[I-169] The process according to any one of [I-1] to [I-156], wherein the metal catalyst in the step iii is selected from a tungsten catalyst and a molybdenum catalyst, with the proviso that any process not comprising the step iii is excluded.
[I-170] The process according to any one of [I-1] to [I-156], wherein the metal catalyst in the step iii is a tungsten catalyst or a molybdenum catalyst, with the proviso that any process not comprising the step iii is excluded.
[I-171] The process according to any one of [I-1] to [I-156], wherein the metal catalyst in the step iii is selected from tungstic acid, tungstic acid salts, metal tungsten, tungsten oxide, tungsten carbide, tungsten chloride and salts thereof, and a mixture thereof, molybdic acid, molybdic acid salts, metal molybdenum, molybdenum carbide, molybdenum oxide, molybdenum chloride, and a mixture thereof, with the proviso that any process not comprising the step iii is excluded.
[I-172] The process according to any one of [I-1] to [I-156], wherein the metal catalyst in the step iii is selected from sodium tungstate and ammonium molybdate, with the proviso that any process not comprising the step iii is excluded.
[I-173] The process according to any one of [I-1] to [I-156], wherein the metal catalyst in the step iii is selected from sodium tungstate dihydrate and ammonium molybdate tetrahydrate, with the proviso that any process not comprising the step iii is excluded.
[I-174] The process according to any one of [I-1] to [I-156], wherein the metal catalyst in the step iii is sodium tungstate or ammonium molybdate, with the proviso that any process not comprising the step iii is excluded.
[I-175] The process according to any one of [I-1] to [I-156], wherein the metal catalyst in the step iii is sodium tungstate dihydrate or ammonium molybdate tetrahydrate, with the proviso that any process not comprising the step iii is excluded.
[I-176] The process according to any one of [I-1] to [I-156], wherein the metal catalyst in the step iii is selected from a tungsten catalyst, a molybdenum catalyst and a niobium catalyst, with the proviso that any process not comprising the step iii is excluded.
[I-177] The process according to any one of [I-1] to [I-156], wherein the metal catalyst in the step iii is selected from tungstic acid, tungstic acid salts, metal tungsten, tungsten oxide, tungsten carbide, tungsten chloride and salts thereof, and a mixture thereof, molybdic acid, molybdic acid salts, metal molybdenum, molybdenum carbide, molybdenum oxide, molybdenum chloride, and a mixture thereof, niobic acid, niobic acid salts, metal niobium, niobium carbide, niobium oxide, niobium chloride, etc., and a mixture thereof, with the proviso that any process not comprising the step iii is excluded.
[I-178] The process according to any one of [I-1] to [I-156], wherein the metal catalyst in the step iii is selected from sodium tungstate, ammonium molybdate and sodium niobate, with the proviso that any process not comprising the step iii is excluded.
[I-179] The process according to any one of [I-1] to [I-156], wherein the metal catalyst in the step iii is selected from a tungsten catalyst, a molybdenum catalyst, a niobium catalyst, a tantalum catalyst, a titanium catalyst and a zirconium catalyst, with the proviso that any process not comprising the step iii is excluded.
[I-180] The process according to any one of [I-1] to [I-156], wherein the metal catalyst in the step iii is selected from tungstic acid, tungstic acid salts, metal tungsten, tungsten oxide, tungsten carbide, tungsten chloride and salts thereof, and a mixture thereof,
   molybdic acid, molybdic acid salts, metal molybdenum, molybdenum carbide, molybdenum oxide, molybdenum chloride, and a mixture thereof,
   niobic acid, niobic acid salts, metal niobium, niobium carbide, niobium oxide, niobium chloride, etc., and a mixture thereof,
   tantalic acid, tantalic acid salts, tantalum oxide, tantalum carbide, tantalum chloride, and a mixture thereof,
   titanic acid, titanic acid salts, titanium oxide, titanium carbide, titanium chloride, and a mixture thereof,
   zirconic acid, zirconic acid salts, zirconium oxide, zirconium carbide, zirconium chloride, and a mixture thereof, with the proviso that any process not comprising the step iii is excluded.
[I-181] The process according to any one of [I-1] to [I-156], wherein the metal catalyst in the step iii is selected from tungstic acid, sodium tungstate, potassium tungstate, calcium tungstate, ammonium tungstate, metal tungsten, tungsten(VI) oxide, tungsten carbide, and a mixture thereof,
   molybdic acid, sodium molybdate, potassium molybdate, ammonium molybdate, molybdenum(VI) oxide, molybdenum carbide, molybdenum(V) chloride, molybdenum(IV) sulfide, phosphomolybdic acid, sodium phosphomolybdate, ammonium phosphomolybdate, silicomolybdic acid, sodium silicomolybdate, and a mixture thereof,
   sodium niobate, potassium niobate, niobium carbide, niobium(V) chloride, niobium(V) pentaethoxide, etc., and a mixture thereof,
   lithium tantalate, potassium tantalate, tantalum pentoxide, tantalum carbide, tantalum(V) chloride, tantalum(V) pentaethoxide, etc., and a mixture thereof,
   titanium acetylacetonate, titanium tetrachloride, titanium trichloride, titanium(IV) tetraisopropoxide, etc., and a mixture thereof,
   zirconium dioxide, zirconium(I) chloride, zirconium(IV) chloride and zirconium chloride oxide, with the proviso that any process not comprising the step iii is excluded.
[I-182] The process according to any one of [I-1] to [I-156], wherein the metal catalyst in the step iii is selected from tungstic acid, sodium tungstate, potassium tungstate, calcium tungstate, ammonium tungstate, metal tungsten, tungsten oxide, tungsten carbide,
   sodium molybdate, potassium molybdate, ammonium molybdate,
   sodium niobate, potassium niobate,
   lithium tantalate and potassium tantalate, with the proviso that any process not comprising the step iii is excluded.
[I-183] The process according to any one of [I-1] to [I-156], wherein the metal catalyst in the step iii is selected from tungstic acid, sodium tungstate, potassium tungstate, calcium tungstate, ammonium tungstate, metal tungsten, tungsten oxide, tungsten carbide,
   sodium molybdate, potassium molybdate, ammonium molybdate,
   sodium niobate and potassium niobate, with the proviso that any process not comprising the step iii is excluded.
[I-184] The process according to any one of [I-1] to [I-156], wherein the metal catalyst in the step iii is selected from tungstic acid, sodium tungstate, potassium tungstate, calcium tungstate, ammonium tungstate, metal tungsten, tungsten oxide, tungsten carbide,
   sodium molybdate, potassium molybdate and ammonium molybdate, with the proviso that any process not comprising the step iii is excluded.
[I-185] The process according to any one of [I-1] to [I-156], wherein the amount of the metal catalyst used is 0.001 to 0.1 mol based on 1 mol of the compound of the formula (4), with the proviso that any process not comprising the step iii is excluded.
[I-186] The process according to any one of [I-1] to [I-156], wherein the amount of the metal catalyst used is 0.01 to 0.1 mol based on 1 mol of the compound of the formula (4), with the proviso that any process not comprising the step iii is excluded.
[I-187] The process according to any one of [I-1] to [I-156], wherein the amount of the metal catalyst used is 0.03 to 0.05 mol based on 1 mol of the compound of the formula (4), with the proviso that any process not comprising the step iii is excluded.
[I-188] The process according to any one of [I-1] to [I-156], wherein the reaction in the step iii is performed in the absence of an acid catalyst, with the proviso that any process not comprising the step iii is excluded.
[I-189] The process according to any one of [I-1] to [I-156], wherein the reaction in the step iii is performed in the presence or absence of an acid catalyst, with the proviso that any process not comprising the step iii is excluded.
[I-190] The process according to any one of [I-1] to [I-156], wherein the reaction in the step iii is performed in the presence of an acid catalyst, with the proviso that any process not comprising the step iii is excluded.
[I-191] The process according to [I-189] or [I-190], wherein the acid catalyst is hydrochloric acid, sulfuric acid, phosphoric acid, methyl phosphate, ethyl phosphate, or phenyl phosphate, with the proviso that any process not comprising the step iii is excluded.
[I-192] The process according to [I-189] or [I-190], wherein the acid catalyst is hydrochloric acid, sulfuric acid, phosphoric acid, or phenyl phosphate, with the proviso that any process not comprising the step iii is excluded.
[I-193] The process according to [1-189] or [1-190], wherein the acid catalyst is sulfuric acid or phenyl phosphate, with the proviso that any process not comprising the step iii is excluded.
[I-194] The process according to [I-189] or [1-190], wherein the acid catalyst is phenyl phosphate, with the proviso that any process not comprising the step iii is excluded.
[I-195] The process according to [I-189] or [I-190], wherein the acid catalyst is sulfuric acid, with the proviso that any process not comprising the step iii is excluded.
[I-196] The process according to any one of [I-188] to [I-190], wherein the amount of the acid catalyst used is 0 (zero) to 0.2 mol based on 1 mol of the compound of the formula (4), with the proviso that any process not comprising the step iii is excluded.
[I-197] The process according to any one of [I-188] to [I-190], wherein the amount of the acid catalyst used is 0 (zero) to 0.1 mol based on 1 mol of the compound of the formula (4), with the proviso that any process not comprising the step iii is excluded.
[I-198] The process according to [I-189] or [I-190], wherein the amount of the acid catalyst used is 0.001 to 0.1 mol based on 1 mol of the compound of the formula (4), with the proviso that any process not comprising the step iii is excluded.
[I-199] The process according to [I-189] or [I-190], wherein the amount of the acid catalyst used is 0.005 to 0.05 mol based on 1 mol of the compound of the formula (4), with the proviso that any process not comprising the step iii is excluded.
[I-200] The process according to any one of [I-1] to [I-199], wherein the reaction in the step iii is performed in the absence of a phase transfer catalyst, with the proviso that any process not comprising the step iii is excluded.
[I-201] The process according to any one of [I-1] to [I-199], wherein the reaction in the step iii is performed in the presence or absence of a phase transfer catalyst, with the proviso that any process not comprising the step iii is excluded.
[I-202] The process according to any one of [I-1] to [I-199], wherein the reaction in the step iii is performed in the presence of a phase transfer catalyst, with the proviso that any process not comprising the step iii is excluded.
[I-203] The process according to [I-202], wherein the phase transfer catalyst is tetrabutylammonium chloride, tetrabutylammonium bromide, or tetrabutylammonium hydrogen sulfate, with the proviso that any process not comprising the step iii is excluded.
[I-204] The process according to [I-202], wherein the phase transfer catalyst is tetrabutylammonium hydrogen sulfate, with the proviso that any process not comprising the step iii is excluded.
[I-205] The process according to any one of [I-200] to [I-204], wherein the amount of the phase transfer catalyst used is 0 (zero) to 0.1 mol based on 1 mol of the compound of the formula (4), with the proviso that any process not comprising the step iii is excluded.
[I-206] The process according to any one of [I-201] to [I-204], wherein the amount of the phase transfer catalyst used is 0.001 to 0.1 mol based on 1 mol of the compound of the formula (4), with the proviso that any process not comprising the step iii is excluded.
[I-207] The process according to any one of [I-201] to [I-204], wherein the amount of the phase transfer catalyst used is 0.005 to 0.05 mol based on 1 mol of the compound of the formula (4), with the proviso that any process not comprising the step iii is excluded.
[I-208] The process according to any one of [I-1] to [I-207], wherein the reaction in the step iii is performed in the presence of an organic solvent and a water solvent, with the proviso that any process not comprising the step iii is excluded.
[I-209] The process according to [I-208], wherein the organic solvent in the reaction in the step iii is an organic solvent having an acceptor number of 0 to 50, with the proviso that any process not comprising the step iii is excluded.
[I-210] The process according to [I-208], wherein the organic solvent in the reaction in the step iii is an organic solvent having an acceptor number of 3 to 45, with the proviso that any process not comprising the step iii is excluded.
[I-211] The process according to [I-208], wherein the organic solvent in the reaction in the step iii is an organic solvent having an acceptor number of 5 to 45, with the proviso that any process not comprising the step iii is excluded.
[I-212] The process according to [I-208], wherein the organic solvent in the reaction in the step iii is an organic solvent having an acceptor number of 5 to 35, with the proviso that any process not comprising the step iii is excluded.
[I-213] The process according to [I-208], wherein the organic solvent in the reaction in the step iii is an organic solvent having an acceptor number of 5 to 30, with the proviso that any process not comprising the step iii is excluded.
[I-214] The process according to [I-208], wherein the organic solvent in the reaction in the step iii is an organic solvent having an acceptor number of 5 to 20, with the proviso that any process not comprising the step iii is excluded.
[I-215] The process according to [1-208], wherein the organic solvent in the reaction in the step iii is an organic solvent having an acceptor number of 8 to 20, with the proviso that any process not comprising the step iii is excluded.
[I-216] The process according to [I-208], wherein the organic solvent in the reaction in the step iii is selected from aromatic hydrocarbon derivatives, halogenated aliphatic hydrocarbons, alcohols, nitriles, carboxylic acid esters, ethers, ketones, amides, ureas, sulfoxides and sulfones, with the proviso that any process not comprising the step iii is excluded.
[I-217] The process according to [I-208], wherein the organic solvent in the reaction in the step iii is one or more (preferably one or two, more preferably one) organic solvents selected from aromatic hydrocarbon derivatives, halogenated aliphatic hydrocarbons, alcohols, nitriles, carboxylic acid esters, ethers, ketones, amides, ureas, sulfoxides and sulfones, with the proviso that any process not comprising the step iii is excluded.
[I-218] The process according to [I-208], wherein the organic solvent in the reaction in the step iii is one or more (preferably one or two, more preferably one) organic solvents selected from alcohols, nitriles, carboxylic acid esters, ethers, amides and sulfones, with the proviso that any process not comprising the step iii is excluded.
[I-219] The process according to [I-208], wherein the organic solvent in the reaction in the step iii is one or more (preferably one or two, more preferably one) organic solvents selected from alcohols, nitriles, carboxylic acid esters, ethers and amides, with the proviso that any process not comprising the step iii is excluded.
[I-220] The process according to [I-208], wherein the organic solvent in the reaction in the step iii is selected from alcohols, nitriles, carboxylic acid esters and amides, with the proviso that any process not comprising the step iii is excluded.
[I-221] The process according to [I-208], wherein the organic solvent in the reaction in the step iii is one or more organic solvents selected from alcohols, nitriles, carboxylic acid esters and amides, with the proviso that any process not comprising the step iii is excluded.
[I-222] The process according to [1-208], wherein the organic solvent in the reaction in the step iii is selected from alcohols, nitriles and carboxylic acid esters, with the proviso that any process not comprising the step iii is excluded.
[I-223] The process according to [I-208], wherein the organic solvent in the reaction in the step iii is one or more (preferably one or two, more preferably one) organic solvents selected from alcohols, nitriles and carboxylic acid esters, with the proviso that any process not comprising the step iii is excluded.
[I-224] The process according to [I-208], wherein the organic solvent in the reaction in the step iii is one or two organic solvents selected from alcohols, nitriles and carboxylic acid esters, with the proviso that any process not comprising the step iii is excluded.
[I-225] The process according to [I-208], wherein the organic solvent in the reaction in the step iii is one organic solvent selected from alcohols, nitriles and carboxylic acid esters, with the proviso that any process not comprising the step iii is excluded.
[I-226] The process according to [1-208], wherein the organic solvent in the reaction in the step iii is selected from nitriles and carboxylic acid esters, with the proviso that any process not comprising the step iii is excluded.
[I-227] The process according to [I-208], wherein the organic solvent in the reaction in the step iii is one or more organic solvents selected from nitriles and carboxylic acid esters, with the proviso that any process not comprising the step iii is excluded.
[I-228] The process according to [I-208], wherein the organic solvent in the reaction in the step iii is one or two organic solvents selected from nitriles and carboxylic acid esters, with the proviso that any process not comprising the step iii is excluded.
[I-229] The process according to [I-208], wherein the organic solvent in the reaction in the step iii is one organic solvent selected from nitriles and carboxylic acid esters, with the proviso that any process not comprising the step iii is excluded.
[I-230] The process according to [I-208], wherein the organic solvent in the reaction in the step iii is a nitrile, with the proviso that any process not comprising the step iii is excluded.
[I-231] The process according to [I-208], wherein the organic solvent in the reaction in the step iii is selected from nitriles, with the proviso that any process not comprising the step iii is excluded.
[I-232] The process according to [I-208], wherein the organic solvent in the reaction in the step iii is one or more organic solvents selected from nitriles, with the proviso that any process not comprising the step iii is excluded.
[I-233] The process according to [I-208], wherein the organic solvent in the reaction in the step iii is one or two organic solvents selected from nitriles, with the proviso that any process not comprising the step iii is excluded.
[I-234] The process according to [I-208], wherein the organic solvent in the reaction in the step iii is one organic solvent selected from nitriles, with the proviso that any process not comprising the step iii is excluded.
[I-235] The process according to [1-208], wherein the organic solvent in the reaction in the step iii is a carboxylic acid ester, with the proviso that any process not comprising the step iii is excluded.
[I-236] The process according to [I-208], wherein the organic solvent in the reaction in the step iii is selected from carboxylic acid esters, with the proviso that any process not comprising the step iii is excluded.
[I-237] The process according to [I-208], wherein the organic solvent in the reaction in the step iii is one or more organic solvents selected from carboxylic acid esters, with the proviso that any process not comprising the step iii is excluded.
[I-238] The process according to [I-208], wherein the organic solvent in the reaction in the step iii is preferably one to five organic solvents selected from carboxylic acid esters, with the proviso that any process not comprising the step iii is excluded.
[I-239] The process according to [I-208], wherein the organic solvent in the reaction in the step iii is preferably one or two organic solvents selected from carboxylic acid esters, with the proviso that any process not comprising the step iii is excluded.
[I-240] The process according to [I-208], wherein the organic solvent in the reaction in the step iii is one organic solvent selected from carboxylic acid esters, with the proviso that any process not comprising the step iii is excluded.
[I-241] The process according to [I-208], wherein the organic solvent in the reaction in the step iii is selected from (C1-C6)alcohols, (C2-C5)alkane nitriles, (C1-C4)alkyl (C1-C4)carboxylates and N,N-di ((C1-C4) alkyl) (C1-C4)alkaneamides, with the proviso that any process not comprising the step iii is excluded.
[I-242] The process according to [I-208], wherein the organic solvent in the reaction in the step iii is one or more organic solvents selected from (C1-C6)alcohols, (C2-C5)alkane nitriles, (C1-C4)alkyl (C1-C4)carboxylates and N,N-di ((C1-C4) alkyl) (C1-C4) alkaneamides, with the proviso that any process not comprising the step iii is excluded.
[I-243] The process according to [I-208], wherein the organic solvent in the reaction in the step iii is one or two organic solvents selected from (C1-C6)alcohols, (C2-C5)alkane nitriles, (C1-C4)alkyl (C1-C4)carboxylates and N,N-di((C1-C4)alkyl)(C1-C4)alkaneamides, with the proviso that any process not comprising the step iii is excluded.
[I-244] The process according to [I-208], wherein the organic solvent in the reaction in the step iii is one organic solvent selected from (C1-C6)alcohols, (C2-C5)alkane nitriles, (C1-C4)alkyl (C1-C4)carboxylates and N,N-di((C1-C4)alkyl)(C1-C4)alkaneamides, with the proviso that any process not comprising the step iii is excluded.
[I-245] The process according to [I-208], wherein the organic solvent in the reaction in the step iii is one or more (preferably one or two, more preferably one) organic solvents selected from (C2-C6)alcohols, (C2-C5)alkane nitriles, (C1-C4)alkyl (C1-C4)carboxylates and N,N-di((C1-C4)alkyl)(C1-C4)alkaneamides, with the proviso that any process not comprising the step iii is excluded.
[I-246] The process according to [I-208], wherein the organic solvent in the reaction in the step iii is one or more (preferably one or two, more preferably one) organic solvents selected from (C1-C4)alcohols, (C2-C5)alkane nitriles, (C1-C4)alkyl (C1-C4)carboxylates and N,N-di((C1-C4)alkyl)(C1-C4)alkaneamides, with the proviso that any process not comprising the step iii is excluded.
[I-247] The process according to [I-208], wherein the organic solvent in the reaction in the step iii is one or more (preferably one or two, more preferably one) organic solvents selected from (C2-C4)alcohols, (C2-C5)alkane nitriles, (C1-C4)alkyl (C1-C4)carboxylates and N,N-di((C1-C4)alkyl)(C1-C4)alkaneamides, with the proviso that any process not comprising the step iii is excluded.
[I-248] The process according to [I-208], wherein the organic solvent in the reaction in the step iii is selected from (C1-C6)alcohols, (C2-C5)alkane nitriles and (C1-C4)alkyl (C1-C4)carboxylates, with the proviso that any process not comprising the step iii is excluded.
[I-249] The process according to [I-208], wherein the organic solvent in the reaction in the step iii is one or more organic solvents selected from (C1-C6)alcohols, (C2-C5)alkane nitriles and (C1-C4)alkyl (C1-C4)carboxylates, with the proviso that any process not comprising the step iii is excluded.
[I-250] The process according to [I-208], wherein the organic solvent in the reaction in the step iii is one or two organic solvents selected from (C1-C6)alcohols, (C2-C5)alkane nitriles and (C1-C4)alkyl (C1-C4)carboxylates, with the proviso that any process not comprising the step iii is excluded.
[I-251] The process according to [I-208], wherein the organic solvent in the reaction in the step iii is one organic solvent selected from (C1-C6)alcohols, (C2-C5)alkane nitriles and (C1-C4)alkyl (C1-C4)carboxylates, with the proviso that any process not comprising the step iii is excluded.
[I-252] The process according to [I-208], wherein the organic solvent in the reaction in the step iii is one or more (preferably one or two, more preferably one) organic solvents selected from (C2-C6)alcohols, (C2-C5)alkane nitriles and (C1-C4)alkyl (C1-C4)carboxylates, with the proviso that any process not comprising the step iii is excluded.
[I-253] The process according to [I-208], wherein the organic solvent in the reaction in the step iii is one or more (preferably one or two, more preferably one) organic solvents selected from (C1-C4)alcohols, (C2-C5)alkane nitriles and (C1-C4)alkyl (C1-C4)carboxylates, with the proviso that any process not comprising the step iii is excluded.
[I-254] The process according to [I-208], wherein the organic solvent in the reaction in the step iii is one or more (preferably one or two, more preferably one) organic solvents selected from (C2-C4)alcohols, (C2-C5)alkane nitriles and (C1-C4)alkyl (C1-C4)carboxylates, with the proviso that any process not comprising the step iii is excluded.
[I-255] The process according to [I-208], wherein the organic solvent in the reaction in the step iii is selected from (C2-C5)alkane nitriles and (C1-C4)alkyl (C1-C4)carboxylates, with the proviso that any process not comprising the step iii is excluded.
[I-256] The process according to [I-208], wherein the organic solvent in the reaction in the step iii is one or more organic solvents selected from (C2-C5) alkane nitrile and (C1-C4) alkyl (C1-C4) carboxylate, with the proviso that any process not comprising the step iii is excluded.
[I-257] The process according to [I-208], wherein the organic solvent in the reaction in the step iii is one or two organic solvents selected from (C2-C5) alkane nitrile and (C1-C4) alkyl (C1-C4) carboxylate, with the proviso that any process not comprising the step iii is excluded.
[I-258] The process according to [I-208], wherein the organic solvent in the reaction in the step iii is one organic solvent selected from (C2-C5) alkane nitrile and (C1-C4) alkyl (C1-C4) carboxylate, with the proviso that any process not comprising the step iii is excluded.
[I-259] The process according to [I-208], wherein the organic solvent in the reaction in the step iii is one or more (preferably one or two, more preferably one) organic solvents selected from toluene, xylenes, chlorobenzene, dichlorobenzenes, dichloromethane, 1,2-dichloroethane, methanol, ethanol, 2-propanol, butanol, tert-butanol, acetonitrile, ethyl acetate, propyl acetate, isopropyl acetate, butyl acetate and isomers thereof, tetrahydrofuran, 1,4-dioxane, diisopropyl ether, dibutyl ether, di-tert-butyl ether, cyclopentyl methyl ether, methyl-tert-butyl ether, 1,2-dimethoxyethane, diglyme, acetone, methyl ethyl ketone, methyl isopropyl ketone, methyl isobutyl ketone, N,N-dimethylformamide, N,N-dimethylacetamide, N-methylpyrrolidone, N,N'-dimethylimidazolidinone, tetramethylurea, dimethyl sulfoxide and sulfolane, with the proviso that any process not comprising the step iii is excluded.
[I-259] The process according to [I-208], wherein the organic solvent in the reaction in the step iii is one or more (preferably one or two, more preferably one) organic solvents selected from methanol, ethanol, 2-propanol, butanol, tert-butanol, acetonitrile, ethyl acetate, propyl acetate, isopropyl acetate, butyl acetate and isomers thereof, tetrahydrofuran, 1,4-dioxane, diisopropyl ether, dibutyl ether, di-tert-butyl ether, cyclopentyl methyl ether, methyl-tert-butyl ether, 1,2-dimethoxyethane, diglyme, N,N-dimethylformamide, N,N-dimethylacetamide, N-methylpyrrolidone and sulfolane, with the proviso that any process not comprising the step iii is excluded.
[I-260] The process according to [I-208], wherein the organic solvent in the reaction in the step iii is one or more (preferably one or two, more preferably one) organic solvents selected from methanol, ethanol, 2-propanol, butanol, tert-butanol, acetonitrile, ethyl acetate, propyl acetate, isopropyl acetate, butyl acetate and isomers thereof, tetrahydrofuran, 1,4-dioxane, diisopropyl ether, dibutyl ether, di-tert-butyl ether, cyclopentyl methyl ether, methyl-tert-butyl ether, 1,2-dimethoxyethane, diglyme, N,N-dimethylformamide, N,N-dimethylacetamide and N-methylpyrrolidone, with the proviso that any process not comprising the step iii is excluded.
[I-261] The process according to [I-208], wherein the organic solvent in the reaction in the step iii is one or more (preferably one or two, more preferably one) organic solvents selected from methanol, ethanol, 2-propanol, butanol, tert-butanol, acetonitrile, ethyl acetate, propyl acetate, isopropyl acetate, butyl acetate and isomers thereof, N,N-dimethylformamide, N,N-dimethylacetamide and N-methylpyrrolidone, with the proviso that any process not comprising the step iii is excluded.
[I-262] The process according to [I-208], wherein the organic solvent in the reaction in the step iii is one organic solvent selected from methanol, ethanol, 2-propanol, butanol, tert-butanol, acetonitrile, ethyl acetate, propyl acetate, isopropyl acetate, butyl acetate and N,N-dimethylformamide, with the proviso that any process not comprising the step iii is excluded.
[I-263] The process according to [I-208], wherein the organic solvent in the reaction in the step iii is one or more (preferably one or two, more preferably one) organic solvents selected from methanol, ethanol, 2-propanol, butanol, tert-butanol, acetonitrile, ethyl acetate, propyl acetate, isopropyl acetate, butyl acetate and isomers thereof, with the proviso that any process not comprising the step iii is excluded.
[I-264] The process according to [I-208], wherein the organic solvent of the reaction in the step iii is one organic solvent selected from methanol, ethanol, 2-propanol, butanol, tert-butanol, acetonitrile, ethyl acetate, propyl acetate, isopropyl acetate and butyl acetate, with the proviso that any process not comprising the step iii is excluded.
[I-265] The process according to [I-208], wherein the organic solvent in the reaction in the step iii is one or more (preferably one or two, more preferably one) organic solvents selected from ethanol, 2-propanol, butanol, tert-butanol, acetonitrile, ethyl acetate, propyl acetate, isopropyl acetate, butyl acetate and isomers thereof (preferably one or more (preferably one or two, more preferably one) organic solvents selected from ethanol, 2-propanol, butanol, tert-butanol and acetonitrile), with the proviso that any process not comprising the step iii is excluded.
[I-266] The process according to [I-208], wherein the organic solvent in the reaction in the step iii is one or more (preferably one or two, more preferably one) organic solvents selected from butanol, acetonitrile, ethyl acetate, propyl acetate, isopropyl acetate and butyl acetate, with the proviso that any process not comprising the step iii is excluded.
[I-267] The process according to [I-208], wherein the organic solvent in the reaction in the step iii is one or more (preferably one or two, more preferably one) organic solvents selected from acetonitrile, ethyl acetate, isopropyl acetate and butyl acetate, with the proviso that any process not comprising the step iii is excluded.
[I-268] The process according to [I-208], wherein the organic solvent in the reaction in the step iii is one organic solvent selected from acetonitrile, ethyl acetate, isopropyl acetate and butyl acetate, with the proviso that any process not comprising the step iii is excluded.
[I-269] The process according to [I-208], wherein the organic solvent in the reaction in the step iii is one or two (preferably one) organic solvents selected from acetonitrile and butyl acetate, with the proviso that any process not comprising the step iii is excluded.
[I-270] The process according to [I-208], wherein the organic solvent in the reaction in the step iii is acetonitrile or butyl acetate, with the proviso that any process not comprising the step iii is excluded.
[I-271] The process according to [I-208], wherein the organic solvent in the reaction in the step iii is acetonitrile, with the proviso that any process not comprising the step iii is excluded.
[I-272] The process according to [I-208], wherein the organic solvent in the reaction in the step iii is butyl acetate, with the proviso that any process not comprising the step iii is excluded.
[I-273] The process according to any one of [I-208] to [I-272], wherein the total amount of the solvent used in the reaction in the step iii is 0.3 to 3 liters based on 1 mol of the compound of the formula (4), with the proviso that any process not comprising the step iii is excluded.
[I-274] The process according to any one of [1-208] to [I-272], wherein the total amount of the solvent used in the reaction in the step iii is 0.5 to 3 liters based on 1 mol of the compound of the formula (4), with the proviso that any process not comprising the step iii is excluded.
[I-275] The process according to any one of [I-208] to [I-272], wherein the total amount of the solvent used in the reaction in the step iii is 0.5 to 2 liters based on 1 mol of the compound of the formula (4), with the proviso that any process not comprising the step iii is excluded.
[I-276] The process according to any one of [I-208] to [I-272], wherein the total amount of the solvent used in the reaction in the step iii is 0.7 to 1.8 liters based on 1 mol of the compound of the formula (4), with the proviso that any process not comprising the step iii is excluded.
[I-277] The process according to any one of [I-208] to [I-276], wherein the amount of the organic solvent used in the reaction in the step iii is 0.3 to 2 liters based on 1 mol of the compound of the formula (4), with the proviso that any process not comprising the step iii is excluded.
[I-279] The process according to any one of [I-208] to [I-276], wherein the amount of the organic solvent used in the reaction in the step iii is 0.4 to 1.5 liters based on 1 mol of the compound of the formula (4), with the proviso that any process not comprising the step iii is excluded.
[I-280] The process according to any one of [I-208] to [I-276], wherein the amount of the organic solvent used in the reaction in the step iii is 0.4 to 1.3 liters based on 1 mol of the compound of the formula (4), with the proviso that any process not comprising the step iii is excluded.
[I-281] The process according to any one of [I-208] to [I-276], wherein the amount of the organic solvent used in the reaction in the step iii is 0.5 to 1.3 liters based on 1 mol of the compound of the formula (4), with the proviso that any process not comprising the step iii is excluded.
[I-282] The process according to any one of [I-208] to [I-281], wherein the amount of the water solvent used in the reaction in the step iii is 0.05 to 1 liter based on 1 mol of the compound of the formula (4), with the proviso that any process not comprising the step iii is excluded.
[I-283] The process according to any one of [I-208] to [I-281], wherein the amount of the water solvent used in the reaction in the step iii is 0.1 to 1 liter based on 1 mol of the compound of the formula (4), with the proviso that any process not comprising the step iii is excluded.
[I-284] The process according to any one of [I-208] to [I-281], wherein the amount of the water solvent used in the reaction in the step iii is 0.1 to 0.8 liters based on 1 mol of the compound of the formula (4), with the proviso that any process not comprising the step iii is excluded.
[I-285] The process according to any one of [I-208] to [I-281], wherein the amount of the water solvent used in the reaction in the step iii is 0.2 to 0.5 liters based on 1 mol of the compound of the formula (4), with the proviso that any process not comprising the step iii is excluded.
[I-286] The process according to any one of [I-208] to [I-281], wherein the amount of the water solvent used in the reaction in the step iii is 0.1 to 0.3 liters based on 1 mol of the compound of the formula (4), with the proviso that any process not comprising the step iii is excluded.
[I-287] The process according to any one of [I-208] to [I-286], wherein the ratio of the organic solvent to the water solvent used in the reaction in the step iii is 90 : 10 to 50 : 50 by volume ratio, with the proviso that any process not comprising the step iii is excluded.
[I-288] The process according to any one of [1-208] to [I-286], wherein the ratio of the organic solvent to the water solvent used in the reaction in the step iii is 85 : 15 to 55 : 45 by volume ratio, with the proviso that any process not comprising the step iii is excluded.
[I-289] The process according to any one of [I-208] to [I-286], wherein the ratio of the organic solvent to the water solvent used in the reaction in the step iii is 80 : 20 to 60 : 40 by volume ratio, with the proviso that any process not comprising the step iii is excluded.
[I-290] The process according to any one of [I-208] to [I-286], wherein the ratio of the organic solvent to the water solvent used in the reaction in the step iii is 75 : 25 to 60 : 40 by volume ratio, with the proviso that any process not comprising the step iii is excluded.
[I-291] The process according to any one of [I-208] to [I-286], wherein in the reaction in the step iii, the amount of the water solvent in the whole solvent composed of the organic solvent and the water solvent is 10 vol% to 50 vol% based on the amount of the whole solvent, with the proviso that any process not comprising the step iii is excluded.
[I-292] The process according to any one of [I-208] to [I-286], wherein in the reaction in the step iii, the amount of the water solvent in the whole solvent composed of the organic solvent and the water solvent is 15 vol% to 45 vol% based on the amount of the whole solvent, with the proviso that any process not comprising the step iii is excluded.
[I-293] The process according to any one of [I-208] to [I-286], wherein in the reaction in the step iii, the amount of the water solvent in the whole solvent composed of the organic solvent and the water solvent is 20 vol% to 40 vol% based on the amount of the whole solvent, with the proviso that any process not comprising the step iii is excluded.
[I-294] The process according to any one of [I-208] to [I-286], wherein in the reaction in the step iii, the amount of the water solvent in the whole solvent composed of the organic solvent and the water solvent is 25 vol% to 40 vol% based on the amount of the whole solvent, with the proviso that any process not comprising the step iii is excluded.
[I-295] The process according to any one of [I-1] to [I-294], wherein the step iii comprises the following steps:
   (step iii-1) adding a compound of the formula (4), an organic solvent, a water solvent and a metal catalyst;
   (step iii-2) adding hydrogen peroxide thereto to react the compound of the formula (4) with the hydrogen peroxide, with the proviso that any process not comprising the step iii is excluded.
[I-296] The process according to any one of [I-1] to [I-294], wherein the step iii comprises the following steps:
   (step iii-1) adding a compound of the formula (4), an organic solvent, a water solvent and a tungsten catalyst; (step iii-2) adding hydrogen peroxide thereto to react the compound of the formula (4) with the hydrogen peroxide, with the proviso that any process not comprising the step iii is excluded and any process not including a tungsten catalyst is excluded.
[I-297] The process according to any one of [I-1] to [I-294], wherein the step iii comprises the following steps:
   (step iii-1) adding a compound of the formula (4), an organic solvent, a water solvent and a molybdenum catalyst;
   (step iii-2) adding hydrogen peroxide thereto to react the compound of the formula (4) with the hydrogen peroxide, with the proviso that any process not comprising the step iii is excluded and any process not including a molybdenum catalyst is excluded.
[I-298] The process according to any one of [1-295] to [I-297], wherein the amount of the water solvent added in the step iii-1 is 0 to 0.5 liters based on 1 mol of the compound of the formula (4), with the proviso that any process not comprising the step iii is excluded.
[I-299] The process according to any one of [1-295] to [I-297], wherein the amount of the water solvent added in the step iii-1 is 0.05 to 0.5 liters based on 1 mol of the compound of the formula (4), with the proviso that any process not comprising the step iii is excluded.
[I-300] The process according to any one of [I-295] to [I-297], wherein the amount of the water solvent added in the step iii-1 is 0.05 to 0.4 liters based on 1 mol of the compound of the formula (4), with the proviso that any process not comprising the step iii is excluded.
[I-301] The process according to any one of [I-295] to [I-297], wherein the amount of the water solvent added in the step iii-1 is 0.08 to 0.2 liters based on 1 mol of the compound of the formula (4), with the proviso that any process not comprising the step iii is excluded.
[I-302] The process according to any one of [1-295] to [I-297], wherein the amount of the water solvent added in the step iii-1 is 0.1 to 0.3 liters based on 1 mol of the compound of the formula (4), with the proviso that any process not comprising the step iii is excluded.
[I-303] The process according to any one of [I-1] to [I-302], wherein the reaction in the step iii is performed at 50°C to 150°C, with the proviso that any process not comprising the step iii is excluded.
[I-304] The process according to any one of [I-1] to [I-302], wherein the reaction in the step iii is performed at 50°C to 90°C, with the proviso that any process not comprising the step iii is excluded.
[I-305] The process according to any one of [I-1] to [I-302], wherein the reaction in the step iii is performed at 60°C to 120°C, with the proviso that any process not comprising the step iii is excluded.
[I-306] The process according to any one of [I-1] to [I-302], wherein the reaction in the step iii is performed at 60°C to 100°C, with the proviso that any process not comprising the step iii is excluded.
[I-307] The process according to any one of [I-1] to [I-302], wherein the reaction in the step iii is performed at 60°C to 90°C, with the proviso that any process not comprising the step iii is excluded.
[I-308] The process according to any one of [I-1] to [I-302], wherein the reaction in the step iii is performed at 70°C to 90°C, with the proviso that any process not comprising the step iii is excluded.
[I-309] The process according to any one of [I-1] to [I-302], wherein the reaction in the step iii is performed at 75°C to 90°C, with the proviso that any process not comprising the step iii is excluded.
[I-310] The process according to any one of [I-1] to [I-302], wherein the reaction in the step iii is performed at 75°C to 80°C, with the proviso that any process not comprising the step iii is excluded.
[I-311] The process according to any one of [I-1] to [I-310], wherein the reaction in the step iii is performed in 3 hours to 48 hours, with the proviso that any process not comprising the step iii is excluded.
[I-312] The process according to any one of [I-1] to [I-310], wherein the reaction in the step iii is performed in 4 hours to 24 hours, with the proviso that any process not comprising the step iii is excluded.
[I-313] The process according to any one of [I-1] to [I-312], wherein
   R¹ is a (C1-C4)alkyl,
   R² is a (C1-C4)perfluoroalkyl, and
   R³ is a (C1-C4)alkyl optionally substituted with 1 to 9 fluorine atoms.
[I-314] The process according to any one of [I-1] to [I-312], wherein
   R¹ is methyl,
   R² is trifluoromethyl, and
   R³ is difluoromethyl.
[I-315] The process according to any one of [I-1] to [I-312], wherein
   R⁴ and R⁵ are each independently a (C1-C4)alkyl.
[I-316] The process according to any one of [I-1] to [I-312], wherein
   R⁴ and R⁵ are methyl.
[I-317] The process according to any one of [I-1] to [I-312], wherein X¹ is a chlorine atom or a bromine atom.
[I-318] The process according to any one of [I-1] to [I-312], wherein
   X¹ is a chlorine atom.
[I-319] The process according to any one of [I-1] to [I-312], wherein
   X² is a chlorine atom, a bromine atom, a sulfate group, a hydrogen sulfate group, a phosphate group, a monohydrogen phosphate group, methanesulfonyloxy, p-toluenesulfonyloxy, or a mixture of two or more (preferably two or three, more preferably two) thereof.
[I-320] The process according to any one of [I-1] to [I-312], wherein
   X² is a halogen atom.
[I-321] The process according to any one of [I-1] to [I-312], wherein
   X² is a chlorine atom, a bromine atom, or a mixture thereof.
[I-322] The process according to any one of [I-1] to [I-312], wherein
   X² is a chlorine atom.
[I-323] The process according to any one of [I-1] to [I-312], wherein
   X² is a bromine atom.
[I-324] Use of a compound of the formula (5) produced by the process according to any one of [I-1] to [I-312] as an agrochemical.
In another aspect, the present invention is as follows.
[II-1] A process for producing a compound of the formula (4), the process comprising the following step ii:
   (step ii) a step of reacting a compound of the formula (2) with a compound of the formula (3) in the presence of a base to produce the compound of the formula (4); 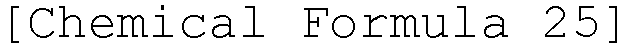 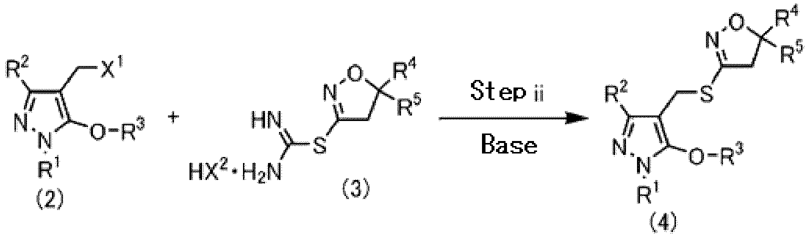
   wherein in the formula (2),
      R¹, R² and R³ are each independently a (C1-C6)alkyl optionally substituted with one or more substituents, a (C3-C6)cycloalkyl optionally substituted with one or more substituents, a (C2-C6)alkenyl optionally substituted with one or more substituents, a (C2-C6)alkynyl optionally substituted with one or more substituents, or a (C6-C10)aryl optionally substituted with one or more substituents, and
      X¹ is a leaving group,
      in the formula (3),
      R⁴ and R⁵ are each independently a (C1-C6)alkyl optionally substituted with one or more substituents, a (C3-C6)cycloalkyl optionally substituted with one or more substituents, a (C2-C6)alkenyl optionally substituted with one or more substituents, a (C2-C6)alkynyl optionally substituted with one or more substituents, a (C1-C6)alkoxy optionally substituted with one or more substituents; or a (C6-C10)aryl optionally substituted with one or more substituents, or
      R⁴ and R⁵, together with the carbon atom to which they are attached, form a 4- to 12-membered carbocyclic ring, wherein the formed ring is optionally substituted with one or more substituents,
      X² is an atom or an atomic group that forms an acid,
      in the formula (4), R¹, R², R³, R⁴ and R⁵ are as defined above.
[II-2] The process according to [II-1], wherein the base in the step ii is an alkali metal hydroxide.
[II-3] The process according to [II-1], wherein the base in the step ii is sodium hydroxide.
[II-4] The process according to any one of [II-1] to [II-3], wherein in the formula (2),
   R¹ is a (C1-C4)alkyl,
   R² is a (C1-C4)perfluoroalkyl,
   R³ is a (C1-C4)alkyl optionally substituted with 1 to 9 fluorine atoms,
   X¹ is a chlorine atom or a bromine atom,
   in the formula (3),
   R⁴ and R⁵ are each independently a (C1-C4)alkyl,
   X² is a chlorine atom, a bromine atom, a sulfate group, a hydrogen sulfate group, a phosphate group, a monohydrogen phosphate group, methanesulfonyloxy, p-toluenesulfonyloxy, or a mixture of two or more thereof, and in the formula (4), R¹, R², R³, R⁴ and R⁵ are as defined above.
[II-5] The process according to any one of [II-1] to [II-3], wherein in the formula (2),
   R¹ is methyl,
   R² is trifluoromethyl,
   R³ is difluoromethyl,
   X¹ is a chlorine atom,
   in the formula (3),
   R⁴ and R⁵ are methyl,
   X² is a chlorine atom, a bromine atom, or a mixture thereof,
   in the formula (4), R¹, R², R3, R⁴ and R⁵ are as defined above.
[II-6] The process according to any one of [II-1] to [II-5], the process further comprising the following step i before the step ii:
   (step i) a step of reacting a compound of the formula (1) with a halogenating agent to produce the compound of the formula (2): 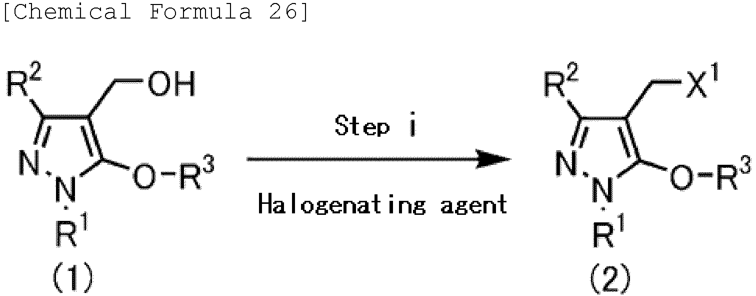
   wherein in the formula (1), R¹, R² and R³ are as defined in [II-1],
   in the formula (2), R¹, R² and R³ are as defined in [II-1], and X¹ is a halogen atom.
[II-7] The process according to [II-6], wherein the halogenating agent in the step i is a chlorinating agent.
[II-8] The process according to [II-6], wherein the halogenating agent in the step i is thionyl chloride.
[II-9] The process according to [II-7] or [II-8], wherein in the formula (1),
   R¹ is a (C1-C4)alkyl,
   R² is a (C1-C4)perfluoroalkyl,
   R³ is a (C1-C4)alkyl optionally substituted with 1 to 9 fluorine atoms,
   in the formula (2),
   R¹, R² and R³ are as defined above,
   X¹ is a chlorine atom.
[II-10] The process according to [II-7] or [II-8], wherein in the formula (1),
   R¹ is methyl,
   R² is trifluoromethyl,
   R³ is difluoromethyl,
   in the formula (2),
   R¹, R² and R³ are as defined above,
   X¹ is a chlorine atom.
[II-11] The process according to any one of [II-1] to [II-10], the process further comprising the following step iii after the step ii:
   (step iii) a step of reacting the compound of the formula (4) with hydrogen peroxide in the presence of a metal catalyst to produce a compound of the formula (5): 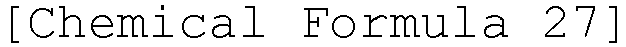 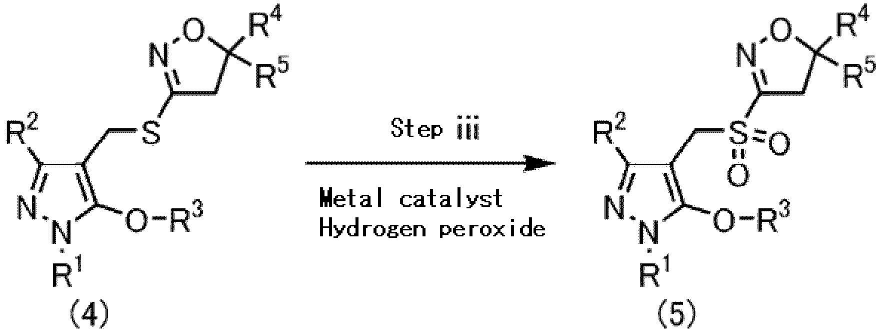
   wherein R¹, R², R³, R⁴ and R⁵ in the formula (4) and the formula (5) are as defined in [II-1].
[II-12] A process for producing a compound of the formula (5) : 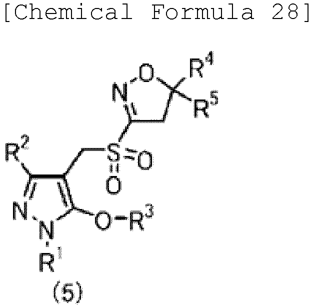 the process comprising the following steps:
   (step ii) producing a compound of the formula (4) by the process according to any one of [II-1] to [II-10]: 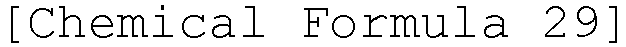 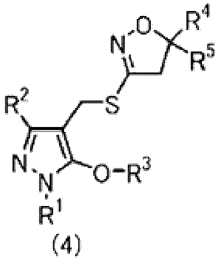
   (step iii) reacting the compound of the formula (4) with hydrogen peroxide in the presence of a metal catalyst to produce a compound of formula (5): 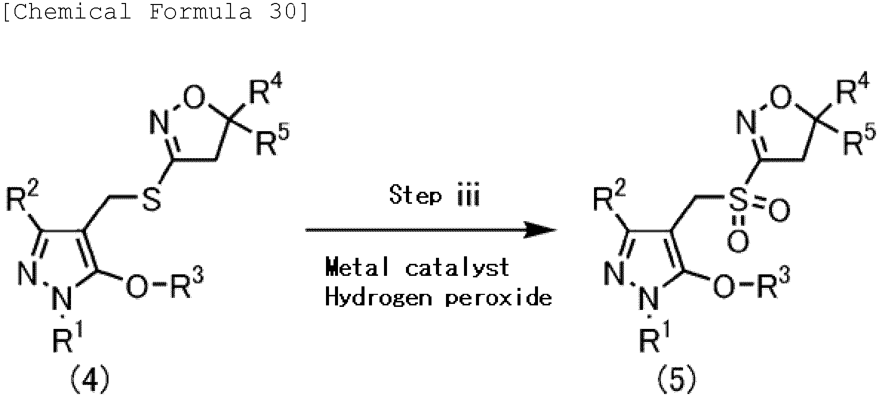
   wherein R¹, R², R³, R⁴ and R⁵ in the formula (4) and the formula (5) are as defined in [II-1].
[II-13] A process for producing a compound of the formula (5) : 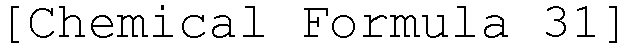 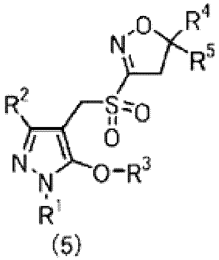 using a compound of the formula (4): 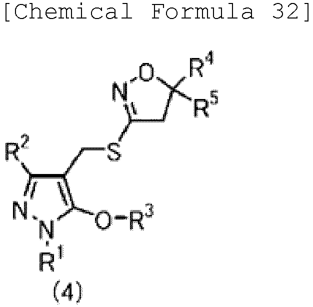 wherein
   the compound of the formula (4) is produced by the process according to any one of [II-1] to [II-10],
   wherein R¹, R², R³, R⁴ and R⁵ in the formula (4) and the formula (5) are as defined in [II-1].
[II-14] A process for producing a compound of the formula (5) : 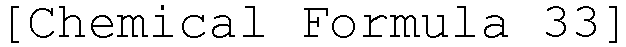 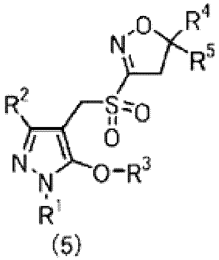 using a compound of the formula (4): 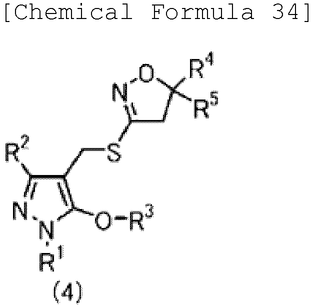 wherein
   as the compound of the formula (4), a compound of the formula (4) produced by the process according to any one of [II-1] to [II-10] is used,
   wherein R¹, R², R³, R⁴ and R⁵ in the formula (4) and the formula (5) are as defined in [II-1].
[II-15] The process according to [II-11] or [II-12], wherein the hydrogen peroxide in the step iii is a 10 to 70 wt% aqueous hydrogen peroxide solution.
[II-16] The process according to [II-11], [II-12], or [II-15], wherein the metal catalyst in the step iii is a tungsten catalyst, a molybdenum catalyst, a niobium catalyst, a tantalum catalyst, a titanium catalyst, or a zirconium catalyst.
[II-17] The process according to [II-11], [II-12], or [II-15], wherein the metal catalyst in the step iii is a tungsten catalyst, a molybdenum catalyst, or a niobium catalyst.
[II-18] The process according to [II-11], [II-12], or [II-15], wherein the metal catalyst in the step iii is a tungsten catalyst.
[II-19] The process according to [II-11], [II-12], or [II-15], wherein the metal catalyst in the step iii is tungstic acid, a tungstic acid salt, metal tungsten, tungsten oxide, tungsten carbide, tungsten chloride, tungsten bromide, tungsten sulfide, phosphotungstic acid or a salt thereof, silicotungstic acid or a salt thereof, or a mixture of them.
[II-20] The process according to [II-11], [II-12], or [II-15], wherein the metal catalyst in the step iii is sodium tungstate dihydrate, ammonium molybdate tetrahydrate, sodium niobate, lithium tantalate, titanium acetylacetonate, or zirconium chloride oxide.
[II-21] The process according to [II-11], [II-12], or [II-15], wherein the metal catalyst in the step iii is sodium tungstate dihydrate, ammonium molybdate tetrahydrate, or sodium niobate.
[II-22] The process according to [II-11], [II-12], or [II-15], wherein the metal catalyst in the step iii is sodium tungstate dihydrate or ammonium molybdate tetrahydrate.
[II-23] The process according to any one of [II-11], [II-12] and [II-15] to [II-22], wherein the reaction in the step iii is performed in the presence of an organic solvent and a water solvent.
[II-24] The process according to [II-23], wherein the organic solvent in the reaction in the step iii is one or more organic solvents selected from alcohols, nitriles, carboxylic acid esters, ethers and amides.
[II-25] The process according to [II-23], wherein the organic solvent in the reaction in the step iii is one or more organic solvents selected from alcohols, nitriles, carboxylic acid esters and amides.
[II-26] The process according to [II-23], wherein the organic solvent in the reaction in the step iii is one or more organic solvents selected from alcohols, nitriles and carboxylic acid esters.
[II-27] The process according to [II-23], wherein the organic solvent in the reaction in the step iii is one or more organic solvents selected from butanol, acetonitrile, ethyl acetate, propyl acetate, isopropyl acetate and butyl acetate.
[II-28] The process according to [II-23], wherein the organic solvent in the reaction in the step iii is acetonitrile or butyl acetate.
[II-29] The process according to any one of [II-11] to [II-26], wherein
   in the formula (4),
   R¹ is a (C1-C4)alkyl,
   R² is a (C1-C4)perfluoroalkyl,
   R³ is a (C1-C4)alkyl optionally substituted with 1 to 9 fluorine atoms,
   R⁴ and R⁵ are each independently a (C1-C4)alkyl,
   in the formula (5), R¹, R², R³, R⁴ and R⁵ are as defined above.
[II-30] The process according to any one of [II-11] to [II-26], wherein
   in the formula (4),
   R¹ is methyl,
   R² is trifluoromethyl,
   R³ is difluoromethyl,
   R⁴ and R⁵ are methyl,
   in the formula (5), R¹, R², R³, R⁴ and R⁵ are as defined above.
[II-31] A process for producing a compound of the formula (5), the process comprising the following step iii, wherein the reaction in the step iii is performed in the presence of an organic solvent having an acceptor number of 0 to 50 and a water solvent:
   (step iii) a step of reacting the compound of the formula (4) with hydrogen peroxide in the presence of a metal catalyst to produce a compound of the formula (5): 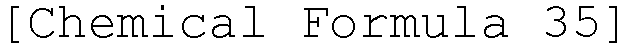 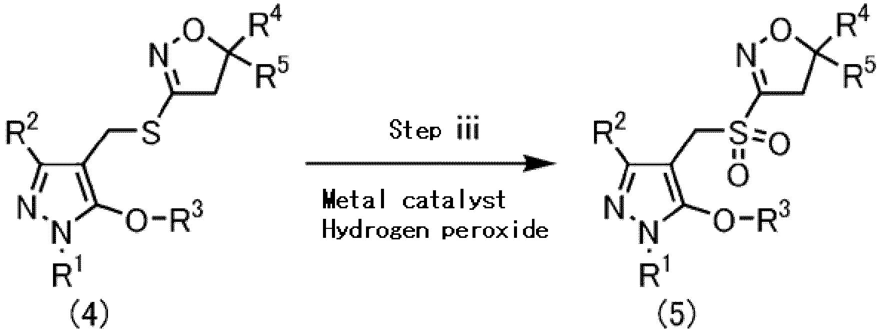
   wherein in the formula (4) and the formula (5),
   R¹, R² and R³ are each independently a (C1-C6)alkyl optionally substituted with one or more substituents, a (C3-C6)cycloalkyl optionally substituted with one or more substituents, a (C2-C6)alkenyl optionally substituted with one or more substituents, a (C2-C6)alkynyl optionally substituted with one or more substituents, or a (C6-C10)aryl optionally substituted with one or more substituents,
   R⁴ and R⁵ are each independently a (C1-C6)alkyl optionally substituted with one or more substituents, a (C3-C6)cycloalkyl optionally substituted with one or more substituents, a (C2-C6)alkenyl optionally substituted with one or more substituents, a (C2-C6)alkynyl optionally substituted with one or more substituents, a (C1-C6)alkoxy optionally substituted with one or more substituents; or a (C6-C10)aryl optionally substituted with one or more substituents, or
   R⁴ and R⁵, together with the carbon atom to which they are attached, form a 4- to 12-membered carbocyclic ring, wherein the formed ring is optionally substituted with one or more substituents.
[II-32] The process according to [II-31], wherein the hydrogen peroxide in the step iii is a 10 to 70 wt% aqueous hydrogen peroxide solution.
[II-33] The process according to [II-31] or [II-32], wherein the metal catalyst in the step iii is a tungsten catalyst, a molybdenum catalyst or a niobium catalyst, a tantalum catalyst, a titanium catalyst, a zirconium catalyst.
[II-34] The process according to [II-31] or [II-32], wherein the metal catalyst in the step iii is a tungsten catalyst, a molybdenum catalyst, or a niobium catalyst.
[II-35] The process according to [II-31] or [II-32], wherein the metal catalyst in the step iii is a tungsten catalyst.
[II-36] The process according to [II-31] or [II-32], wherein the metal catalyst in the step iii is tungstic acid, a tungstic acid salt, metal tungsten, tungsten oxide, tungsten carbide, tungsten chloride, tungsten bromide, tungsten sulfide, phosphotungstic acid or a salt thereof, silicotungstic acid or a salt thereof, or a mixture of them.
[II-37] The process according to [II-31] or [II-32], wherein the metal catalyst in the step iii is sodium tungstate dihydrate, ammonium molybdate tetrahydrate, sodium niobate, lithium tantalate, titanium acetylacetonate, or zirconium chloride oxide.
[II-38] The process according to [II-31] or [II-32], wherein the metal catalyst in the step iii is sodium tungstate dihydrate, ammonium molybdate tetrahydrate, or sodium niobate.
[II-39] The process according to [II-31] or [II-32], wherein the metal catalyst in the step iii is sodium tungstate dihydrate or ammonium molybdate tetrahydrate.
[II-40] The process according to [II-31] or [II-32], wherein the metal catalyst in the step iii is sodium tungstate dihydrate.
[II-41] The process according to any one of [11-31] to [II-40], wherein the organic solvent in the reaction in the step iii is one or more organic solvents selected from alcohols, nitriles, carboxylic acid esters, ethers and amides.
[II-42] The process according to any one of [II-31] to [II-40], wherein the organic solvent in the reaction in the step iii is one or more organic solvents selected from alcohols, nitriles, carboxylic acid esters and amides.
[II-43] The process according to any one of [II-31] to [II-40], wherein the organic solvent in the reaction in the step iii is one or more organic solvents selected from alcohols, nitriles and carboxylic acid esters.
[II-44] The process according to any one of [II-31] to [II-40], wherein the organic solvent in the reaction in the step iii is one or more organic solvents selected from butanol, acetonitrile, ethyl acetate, propyl acetate, isopropyl acetate and butyl acetate.
[II-45] The process according to any one of [II-31] to [II-40], wherein the organic solvent in the reaction in the step iii is acetonitrile or butyl acetate.
[II-46] The process according to any one of [11-31] to [II-45], wherein
   in the formula (4),
   R¹ is a (C1-C4)alkyl,
   R² is a (C1-C4)perfluoroalkyl,
   R³ is a (C1-C4)alkyl optionally substituted with 1 to 9 fluorine atoms,
   R⁴ and R⁵ are each independently a (C1-C4)alkyl,
   in the formula (5), R¹, R², R³, R⁴ and R⁵ are as defined above.
[II-47] The process according to any one of [II-31] to [II-45], wherein
   in the formula (4),
   R¹ is methyl,
   R² is trifluoromethyl,
   R³ is difluoromethyl,
   R⁴ and R⁵ are methyl,
   in the formula (5), R¹, R², R³, R⁴ and R⁵ are as defined above.
[II-48] The process according to any one of [II-23] to [II-30], wherein the total amount of the solvent used in the reaction in the step iii is 0.3 to 3 liters based on 1 mol of the compound of the formula (4).
[II-49] The process according to any one of [11-23] to [II-30], wherein the total amount of the solvent used in the reaction in the step iii is 0.5 to 2 liters based on 1 mol of the compound of the formula (4).
[II-50] The process according to any one of [II-23] to [II-30], [II-48] and [II-49], wherein the amount of the organic solvent used in the reaction in the step iii is 0.3 to 2 liters based on 1 mol of the compound of the formula (4).
[II-51] The process according to any one of [II-23] to [II-30], [II-48] and [II-49], wherein the amount of the organic solvent used in the reaction in the step iii is 0.4 to 1.5 liters based on 1 mol of the compound of the formula (4).
[II-52] The process according to any one of [II-23] to [II-30] and [II-48] to [II-51], wherein the amount of the water solvent used in the reaction in the step iii is 0.1 to 1 liter based on 1 mol of the compound of the formula (4).
[II-53] The process according to any one of [II-23] to [II-30] and [II-48] to [II-51], wherein the amount of the water solvent used in the reaction in the step iii is 0.1 to 0.3 liters based on 1 mol of the compound of the formula (4).
[II-54] The process according to any one of [II-23] to [II-30] and [II-48] to [II-53], wherein the ratio of the organic solvent to the water solvent used in the reaction in the step iii is 90 : 10 to 50 : 50 by volume ratio.
[II-55] The process according to any one of [II-23] to [II-30] and [II-48] to [II-53], wherein the ratio of the organic solvent to the water solvent used in the reaction in the step iii is 80 : 20 to 60 : 40 by volume ratio.
[II-56] The process according to any one of [II-23] to [II-30] and [II-48] to [II-55], wherein the step iii comprises the following steps:
   (step iii-1) adding the compound of the formula (4), the organic solvent, the water solvent and a tungsten catalyst; (step iii-2) adding hydrogen peroxide thereto to react the compound of the formula (4) with the hydrogen peroxide.
[II-57] The process according to [II-56], wherein the amount of the water solvent added in the step iii-1 is 0.05 to 0.4 liters based on 1 mol of the compound of the formula (4) .
[II-58] The process according to [11-56], wherein the amount of the water solvent added in the step iii-1 is 0.08 to 0.2 liters based on 1 mol of the compound of the formula (4) .
[II-59] The process according to any one of [II-31] to [II-47], wherein the total amount of the solvent used in the reaction in the step iii is 0.3 to 3 liters based on 1 mol of the compound of the formula (4).
[II-60] The process according to any one of [II-31] to [II-47], wherein the total amount of the solvent used in the reaction in the step iii is 0.5 to 2 liters based on 1 mol of the compound of the formula (4).
[II-61] The process according to any one of [II-31] to [II-47], [II-59] and [II-60], wherein the amount of the organic solvent used in the reaction in the step iii is 0.3 to 2 liters based on 1 mol of the compound of the formula (4).
[II-62] The process according to any one of [II-31] to [II-47], [II-59] and [II-60], wherein the amount of the organic solvent used in the reaction in the step iii is 0.4 to 1.5 liters based on 1 mol of the compound of the formula (4).
[II-63] The process according to any one of [11-31] to [II-47] and [II-59] to [II-62], wherein the amount of the water solvent used in the reaction in the step iii is 0.1 to 1 liter based on 1 mol of the compound of the formula (4).
[II-64] The process according to any one of [II-31] to [II-47] and [II-59] to [II-62], wherein the amount of the water solvent used in the reaction in the step iii is 0.1 to 0.3 liters based on 1 mol of the compound of the formula (4).
[II-65] The process according to any one of [II-31] to [II-47] and [II-59] to [II-64], wherein the ratio of the organic solvent to the water solvent used in the reaction in the step iii is 90 : 10 to 50 : 50 by volume ratio.
[II-66] The process according to any one of [II-31] to [II-47] and [II-59] to [II-64], wherein the ratio of the organic solvent to the water solvent used in the reaction in the step iii is 80 : 20 to 60 : 40 by volume ratio.
[II-67] The process according to any one of [II-31] to [II-47] and [II-59] to [II-66], wherein the step iii comprises the following steps:
   (step iii-1) adding the compound of the formula (4), the organic solvent, the water solvent and a tungsten catalyst; (step iii-2) adding hydrogen peroxide thereto to react the compound of the formula (4) with the hydrogen peroxide.
[II-68] The process according to [II-67], wherein the amount of the water solvent added in the step iii-1 is 0.05 to 0.4 liters based on 1 mol of the compound of the formula (4) .
[II-69] The process according to [II-67], wherein the amount of the water solvent added in the step iii-1 is 0.08 to 0.2 liters based on 1 mol of the compound of the formula (4) .
In another aspect, the present invention is as follows.
[III-1] A crystal of pyroxasulfone wherein the crystal exhibits a spectrum having peaks at diffraction angles 2Θ at least in the range of 17.8 to 17.9°, 18.0 to 18.1° and 19.9 to 20.0° in powder X-ray diffraction measurement by a transmission method using Cu-Kα ray, and the peak height of 19.9 to 20.0° is maximum among the three peaks.
[III-2] The crystal according to [III-1], wherein the crystal exhibits a spectrum further having peaks at diffraction angles 2Θ of 9.9 to 10.0°, 20.6 to 20.7° and 30.1 to 30.3° in the powder X-ray diffraction measurement.
[III-3] The crystal according to [III-1] or [III-2], wherein the crystal exhibits a spectrum having further peaks at diffraction angles 2Θ of 4.9 to 5.0° in the powder X-ray diffraction measurement.
[III-4] The crystal according to any one of [III-1] to [III-3], wherein the crystal exhibits a spectrum further having peaks at diffraction angles 2Θ of 20.3 to 20.4° in the powder X-ray diffraction measurement.
[III-5] The crystal according to any one of [III-1] to [III-4], wherein the crystal exhibits a spectrum further having peaks at diffraction angles 2Θ of 21.8 to 21.9° in the powder X-ray diffraction measurement.
[III-6] The crystal according to any one of [III-1] to [III-5], wherein the crystal exhibits a spectrum further having peaks at diffraction angles 2Θ of 22.3 to 22.4° in the powder X-ray diffraction measurement.
[III-7] The crystal according to any one of [III-1] to [III-6], wherein the crystal exhibits a spectrum further having peaks at diffraction angles 2Θ of 25.4 to 25.5° in the powder X-ray diffraction measurement.
[III-8] The crystal according to any one of [III-1] to [III-7], wherein the crystal exhibits a spectrum further having peaks at diffraction angles 2Θ of 26.6 to 26.7° in the powder X-ray diffraction measurement.
[III-9] The crystal according to any one of [III-1] to [III-8], wherein the crystal exhibits a spectrum further having peaks at diffraction angles 2Θ of 26.9 to 27.0° in the powder X-ray diffraction measurement.
[III-10] The crystal according to any one of [III-1] to [III-9], wherein the crystal exhibits a spectrum further having peaks at diffraction angles 2Θ of 27.1 to 27.2° in the powder X-ray diffraction measurement.
[III-11] The crystal according to any one of [III-1] to [III-10], wherein the crystal exhibits a spectrum further having peaks at diffraction angles 2Θ of 35.5 to 35.6° in the powder X-ray diffraction measurement.
[III-12] The crystal according to any one of [III-1] to [III-11], wherein the crystal exhibits a spectrum further having peaks at diffraction angles 2Θ of 14.3 to 14.4°, 20.8 to 20.9°, 26.2 to 26.3°, 28.3 to 28.4°, 32.4 to 32.5°, 35.3 to 35.4°, 36.1 to 36.2°, 38.0 to 38.1° and 38.6 to 38.7° in the powder X-ray diffraction measurement.
[III-13] The crystal according to any one of [III-1] to [III-12], wherein the ratio of the peak height at 19.9 to 20.0° to the peak height at 17.7 to 17.8° is 1 : 0.02 to 1 : 0.95 in the powder X-ray diffraction measurement.
[III-14] The crystal according to [III-13], wherein the ratio of the peak height of 19.9 to 20.0° to the peak height of 17.7 to 17.8° is 1 : 0.1 to 1 : 0.85 in the powder X-ray diffraction measurement.
[III-15] The crystal according to [III-14], wherein the ratio of the peak height at 19.9 to 20.0° to the peak height at 17.7 to 17.8° is 1 : 0.3 to 1 : 0.75 in the powder X-ray diffraction measurement.
[III-16] The crystal according to any one of [III-13] to [III-15], wherein the ratio of the peak height at 19.9 to 20.0° to the peak height at 18.0 to 18.1° is 1 : 0.02 to 1 : 0.95 in the powder X-ray diffraction measurement.
[III-17] The crystal according to [III-16], wherein the ratio of the peak height at 19.9 to 20.0° to the peak height at 18.0 to 18.1° is 1 : 0.04 to 1 : 0.8 in the powder X-ray diffraction measurement.
[III-18] The crystal according to [III-17], wherein the ratio of the peak height at 19.9 to 20.0° to the peak height at 18.0 to 18.1° is 1 : 0.07 to 1 : 0.6 in the powder X-ray diffraction measurement.
[III-19] The crystal according to any one of [III-1] to [III-18], wherein the crystal is obtained by distilling off an organic solvent from a solution of pyroxasulfone in a medium composed of a liquid comprising the organic solvent as a main component to precipitate pyroxasulfone, the organic solvent being one selected from the group consisting of nitriles, carboxylic acids, carboxylic acid esters, ketones, amides and dihalogenated aliphatic hydrocarbons.
[III-20] The crystal according to [III-19], wherein the organic solvent is one selected from the group consisting of C2-C5 alkanenitriles, C1-C4 carboxylic acids, C1-C4 alkyl C1-C4 carboxylates, C1-C4 alkyl C1-C4 alkyl ketones, N,N-di(C1-C4 alkyl) C1-C4 alkanamides and C1-C4 dihaloalkanes.
[III-21] The crystal according to [III-20], wherein the organic solvent is one selected from the group consisting of acetonitrile, acetic acid, ethyl acetate, methyl isobutyl ketone, N,N-dimethylformamide, N,N-dimethylacetamide and dichloromethane.
[III-22] The crystal according to [III-19], wherein the medium is one selected from the group consisting of C2-C5 alkanenitrile/C1-C4 alcohol mixtures, hydrous C2-C5 alkanenitriles, C1-C4 carboxylic acids, C1-C4 alkyl C1-C4 carboxylates, C1-C4 alkyl C1-C4 alkyl ketones, N,N-di(C1-C4 alkyl) C1-C4 alkanamides and C1-C4 dihaloalkane/C1-C4 alcohol mixtures.
[III-23] The crystal according to [III-22], wherein the medium is one selected from the group consisting of an acetonitrile/methanol mixture, hydrous acetonitrile, acetic acid, ethyl acetate, methyl isobutyl ketone, N,N-dimethylformamide, N,N-dimethylacetamide and a dichloromethane/ethanol mixture.
[III-24] The crystal according to any one of [III-1] to [III-18], wherein the crystal is one obtained by adding an antisolvent for pyroxasulfone selected from the group consisting of ethers, carboxylic acid esters, ketones, aromatic hydrocarbon derivatives, aliphatic hydrocarbons, alcohols and water to the solution of pyroxasulfone in a medium composed of a liquid comprising one organic solvent selected from the group consisting of nitriles, ketones and carboxylic acid esters as a main component, and precipitating pyroxasulfone.
[III-25] The crystal according to [III-24], wherein the organic solvent is one selected from the group consisting of C2-C5 alkanenitriles, C1-C4 alkyl C1-C4 alkyl ketones and C1-C4 alkyl C1-C4 carboxylates.
[III-26] The crystal according to [III-25], wherein the organic solvent is one selected from the group consisting of acetonitrile, acetone and ethyl acetate.
[III-27] The crystal according to any one of [III-24] to [III-26], wherein the antisolvent for pyroxasulfone is C1-C4 alcohol.
[III-28] The crystal according to [III-27], wherein the antisolvent for pyroxasulfone is one selected from the group consisting of ethanol and isopropanol.
[III-29] The crystal according to any one of [III-19] to [III-28], wherein the solution of pyroxasulfone is a reaction solution prepared by producing pyroxasulfone by reacting 3-[(5-difluoromethoxy-1-methyl-3-trifluoromethylpyrazol-4-yl)methylthio]-4,5-dihydro-5,5-dimethylisoxazole with hydrogen peroxide in the presence of a metal catalyst in the liquid comprising the organic solvent described above as a main component.
[III-30] The crystal according to any one of [III-1] to [III-29], wherein the crystal has a short columnar or columnar appearance.
[III-31] An agrochemical composition comprising the crystal of pyroxasulfone according to any one of [III-1] to [III-30] and a surfactant.
[III-32] The agrochemical composition according to [III-31], further comprising water or an oil-based dispersion medium and having a dosage form of an aqueous suspension concentrate or an oil dispersion.
[III-33] The agrochemical composition according to [III-31], further comprising a solid carrier and having a dosage form of a wettable powder or a water-dispersible granule.
[III-34] The agrochemical composition comprising a crystal of pyroxasulfone and a surfactant, wherein the composition is one produced using the crystal of pyroxasulfone according to any one of [III-1] to [III-30].
[III-35] The agrochemical composition according to [III-34], further comprising water or an oil-based dispersion medium and having a dosage form of an aqueous suspension concentrate or an oil dispersion.
[III-36] The agrochemical composition according to [III-34], further comprising a solid carrier and having a dosage form of a wettable powder or a water-dispersible granule.
[III-37] A process for producing a crystal of pyroxasulfone, wherein an organic solvent is distilled off from a solution of pyroxasulfone in a medium composed of a liquid comprising the organic solvent as a main component to precipitate pyroxasulfone, the organic solvent being one selected from the group consisting of nitriles, carboxylic acids, carboxylic acid esters, ketones, amides and dihalogenated aliphatic hydrocarbons.
[III-38] The process for producing according to [III-37], wherein the organic solvent is one selected from the group consisting of C2-C5 alkanenitriles, C1-C4 carboxylic acids, C1-C4 alkyl C1-C4 carboxylates, C1-C4 alkyl C1-C4 alkyl ketones, N,N-di(C1-C4 alkyl) C1-C4 alkanamides and C1-C4 dihaloalkanes.
[III-39] The process for producing according to [III-38], wherein the organic solvent is acetonitrile, acetic acid, ethyl acetate, methyl isobutyl ketone, N,N-dimethylformamide, N,N-dimethylacetamide or dichloromethane.
[III-40] The process for producing according to [III-37], wherein the medium is one selected from the group consisting of C2-C5 alkanenitrile/C1-C4 alcohol mixtures, hydrous C2-C5 alkanenitriles, C1-C4 carboxylic acids, C1-C4 alkyl C1-C4 carboxylates, C1-C4 alkyl C1-C4 alkyl ketones, N,N-di(C1-C4 alkyl) C1-C4 alkanamides and C1-C4 dihaloalkane/C1-C4 alcohol mixtures.
[III-41] The process for producing according to [III-40], wherein the medium is one selected from the group consisting of an acetonitrile/methanol mixture, hydrous acetonitrile, acetic acid, ethyl acetate, methyl isobutyl ketone, N,N-dimethylformamide, N,N-dimethylacetamide and a dichloromethane/ethanol mixture.
[III-42] A process for producing a crystal of pyroxasulfone, wherein an antisolvent for pyroxasulfone selected from the group consisting of ethers, carboxylic acid esters, ketones, aromatic hydrocarbon derivatives, aliphatic hydrocarbons, alcohols and water is added to a solution of pyroxasulfone in a medium composed of a liquid comprising one organic solvent selected from the group consisting of nitriles, ketones and carboxylic acid esters as a main component to precipitate pyroxasulfone.
[III-43] The process for producing according to [III-42], wherein the organic solvent is one selected from the group consisting of C2-C5 alkanenitriles, C1-C4 alkyl C1-C4 alkyl ketones and C1-C4 alkyl C1-C4 carboxylates.
[III-44] The process for producing according to [III-43], wherein the organic solvent is one selected from the group consisting of acetonitrile, acetone and ethyl acetate.
[III-45] The process for producing according to any one of [III-42] to [III-44], wherein the antisolvent for pyroxasulfone is C1-C4 alcohol.
[III-46] The crystal according to [III-45], wherein the antisolvent for pyroxasulfone is one selected from the group consisting of ethanol and isopropanol.
[III-47] A process for producing a crystal of pyroxasulfone, wherein 3-[(5-difluoromethoxy-1-methyl-3-trifluoromethylpyrazol-4-yl)methylthio]-4,5-dihydro-5,5-dimethylisoxazole is reacted with hydrogen peroxide in the presence of a metal catalyst in a liquid comprising one organic solvent selected from the group consisting of nitriles, carboxylic acids, carboxylic acid esters, ketones, amides and dihalogenated aliphatic hydrocarbons as a main component to produce pyroxasulfone, and the organic solvent is distilled off from the obtained reaction solution of pyroxasulfone to precipitate pyroxasulfone.
[III-48] The process for producing according to [III-47], further comprising the step of producing pyroxasulfone by the step (iii) described in [I-1].
[III-49] The process for producing according to [III-47] or [III-48], wherein the organic solvent is one selected from the group consisting of C2-C5 alkanenitriles, C1-C4 carboxylic acids, C1-C4 alkyl C1-C4 carboxylates, C1-C4 alkyl C1-C4 alkyl ketones, N,N-di(C1-C4 alkyl)C1-C4 alkanamides and C1-C4 dihaloalkanes.
[III-50] The process for producing according to [III-49], wherein the organic solvent is one selected from the group consisting of acetonitrile, acetic acid, ethyl acetate, methyl isobutyl ketone, N,N-dimethylformamide, N,N-dimethylacetamide and dichloromethane.
[III-51] The process for producing according to [III-47] or [III-48], wherein the organic solvent is one selected from the group consisting of C2-C5 alkanenitriles, C1-C4 alkyl C1-C4 carboxylates and N,N-di(C1-C4 alkyl)C1-C4 alkanamides.
[III-52] The process for producing according to [III-51], wherein the organic solvent is one selected from the group consisting of acetonitrile, ethyl acetate and N,N-dimethylformamide.
[III-53] The process for producing according to [III-47] or [III-48], wherein the liquid is one selected from the group consisting of C2-C5 alkanenitrile/C1-C4 alcohol mixtures, hydrous C2-C5 alkanenitriles, C1-C4 carboxylic acids, C1-C4 alkyl C1-C4 carboxylates, C1-C4 alkyl C1-C4 alkyl ketones, N,N-di(C1-C4 alkyl) C1-C4 alkanamides and C1-C4 dihaloalkane/C1-C4 alcohol mixtures.
[III-54] The process for producing according to [III-53], wherein the liquid is one selected from the group consisting of acetonitrile/methanol mixtures, hydrous acetonitrile, acetic acid, ethyl acetate, methyl isobutyl ketone, N,N-dimethylformamide, N,N-dimethylacetamide and dichloromethane/ethanol mixtures.
[III-55] The process for producing according to [III-47] or [III-48], wherein the liquid is one selected from the group consisting of C2-C5 alkanenitrile/C1-C4 alcohol mixtures, hydrous C2-C5 alkanenitriles, C1-C4 alkyl C1-C4 carboxylates and N,N-di(C1-C4 alkyl)C1-C4 alkanamides.
[III-56] The process for producing according to [III-55], wherein the liquid is one selected from the group consisting of acetonitrile/methanol mixtures, hydrous acetonitrile, ethyl acetate and N,N-dimethylformamide.
[III-57] A process for producing a crystal of pyroxasulfone, wherein 3-[(5-difluoromethoxy-1-methyl-3-trifluoromethylpyrazol-4-yl)methylthio]-4,5-dihydro-5,5-dimethylisoxazole is reacted with hydrogen peroxide in the presence of a metal catalyst in a liquid comprising one organic solvent selected from the group consisting of nitriles, ketones and carboxylic acid esters as a main component to produce pyroxasulfone, and then an antisolvent for pyroxasulfone selected from the group consisting of ethers, carboxylic acid esters, ketones, aromatic hydrocarbon derivatives, aliphatic hydrocarbons, alcohols and water is added to the obtained solution of pyroxasulfone to precipitate pyroxasulfone.
[III-58] The process for producing according to [III-57], further comprising the step of producing pyroxasulfone by the step (iii) defined in [I-1].
[III-59] The process for producing according to [III-57] or [III-58], wherein the organic solvent is one selected from the group consisting of C2-C5 alkanenitriles, C1-C4 alkyl C1-C4 alkyl ketones and C1-C4 alkyl C1-C4 carboxylates.
[III-60] The process for producing according to [III-59], wherein the organic solvent is one selected from the group consisting of acetonitrile, acetone and ethyl acetate.
[III-61] The process for producing according to [III-57] or [III-58], wherein the organic solvent is one selected from the group consisting of C2-C5 alkanenitriles and C1-C4 alkyl C1-C4 carboxylates.
[III-62] The process for producing according to [III-61], wherein the organic solvent is one selected from the group consisting of acetonitrile and ethyl acetate.
[III-63] The process for producing according to any one of [III-57] to [III-62], wherein the antisolvent for pyroxasulfone is C1-C4 alcohol.
[III-64] The process for producing according to [III-63], wherein the antisolvent for pyroxasulfone is one selected from the group consisting of ethanol and isopropanol.
[III-65] A process for producing an agrochemical composition having a dosage form of a wettable powder, the process comprising a step of pulverizing a powder comprising the crystal of pyroxasulfone according to any one of [III-1] to [III-30], and a step of mixing the whole raw material comprising the pulverized crystal of pyroxasulfone, a surfactant and a solid carrier to homogenize the mixture.
[III-66] A process for producing an agrochemical composition having a dosage form of a water-dispersible granule, the process comprising a step of pulverizing a powder or a slurry comprising the crystal of pyroxasulfone according to any one of [III-1] to [III-30], a step of, while homogenizing the whole raw material comprising the pulverized crystal of pyroxasulfone, a surfactant and a solid carrier, further adding a slight amount of water and kneading the mixture, a step of granulating the kneaded product obtained in the preceding step, and a step of drying the granulated product obtained in the preceding step.
[III-67] A process for producing an agrochemical composition having a dosage form of an aqueous suspension concentrate, the process comprising a step of pulverizing a powder or slurry comprising the crystal of pyroxasulfone according to any one of [III-1] to [III-30], and a step of mixing the whole raw material comprising the pulverized crystal of pyroxasulfone, a surfactant and water to homogenize the mixture.
[III-68] A process for producing an agrochemical composition having a dosage form of an oil dispersion, the process comprising a step of pulverizing a powder or slurry comprising the crystal of pyroxasulfone according to any one of [III-1] to [III-30], and a step of mixing the whole raw material comprising the pulverized crystal of pyroxasulfone, a surfactant and an oil-based dispersion medium to homogenize the mixture.
[III-69] A process for producing an agrochemical composition having a dosage form of a wettable powder, the process comprising a step of pulverizing a powder comprising a crystal of pyroxasulfone prepared by the process for producing according to any one of [III-37] to [III-64], and a step of mixing the whole raw material comprising the pulverized crystal of pyroxasulfone, a surfactant and a solid carrier to homogenize the mixture.
[III-70] A process for producing an agrochemical composition having a dosage form of a water-dispersible granule, the process comprising a step of pulverizing a powder or a slurry comprising a crystal of pyroxasulfone prepared by the process for producing according to any one of [III-37] to [III-64], a step of, while homogenizing the whole raw material comprising the pulverized crystal of pyroxasulfone, a surfactant and a solid carrier, further adding a slight amount of water and kneading the mixture, a step of granulating the kneaded product obtained in the preceding step, and a step of drying the granulated product obtained in the preceding step.
[III-71] A process for producing an agrochemical composition having a dosage form of an aqueous suspension concentrate, the process comprising a step of pulverizing a powder or a slurry comprising a crystal of pyroxasulfone prepared by the process for producing according to any one of [III-37] to [III-64] and a step of mixing the whole raw material comprising the pulverized crystal of pyroxasulfone, a surfactant and water to homogenize the mixture.
[III-72] A process for producing an agrochemical composition having a dosage form of an oil dispersion, the process comprising a step of pulverizing a powder or slurry comprising a crystal of pyroxasulfone prepared by the process for producing according to any one of [III-37] to [III-64], and a step of mixing the whole raw material comprising the pulverized crystal of pyroxasulfone, a surfactant and an oil-based dispersion medium to homogenize the mixture.

The symbols and terms described in the present description will be explained.

Herein, the following abbreviations and prefixes may be used, and their meanings are as follows:
Me: methyl
Et: ethyl
Pr, n-Pr and Pr-n: propyl (i.e., normal propyl)
i-Pr and Pr-i: isopropyl
Bu, n-Bu and Bu-n: butyl (i.e., normal butyl)
s-Bu and Bu-s: sec-butyl (i.e., secondary butyl)
i-Bu and Bu-i: isobutyl
t-Bu and Bu-t: tert-butyl (i.e., tertiary butyl)
Ph: phenyl
n-: normal
s- and sec-: secondary
i- and iso-: iso
t- and tert-: tertiary
c- and cyc-: cyclo
o-: ortho
m-: meta
p-: para

The term "nitro" means the substituent "-NO₂".

The term "cyano" or "nitrile" means the substituent "-CN".

The term "hydroxy" means the substituent "-OH".

The term "amino" means the substituent "-NH₂".

(Ca-Cb) means that the number of carbon atoms is a to b. For example, "(C1-C4)" in "(C1-C4)alkyl" means that the number of the carbon atoms in the alkyl is 1 to 4, and "(C2-C5)" means that the number of the carbon atoms in the alkyl is 2 to 5. "(Ca-Cb)" meaning the number of carbon atoms may be written as "Ca-Cb" without parentheses. Thus, for example, "C1-C4" in "C1-C4 alkyl" means that the number of the carbon atoms in the alkyl is 1 to 4.

Herein, it is to be interpreted that generic terms such as "alkyl" include both the straight chain and the branched chain such as butyl and tert-butyl. Meanwhile, for example, the specific term "butyl" refers to straight "normal butyl", and does not refer to branched "tert-butyl". Branched chain isomers such as "tert-butyl" are referred to specifically when intended.

Examples of the halogen atom include fluorine atom, chlorine atom, bromine atom and iodine.

The (C1-C6)alkyl means a straight or branched alkyl having 1 to 6 carbon atoms. Examples of the (C1-C6)alkyl include, but are not limited to, methyl, ethyl, propyl, isopropyl, butyl, sec-butyl, isobutyl, tert-butyl, pentyl and hexyl.

The (C1-C4)alkyl means a straight or branched alkyl having 1 to 4 carbon atoms. Examples of the (C1-C4)alkyl include, appropriate examples of the examples of the (C1-C6)alkyl above-mentioned.

The (C3-C6)cycloalkyl means a cycloalkyl having 3 to 6 carbon atoms. Examples of the (C3-C6)cycloalkyl are cyclopropyl, cyclobutyl, cyclopentyl and cyclohexyl.

The (C2-C6)alkenyl means a straight or branched alkenyl having 2 to 6 carbon atoms. Examples of the (C2-C6)alkenyl include, but are not limited to, vinyl, 1-propenyl, isopropenyl, 2-propenyl, 1-butenyl, 1-methyl-1-propenyl, 2-methyl-1-propenyl, 2-butenyl, 3-butenyl, 1,3-butadienyl, 1-pentenyl and 1-hexenyl.

The (C2-C6)alkynyl means a straight or branched alkynyl having 2 to 6 carbon atoms. Examples of the (C2-C6)alkynyl include, but are not limited to, ethynyl, 1-propynyl, 2-propynyl, 1-butynyl, 1-methyl-2-propynyl, 2-butynyl, 3-butynyl, 1-pentynyl and 1-hexynyl.

Examples of the (C6-C10)aryl are phenyl, 1-naphthyl and 2-naphthyl.

The (C1-C6)haloalkyl means a straight or branched alkyl having 1 to 6 carbon atoms which is substituted with 1 to 13 same or different halogen atoms, wherein the halogen atoms have the same meaning as defined above. Examples of the (C1-C6)haloalkyl include, but are not limited to, fluoromethyl, chloromethyl, bromomethyl, difluoromethyl, dichloromethyl, trifluoromethyl, trichloromethyl, chlorodifluoromethyl, bromodifluoromethyl, 2-fluoroethyl, 1-chloroethyl, 2-chloroethyl, 2,2,2-trifluoroethyl, pentafluoroethyl, 3-fluoropropyl, 3-chloropropyl, 2-chloro-1-methylethyl, 2,2,3,3,3-pentafluoropropyl, 2,2,2-trifluoro-1-trifluoromethylethyl, heptafluoropropyl, 1,2,2,2-tetrafluoro-1-trifluoromethylethyl, 4-fluorobutyl, 4-chlorobutyl, 2,2,3,3,4,4,4-heptafluorobutyl, nonafluorobutyl, 1,1,2,3,3,3-hexafluoro-2-trifluoromethylpropyl, 2,2,2-trifluoro-1,1-di(trifluoromethyl)ethyl, undecafluoropentyl and tridecafluorohexyl.

The (C1-C4)perfluoroalkyl means a straight or branched alkyl having 1 to 4 carbon atoms, wherein all hydrogen atoms are substituted with fluorine atoms. Examples of the (C1-C4)perfluoroalkyl are trifluoromethyl (i.e., -CF₃), pentafluoroethyl (i.e., -CF₂CF₃), heptafluoropropyl (i.e., -CF₂CF₂CF₃), 1,2,2,2-tetrafluoro-1-trifluoromethylethyl (i.e., -CF(CF₃)₂), nonafluorobutyl, (i.e., -CF₂CF₂CF₂CF₃), 1,2,2,3,3,3-hexafluoro-1-trifluoromethylpropyl (i.e., - CF (CF₃) CF₂CF₃), 1,1,2,3,3, 3-hexafluoro-2-trifluoromethylpropyl (i.e., -CF₂CF(CF₃)₂) and 2,2,2-trifluoro-1,1-di(trifluoromethyl) ethyl (i.e., -C(CF₃)₃).

The (C1-C6)alkoxy means a (C1-C6)alkyl-O-, wherein the (C1-C6)alkyl moiety has the same meaning as defined above. Examples of the (C1-C6)alkoxy include, but are not limited to, methoxy, ethoxy, propoxy, isopropoxy, butoxy, sec-butoxy, isobutoxy, tert-butoxy, pentyloxy, isopentyloxy, neopentyloxy and hexyloxy.

The (C1-C6)alcohol means a (C1-C6)alkyl-OH, wherein the (C1-C6)alkyl moiety has the same meaning as defined above. Examples of the (C1-C4)alcohol include, but are not limited to, methanol, ethanol, propanol (i.e., 1-propanol), 2-propanol, butanol (i.e., 1-butanol), sec-butanol, isobutanol, tert-butanol, pentanol (i.e., 1-pentanol), sec-amyl alcohol, 3-pentanol, 2-methyl-1-butanol, isoamyl alcohol, tert-amyl alcohol, hexanol (i.e., 1-hexanol) and cyclohexanol. Polyols having 1 to 6 carbons (e.g., diols and triols) such as ethylene glycol, propylene glycol and glycerol are equivalents of (C1-C6)alcohols.

The (C1-C4)alcohol means a (C1-C4)alkyl-OH, wherein the (C1-C4)alkyl moiety has the same meaning as defined above. Examples of the (C1-C4)alcohol include, but are not limited to, methanol, ethanol, propanol (i.e., 1-propanol), 2-propanol, butanol, sec-butanol, isobutanol and tert-butanol. Polyols having 1 to 4 carbons (e.g., diols and triols) such as ethylene glycol, propylene glycol and glycerol are equivalents of (C1-C4)alcohols.

The (C2-C5)alkanenitrile means (C1-C4)alkyl-CN, wherein the (C1-C4)alkyl moiety means a linear or branched alkyl having 1 to 5 carbon atoms; examples of the (C1-C5)alkyl include appropriate examples among the examples of the (C1-C6)alkyl described above. Examples of the (C2-C5)alkanenitrile include, but are not limited to, acetonitrile and propionitrile. Herein, the (C2-C5)alkanenitrile is also referred to as C2-C5 alkanenitrile. C2 alkanenitrile is acetonitrile. In other words, acetonitrile is ethanenitrile in accordance with the IUPAC nomenclature and is a C2 alkanenitrile having two carbon atoms. Similarly, propionitrile is a C3 alkanenitrile.

Examples of the (C1-C4)alkyl (C1-C4)carboxylates include, but are not limited to, ethyl acetate, propyl acetate, isopropyl acetate, butyl acetate and isomers thereof, ethyl propionate, propyl propionate, isopropyl propionate, butyl propionate and isomers thereof, and preferably ethyl acetate, propyl acetate, isopropyl acetate, butyl acetate and isomers thereof. Herein, (C1-C4)alkyl (C1-C4)carboxylate is also referred to as C1-C4 alkyl C1-C4 carboxylate.

Examples of the N,N-di((C1-C4)alkyl) (C1-C4)alkanamides include, but are not limited to, N,N-dimethylformamide, N,N-dimethylacetamide, N,N-diethylformamide and N,N-diethylacetamide, and preferably N,N-dimethylformamide and N,N-dimethylacetamide. Herein, N,N-di((C1-C4)alkyl) (C1-C4)alkanamide is also referred to as N,N-di(C1-C4 alkyl)C1-C4 alkanamide. N,N-di(C1 alkyl)C1 alkanamide is N,N-dimethylformamide. N,N-di(C1 alkyl)C2 alkanamide is N,N-dimethylacetamide.

The (C1-C4)carboxylic acid means a (C1-C4)alkyl-COOH, i.e., (C1-C4)alkyl-C(=O)-OH, wherein the (C1-C4)alkyl moiety has the same meaning as defined above. Examples of the (C1-C4)carboxylic acids include, but are not limited to, acetic acid and propionic acid, and preferably acetic acid. Herein, (C1-C4)carboxylic acid is also referred to as C1-C4 carboxylic acid.

Examples of the (C1-C4)alkyl (C1-C4)alkyl ketones include, but are not limited to, acetone, methyl ethyl ketone (MEK), methyl isopropyl ketone (MIPK) and methyl isobutyl ketone (MIBK). Herein, (C1-C4) alkyl (C1-C4) alkyl ketone is also referred to as C1-C4 alkyl C1-C4 alkyl ketone.

Examples of the (C1-C4)dihaloalkanes include, but are not limited to, dichloromethane and 1,2-dichloroethane. Herein, (C1-C4)dihaloalkane is also referred to as C1-C4 dihaloalkane.

The cyclic hydrocarbon group means a cyclic group which is monocyclic or multicyclic, wherein all of the ring-constituting atoms are carbon atoms. In one embodiment, examples of the cyclic hydrocarbon group include, but are not limited to, a 3- to 14-membered (preferably 5- to 14-membered, more preferably 5- to 10-membered) cyclic hydrocarbon group which is aromatic or non-aromatic and is monocyclic, bicyclic or tricyclic. In another embodiment, examples of the cyclic hydrocarbon group include, but are not limited to, a 4- to 8-membered (preferably 5- to 6-membered) cyclic hydrocarbon group which is aromatic or non-aromatic and is monocyclic or bicyclic (preferably monocyclic). Examples of the cyclic hydrocarbon group include, but are not limited to, cycloalkyls and aryls. Examples of the cycloalkyl include the examples of the (C3-C6)cycloalkyl described above. The aryls are aromatic cyclic groups among the cyclic hydrocarbon groups as defined above. Examples of the aryl include the examples of the (C6-C10)aryl described above. The cyclic hydrocarbon group as defined or exemplified above may include a non-condensed cyclic group (e.g., a monocyclic group or a spirocyclic group) and a condensed cyclic group, when possible. The cyclic hydrocarbon group as defined or exemplified above may be unsaturated, partially saturated or saturated, when possible. The cyclic hydrocarbon group as defined or exemplified above is also referred to as a carbocyclic ring group. The carbocyclic ring is a ring which corresponds to the cyclic hydrocarbon group as defined or exemplified above. Examples of the carbocyclic ring include, but are not limited to, cyclopropane, cyclobutane, cyclopentane, cyclohexane, cyclopentene and cyclohexene.

Herein, there are no particular limitations on the "substituent(s)" for the phrase "optionally substituted with one or more substituents" as long as they are chemically acceptable and exhibit the effects of the present invention.

Herein, examples of the "substituent(s)" for the phrase "optionally substituted with one or more substituent(s)" include, but are not limited to, one or more substituents (preferably 1 to 3 substituents) selected independently from Substituent Group (a).

Substituent Group (a) is a group consisting of a halogen atom; a nitro group, a cyano group, a hydroxy group, an amino group, (C1-C6)alkyl, (C1-C6)haloalkyl, (C3-C6)cycloalkyl, (C2-C6)alkenyl, (C2-C6)alkynyl, (C1-C6)alkoxy, phenyl and phenoxy.

In addition, one or more substituents (preferably 1 to 3 substituents) selected independently from Substituent Group (a) may each independently be substituted with one or more substituents (preferably 1 to 3 substituents) selected independently from Substituent Group (b). In this context, Substituent Group (b) is the same as Substituent Group (a).

Examples of the "(C1-C6)alkyl optionally substituted with one or more substituents" include, but are not limited to, (C1-C6)haloalkyl, (C1-C4)perfluoroalkyl and (C1-C4)alkyl optionally substituted with 1 to 9 fluorine atoms.

Examples of the (C1-C4)alkyl optionally substituted with 1 to 9 fluorine atoms include, but are not limited to, fluoromethyl (i.e., -CH₂F), difluoromethyl (i.e., -CHF₂), trifluoromethyl (i.e., -CF₃), 2-fluoroethyl, 2,2,2-trifluoroethyl, pentafluoroethyl, 3-fluoropropyl, 2,2,3,3,3-pentafluoropropyl, 2,2,2-trifluoro-1-trifluoromethylethyl, heptafluoropropyl, 1,2,2,2-tetrafluoro-1-trifluoromethylethyl, 4-fluorobutyl, 2,2,3,3,4,4,4-heptafluorobutyl, nonafluorobutyl, 1,1,2,3,3,3-hexafluoro-2 trifluoromethylpropyl and 2,2,2-trifluoro-1,1-di(trifluoromethyl)ethyl.

Herein, the phrase "as described herein" and similar phrases used when referring to substituents (for example, R¹, R², R³, R⁴, R⁵, X¹ and X²) incorporate by reference all definitions of the substituents and, if any, all of their examples, preferred examples, more preferred examples, further preferred examples and particularly preferred examples in this specification.

### (Acceptor Number)

Herein, regarding the acceptor number, the following document can be referred to, for example,

Christian Reichardt, "Solvents and Solvent Effects in Organic Chemistry", 3rd, updated and enlarged edition, WILEY-VCH, 2003, p. 25-26

The definition of the acceptor number utilizing ³¹P-NMR chemical shift values are described in the above document, which is incorporated into the present invention by reference.

Examples of the solvent having a specified acceptor number are described in the above document, which are incorporated into the present invention by reference, but are not limited thereto. The acceptor numbers of typical solvents are as follows:
hexane: 0.0, ethyl acetate: 9.3, acetonitrile: 18.9, butanol: 32.2, ethanol, 37.1, acetic acid: 52.9, and water: 54.8.

As used herein, the non-limiting term "comprise(s)/comprising" can each optionally be replaced by the limiting phrase "consist(s) of/consisting of".

Unless otherwise stated, all technical and scientific terms used herein have the same meaning as commonly understood by a person skilled in the art to which the present disclosure belongs.

Unless otherwise indicated, it is understood that numbers used herein to express characteristics such as quantities, sizes, concentrations, and reaction conditions are modified by the term "about". In some embodiments, disclosed numerical values are interpreted applying the reported number of significant digits and conventional rounding techniques. In some embodiments, disclosed numerical values are interpreted as containing certain errors necessarily resulting from the standard deviation found in their respective testing measurements.

### (Step i)

The step i will be described.

The step i is one of the processes for producing the compound of the formula (2) when X¹ in the formula (2) is a halogen atom.

The step i is a step of reacting a compound of the formula (1) with a halogenating agent to produce the compound of the formula (2), with the proviso that X¹ in the formula (2) is a halogen atom: 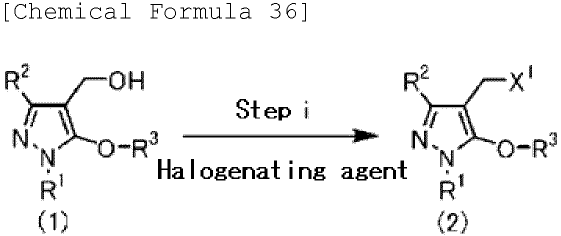 wherein R¹, R² and R³ are as described herein and X¹ is a halogen atom.

The reaction in the step i is a halogenation reaction of a hydroxy group.

### (Raw Material in Step i; Compound of Formula (1))

A compound of the formula (1) is used as a raw material in the step i.

The compound of the formula (1) may be a known compound or may be produced from a known compound according to a known process. For example, the preparation of the compound of the formula (1) can be performed by the process described in WO 2007/094225 A1 (Patent Document 5), Example 1 as shown below, or by a process similar thereto.

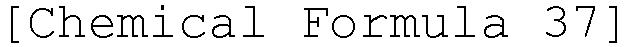

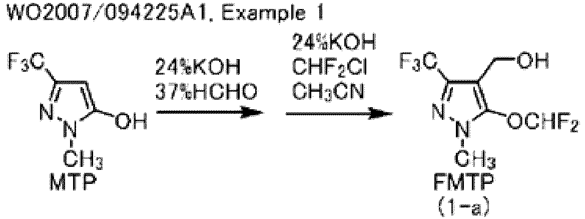

WO 2007/094225 A1 (Patent Document 5) is summarized below. For example, WO 2007/094225 A1 (Patent Document 5) discloses that the compound of the formula (1-a) has been produced from an acetoacetic acid ester derivative as shown in the following scheme.

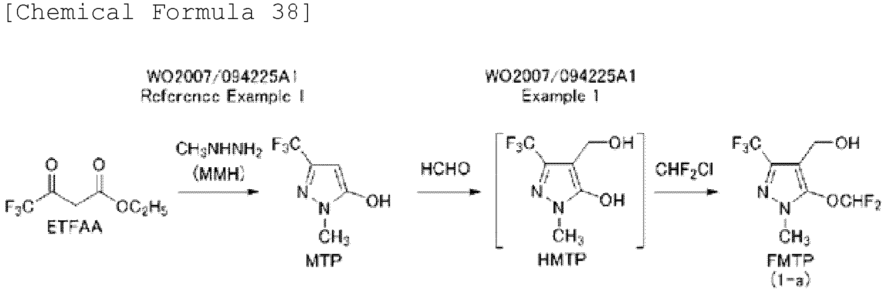

In the formula (1), R¹, R² and R³ are each independently a (C1-C6)alkyl optionally substituted with one or more substituents, a (C3-C6)cycloalkyl optionally substituted with one or more substituents, a (C2-C6)alkenyl optionally substituted with one or more substituents, a (C2-C6)alkynyl optionally substituted with one or more substituents, or a (C6-C10)aryl optionally substituted with one or more substituents.

From the viewpoints of yield, availability, price, usefulness of the product, etc., preferred examples of R¹ in the formula (1) include (C1-C6)alkyls optionally substituted with one or more substituents, more preferably (C1-C6)alkyls, further preferably (C1-C4)alkyls, and particularly preferably methyl.

From the same viewpoints as described above, preferred examples of R² in the formula (1) include (C1-C6)alkyls optionally substituted with one or more substituents, more preferably (C1-C6)haloalkyls, further preferably (C1-C4)perfluoroalkyls, and particularly preferably trifluoromethyl.

From the same viewpoints as described above, preferred examples of R³ in the formula (1) include (C1-C6)alkyls optionally substituted with one or more substituents, more preferably (C1-C6)haloalkyls,
further preferably (C1-C4)alkyls optionally substituted with 1 to 9 fluorine atoms, and particularly preferably difluoromethyl.

A particularly preferred example of the compound of the formula (1) is as follows:

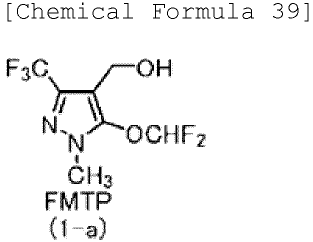

### (Product in Step i; Compound of Formula (2))

The product in the step i is a compound of the formula (2) corresponding to the compound of the formula (1) used as a raw material.

In the formula (2), R¹, R² and R³ are as defined in the formula (1). In the formula (2), examples, preferred examples, more preferred examples, and particularly preferred examples of R¹, R² and R³ are the same as those in the formula (1) described above, respectively.

In the step i, in the formula (2), X¹ is a halogen atom. From the viewpoint of yield, availability, price, etc., more preferred examples of X¹ in the formula (2) include chlorine atom and bromine atom, and particularly preferably chlorine atom.

Specific examples of the compound of the formula (2) include, but are not limited to, the following:

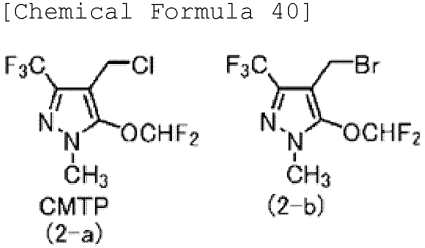

Particularly preferred specific examples of the compound of the formula (2) are as follows:

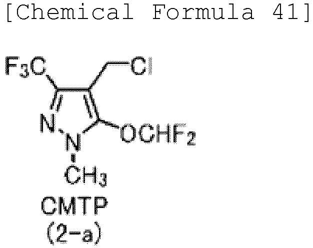

### (Halogenating Agent in Step i)

The halogenating agent may be any halogenating agent as long as the reaction proceeds. Examples of the halogenating agent include, but are not limited to, chlorinating agents and brominating agents, and preferably chlorinating agents.

Specific examples of the halogenating agent include, but are not limited to, chlorine (Cl₂), hydrogen chloride (e.g., hydrogen chloride gas, 30% to 35% hydrochloric acid), thionyl chloride, sulfuryl chloride, phosphorus trichloride, phosphorus pentachloride, phosphorus oxychloride, oxalyl chloride, bromine (Br₂), hydrogen bromide (e.g., 48% hydrobromic acid), thionyl bromide, phosphorus tribromide, phosphorus oxybromide, etc.), phosgene and benzoyl chloride. From the viewpoints of yield, suppression of by-products, economic efficiency, etc., preferred specific examples of the halogenating agent include chlorine, hydrogen chloride, thionyl chloride, sulfuryl chloride, phosphorus trichloride, phosphorus pentachloride, phosphorus oxychloride, bromine, hydrogen bromide and phosphorus tribromide, more preferably chlorine, thionyl chloride, sulfuryl chloride, phosphorus trichloride, phosphorus pentachloride and phosphorus oxychloride, further preferably thionyl chloride and sulfuryl chloride, and further preferably thionyl chloride.

The halogenating agent in the step i may be used singly or in a combination of two or more kinds thereof in any ratio. The form of the halogenating agent in the step i may be any form as long as the reaction proceeds.
The form of the halogenating agent in the step i can be appropriately selected by a person skilled in the art. The amount of the halogenating agent used in the step i may be any amount as long as the reaction proceeds. The amount of the halogenating agent used in the step i may be appropriately adjusted by a person skilled in the art. However, from the viewpoint of yield, suppression of by-products, economic efficiency, etc., the amount of the halogenating agent used in the step i is, for example, 1.0 to 3.0 mol, preferably 1.0 to 2.0 mol, more preferably 1.0 to 1.5 mol, and still more preferably 1.0 to 1.1 mol, based on 1 mol of the compound of the formula (1) (raw material).

### (Catalyst in Step i: Amide, etc.)

The reaction in the step i may be performed in the presence or absence of a catalyst. Whether or not to use a catalyst in the reaction in the step i can be appropriately determined by a person skilled in the art. However, it is apparent from the Examples disclosed herein that the reaction proceeds sufficiently in the absence of a catalyst. When a catalyst is used in the step i, any catalyst may be used as long as the reaction proceeds. Examples of the catalyst in the step i include, but are not limited to, amides (e.g., N,N-dimethylformamide (DMF), N,N-dimethylacetamide (DMAC) and N-methylpyrrolidone (NMP)) and ureas (e.g., N,N'-dimethylimidazolidinone (DMI) and tetramethylurea). From the viewpoint of yield, suppression of by-products, economic efficiency, etc., preferred examples of the catalyst in the step i include N,N-dimethylformamide (DMF) and N-methylpyrrolidone (NMP), and more preferably N,N-dimethylformamide (DMF).

The catalyst in the step i may be used singly or in a combination of two or more kinds thereof in any ratio. The catalyst in the step i may be in any form as long as the reaction proceeds. The form of the catalyst in the step i can be appropriately selected by a person skilled in the art. The amount of the catalyst used in the step i may be any amount as long as the reaction proceeds. In the step i, no catalyst may be used. When a catalyst is used in the step i, the amount of the catalyst used in the step i may be appropriately adjusted by a person skilled in the art. However, from the viewpoint of yield, suppression of by-products, economic efficiency, etc., in one embodiment, the amount of the catalyst used in the step i is, for example, 0 (zero) to 0.1 mol, and preferably 0 (zero) to 0.05 mol, based on 1 mol of the compound of the formula (1) (raw material). In another embodiment, the amount of the catalyst used in the step i is, for example, 0.001 to 0.1 mol, and preferably 0.01 to 0.05 mol, based on 1 mol of the compound of the formula (1) (raw material).

### (Reaction Solvent in Step i)

The reaction in the step i may be performed in the absence or presence of a solvent. Whether or not to use a solvent in the reaction in the step i can be appropriately determined by a person skilled in the art. When a solvent is used in the reaction in the step i, the solvent may be any solvent as long as the reaction proceeds. The solvent in the reaction in the step i can be selected appropriately by a person skilled in the art. Examples of the solvent in the reaction in the step i include, but are not limited to, the following: aromatic hydrocarbon derivatives (e.g., benzene, toluene, xylenes, chlorobenzene, dichlorobenzenes, trichlorobenzenes and nitrobenzene), halogenated aliphatic hydrocarbons (e.g., dichloromethane and 1,2-dichloroethane (EDC)), aliphatic hydrocarbons (e.g., hexane, cyclohexane and ethylcyclohexane), nitriles (e.g., acetonitrile and propionitrile), ethers (e.g., tetrahydrofuran (THF), 1,4-dioxane, diisopropyl ether, dibutyl ether, di-tert-butyl ether, cyclopentyl methyl ether (CPME), methyl-tert-butyl ether, 1,2-dimethoxyethane (DME) and diglyme), and any combination thereof in any ratio. Among the aromatic hydrocarbon derivatives, halogenated aromatic hydrocarbons (e.g., chlorobenzene, dichlorobenzene and trichlorobenzene) are preferable.

From the viewpoint of yield, suppression of by-products, economic efficiency, etc., preferably, the reaction in the step i is performed in the absence of a solvent or in the presence of a solvent selected from the following: aromatic hydrocarbon derivatives, halogenated aliphatic hydrocarbons, nitriles, and any combination thereof in any ratio. More preferably, the reaction in the step i is performed in the absence of a solvent or in the presence of a solvent selected from the following: chlorobenzene, dichlorobenzenes, trichlorobenzenes, dichloromethane, 1,2-dichloroethane, acetonitrile, and any combination thereof in any ratio. More preferably, the reaction in the step i is performed in the absence of a solvent or in the presence of a solvent selected from the following: dichloromethane and acetonitrile. More preferably, the reaction in the step i is performed in the absence of a solvent or in the presence of acetonitrile as a solvent. Particularly preferably, the reaction in the step i is performed in the presence of acetonitrile as a solvent.

The amount of the solvent used in the reaction in the step i is not particularly limited as long as the reaction system can be sufficiently stirred. However, from the viewpoint of yield, suppression of by-products, economic efficiency, etc., in one embodiment, the amount of the solvent used in the reaction in the step i is, for example, 0 (zero) to 3 L (liters), preferably 0 (zero) to 2 L, more preferably 0 (zero) to 1.5 L, further preferably 0 (zero) to 1 L, and further preferably 0 (zero) to 0.8 L, based on 1 mol of the compound of the formula (1) (raw material). In another embodiment, the amount of the solvent used in the reaction is, for example, 0.3 to 3 L (liters), preferably 0.4 to 2 L, more preferably 0.5 to 1.5 L, and further preferably 0.6 to 1.2 L, based on 1 mol of the compound of the formula (1) (raw material). When a combination of two or more solvents is used, the ratio of the two or more solvents may be any ratio as long as the reaction proceeds.

### (Reaction Temperature in Step i)

The reaction temperature in the step i is not particularly limited. However, from the viewpoint of yield, suppression of by-products, economic efficiency, etc., the reaction temperature in the step i is, for example, -10°C (minus 10°C) to 100°C, preferably -5°C (minus 5°C) to 80°C, more preferably 0°C (zero °C) to 50°C, further preferably 0°C (zero °C) to 40°C, and further preferably 0°C (zero °C) to 30°C.

### (Reaction Time in Step i)

The reaction time in the step i is not particularly limited. However, from the viewpoint of yield, suppression of by-products, economic efficiency, etc., the reaction time in the step i is, for example, 0.5 hours to 48 hours, preferably 1 hour to 24 hours, more preferably 1 hour to 12 hours, and further preferably 2 hours to 12 hours.
However, the reaction time can be adjusted appropriately by a person skilled in the art.

### (Working-up in Step i; Isolation and/or Purification)

The compound of the formula (2), especially the compound (2-a), which is the product in the step i, can be used as a raw material in the step ii. The compound of the formula (2) obtained in the step i may be used in the next step after isolation and/or purification, or may be used in the next step without isolation. Whether or not to perform the working-up (isolation and/or purification) can be appropriately determined by a person skilled in the art according to the purpose and situation.

In the step i, after the completion of the reaction, the gas, the volatile components and the low-boiling components may be removed by purging with an inert gas (e.g., nitrogen), bubbling and/or reducing pressure. For example, acidic components may be removed.

The compound of the formula (2), especially the compounds (2-a), which is the target product in the step i, may be isolated and purified from the reaction mixture, if possible, by a method known to a person skilled in the art (e.g., if possible, distillation, extraction, washing, crystallization including recrystallization, crystal washing and/or other procedures) and an improved method thereof, and any combination thereof.

If possible, the product dissolved or suspended in an organic solvent may be washed with water, hot water, an aqueous alkaline solution (e.g., a 5% or more saturated aqueous sodium hydrogen carbonate solution), or an acidic aqueous solution (e.g., 5 to 35% hydrochloric acid or 5 to 35% sulfuric acid). Such washing procedures may be combined.

When performing crystallization of the product including recrystallization and washing of crystals, the description in the step iii described later may be referred to.

In any of the above procedures, the temperature can be appropriately adjusted by a person skilled in the art according to the purpose and situation.

In any procedure of the working-up and the procedure of using the product in the next step, the amount of a solvent can be appropriately adjusted by a person skilled in the art by addition and removal thereof. Furthermore, recovery and recycle of the solvent may be optionally performed. For example, the recovery and recycle of the solvent used in the reaction may be performed, and the recovery and recycle of the solvent used in the working-up (isolation and/or purification) may be performed.

Working-up (isolation and/or purification) can be performed by appropriately combining all or some of the procedures described above. Optionally, the above procedure may be repeated according to the purpose. In addition, a person skilled in the art can appropriately select a combination of any of the above procedures and their order.

### (Step ii)

The step ii will be described.

The step ii is a step of reacting a compound of the formula (2) with a compound of the formula (3) in the presence of a base to produce a compound of the formula (4) : 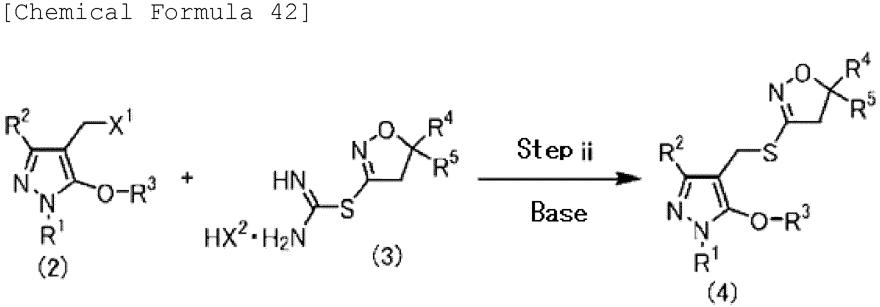 wherein R¹, R², R³, R⁴, R⁵, X¹ and X² are as described herein.

The reaction in the step ii is a condensation reaction.

### (Raw Material in Step ii; Compound of Formula (2))

A compound of the formula (2) is used as a raw material in the step ii. The compound of the formula (2) may be a known compound or may be produced from a known compound according to a known process. For example, the preparation of the compounds of the formula (2) is described in WO 2004/013106 A1 (Patent Document 2), Examples 13 and 14, which are shown below.

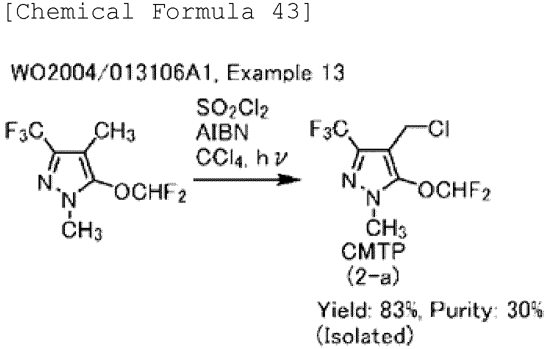

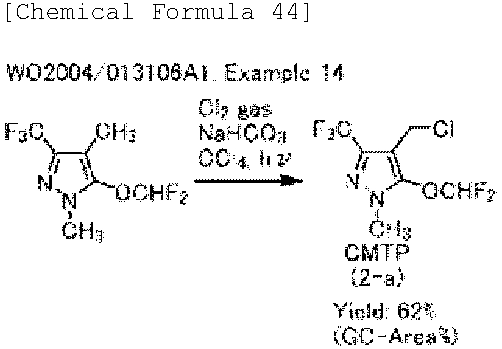

However, it is preferred that the compound of the formula (2) is produced by the process of the present invention. That is, when X¹ in the formula (2) is a halogen atom, the compound of the formula (2) is preferably produced by the process comprising the step i described herein.

In the formula (2), R¹, R² and R³ are as defined above. In the formula (2), examples, preferred examples, more preferred examples, and particularly preferred examples of R¹, R² and R³ are the same as those described above.

X¹ in the formula (2) is a leaving group. X¹ in the formula (2) may be any atom or atomic group as long as it functions as a leaving group in the reaction in the step ii.

From the viewpoint of yield, availability, price, etc., preferred examples of X¹ in the formula (2) include halogen atoms, (C1-C4)alkylsulfonyloxys, (C1-C4)haloalkylsulfonyloxys, (C1-C4)alkyls, or benzenesulfonyloxy optionally having a halogen atom, more preferably a chlorine atom, a bromine atom, an iodine atom, methanesulfonyloxy, ethanesulfonyloxy, trifluoromethanesulfonyloxy, benzenesulfonyloxy, p-toluenesulfonyloxy and p-chlorobenzenesulfonyloxy, further preferably a chlorine atom and a bromine atom, and particularly preferably a chlorine atom.

Specific examples and particularly preferred specific examples of the compound of the formula (2) are as described above.

### (Raw Material in Step ii; Compound of Formula (3))

A compound of the formula (3) is used as a raw material in the step ii.

The compound of the formula (3) may be a known compound or may be produced from a known compound according to a known process. For example, the preparation of the compound of the formula (3) can be performed by the processes described in WO 2006/068092 A1 (Patent Document 6), JP 2013-512201 A (Patent Document 7) and WO 2019/131715 A1 (Patent Document 8), or by processes similar thereto. JP 2013-512201 A, paragraph 0004 (US 2012/264947 A1, paragraph 0007) (Patent Document 7) disclose a process for producing the raw material used in the process described in WO 2006/068092 A1 (Patent Document 6) by citing JP 2008-001597 A and WO 2006/038657 A1. These are summarized in the following scheme.

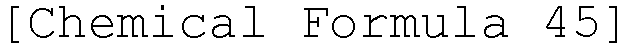

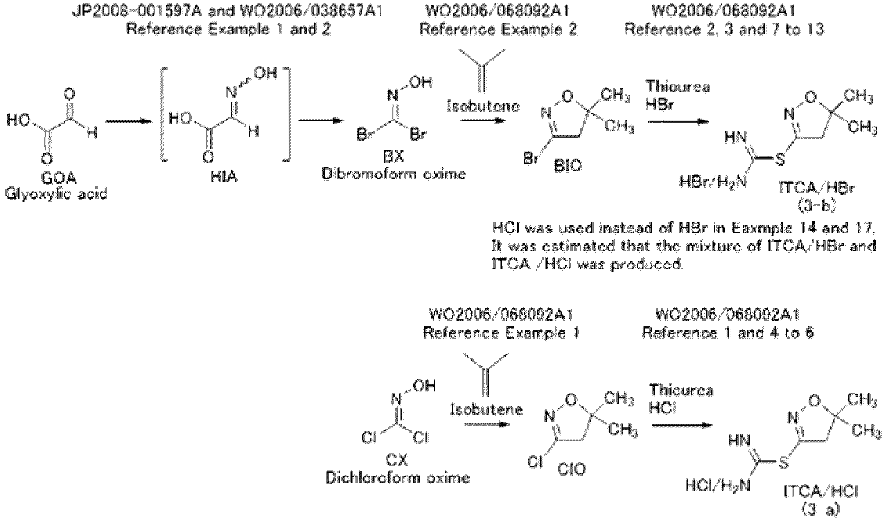

In the formula (3), R⁴ and R⁵ are each independently a (C1-C6)alkyl optionally substituted with one or more substituents, a (C3-C6)cycloalkyl optionally substituted with one or more substituents, a (C2-C6)alkenyl optionally substituted with one or more substituents, a (C2-C6)alkynyl optionally substituted with one or more substituents, a (C1-C6)alkoxy optionally substituted with one or more substituents, or a (C6-C10)aryl optionally substituted with one or more substituents; or R⁴ and R⁵, together with the carbon atom to which they are attached, form a 4- to 12-membered carbocyclic ring, wherein the formed ring is optionally substituted with one or more substituents.

From the viewpoints of yield, availability, price, usefulness of the product, etc., preferred examples of R⁴ and R⁵ in the formula (1) each independently include (C1-C6)alkyls optionally substituted with one or more substituents, more preferably (C1-C6)alkyls, further preferably (C1-C4)alkyls, and particularly preferably methyl.

X² in the formula (3) is an atom or an atomic group that forms an acid. Thus, HX² is an acid.

From the viewpoint of yield, availability, price, usefulness of the product, etc., preferred examples of X¹ in the formula (2) include:
halogen atoms, a sulfate group, a hydrogen sulfate group, a phosphate group, a monohydrogen phosphate group, a dihydrogen phosphate group, (C1-C4)alkylsulfonyloxys, (C1-C4)haloalkylsulfonyloxys, benzenesulfonyloxys optionally having an (C1-C4)alkyl or a halogen atom, and mixtures of two or more (preferably two or three, more preferably two) thereof, more preferably a chlorine atom, a bromine atom, an iodine atom, a sulfate group, a hydrogen sulfate group, a phosphate group, a monohydrogen phosphate group, a dihydrogen phosphate group, methanesulfonyloxy, ethanesulfonyloxy, trifluoromethanesulfonyloxy, benzenesulfonyloxy, p-toluenesulfonyloxy, p-chlorobenzenesulfonyloxy and mixtures of two or more (preferably two or three, more preferably two) thereof, still more preferably a chlorine atom, a bromine atom, a sulfate group, a hydrogen sulfate group, a phosphate group, a monohydrogen phosphate group, methanesulfonyloxy, p-toluenesulfonyloxy, and mixtures of two or more (preferably two or three, more preferably two) thereof, and particularly preferably a chlorine atom, a bromine atom and a mixture thereof.

Particularly preferred specific examples of the compound of the formula (3) are the following compounds and a mixture thereof.

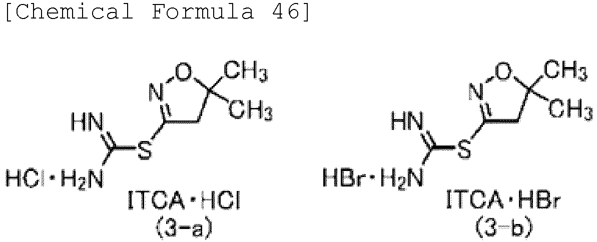

In addition, when "X₂H" is a polyvalent acid such as sulfuric acid or phosphoric acid, it is within the scope of the present invention that the ratio between "X2 of the acid moiety" and "(4,5-dihydroisoxazolo-3-yl)thiocarboxamidine moiety in the following formula (3-1)" can be a ratio corresponding to all possible valences of the polyvalent acid.

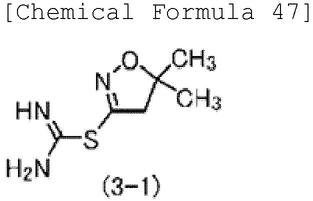

In other words, for example, the compound of the following formula (3-c) is an equivalent of the compound of the formula (3).

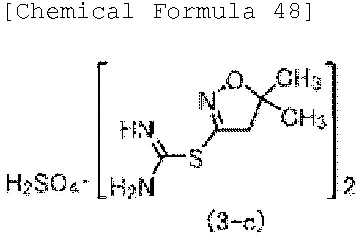

In the reaction in the step ii, it was presumed that the isothiouronium group in the compound of the formula (3) produced the corresponding thiol group and/or a salt thereof (e.g., generally -S⁻Na⁺ or -S⁻K⁺) and/or an analog thereof. Compounds having thiol groups and/or salts thereof and/or analogs thereof corresponding to the compounds of the formula (3) are equivalents of the compounds of the formula (3), and processes using the equivalents are within the scope of the present invention as defined by the appended claims.

### (Raw material in Step ii: Amount of Compound of Formula (3) Used)

The amount of the formula (3) used in the step ii may be any amount as long as the reaction proceeds. The amount of the formula (3) used in the step ii may be appropriately adjusted by a person skilled in the art. However, from the viewpoint of yield, suppression of by-products, economic efficiency, etc., the amount of the compound of the formula (3) used in the step ii is, for example, 0.5 to 2.0 mol or more, preferably 0.8 to 1.5 mol, more preferably 1.0 to 1.5 mol, and still more preferably 1.0 to 1.1 mol, based on 1 mol of the compound of the formula (2) (raw material).

### (Product in Step ii; Compound of Formula (4))

The product in the step ii is a compound of the formula (4) corresponding to the compound of the formula (2) and the compound of the formula (3) used as raw materials.

In the formula (4), R¹, R² and R³ are as defined in the formula (1). In the formula (4), R⁴ and R⁵ are as defined in the formula (3). In the formula (4), examples, preferred examples, more preferred examples, and particularly preferred examples of R¹, R², R³, R⁴ and R⁵ are the same as those in the formula (1) and the formula (3) described above, respectively.

Particularly preferred specific examples of the compound of the formula (4) are as follows:

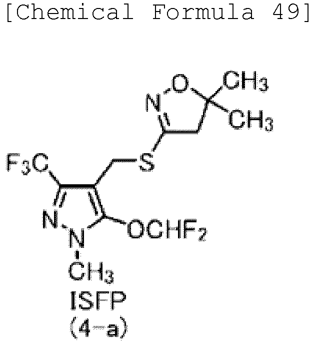

### (Base in Step ii)

The reaction in the step ii is performed in the presence of a base. The base may be any base as long as the reaction proceeds. Examples of the base in the step ii include, but are not limited to, the following:
alkali metal hydroxides (e.g., lithium hydroxide, sodium hydroxide and potassium hydroxide), alkaline earth metal hydroxides (e.g., magnesium hydroxide, calcium hydroxide and barium hydroxide), alkali metal carbonates (e.g., lithium carbonate, sodium carbonate, potassium carbonate and cesium carbonate), alkaline earth metal carbonates (e.g., magnesium carbonate and calcium carbonate), alkali metal hydrogen carbonates (e.g., lithium hydrogen carbonate, sodium hydrogen carbonate and potassium hydrogen carbonate), alkaline earth metal hydrogen carbonates (e.g., calcium hydrogen carbonate), phosphate salts (e.g., sodium phosphate, potassium phosphate and calcium phosphate), hydrogen phosphate salts (e.g., sodium hydrogen phosphate, potassium hydrogen phosphate and calcium hydrogen phosphate), amines (e.g., triethylamine, tributylamine, diisopropylethylamine, 1,8-diazabicyclo[5.4.0]-7-undec-7-ene (DBU), 1,4-diazabicyclo[2.2.2]octane (DABCO), pyridine and 4-(dimethylamino)-pyridine (DMAP)), ammonia, and a mixture thereof.

From the viewpoint of yield, suppression of by-products, economic efficiency, etc., preferred examples of the base in the step ii include alkali metal hydroxides, alkali metal carbonates, alkali metal hydrogen carbonates, and a mixture thereof, more preferably alkali metal hydroxides, alkali metal carbonates, and a mixture thereof, and still more preferably alkali metal hydroxides.

From the same viewpoint as above, preferred specific examples of the base in the step ii include lithium hydroxide, sodium hydroxide, potassium hydroxide, lithium carbonate, sodium carbonate, potassium carbonate, lithium hydrogen carbonate, sodium hydrogen carbonate, potassium hydrogen carbonate and a mixture thereof, more preferably lithium hydroxide, sodium hydroxide, potassium hydroxide, lithium carbonate, sodium carbonate, potassium carbonate and a mixture thereof, still more preferably sodium hydroxide, potassium hydroxide, sodium carbonate, potassium carbonate and a mixture thereof, further preferably sodium hydroxide, potassium hydroxide and a mixture thereof, and particularly preferably sodium hydroxide.

The base in the step ii may be used singly or in a combination of two or more kinds thereof in any ratio. The base in the step ii may be in any form as long as the reaction proceeds. Examples of the form of the base in the step ii include a base-only solid and an aqueous solution with any concentration. Specific examples of the form of the base include, but are not limited to, flake, pellet, bead, powder and 10 to 50% aqueous solution, and preferably 20 to 50% aqueous solution (e.g., 25% aqueous sodium hydroxide solution and 48% aqueous sodium hydroxide solution, preferably 48% aqueous sodium hydroxide solution). The form of the base in the step ii can be appropriately selected by a person skilled in the art.

The amount of the base used in the step ii may be any amount as long as the reaction proceeds. The amount of the base used in the step ii may be appropriately adjusted by a person skilled in the art. However, from the viewpoint of yield, suppression of by-products, economic efficiency, etc., in one embodiment, the amount of the base used in the step ii is, for example, 5 to 10 equivalents, preferably 5 to 8 equivalents, more preferably 5 to 7 equivalents, and still more preferably 5 to 6 equivalents, based on 1 equivalent of the compound of the formula (2) (raw material). In another embodiment, for example, the amount of the base used in the step ii is 1 to 15 equivalents, preferably 1 to 10 equivalents, more preferably 2 to 9 equivalents, still more preferably 4 to 8 equivalents, and further preferably 5 to 6 equivalents, based on 1 equivalent of the compound of the formula (2) (raw material).

### (Reaction Solvent in Step ii)

From the viewpoint of allowing the reaction to smoothly proceed, the reaction in the step ii is preferably performed in the presence of a solvent.

The solvent in the reaction in the step ii may be any solvent as long as the reaction proceeds.

In one embodiment, examples of the solvent in the reaction in the step ii include, but are not limited to, the following: organic solvents having an acceptor number from 0 (zero) to 50 (preferably 3 to 45, more preferably 5 to 45, still more preferably 5 to 35, further preferably 5 to 30, further preferably 5 to 20, and further preferably 8 to 20), water, and any combination thereof in any ratio.

In another embodiment, examples of the solvent in the reaction in the step ii include, but are not limited to, the following:
aromatic hydrocarbon derivatives (e.g., benzene, toluene, xylenes, chlorobenzene, dichlorobenzenes, trichlorobenzenes and nitrobenzene), halogenated aliphatic hydrocarbons (e.g., dichloromethane and 1,2-dichloroethane (EDC)), alcohols (e.g., methanol, ethanol, propanol, 2-propanol, butanol, sec-butanol, isobutanol and tert-butanol (tert-butanol is also referred to as tert-butyl alcohol), pentanol, sec-amyl alcohol, 3-pentanol, 2-methyl-1-butanol, isoamyl alcohol, tert-amyl alcohol, hexanol and cyclohexanol), nitriles (e.g., acetonitrile and propionitrile), carboxylic acid esters (e.g., methyl acetate, ethyl acetate, propyl acetate, isopropyl acetate, butyl acetate and isomers thereof and pentyl acetate and isomers thereof), ethers (e.g., tetrahydrofuran (THF), 1,4-dioxane, diisopropyl ether, dibutyl ether, di-tert-butyl ether, cyclopentyl methyl ether (CPME), methyl tert-butyl ether, 1,2-dimethoxyethane (DME) and diglyme), ketones (e.g., acetone, methyl ethyl ketone (MEK), methyl isopropyl ketone (MIPK) and methyl isobutyl ketone (MIBK)), amides (e.g., N,N-dimethylformamide (DMF), N,N-dimethylacetamide (DMAC) and N-methylpyrrolidone (NMP)), ureas (e.g., N,N'-dimethylimidazolidinone (DMI) and tetramethylurea), sulfoxides (e.g., dimethyl sulfoxide (DMSO)), sulfones (e.g., sulfolane), water, and any combination thereof in any ratio. "2-Propanol" is also referred to as "isopropyl alcohol" or "isopropanol".

However, from the viewpoint of yield, suppression of by-products, economic efficiency, etc., preferred examples of the solvent in the reaction in the step ii include combinations of one or more (preferably one or two, more preferably one) organic solvents selected from aromatic hydrocarbon derivatives, halogenated aliphatic hydrocarbons, alcohols, nitriles, carboxylic acid esters, ethers, ketones, amides, ureas, sulfoxides, and sulfones, with a water solvent in any ratio.

More preferred examples of the solvent in the reaction in the step ii include combinations of one or more (preferably one or two, more preferably one) organic solvents selected from alcohols, nitriles, carboxylic acid esters, ethers, amides and sulfones with a water solvent in any ratio.

More preferred examples of the solvent in the reaction in the step ii include combinations of one or more (preferably one or two, more preferably one) organic solvents selected from alcohols, nitriles, carboxylic acid esters, ethers and amides with a water solvent in any ratio.

More preferred examples of the solvent in the reaction in the step ii include combinations of one or more (preferably one or two, more preferably one) organic solvents selected from alcohols, nitriles, carboxylic acid esters and amides with a water solvent in any ratio.

More preferred examples of the solvent in the reaction in the step ii include combinations of one or more (preferably one or two, more preferably one) organic solvents selected from alcohols, nitriles and carboxylic acid esters with a water solvent in any ratio.

More preferred examples of the solvent in the reaction of the step ii include combinations of one or more (preferably one or two, more preferably one) organic solvents selected from nitriles and carboxylic acid esters with a water solvent in any ratio.

In one embodiment, particularly preferred examples of the solvent in the reaction in the step ii include combinations of nitriles with a water solvent in any ratio.

In another embodiment, particularly preferred examples of the solvent in the reaction in the step ii include combinations of carboxylic acid esters with a water solvent in any ratio.

From the same viewpoint as described above, preferred specific examples of the solvent in the reaction in the step ii include combinations of one or more (preferably one or two, more preferably one) organic solvents selected from toluene, xylenes, chlorobenzene, dichlorobenzenes, dichloromethane, 1,2-dichloroethane, methanol, ethanol, propanol, 2-propanol, butanol, sec-butanol, isobutanol, tert-butanol, pentanol, sec-amyl alcohol, 3-pentanol, 2-methyl-1-butanol, isoamyl alcohol, tert-amyl alcohol, acetonitrile, ethyl acetate, propyl acetate, isopropyl acetate, butyl acetate and isomers thereof (in the present invention, the "isomer of butyl acetate" is an equivalent of "butyl acetate"), tetrahydrofuran (THF), 1,4-dioxane, diisopropyl ether, dibutyl ether, di-tert-butyl ether, cyclopentyl methyl ether (CPME), methyl-tert-butyl ether, 1,2-dimethoxyethane (DME), diglyme, acetone, methyl ethyl ketone (MEK), methyl isopropyl ketone (MIPK), methyl isobutyl ketone (MIBK), N,N-dimethylformamide (DMF), N,N-dimethylacetamide (DMAC), N-methylpyrrolidone (NMP), N,N'-dimethylimidazolidinone (DMI), tetramethylurea, dimethyl sulfoxide (DMSO) and sulfolane with a water solvent in any ratio.

From the same viewpoint as described above, more preferred specific examples of the solvent in the reaction in the step ii include combinations of one or more (preferably one or two, more preferably one) organic solvents selected from toluene, xylenes, chlorobenzene, dichlorobenzenes, dichloromethane, 1,2-dichloroethane, methanol, ethanol, propanol, 2-propanol, butanol, sec-butanol, isobutanol, tert-butanol, acetonitrile, ethyl acetate, propyl acetate, isopropyl acetate, butyl acetate and isomers thereof (in the present invention, the "isomer of butyl acetate" is an equivalent of "butyl acetate"), tetrahydrofuran (THF), 1,4-dioxane, diisopropyl ether, dibutyl ether, di-tert-butyl ether, cyclopentyl methyl ether (CPME), methyl-tert-butyl ether, 1,2-dimethoxyethane (DME), diglyme, acetone, methyl ethyl ketone (MEK), methyl isopropyl ketone (MIPK), methyl isobutyl ketone (MIBK), N,N-dimethylformamide (DMF), N,N-dimethylacetamide (DMAC), N-methylpyrrolidone (NMP), N,N'-dimethylimidazolidinone (DMI), tetramethylurea, dimethyl sulfoxide (DMSO) and sulfolane with a water solvent in any ratio.

From the same viewpoint as described above, still more preferred specific examples of the solvent in the reaction in the step ii include combinations of one or more (preferably one or two, more preferably one) organic solvents selected from toluene, xylenes, chlorobenzene, dichlorobenzenes, dichloromethane, 1,2-dichloroethane, methanol, ethanol, 2-propanol, butanol, tert-butanol, acetonitrile, ethyl acetate, propyl acetate, isopropyl acetate, butyl acetate and isomers thereof (in the present invention, the "isomer of butyl acetate" is an equivalent of "butyl acetate"), tetrahydrofuran (THF), 1,4-dioxane, diisopropyl ether, dibutyl ether, di-tert-butyl ether, cyclopentyl methyl ether (CPME), methyl-tert-butyl ether, 1,2-dimethoxyethane (DME), diglyme, acetone, methyl ethyl ketone (MEK), methyl isopropyl ketone (MIPK), methyl isobutyl ketone (MIBK), N,N-dimethylformamide (DMF), N,N-dimethylacetamide (DMAC), N-methylpyrrolidone (NMP), N,N'-dimethylimidazolidinone (DMI), tetramethylurea, dimethyl sulfoxide (DMSO) and sulfolane with a water solvent in any ratio.

More preferred specific examples of the solvent in the reaction in the step ii include combinations of one or more (preferably one or two, more preferably one) organic solvents selected from methanol, ethanol, 2-propanol, butanol, tert-butanol, acetonitrile, ethyl acetate, propyl acetate, isopropyl acetate, butyl acetate and isomers thereof with a water solvent in any ratio.

Still more preferred specific examples of the solvent in the reaction in the step ii include combinations of one or more (preferably one or two, more preferably one) organic solvents selected from butanol, acetonitrile, ethyl acetate, propyl acetate, isopropyl acetate and butyl acetate with a water solvent in any ratio.

Further preferred specific examples of the solvent in the reaction in the step ii include combinations of one or more (preferably one or two, more preferably one) organic solvents selected from acetonitrile, ethyl acetate, isopropyl acetate and butyl acetate with a water solvent in any ratio.

Further preferred specific examples of the solvent in the reaction in the step ii include combinations of one or two (preferably one) organic solvents selected from acetonitrile and butyl acetate with a water solvent in any ratio.

In one embodiment, particularly preferred specific examples of the solvent in the reaction in the step ii include combinations of an acetonitrile solvent with a water solvent in any ratio.

In another embodiment, particularly preferred specific examples of the solvent in the reaction in the step ii include combinations of a butyl acetate solvent with a water solvent in any ratio.

In any case, the solvent may be in a single layer or may be separated into two layers as long as the reaction proceeds.

The amount of the solvent used in the reaction in the step ii will be described. The "total amount of the solvent used in the reaction" is the sum total of the amounts of all the organic solvents and the amount of the water solvent used in the reaction. The organic solvent and the water solvent used in the working-up (e.g., isolation and purification) after the reaction are not included. The "organic solvent" used in the reaction includes the organic solvent in the raw material solution and that in the reactant solution. The "water solvent" used in the reaction includes the water in the raw material solution and that in the reactant solution (e.g., the water in a 48% aqueous sodium hydroxide solution).

The total amount of the solvent used in the reaction in the step ii is not particularly limited as long as the reaction system can be sufficiently stirred. However, from the viewpoint of yield, suppression of by-products, economic efficiency, etc., in one embodiment, the total amount of the solvent used in the reaction in the step ii is, for example, 0.1 to 10 L (liters), preferably 0.5 to 5 L, more preferably 1 to 5 L, still more preferably 1 to 3 L, and further preferably 1 to 2 L, based on 1 mol of the compound of the formula (2) (raw material). In another embodiment, the total amount of the solvent used in the reaction in the step ii is, for example, 1.5 to 3.0 L (liters), preferably 1.5 to 2.5 L, and more preferably 1.5 to 2.0 L, based on 1 mol of the compound of the formula (2) (raw material). In another embodiment, the total amount of the solvent used in the reaction in the step ii is, for example, 1.7 to 3.0 L (liters), preferably 1.7 to 2.5 L, and more preferably 1.7 to 2.0 L, based on 1 mol of the compound of the formula (2) (raw material).

From the same viewpoint as described above, in one embodiment, the amount of the organic solvent used in the reaction in the step ii is, for example, 0 (zero) to 5 L (liters), preferably 0.4 to 2.0 L, more preferably 0.5 to 1.5 L, still more preferably 0.6 to 1.0 L, and further preferably 0.7 to 0.9 L, based on 1 mol of the compound of the formula (2) (raw material). In another embodiment, the amount of the organic solvent used in the reaction in the step ii is, for example, 0.1 to 5 L (liters), preferably 0.3 to 2.0 L, more preferably 0.4 to 1.5 L, still more preferably 0.5 to 1.0 L, and further preferably 0.6 to 0.8 L, based on 1 mol of the compound of the formula (2) (raw material).

From the same viewpoint as described above, the amount of the water solvent used in the reaction in the step ii is, for example, 0.1 to 5 L (liters), preferably 0.5 to 2.0 L, more preferably 0.5 to 1.5 L, still more preferably 0.7 to 1.4 L, and further preferably 0.9 to 1.2 L, based on 1 mol of the compound of the formula (2) (raw material).

When a combination of two or more organic solvents is used, the ratio of the two or more organic solvents may be any ratio as long as the reaction proceeds.

When a combination of an organic solvent and a water solvent is used, the ratio of the organic solvent to the water solvent may be any ratio as long as the reaction proceeds. However, from the viewpoint of yield, suppression of by-products, economic efficiency, etc., the ratio of the organic solvent to the water solvent is, for example, 90 : 10 to 0 : 100 by volume ratio, preferably 90 : 10 to 10 : 90 by volume ratio, more preferably 70 : 30 to 30 : 70 by volume ratio, still more preferably 50 : 50 to 35 : 65 by volume ratio, and further preferably 50 : 50 to 40 : 60 by volume ratio.

In other words, the amount of the water solvent in the whole solvent composed of the organic solvent and the water solvent is, for example, 10 vol% to 100 vol%, preferably 10 vol% to 90 vol%, more preferably 30 vol% to 70 vol%, still more preferably 50 vol% to 65 vol%, and further preferably 50 vol% to 60 vol%, based on the amount of the whole solvent (100 vol%).

### (Reaction Temperature in Step ii)

The reaction temperature in the step ii is not particularly limited. However, from the viewpoint of yield, suppression of by-products, economic efficiency, etc., the reaction temperature in the step ii is, for example, -10 (minus 10)°C to 100°C, preferably -10°C to 70°C, more preferably -10°C to 50°C, still more preferably 0 (zero) °C to 40°C, further preferably 0°C to 30°C, and further preferably 0°C to 25°C.

### (Reaction Time in Step ii)

The reaction time in the step ii is not particularly limited. However, from the viewpoint of yield, suppression of by-products, economic efficiency, etc., in one embodiment, the reaction time in the step ii is, for example, 4 hours to 48 hours, preferably 4 hours to 24 hours, more preferably 4 hours to 18 hours, and still more preferably 4 hours to 12 hours. In another embodiment, the reaction time in the step ii is, for example, 1 hour to 48 hours, preferably 1 hour to 24 hours, more preferably 3 hours to 18 hours, and still more preferably 3 hours to 12 hours. However, the reaction time can be adjusted appropriately by a person skilled in the art.

### (Adding Method in Step ii)

The order of adding the compound of the formula (2), the compound of the formula (3), the base, the solvent, etc. is not particularly limited. As long as the reaction proceeds, the addition order thereof may be any order. For example, the base may be added dropwise to a mixture comprising the compound of the formula (2), the compound of the formula (3) and the solvent in a reaction vessel. As another example, the compound of the formula (2) may be added dropwise to the reaction vessel after adding the compound of the formula (3), the base and the solvent. As still another example, the compound of the formula (2) and the compound of the formula (3) may be successively added dropwise to the reaction vessel after adding the base and the solvent.

### (Working-up in Step ii; Isolation and/or Purification)

The compound of the formula (4), especially the compound (4-a), which is the product in the step ii, can be used as a raw material in the step iii. The compound of the formula (4) obtained in the step ii may be isolated and/or purified and then used in the next step, or may be used in the next step without being isolated. Whether or not to perform the working-up (isolation and/or purification) can be appropriately determined by a person skilled in the art according to the purpose and situation.

The compounds of the formula (4), especially the compound (4-a), which is the target product in the step ii, can be isolated and purified from the reaction mixture by methods known to a person skilled in the art (e.g., extraction, washing, crystallization including recrystallization, crystal washing and/or other procedures) and improved methods thereof, and any combination thereof.

In the working-up step (isolation and/or purification), the following procedures may be performed, but are not limited thereto: in the working-up, an extraction procedure and a washing procedure which include separation of an organic layer and an aqueous layer may be performed. When the mixture is separated into an organic layer and an aqueous layer, the mixture may be separated while being hot. For example, when separating the organic layer from the aqueous layer, a hot mixture may be used, or the mixture may be heated. Impurities may be removed by a filtration procedure including hot filtration.

In the washing procedure, if possible, the product dissolved or suspended in an organic solvent may be washed with water, hot water, an aqueous alkaline solution (e.g., a 5% to saturated aqueous sodium hydrogen carbonate solution or a 1 to 10% aqueous sodium hydroxide solution), or an acidic aqueous solution (e.g., 5 to 35% hydrochloric acid or 5 to 35% sulfuric acid). Such washing procedures may be combined.

When performing crystallization of the product including recrystallization and washing of crystals, the description in the step iii described later may be referred to.

In any of the above procedures, the temperature can be appropriately adjusted by a person skilled in the art according to the purpose and situation.

In any procedure of the working-up and the procedure of using the product in the next step, the amount of a solvent can be appropriately adjusted by a person skilled in the art by addition and removal thereof. Furthermore, recovery and recycle of the solvent may be optionally performed. For example, the recovery and recycle of the solvent used in the reaction may be performed, and the recovery and recycle of the solvent used in the working-up (isolation and/or purification) may be performed.

Working-up (isolation and/or purification) can be performed by appropriately combining all or some of the procedures described above. Optionally, the above procedure may be repeated according to the purpose. In addition, a person skilled in the art can appropriately select a combination of any of the above procedures and their order.

### (Step iii)

The step iii will be described.

The reaction in the step iii is a step of producing a compound of the formula (5) from a compound of the formula (4) by an oxidation reaction in the presence of a metal catalyst.

Examples of the oxidation reaction in the step iii include a method using an oxidizing agent such as hydrogen peroxide, hypochlorite, peroxide, permanganate, manganese dioxide, or chromic acid, dimethyl sulfoxide oxidation such as Jones oxidation, ozone oxidation, or Swern oxidation, and Oxone oxidation. Performing the reaction in the step iii using a hypochlorite such as sodium hypochlorite or potassium hypochlorite, sodium peroxodisulfate, potassium peroxymonosulfate (Oxone (registered trademark)), or the like in place of hydrogen peroxide is an equivalent of the present invention and is within the scope of the present invention.

The step iii is preferably a step of reacting the compound of the formula (4) with hydrogen peroxide in the presence of a metal catalyst to produce the compound of the formula (5): 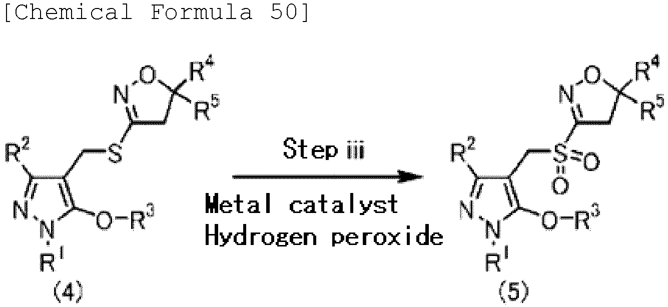 wherein R¹, R², R³, R⁴ and R⁵ are as described herein.

### (Raw Material in Step iii; Compound of Formula (4))

A compound of the formula (4) is used as a raw material in the step iii. The compound of the formula (4) may be a known compound or may be produced from a known compound according to a known process. As described above, for example, the preparation of the compound of the formula (4) is described in WO 2004/013106 A1 (Patent Document 2), Reference Examples 1-1, 1-2 and 1-3, WO 2005/105755 A1 (Patent Document 3), Examples 3 to 5 and WO 2005/095352 A1 (Patent Document 4), Examples 1 to 5. In addition, the preparation of the compound of the formula (4) can be performed by a similar method. However, it is preferred that the compound of the formula (4) is produced by the process of the present invention. That is, the compound of the formula (4) is preferably produced by the process comprising the step ii described herein.

In the formula (4), R¹, R² and R³ are as defined in the formula (1). In the formula (4), R⁴ and R⁵ are as defined in the formula (3). In the formula (4), examples, preferred examples, more preferred examples, and particularly preferred examples of R¹, R², R³, R⁴ and R⁵ are the same as those in the formula (1) and the formula (3) described above, respectively. Particularly preferred specific examples of the compound of the formula (4) are as described above.

### (Product in Step iii; Compound of Formula (5))

The product in the step iii is a compound of the formula (5) corresponding to the compound of the formula (4) used as a raw material.

In the formula (5), R¹, R² and R³ are as defined in the formula (1). In the formula (5), R⁴ and R⁵ are as defined in the formula (3). In the formula (5), examples, preferred examples, more preferred examples, and particularly preferred examples of R¹, R², R³, R⁴ and R⁵ are the same as those in the formula (1) and the formula (3) described above, respectively. 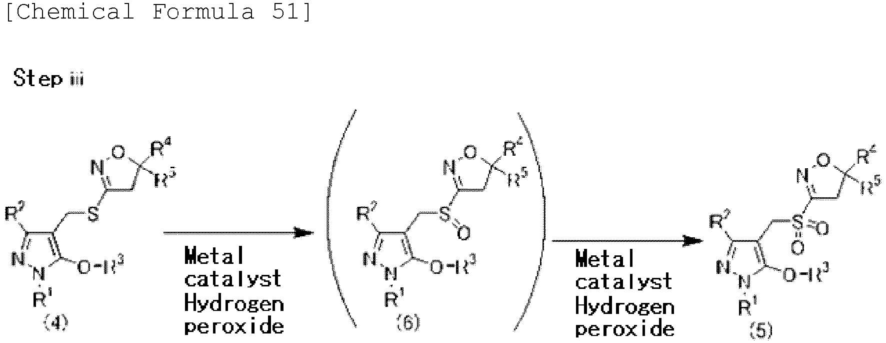 After the formula (4) is oxidized to afford the formula (6), oxidation to the formula (5) may be performed.

Particularly preferred specific examples of the compound of the formula (5) are as follows:

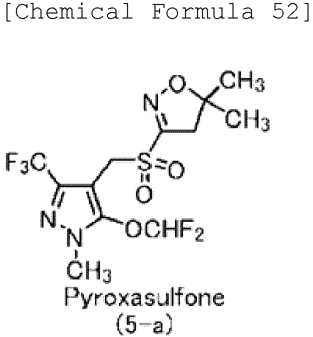

As described above, in the process of producing the compound of the formula (5) (SO₂ derivative) from the compound of the formula (4) (S derivative), it is desired that the oxidation reaction sufficiently proceeds and the proportion of the compound of the formula (6) (SO derivative) in the product is sufficiently low. For example, in the reaction mixture after the reaction in the step iii, the ratio of the compound of the formula (6) (SO derivative) is preferably 10% or less, more preferably 5% or less, still more preferably 3% or less, further preferably 2% or less, and further preferably 1% or less.

### (Oxidizing Agent in Step iii: Hydrogen Peroxide)

In the reaction in the step iii, the hypochlorite, peroxide, permanganate, manganese dioxide, chromic acid, etc. described above can be used as an oxidizing agent. Preferably, hydrogen peroxide is used.

The form of the hydrogen peroxide in the step iii may be any form as long as the reaction proceeds. The form of the hydrogen peroxide in the step iii can be suitably selected by a person skilled in the art. However, in view of safety, danger, economic efficiency, etc., preferred examples of the form of the hydrogen peroxide include a 10 to 70 wt% aqueous hydrogen peroxide solution, more preferably a 25 to 65 wt% aqueous hydrogen peroxide solution, still more preferably a 30 to 65 wt% aqueous hydrogen peroxide solution, and particularly preferably a 30 to 60 wt% aqueous hydrogen peroxide solution. Specific examples of the form of the hydrogen peroxide include, but are not limited to, a 30 wt% aqueous hydrogen peroxide solution, a 35 wt% aqueous hydrogen peroxide solution, a 50 wt% aqueous hydrogen peroxide solution and a 60 wt% aqueous hydrogen peroxide solution.

The amount of the hydrogen peroxide used in the step iii may be any amount as long as the reaction proceeds. The amount of the hydrogen peroxide used in the step iii may be appropriately adjusted by a person skilled in the art. However, from the viewpoint of yield, suppression of by-products, economic efficiency, safety, risk, etc., the amount of the hydrogen peroxide used in the step iii is, for example, 2 mol or more, preferably 2 to 8 mol, more preferably 2 to 6 mol, still more preferably 2 to 5 mol, further preferably 2 to 4 mol, further preferably 2 to 3, and further preferably 2.3 to 3 mol, based on 1 mol of the compound of the formula (4) (raw material).

### (Catalyst in Step iii: Metal Catalyst)

The reaction in the step iii is performed in the presence of a metal catalyst. The metal catalyst may be any metal catalyst as long as the reaction proceeds. Examples of the metal catalyst in the step iii include, but are not limited to, the following:
tungsten catalysts (e.g., tungstic acid, tungstic acid salts (e.g., sodium tungstates (including sodium tungstate dihydrate and sodium tungstate decahydrate), potassium tungstate, calcium tungstate and ammonium tungstate), metal tungsten, tungsten oxides (e.g., tungsten(VI) oxide;
tungsten(VI) oxide is also called tungsten trioxide), tungsten carbide, tungsten chlorides (e.g., tungsten(VI) chloride; tungsten(VI) chloride is also called tungsten hexachloride), tungsten bromides (e.g., tungsten(V) bromide), tungsten sulfides (e.g., tungsten(IV) sulfide;
tungsten(IV) sulfide is also called tungsten disulfide), phosphotungstic acid and salts thereof (e.g., phosphotungstic acid, sodium phosphotungstate and ammonium phosphotungstate), silicotungstic acid and salts thereof (e.g., silicotungstic acid and sodium silicotungstate), and a mixture thereof),
molybdenum catalysts (e.g., molybdic acid, molybdic acid salts, (e.g., sodium molybdate (including sodium molybdate dihydrate), potassium molybdate, ammonium molybdate (including ammonium molybdate tetrahydrate), metal molybdenum, molybdenum oxides (e.g., molybdenum(VI) oxide;
molybdenum(VI) oxide is also called molybdenum trioxide), molybdate chlorides (molybdenum(V) chloride; molybdenum(V) chloride is also called molybdenum pentachloride), molybdenum sulfides (e.g., molybdenum(IV) sulfide;
molybdenum(IV) sulfide is also called molybdenum disulfide), phosphomolybdic acid and salts thereof (e.g., phosphomolybdic acid, sodium phosphomolybdate and ammonium phosphomolybdate), silicomolybdic acid and salts thereof (e.g., silicomolybdic acid and sodium silicomolybdate), bis(2,4-pentandionato)molybdenum(VI) dioxide, and a mixture thereof),
iron catalysts (e.g., iron(I) acetylacetoneate, iron(I) chloride and iron(I) nitrate, and a mixture thereof), manganese catalysts (e.g., potassium permanganate, manganese(II) oxide and manganese(II) chloride, and a mixture thereof),
vanadium catalysts (e.g., vanadyl acetylacetonate,
vanadium(V) oxide, vanadium(V) oxytrichloride, vanadium(V) oxytriethoxyde and vanadium(V) oxytriisopropoxide, and a mixture thereof),
niobium catalysts (e.g., niobium carbide, niobium(V) chloride and niobium(V) pentaethoxyde, and a mixture thereof),
tantalum catalysts (e.g., tantalum carbide (TaC), tantalum(V) chloride (TaCl₅) and tantalum(V) pentaethoxyde (Ta(OEt)₅), and a mixture thereof),
titanium catalysts (e.g., titanium tetrachloride, titanium trichloride and titanium(IV) tetraisopropoxide, and a mixture thereof),
zirconium catalysts (e.g., zirconium dioxide, zirconium(I) chloride, zirconium(IV) chloride, zirconium chloride oxide, and a mixture thereof),
copper catalysts (e.g., copper(I) acetate, copper(II) acetate, copper(I) bromide and copper(I) iodide, and a mixture thereof),
thallium catalysts (e.g., thallium(I) nitrate, thallium(I) acetate and thallium (I) trifluoroacetate, and a mixture thereof).

Herein, an acid that can be in the form of a hydrate and a salt thereof may be in the form of a hydrate thereof, and any form is within the scope of the present invention.

Thus, for example, "sodium tungstate" encompasses "sodium tungstate dihydrate" and "sodium tungstate decahydrate".

Herein, an acid that can be in the form of a polyacid and a salt thereof (e.g., tungstic acid and salts thereof) may be in the form of a polyacid, and any form is within the scope of the present invention.
"Bis(2,4-pentanedionato)molybdenum(VI) dioxide" is also referred to as "MoO₂(acac)₂", "molybdenum(VI) dioxyacetylacetonate", or "bis(acetylacetonato)molybdenum(IV) oxide".
"Iron(I) acetylacetonate" is also referred to as "Fe(acac)₃" or "tris (2, 4-pentanedionato)iron(I)".
"Vanadyl acetylacetonate" is also referred to as "VO(acac)2", "bis(2,4-pentanedionato)vanadium(IV) oxide", "vanadium(IV) oxyacetylacetonate", or "vanadium(IV) bis(acetylacetonate)oxide".
"Vanadium(V) oxytrichloride" is also referred to as "VOCl₃", "vanadium(V) trichloride oxide", or "vanadyl trichloride".
"Vanadium(V) oxytriethoxide" is also referred to as "VO(OEt)₃", "vanadium(V) triethoxide", or "triethoxyvanadium(V) oxide".
"Vanadium(V) oxytriisopropoxide" is also referred to as (VO(OPr-i)₃", "vanadium(V) triisopropoxide oxide" or "triisopropoxyvanadium(V) oxide".
Titanium(IV) tetraisopropoxide is also referred to as titanium(IV) isopropoxide or tetraisopropoxytitanium(IV)).

The metal of the metal catalyst in the step iii is preferably a transition metal. Specific examples thereof include Group 3 elements (Sc, Y, etc.), Group 4 elements (Ti, Zr, Hf), Group 5 elements (V, Nb, Ta), Group 6 elements (Cr, Mo, W), Group 7 elements (Mn, Tc, Re), Group 8 elements (Fe, Ru, Os), Group 9 elements (Co, Rh, Ir), Group 10 elements (Ni, Pd, Pt) and Group 11 elements (Cu, Ag, Au).

The transition metal of the metal catalyst in the step iii is preferably a metal of Group 4, 5, or 6 of the periodic table.

The transition metal of the metal catalyst in the step iii is preferably Group 5 or Group 6.

The transition metal of the metal catalyst in the step iii is preferably Group 5 or Group 6 rather than Group 7, Group 8 and Group 9.

Preferred examples of the metal catalyst in the step iii are tungsten catalysts, molybdenum catalysts, niobium catalysts, tantalum catalysts, titanium catalysts and zirconium catalysts.

Preferred examples of the metal catalyst in the step iii are tungsten catalysts, molybdenum catalysts, niobium catalysts and tantalum catalysts.

Preferred examples of the metal catalyst in the step iii are tungsten catalysts, molybdenum catalysts and niobium catalysts.

Preferred examples of the metal catalyst in the step iii are tungsten catalysts and molybdenum catalysts.

Preferred examples of the metal catalyst in the step iii are tungsten catalysts.

Preferred examples of the metal catalyst in the step iii are molybdenum catalysts.

From the viewpoint of yield, suppression of by-products, economic efficiency, etc., preferred examples of the tungsten catalyst in the step iii include tungstic acid, tungstic acid salts, metal tungsten, tungsten oxide, tungsten carbide, tungsten chloride, tungsten sulfide, phosphotungstic acid, silicotungstic acid and salts thereof, and a mixture thereof,
more preferably tungstic acid, tungstic acid salts, metal tungsten, tungsten oxide, tungsten carbide, tungsten chloride and salts thereof, and a mixture thereof,
still more preferably tungstic acid, tungstic acid salts, metal tungsten, tungsten oxide, tungsten carbide, and a mixture thereof,
further preferably tungstic acid, sodium tungstate, potassium tungstate, calcium tungstate, ammonium tungstate, metal tungsten, tungsten(VI) oxide, tungsten carbide, and a mixture thereof,
further preferably tungstic acid, sodium tungstate, metal tungsten, tungsten carbide, and a mixture thereof,
further preferably tungstic acid and sodium tungstate, and particularly preferably sodium tungstate.

The metal catalyst (especially, a tungsten catalyst) in the step iii may be used singly or in a combination of two or more kinds thereof in any ratio. The form of the metal catalyst (especially, a tungsten catalyst) in the step iii may be any form as long as the reaction proceeds. The form thereof can be appropriately selected by a person skilled in the art. The amount of the metal catalyst (especially, a tungsten catalyst) used may be any amount as long as the reaction proceeds. The use amount may be appropriately adjusted by a person skilled in the art. However, from the viewpoint of yield, suppression of by-products, economic efficiency, etc., the use amount thereof is, for example, 0.001 to 0.1 mol, preferably 0.01 to 0.1 mol, more preferably 0.01 to 0.05 mol, and still more preferably 0.03 to 0.05 mol, based on 1 mol of the compound of the formula (4) (raw material).

From the viewpoint of yield, suppression of by-products, economic efficiency, etc., preferred examples of the molybdenum catalyst in the step iii include molybdic acid, molybdic acid salts, metal molybdenum, molybdenum oxide, molybdenum carbide, molybdenum chloride, molybdenum sulfide, molybdenum bromide, phosphomolybdic acid, silicomolybdic acid and salts thereof, and a mixture thereof,
more preferably molybdic acid, molybdic acid salts, metal molybdenum, molybdenum carbide, molybdenum oxide, molybdenum chloride, and a mixture thereof,
still more preferably molybdic acid, sodium molybdate, potassium molybdate, ammonium molybdate, molybdenum(VI) oxide, molybdenum carbide, molybdenum(V) chloride, molybdenum(IV) sulfide, phosphomolybdic acid, sodium phosphomolybdate, ammonium phosphomolybdate, silicomolybdic acid, sodium silicomolybdate, and a mixture thereof, further preferably molybdic acid, sodium molybdate, potassium molybdate, ammonium molybdate, molybdenum(VI) oxide, molybdenum(V) chloride, and a mixture thereof, further preferably sodium molybdate, potassium molybdate and ammonium molybdate, and
particularly preferably ammonium molybdate.

From the viewpoint of yield, suppression of by-products, economic efficiency, etc., preferred examples of the niobium catalyst in the step iii include niobic acid, niobic acid salts, metal niobium, niobium carbide, niobium oxide, niobium chloride, niobium nitride, niobium silicide, niobium boride, and a mixture thereof,
more preferably niobic acid, niobic acid salts, metal niobium, niobium carbide, niobium oxide, niobium chloride, etc., and a mixture thereof,
still more preferably sodium niobate, potassium niobate, niobium carbide, niobium(V) chloride, niobium(V) pentaethoxide, etc., and a mixture thereof,
further preferably sodium niobate and potassium niobate, and particularly preferably sodium niobate.

From the viewpoint of yield, suppression of by-products, economic efficiency, etc., preferred examples of the tantalum catalyst in the step iii include tantalic acid, tantalic acid salts, tantalum oxide, tantalum carbide, tantalum chloride, tantalum nitride, tantalum silicide, tantalum boride, and a mixture thereof,
more preferably tantalic acid, tantalic acid salts, tantalum oxide, tantalum carbide, tantalum chloride, and a mixture thereof,
more preferably lithium tantalate, potassium tantalate, tantalum pentoxide, tantalum carbide, tantalum(V) chloride, tantalum(V) pentaethoxide, etc., and a mixture thereof, still more preferably lithium tantalate and potassium tantalate, and particularly preferably lithium tantalate.

From the viewpoint of yield, suppression of by-products, economic efficiency, etc., preferred examples of the titanium catalyst in the step iii are titanic acid, titanic acid salts, titanium oxide, titanium carbide, titanium chloride, titanium nitride, and a mixture thereof, more preferably titanic acid, titanic acid salts, titanium oxide, titanium carbide, titanium chloride, and a mixture thereof,
still more preferably titanium acetylacetonate, titanium tetrachloride, titanium trichloride, titanium(IV) tetraisopropoxide, etc., and a mixture thereof, and
particularly preferably titanium acetylacetonate.

From the viewpoint of yield, suppression of by-products, economic efficiency, etc., more preferred examples of the zirconium catalyst in the step iii include zirconic acid, zirconic acid salts, zirconium oxide, zirconium carbide, zirconium chloride, and a mixture thereof,
still more preferably zirconium dioxide, zirconium(I) chloride, zirconium(IV) chloride, zirconium chloride oxide, etc., and a mixture thereof, and particularly preferably zirconium chloride oxide.

From the viewpoint of yield, suppression of by-products, economic efficiency, etc., more preferred examples of the metal catalyst in the step iii include tungstic acid, tungstic acid salts, metal tungsten oxide, tungsten carbide, tungsten chloride and salts thereof, and a mixture thereof,
molybdic acid, molybdic acid salts, metal molybdenum, molybdenum carbide, molybdenum oxide, molybdenum chloride, and a mixture thereof,
niobic acid, niobic acid salts, metal niobium, niobium carbide, niobium oxide, niobium chloride, etc., and a mixture thereof,
tantalic acid, tantalic acid salts, tantalum oxide, tantalum carbide, tantalum chloride, and a mixture thereof, titanic acid, titanic acid salts, titanium oxide, titanium carbide, titanium chloride, and a mixture thereof, zirconic acid, zirconic acid salts, zirconium oxide, zirconium carbide, zirconium chloride, and a mixture thereof.

From the viewpoint of yield, suppression of by-products, economic efficiency, etc., more preferred examples of the metal catalyst in the step iii include tungstic acid, tungstic acid salts, metal tungsten oxide, tungsten carbide, tungsten chloride and salts thereof, and a mixture thereof,
molybdic acid, molybdic acid salts, metal molybdenum, molybdenum carbide, molybdenum oxide, molybdenum chloride, and a mixture thereof,
niobic acid, niobic acid salts, metal niobium, niobium carbide, niobium oxide, niobium chloride, etc., and a mixture thereof,
tantalic acid, tantalic acid salts, tantalum oxide, tantalum carbide, tantalum chloride, and a mixture thereof.

From the viewpoint of yield, suppression of by-products, economic efficiency, etc., more preferred examples of the metal catalyst in the step iii include tungstic acid, tungstic acid salts, metal tungsten, tungsten oxide, tungsten carbide, tungsten chloride and salts thereof, and a mixture thereof,
molybdic acid, molybdic acid salts, metal molybdenum, molybdenum carbide, molybdenum oxide, molybdenum chloride, and a mixture thereof,
niobic acid, niobic acid salts, metal niobium, niobium carbide, niobium oxide, niobium chloride, etc., and a mixture thereof.

From the viewpoint of yield, suppression of by-products, economic efficiency, etc., more preferred examples of the metal catalyst in the step iii include tungstic acid, tungstic acid salts, metal tungsten oxide, tungsten carbide, tungsten chloride and salts thereof, and a mixture thereof,
molybdic acid, molybdic acid salts, metal molybdenum, molybdenum carbide, molybdenum oxide, molybdenum chloride, and a mixture thereof.

From the viewpoint of yield, suppression of by-products, economic efficiency, etc., more preferred examples of the metal catalyst in the step iii include tungstic acid, sodium tungstate, potassium tungstate, calcium tungstate, ammonium tungstate, metal tungsten, tungsten(VI) oxide, tungsten carbide, and a mixture thereof,
molybdic acid, sodium molybdate, potassium molybdate, ammonium molybdate, molybdenum(VI) oxide, molybdenum carbide, molybdenum(V) chloride, molybdenum(IV) sulfide, phosphomolybdic acid, sodium phosphomolybdate, ammonium phosphomolybdate, silicomolybdic acid, sodium silicomolybdate, and a mixture thereof,
sodium niobate, potassium niobate, niobium carbide, niobium(V) chloride, niobium(V) pentaethoxide, etc., and a mixture thereof,
lithium tantalate, potassium tantalate, tantalum pentoxide, tantalum carbide, tantalum(V) chloride, tantalum(V) pentaethoxide, etc., and a mixture thereof,
titanium acetylacetonate, titanium tetrachloride, titanium trichloride, titanium(IV) tetraisopropoxide, etc., and a mixture thereof,
zirconium dioxide, zirconium(I) chloride, zirconium(IV) chloride, zirconium chloride oxide, etc., and a mixture thereof.

From the viewpoint of yield, suppression of by-products, economic efficiency, etc., more preferred examples of the metal catalyst in the step iii include tungstic acid, sodium tungstate, potassium tungstate, calcium tungstate, ammonium tungstate, metal tungsten, tungsten(VI) oxide, tungsten carbide, and a mixture thereof,
molybdic acid, sodium molybdate, potassium molybdate, ammonium molybdate, molybdenum(VI) oxide, molybdenum carbide, molybdenum(V) chloride, molybdenum(IV) sulfide, phosphomolybdic acid, sodium phosphomolybdate, ammonium phosphomolybdate, silicomolybdic acid, sodium silicomolybdate, and a mixture thereof,
sodium niobate, potassium niobate, niobium carbide, niobium(V) chloride, niobium(V) pentaethoxide, etc., and a mixture thereof,
lithium tantalate, potassium tantalate, tantalum pentoxide, tantalum carbide, tantalum(V) chloride, tantalum(V) pentaethoxide, etc., and a mixture thereof.

From the viewpoint of yield, suppression of by-products, economic efficiency, etc., more preferred examples of the metal catalyst in the step iii include tungstic acid, sodium tungstate, potassium tungstate, calcium tungstate, ammonium tungstate, metal tungsten, tungsten(VI) oxide, tungsten carbide, and a mixture thereof,
molybdic acid, sodium molybdate, potassium molybdate, ammonium molybdate, molybdenum(VI) oxide, molybdenum carbide, molybdenum(V) chloride, molybdenum(IV) sulfide, phosphomolybdic acid, sodium phosphomolybdate, ammonium phosphomolybdate, silicomolybdic acid, sodium silicomolybdate, and a mixture thereof,
sodium niobate, potassium niobate, niobium carbide, niobium(V) chloride, niobium(V) pentaethoxide, etc., and a mixture thereof.

From the viewpoint of yield, suppression of by-products, economic efficiency, etc., more preferred examples of the metal catalyst in the step iii include tungstic acid, sodium tungstate, potassium tungstate, calcium tungstate, ammonium tungstate, metal tungsten, tungsten(VI) oxide, tungsten carbide, and a mixture thereof,
molybdic acid, sodium molybdate, potassium molybdate, ammonium molybdate, molybdenum(VI) oxide, molybdenum carbide, molybdenum(V) chloride, molybdenum(IV) sulfide, phosphomolybdic acid, sodium phosphomolybdate, ammonium phosphomolybdate, silicomolybdic acid, sodium silicomolybdate, and a mixture thereof.

From the viewpoint of yield, suppression of by-products, economic efficiency, etc., more preferred examples of the metal catalyst in the step iii include tungstic acid, sodium tungstate, potassium tungstate, calcium tungstate, ammonium tungstate, metal tungsten, tungsten oxide, tungsten carbide,
sodium molybdate, potassium molybdate, ammonium molybdate, sodium niobate, potassium niobate,
lithium tantalate and potassium tantalate.

From the viewpoint of yield, suppression of by-products, economic efficiency, etc., more preferred examples of the metal catalyst in the step iii include tungstic acid, sodium tungstate, potassium tungstate, calcium tungstate, ammonium tungstate, metal tungsten, tungsten oxide, tungsten carbide,
sodium molybdate, potassium molybdate, ammonium molybdate, sodium niobate and potassium niobate.

From the viewpoint of yield, suppression of by-products, economic efficiency, etc., more preferred examples of the metal catalyst in the step iii include tungstic acid, sodium tungstate, potassium tungstate, calcium tungstate, ammonium tungstate, metal tungsten, tungsten oxide, tungsten carbide,
sodium molybdate, potassium molybdate and ammonium molybdate.

From the viewpoint of yield, suppression of by-products, economic efficiency, etc., more preferred examples of the metal catalyst in the step iii include tungstic acid, sodium tungstate,
sodium molybdate, potassium molybdate, ammonium molybdate, sodium niobate, potassium niobate,
lithium tantalate and potassium tantalate.

From the viewpoint of yield, suppression of by-products, economic efficiency, etc., more preferred examples of the metal catalyst in the step iii include tungstic acid, sodium tungstate,
sodium molybdate, potassium molybdate, ammonium molybdate, sodium niobate and potassium niobate.

From the viewpoint of yield, suppression of by-products, economic efficiency, etc., more preferred examples of the metal catalyst in the step iii include tungstic acid, sodium tungstate,
sodium molybdate, potassium molybdate and ammonium molybdate.

From the viewpoint of yield, suppression of by-products, economic efficiency, etc., particularly preferred examples of the metal catalyst in the step iii include sodium tungstate, ammonium molybdate and sodium niobate.

From the viewpoint of yield, suppression of by-products, economic efficiency, etc., sodium tungstate and ammonium molybdate are particularly preferred as the metal catalyst in the step iii.

### (Acid Catalyst in Step iii)

The reaction in the step iii may be performed in the presence of an acid catalyst. The acid catalyst may not be used. Whether or not to use an acid catalyst can be appropriately determined by a person skilled in the art. Examples of the acid catalyst in the step iii include, but are not limited to, the following: hydrochloric acid, sulfuric acid, methanesulfonic acid, trifluoromethanesulfonic acid, benzenesulfonic acid, p-toluenesulfonic acid, phosphoric acid, methyl phosphate, ethyl phosphate or phenyl phosphate, preferably sulfuric acid, phosphoric acid or phenyl phosphate, more preferably sulfuric acid or phenyl phosphate, and still more preferably phenyl phosphate.

The acid catalyst in the step iii may be used singly or in a combination of two or more kinds thereof in any ratio. The form of the acid catalyst in the step iii may be any form as long as the reaction proceeds. The form of the acid catalyst can be appropriately selected by a person skilled in the art. The amount of the acid catalyst used in the step iii may be any amount as long as the reaction proceeds. The amount of the acid catalyst used may be appropriately adjusted by a person skilled in the art. However, from the viewpoint of yield, suppression of by-products, economic efficiency, etc., in one embodiment, the amount of the acid catalyst used is, for example, 0 (zero) to 0.1 mol, and preferably 0 (zero) to 0.05 mol, based on 1 mol of the compound of the formula (4) (raw material). In another embodiment, the amount of the acid catalyst used is, for example, 0.001 to 0.1 mol, preferably 0.005 to 0.1 mol, more preferably 0.005 to 0.05 mol, and still more preferably 0.01 to 0.05 mol, based on 1 mol of the compound of the formula (4) (raw material).

### (Phase Transfer Catalyst in Step iii)

The reaction in the step iii may be performed in the presence of a phase transfer catalyst. The phase transfer catalyst may not be used. Whether or not to use a phase transfer catalyst can be appropriately determined by a person skilled in the art. Examples of the phase transfer catalyst in the step iii include, but are not limited to, the following: quaternary ammonium salts (e.g., tetrabutylammonium chloride, tetrabutylammonium bromide, tetrabutylammonium iodide, tetrabutylammonium hydrogen sulfate, benzyltrimethylammonium chloride, benzyltrimethylammonium bromide, octyltrimethylammonium chloride, octyltrimethylammonium bromide, trioctylmethylammonium chloride, trioctylmethylammonium bromide, benzyllauryldimethylammonium chloride (benzyldodecyldimethylammonium chloride), benzyllauryldimethylammonium bromide (benzyldodecyldimethylammonium bromide), myristyltrimethylammonium chloride (tetradecyltrimethylammonium chloride), myristyltrimethylammonium bromide (tetradecyltrimethylammonium bromide), benzyldimethylstearylammonium chloride (benzyloctadecyldimethylammonium chloride), benzyldimethylstearylammonium bromide (benzyloctadecyldimethylammonium bromide), etc.), quaternary phosphonium salts (tetrabutylphosphonium bromide, tetraoctylphosphonium bromide, tetraphenylphosphonium bromide, etc.) and crown ethers (e.g., 12-crown-4, 15-crown-5 and 18-crown-6). From the viewpoint of yield, suppression of by-products, economic efficiency, etc., preferred examples of the phase transfer catalyst in the step iii include tetrabutylammonium chloride, tetrabutylammonium bromide and tetrabutylammonium hydrogen sulfate, and more preferably tetrabutylammonium hydrogen sulfate. Tetrabutylammonium hydrogen sulfate may be abbreviated as TBAHS.

The phase transfer catalyst in the step iii may be used singly or in a combination of two or more kinds thereof in any ratio. The form of the phase transfer catalyst in the step iii may be any form as long as the reaction proceeds. The form of the phase transfer catalyst can be appropriately selected by a person skilled in the art. The amount of the phase transfer catalyst used in the step iii may be any amount as long as the reaction proceeds. The amount of the phase transfer catalyst used may be appropriately adjusted by a person skilled in the art. However, from the viewpoint of yield, suppression of by-products, economic efficiency, etc., in one embodiment, the amount of the phase transfer catalyst used is, for example, 0 (zero) to 0.1 mol, and preferably 0 (zero) to 0.05 mol, based on 1 mol of the compound of the formula (4) (raw material). In another embodiment, the amount of the phase transfer catalyst used is, for example, 0.001 to 0.1 mol, preferably 0.005 to 0.05 mol, and more preferably 0.01 to 0.05 mol, based on 1 mol of the compound of the formula (4) (raw material).

For example, when water and an alcohol-based solvent such as butanol are used as the solvent in the step iii, the reaction is preferably performed in the presence of an acid catalyst and a phase transfer catalyst. However, in a mixed solvent of water and a certain organic solvent, the reaction sufficiently proceeds in the absence of an acid catalyst and a phase transfer catalyst.

### (Reaction Solvent in Step iii)

From the viewpoint of allowing the reaction to smoothly proceed, the reaction in the step iii is preferably performed in the presence of a solvent. The solvent in the reaction in the step iii may be any solvent as long as the reaction proceeds.

In one embodiment, examples of the solvent in the reaction in the step iii include, but are not limited to, the following: organic solvents having an acceptor number from 0 (zero) to 50 (preferably 3 to 45, more preferably 5 to 45, still more preferably 5 to 35, further preferably 5 to 30, further preferably 5 to 20, and further preferably 8 to 20), water, and any combination thereof in any ratio.

In another aspect, examples of the solvent in the reaction in the step iii include, but are not limited to, the following: aromatic hydrocarbon derivatives (e.g., benzene, toluene, xylenes, chlorobenzene, dichlorobenzenes, trichlorobenzenes and nitrobenzene), halogenated aliphatic hydrocarbons (e.g., dichloromethane and 1,2-dichloroethane (EDC)), alcohols (e.g., methanol, ethanol, propanol, 2-propanol, butanol, sec-butanol, isobutanol and tert-butanol (tert-butanol is also referred to as tert-butyl alcohol), pentanol, sec-amyl alcohol, 3-pentanol, 2-methyl-1-butanol, isoamyl alcohol, tert-amyl alcohol, hexanol and cyclohexanol), nitriles (e.g., acetonitrile and propionitrile), carboxylic acid esters (e.g., methyl acetate, ethyl acetate, propyl acetate, isopropyl acetate, butyl acetate and isomers thereof and pentyl acetate and isomers thereof (in the present invention, the "isomer of butyl acetate" is an equivalent of "butyl acetate" and the "isomer of pentyl acetate" is an equivalent of "pentyl acetate")), ethers (e.g., tetrahydrofuran (THF), 1,4-dioxane, diisopropyl ether, dibutyl ether, di-tert-butyl ether, cyclopentyl methyl ether (CPME), methyl tert-butyl ether, 1,2-dimethoxyethane (DME) and diglyme), ketones (e.g., acetone, methyl ethyl ketone (MEK), methyl isopropyl ketone (MIPK) and methyl isobutyl ketone (MIBK)), amides (e.g., N,N-dimethylformamide (DMF), N,N-dimethylacetamide (DMAC) and N-methylpyrrolidone (NMP)), ureas (e.g., N,N'-dimethylimidazolidinone (DMI) and tetramethylurea), sulfoxides (e.g., dimethyl sulfoxide (DMSO)), sulfones (e.g., sulfolane),
water, and any combination thereof in any ratio. "2-Propanol" is also referred to as "isopropyl alcohol" or "isopropanol".

However, from the viewpoint of yield, suppression of by-products, economic efficiency, etc., preferred examples of the solvent in the reaction in the step iii include combinations of one or more (preferably one or two, more preferably one) organic solvents selected from aromatic hydrocarbon derivatives, halogenated aliphatic hydrocarbons, alcohols, nitriles, carboxylic acid esters, ethers, ketones, amides, ureas, sulfoxides and sulfones with a water solvent in any ratio.

More preferred examples of the solvent in the reaction in the step iii include combinations of one or more (preferably one or two, more preferably one) organic solvents selected from alcohols, nitriles, carboxylic acid esters, ethers, amides and sulfones with a water solvent in any ratio.

More preferred examples of the solvent in the reaction in the step iii include combinations of one or more (preferably one or two, more preferably one) organic solvents selected from alcohols, nitriles, carboxylic acid esters, ethers and amides with a water solvent in any ratio.

More preferred examples of the solvent in the reaction in the step iii include combinations of one or more (preferably one or two, more preferably one) organic solvents selected from alcohols, nitriles, carboxylic acid esters and amides with a water solvent in any ratio.

More preferred examples of the solvent in the reaction in the step iii include combinations of one or more (preferably one or two, more preferably one) organic solvents selected from alcohols, nitriles and carboxylic acid esters with a water solvent in any ratio.

Still more preferred examples of the solvent in the reaction in the step iii include combinations of one or more (preferably one or two, more preferably one) organic solvents selected from nitriles and carboxylic acid esters with a water solvent in any ratio.

In one embodiment, particularly preferred examples of the solvent in the reaction in the step iii include combinations of nitriles with a water solvent in any ratio.

In another embodiment, particularly preferred examples of the solvent in the reaction in the step iii include combinations of carboxylic acid esters with a water solvent in any ratio.

From the same viewpoint as described above, preferred specific examples of the solvent in the reaction in the step iii include combinations of one or more (preferably one or two, more preferably one) organic solvents selected from toluene, xylenes, chlorobenzene, dichlorobenzenes, dichloromethane, 1,2-dichloroethane, methanol, ethanol, propanol, 2-propanol, butanol, sec-butanol, isobutanol, tert-butanol, pentanol, sec-amyl alcohol, 3-pentanol, 2-methyl-1-butanol, isoamyl alcohol, tert-amyl alcohol, acetonitrile, ethyl acetate, propyl acetate, isopropyl acetate, butyl acetate and isomers thereof (in the present invention, the "isomer of butyl acetate" is an equivalent of "butyl acetate"), tetrahydrofuran (THF), 1,4-dioxane, diisopropyl ether, dibutyl ether, di-tert-butyl ether, cyclopentyl methyl ether (CPME), methyl-tert-butyl ether, 1,2-dimethoxyethane (DME), diglyme, acetone, methyl ethyl ketone (MEK), methyl isopropyl ketone (MIPK), methyl isobutyl ketone (MIBK), N,N-dimethylformamide (DMF), N,N-dimethylacetamide (DMAC), N-methylpyrrolidone (NMP), N,N'-dimethylimidazolidinone (DMI), tetramethylurea, dimethyl sulfoxide (DMSO) and sulfolane with a water solvent in any ratio.

From the same viewpoint as described above, more preferred specific examples of the solvent in the reaction in the step iii include combinations of one or more (preferably one or two, more preferably one) organic solvents selected from toluene, xylenes, chlorobenzene, dichlorobenzenes, dichloromethane, 1,2-dichloroethane, methanol, ethanol, propanol, 2-propanol, butanol, sec-butanol, isobutanol, tert-butanol, acetonitrile, ethyl acetate, propyl acetate, isopropyl acetate, butyl acetate and isomers thereof (in the present invention, the "isomer of butyl acetate" is an equivalent of "butyl acetate"), tetrahydrofuran (THF), 1,4-dioxane, diisopropyl ether, dibutyl ether, di-tert-butyl ether, cyclopentyl methyl ether (CPME), methyl-tert-butyl ether, 1,2-dimethoxyethane (DME), diglyme, acetone, methyl ethyl ketone (MEK), methyl isopropyl ketone (MIPK), methyl isobutyl ketone (MIBK), N,N-dimethylformamide (DMF), N,N-dimethylacetamide (DMAC), N-methylpyrrolidone (NMP), N,N'-dimethylimidazolidinone (DMI), tetramethylurea, dimethyl sulfoxide (DMSO) and sulfolane with a water solvent in any ratio.

From the same viewpoint as described above, still more preferred specific examples of the solvent in the reaction in the step iii include combinations of one or more (preferably one or two, more preferably one) organic solvents selected from toluene, xylenes, chlorobenzene, dichlorobenzenes, dichloromethane, 1,2-dichloroethane, methanol, ethanol, 2-propanol, butanol, tert-butanol, acetonitrile, ethyl acetate, propyl acetate, isopropyl acetate, butyl acetate and isomers thereof (in the present invention, the "isomer of butyl acetate" is an equivalent of "butyl acetate"), tetrahydrofuran (THF), 1,4-dioxane, diisopropyl ether, dibutyl ether, di-tert-butyl ether, cyclopentyl methyl ether (CPME), methyl-tert-butyl ether, 1,2-dimethoxyethane (DME), diglyme, acetone, methyl ethyl ketone (MEK), methyl isopropyl ketone (MIPK), methyl isobutyl ketone (MIBK), N,N-dimethylformamide (DMF), N,N-dimethylacetamide (DMAC), N-methylpyrrolidone (NMP), N,N'-dimethylimidazolidinone (DMI), tetramethylurea, dimethyl sulfoxide (DMSO) and sulfolane with a water solvent in any ratio.

More preferred specific examples of the solvent in the reaction in the step iii include combinations of one or more (preferably one or two, more preferably one) organic solvents selected from methanol, ethanol, 2-propanol, butanol, tert-butanol, acetonitrile, ethyl acetate, propyl acetate, isopropyl acetate, butyl acetate, 1,4-dioxane, diglyme, N,N-dimethylformamide (DMF), N-methylpyrrolidone (NMP), sulfolane and isomers thereof with a water solvent in any ratio.

More preferred specific examples of the solvent in the reaction in the step iii include combinations of one or more (preferably one or two, more preferably one) organic solvents selected from methanol, ethanol, 2-propanol, butanol, tert-butanol, acetonitrile, ethyl acetate, propyl acetate, isopropyl acetate, butyl acetate, N,N-dimethylformamide (DMF), N-methylpyrrolidone (NMP) and isomers thereof with a water solvent in any ratio.

More preferred specific examples of the solvent in the reaction in the step iii include combinations of one or more (preferably one or two, more preferably one) organic solvents selected from methanol, ethanol, 2-propanol, butanol, tert-butanol, acetonitrile, ethyl acetate, propyl acetate, isopropyl acetate, butyl acetate and isomers thereof with a water solvent in any ratio.

Still more preferred specific examples of the solvent in the reaction in the step iii include combinations of one or more (preferably one or two, more preferably one) organic solvents selected from butanol, acetonitrile, ethyl acetate, propyl acetate, isopropyl acetate and butyl acetate with a water solvent in any ratio.

Still more preferred specific examples of the solvent in the reaction in the step iii include combinations of one or more (preferably one or two, more preferably one) organic solvents selected from ethanol, 2-propanol, butanol, tert-butanol and acetonitrile with a water solvent in any ratio.

Further preferred specific examples of the solvent in the reaction in the step iii include combinations of one or more (preferably one or two, more preferably one) organic solvents selected from acetonitrile, ethyl acetate, isopropyl acetate and butyl acetate with a water solvent in any ratio.

Further preferred specific examples of the solvent in the reaction in the step iii include combinations of one or two (preferably one) organic solvents selected from acetonitrile and butyl acetate with a water solvent in any ratio.

In one embodiment, particularly preferred specific examples of the solvent in the reaction in the step iii include combinations of an acetonitrile solvent with a water solvent in any ratio.

In another embodiment, particularly preferred specific examples of the solvent in the reaction in the step iii include combinations of a butyl acetate with a water solvent in any ratio.

In any case, the solvent may be in a single layer or may be separated into two layers as long as the reaction proceeds.

The amount of the solvent used in the reaction in the step iii will be described. The "total amount of the solvent used in the reaction" is the sum total of the amounts of all the organic solvents and the amount of the water solvent used in the reaction. The organic solvent and the water solvent used in the working-up (e.g., isolation and purification) after the reaction are not included. The "organic solvent" used in the reaction includes the organic solvent in the raw material solution and that in the reactant solution. The "water solvent" used in the reaction includes the water in the raw material solution and that in the reactant solution (e.g., water in an aqueous hydrogen peroxide solution).

The total amount of solvent used in the reaction in the step iii is not particularly limited as long as the reaction system can be sufficiently stirred. However, from the viewpoints of yield, suppression of by-products, economic efficiency, etc., the total amount of the solvent used in the reaction in the step iii is, for example, 0.1 to 10 L (liters) 0.3 to 5 L, preferably 0.3 to 3 L, more preferably 0.5 to 3 L, still more preferably 0.5 to 2 L, and further preferably 0.7 to 1.8 L, based on 1 mol of the compound of the formula (4) (raw material).

From the same viewpoint as described above, in one embodiment, the amount of the organic solvent used in the reaction in the step iii is, for example, 0.1 to 10 L (liters), preferably 0.2 to 5 L, more preferably 0.3 to 2 L, and still more preferably 0.3 to 1 L, based on 1 mol of the compound of the formula (4) (raw material).

In another embodiment, the amount of the organic solvent used in the reaction in the step iii is, for example, 0.4 to 5 L, preferably 0.4 to 2 L, more preferably 0.4 to 1.5 L, and still more preferably 0.4 to 1.3 L, based on 1 mol of the compound of the formula (4) (raw material). In another embodiment, the amount of the organic solvent used in the reaction in the step iii is, for example, 0.5 to 5L (liters), preferably 0.5 to 2 L, more preferably 0.5 to 1.5 L, and still more preferably 0.5 to 1.3 L, based on 1 mol of the compound of the formula (4) (raw material).

From the same viewpoint as described above, in one embodiment, the amount of the water solvent used in the reaction in the step iii is, for example, 0.01 to 2 L (liters), preferably 0.05 to 1 L, more preferably 0.1 to 1 L, and still more preferably 0.2 to 1 L, based on 1 mol of the compound of the formula (4) (raw material).

In another embodiment, the amount of the water solvent used in the reaction in the step iii is, for example, 0.05 to 0.8 L (liters), preferably 0.1 to 0.8 L, more preferably 0.2 to 0.8 L, and still more preferably 0.3 to 0.8 L, based on 1 mol of the compound of the formula (4) (raw material). In another embodiment, the amount of the water solvent used in the reaction in the step iii is, for example, 0.05 to 0.5 L (liters), preferably 0.1 to 0.5 L, more preferably 0.2 to 0.5 L, and still more preferably 0.3 to 0.5 L, based on 1 mol of the compound of the formula (4) (raw material). In another embodiment, the amount of the water solvent used in the reaction in the step iii is, for example, 0.1 to 0.3 L (liters), and preferably 0.2 to 0.3 L, based on 1 mol of the compound of the formula (4) (raw material).

When a combination of two or more organic solvents is used, the ratio of the two or more organic solvents may be any ratio as long as the reaction proceeds.

When a combination of an organic solvent and a water solvent is used, the ratio of the organic solvent to the water solvent may be any ratio as long as the reaction proceeds. However, from the viewpoint of yield, suppression of by-products, economic efficiency, etc., the ratio of the organic solvent to the water solvent is, for example, 90 : 10 to 10 : 90 by volume ratio, preferably 90 : 10 to 50 : 50 by volume ratio, more preferably 85 : 15 to 55 : 45 by volume ratio, still more preferably 80 : 20 to 60 : 40 by volume ratio, and further preferably 75 : 25 to 60 : 40 by volume ratio.

In other words, the amount of the water solvent in the whole solvent composed of the organic solvent and the water solvent is, for example, 10 vol% to 90 vol%, preferably 10 vol% to 50 vol%, more preferably 15 vol% to 45 vol%, still more preferably 20 vol% to 40 vol%, and further preferably 25 vol% to 40 vol%, based on the amount of the whole solvent (100 vol%).

### (Pre-added Water in Step iii)

The "pre-added water" means water previously added as a solvent in the reaction system before the aqueous hydrogen peroxide solution is added. From the viewpoint of safety, smooth start of reaction, stable reaction, etc., and from the viewpoint described later, in one embodiment, the amount of the pre-added water used is, for example, 0 (zero) to 1 L (liter), preferably 0.01 to 1 L, more preferably 0.05 to 1 L, still more preferably 0.08 to 1 L, and further preferably 0.1 to 1 L, based on 1 mol of the compound of the formula (4). In another embodiment, the amount of the pre-added water used is, for example, 0 (zero) to 0.5 L (liters), preferably 0.01 to 0.5 L, more preferably 0.05 to 0.5 L, still more preferably 0.08 to 0.5 L, and further preferably 0.1 to 0.5 L, based on 1 mol of the compound of the formula (4). In another embodiment, the amount of the pre-added water used is, for example, 0 (zero) to 0.4 L (liters), preferably 0.01 to 0.4 L, more preferably 0.05 to 0.4 L, still more preferably 0.08 to 0.2 L, and further preferably 0.1 to 0.2 L, based on 1 mol of the compound of the formula (4). In another embodiment, the amount of the pre-added water used is, for example, 0 (zero) to 0.3 L (liters), preferably 0.01 to 0.3 L, more preferably 0.05 to 0.3 L, still more preferably 0.08 to 0.3 L, and further preferably 0.1 to 0.3 L, based on 1 mol of the compound of the formula (4).

### (Reaction Temperature in Step iii)

The reaction temperature in the step iii is not particularly limited. However, from the viewpoint of yield, suppression of by-products, economic efficiency, etc., in one embodiment, the reaction temperature in the step iii is, for example, 0 (zero) °C to 100°C, preferably 20°C to 100°C, more preferably 50°C to 90°C, preferably 60°C to 90°C, still more preferably 70°C to 90°C, further preferably 75°C to 90°C, and further preferably 75°C to 80°C. From the same viewpoint, in another embodiment, the reaction temperature in the step iii is, for example, 50°C to 150°C, 50°C to 120°C, or 50°C to 100°C, and preferably 60°C to 150°C, 60°C to 120°C, or 60°C to 100°C.

### (Reaction Time in Step iii)

The reaction time in the step iii is not particularly limited. However, from the viewpoint of yield, suppression of by-products, economic efficiency, etc., the reaction time in the step iii is, for example, 1 hour to 48 hours, preferably 3 hours to 48 hours, more preferably 3 hours to 24 hours, still more preferably 4 hours to 24 hours, further preferably 4 hours to 12 hours, and further preferably 4 hours to 8 hours. However, the reaction time can be adjusted appropriately by a person skilled in the art.

### (Adding Method in Step iii)

The order of adding the raw materials, the oxidizing agent, the catalyst, the solvent, etc. is not particularly limited. As long as the reaction proceeds, the addition order thereof may be any order. However, it has been found that when water as a solvent and a catalyst are added in advance into the reaction system before an aqueous hydrogen peroxide solution is added, the reaction can be smoothly started and the initial stage of the reaction is stabilized. This means that a safer and industrially preferable process has been found. Therefore, it is preferable to add water as a solvent into the reaction system before adding an aqueous hydrogen peroxide solution. That is, it is preferable to use the above-described "pre-added water".

### (Working-up in Step iii; Isolation and Purification)

The compounds of the formula (5), especially pyroxasulfone (5-a), which is the target product in the step iii, can be isolated and purified from the reaction mixture by methods known to a person skilled in the art (e.g., extraction, washing, crystallization including recrystallization, crystal washing and/or other procedures) and improved methods thereof, and any combination thereof.

In the step iii, as shown in Examples, it is preferable to decompose an unreacted peroxide such as hydrogen peroxide by treating the reaction mixture with a reducing agent (e.g., an aqueous sodium sulfite solution) after the reaction.

In the working-up step (isolation and/or purification), the following procedures may be performed, but are not limited thereto: in the working-up, an extraction procedure and/or a washing procedure including separation of an organic layer and an aqueous layer may be performed. When the mixture is separated into an organic layer and an aqueous layer, the mixture may be separated while being hot. For example, when separating the organic layer from the aqueous layer, a hot mixture may be used, or the mixture may be heated. Impurities may be removed by a filtration procedure including hot filtration.

In the working-up, crystallization of the target product including recrystallization and washing of crystals may be performed. The crystallization of the target product including recrystallization may be performed by a conventional method known to a person skilled in the art. For example, an antisolvent may be added to a solution of the target product in a good solvent. As another example, a saturated solution of the target product may be cooled.

For still another example, from the solution of the target product in an organic solvent (including the reaction mixture), the solvent may be removed. In this case, examples of the organic solvent that can be used include the examples, the preferred examples, the more preferred examples, and the further preferred examples of the water-miscible organic solvent described later. The organic solvent may be removed after adding water in advance into the system. In this case, the organic solvent may be removed by azeotropy with the water. The organic solvent may be removed under heating, under reduced pressure and under normal pressure. As still another example, water may be added to a solution of the target product in a water-miscible organic solvent. Examples of the water-miscible organic solvent include, but are not limited to, alcohols (e.g., methanol, ethanol, 2-propanol, butanol and t-butanol), nitriles (e.g., acetonitrile), ethers (e.g., tetrahydrofuran (THF) and 1,4-dioxane), ketones (e.g., acetone), amides (e.g., N,N-dimethylformamide (DMF), N,N-dimethylacetamide (DMAC) and N-methylpyrrolidone (NMP)), sulfoxides (e.g., dimethyl sulfoxide (DMSO)), and combinations thereof, preferably methanol, ethanol, 2-propanol, butanol, acetonitrile, acetone, and combinations thereof, and more preferably ethanol, 2-propanol, butanol, acetonitrile, and combinations thereof. The "water-miscible organic solvent" has the same meaning as "water-soluble organic solvent". "2-Propanol" is also referred to as "isopropyl alcohol" or "isopropanol".

In any of the above cases, a seed crystal may be used.

In the crystal washing procedure, the crystals collected by filtration may be washed with a solvent. A suspension (slurry) of crystals may be stirred and then filtered. In any case, examples of the solvent that can be used include the examples, the preferred examples, the more preferred examples, the further preferred examples of the water-miscible organic solvent described above and water.

In any of the above cases (crystallization procedures including recrystallization, crystal washing procedure, etc.), the amount of the solvent such as the water-miscible organic solvent and the amount of water may be at any ratio as long as the purpose is achieved. When a combination of a water-miscible organic solvent and water is employed, the ratio thereof may be any ratio as long as the purpose is achieved. When a combination of two or more solvents such as water-miscible organic solvents is employed, the ratio thereof may be any ratio as long as the purpose is achieved. Their amounts and ratios can be appropriately adjusted by a person skilled in the art depending on the purpose and situation.

In any of the above procedures (extraction procedure, washing procedure, crystallization procedures including recrystallization, crystal washing procedure, etc.), the temperature can be appropriately adjusted by a person skilled in the art. However, from the viewpoint of yield, purity, economic efficiency, etc., for example, the temperature is 0°C (zero °C) to 100°C, preferably 5°C to 90°C, and more preferably 10°C to 80°C. Heating and cooling may be performed in these temperature ranges.

In any of the above procedures (extraction procedure, washing procedure, crystallization procedures including recrystallization, crystal washing procedure, etc.), the amount of the organic solvent (including the water-miscible organic solvent) and/or water can be appropriately adjusted by a person skilled in the art by addition and removal thereof. Furthermore, recovery and recycling of the solvent may be optionally performed. For example, the recovery and recycle of the solvent used in the reaction may be performed, and the recovery and recycle of the solvent used in the working-up (isolation and/or purification) may be performed.

Working-up (isolation and/or purification) can be performed by appropriately combining all or some of the procedures described above. Optionally, the above procedures may be repeated according to the purpose such as isolation and/or purification. In addition, a person skilled in the art can appropriately select a combination of any of the above procedures and their order.

### (Crystal of Pyroxasulfone)

One aspect of the present invention includes a novel crystal of pyroxasulfone and a process for producing the same.

The process of crystallization of the pyroxasulfone is described below.

The crystal of pyroxasulfone of the present invention is identified by a characteristic pattern observed in a powder X-ray diffraction spectrum obtained by measurement by the transmission method using Cu-Kα rays.

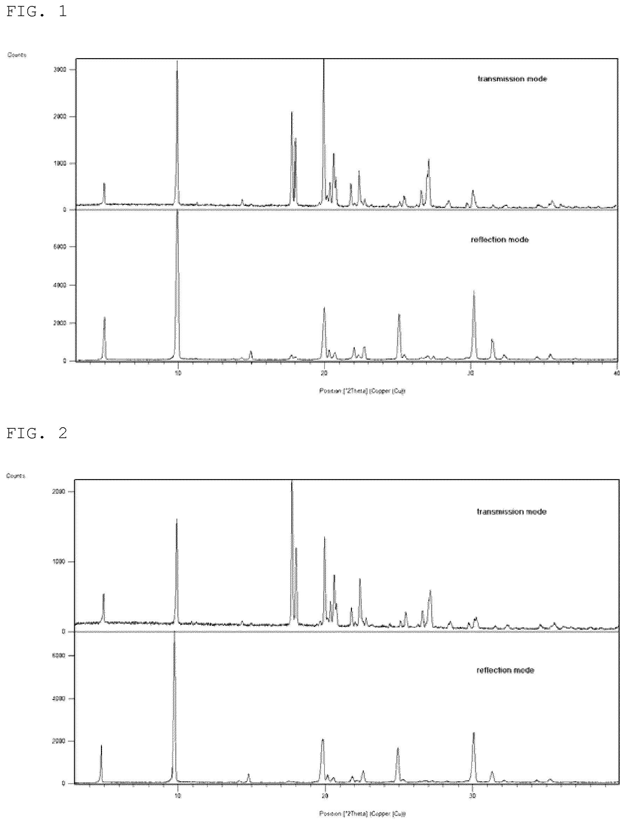

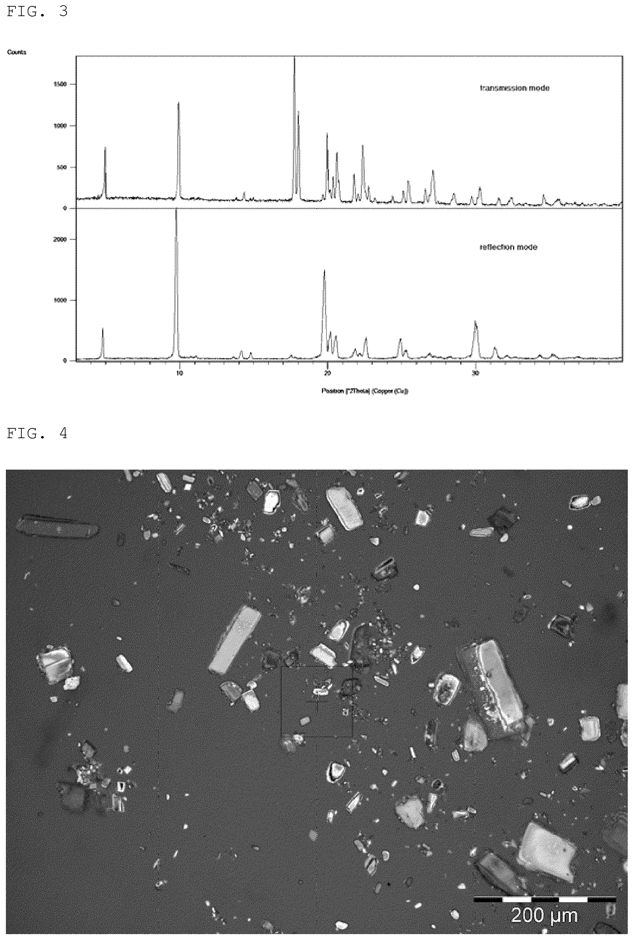

One example of a powder X-ray diffraction spectrum of a crystal of pyroxasulfone of the present invention is shown in FIG. 1. In addition, powder X-ray diffraction spectra of crystals of pyroxasulfone produced by the processes disclosed in Patent Documents 2 and 10 are shown in FIGs. 2 and 3. Of the upper and lower two charts shown in each figure, the upper one is a powder X-ray diffraction spectrum measured by the transmission method, and the lower one is a powder X-ray diffraction spectrum measured by the reflection method. In the present invention, powder X-ray diffraction spectra measured by the transmission method are employed in the discussion.

Comparing the powder X-ray diffraction spectrum measured by the transmission method for the crystal of pyroxasulfone of the present invention and that measured for the crystal of pyroxasulfone produced by a known process, they are common in that characteristic peaks are observed at diffraction angles 2Θ at least in the ranges of 17.7 to 17.8°, 18.0 to 18.1° and 19.9 to 20.0°, but they are different in terms of the order of the peak heights. Specifically, the crystal of pyroxasulfone produced by the known process has a maximum peak height at the peak of 17.7 to 17.8°, whereas the crystal of pyroxasulfone of the present invention has a maximum peak height at the peak of 19.9 to 20.0°. Herein, when there is a plurality of peaks at a peak position designated by a range, the highest peak among the plurality of peaks is recognized as the peak at the position.

Surprisingly, the difference in the order of peak intensities between those crystals of pyroxasulfone affects the physicochemical properties of the agrochemical formulations prepared using such crystals. Details of this point will be described in the section of agrochemical formulation described later.

In one embodiment, the powder X-ray diffraction spectrum of the crystal of pyroxasulfone of the present invention has characteristic peaks at diffraction angles 2Θ at least in the ranges of 17.7 to 17.8°, 18.0 to 18.1° and 19.9 to 20.0°, and the peak height at the peak of 19.9 to 20.0° is maximum among the three peaks.

The ratio of the peak height at 19.9 to 20.0° to the peak height at 17.7 to 17.8° in the powder X-ray diffraction spectrum of the crystal of pyroxasulfone of the present invention is not particularly limited, but usually includes a range of 1 : 0.01 to 1 : 0.99, preferably 1 : 0.02 to 1 : 0.95, more preferably 1 : 0.1 to 1 : 0.85, still more preferably 1 : 0.3 to 1 : 0.75, and particularly preferably 1 : 0.4 to 1 : 0.65.

The ratio of the peak height at 19.9 to 20.0° to the peak height at 18.0 to 18.1° in the powder X-ray diffraction spectrum of the crystal of pyroxasulfone of the present invention is not particularly limited, but usually includes a range of 1 : 0.01 to 1 : 0.99, preferably 1 : 0.02 to 1 : 0.95, more preferably 1 : 0.04 to 1 : 0.8, even more preferably 1 : 0.07 to 1 : 0.6, and particularly preferably 1 : 0.1 to 1 : 0.5.

In another embodiment, the powder X-ray diffraction spectrum of the crystal of pyroxasulfone of the present invention has characteristic peaks at diffraction angles 2Θ at least in the ranges of 17.7 to 17.8°, 18.0 to 18.1° and 19.9 to 20.0° and the peak height at the peak of 19.9 to 20.0° is maximum among the three peaks, and also has peaks at diffraction angles 2Θ at least in the ranges of 9.9 to 10.0°, 20.6 to 20.7° and 30.1 to 30.3°.

In another embodiment, the powder X-ray diffraction spectrum of the crystal of pyroxasulfone of the present invention has characteristic peaks at diffraction angles 2Θ at least in the ranges of 17.7 to 17.8°, 18.0 to 18.1° and 19.9 to 20.0° and the peak height at the peak of 19.9 to 20.0° is maximum among the three peaks, and also has peaks at diffraction angles 2Θ at least in the ranges of 4.9 to 5.0°, 9.9 to 10.0°, 20.6 to 20.7° and 30.1 to 30.3°.

In another embodiment, the powder X-ray diffraction spectrum of the crystal of pyroxasulfone of the present invention has characteristic peaks at diffraction angles 2Θ at least in the ranges of 17.7 to 17.8°, 18.0 to 18.1° and 19.9 to 20.0° and the peak height at the peak of 19.9 to 20.0° is maximum among the three peaks, and also has peaks at diffraction angles 2Θ at least in the ranges of 9.9 to 10.0°, 20.3 to 20.4°, 20.6 to 20.7° and 30.1 to 30.3°.

In another embodiment, the powder X-ray diffraction spectrum of the crystal of pyroxasulfone of the present invention has characteristic peaks at diffraction angles 2Θ at least in the ranges of 17.7 to 17.8°, 18.0 to 18.1° and 19.9 to 20.0° and the peak height at the peak of 19.9 to 20.0° is maximum among the three peaks, and also has peaks at diffraction angles 2Θ at least in the ranges of 9.9 to 10.0°, 20.6 to 20.7°, 21.8 to 21.9° and 30.1 to 30.3°.

In another embodiment, the powder X-ray diffraction spectrum of the crystal of pyroxasulfone of the present invention has characteristic peaks at diffraction angles 2Θ at least in the ranges of 17.7 to 17.8°, 18.0 to 18.1° and 19.9 to 20.0° and the peak height at the peak of 19.9 to 20.0° is maximum among the three peaks, and also has peaks at diffraction angles 2Θ at least in the ranges of 9.9 to 10.0°, 20.6 to 20.7°, 22.3 to 22.4° and 30.1 to 30.3°.

In another embodiment, the powder X-ray diffraction spectrum of the crystal of pyroxasulfone of the present invention has characteristic peaks at diffraction angles 2Θ at least in the ranges of 17.7 to 17.8°, 18.0 to 18.1° and 19.9 to 20.0° and the peak height at the peak of 19.9 to 20.0° is maximum among the three peaks, and also has peaks at diffraction angles 2Θ at least in the ranges of 9.9 to 10.0°, 20.6 to 20.7°, 25.4 to 25.5° and 30.1 to 30.3°.

In another embodiment, the powder X-ray diffraction spectrum of the crystal of pyroxasulfone of the present invention has characteristic peaks at diffraction angles 2Θ at least in the ranges of 17.7 to 17.8°, 18.0 to 18.1° and 19.9 to 20.0° and the peak height at the peak of 19.9 to 20.0° is maximum among the three peaks, and also has peaks at diffraction angles 2Θ at least in the ranges of 9.9 to 10.0°, 20.6 to 20.7°, 26.6 to 26.7° and 30.1 to 30.3°.

In another embodiment, the powder X-ray diffraction spectrum of the crystal of pyroxasulfone of the present invention has characteristic peaks at diffraction angles 2Θ at least in the ranges of 17.7 to 17.8°, 18.0 to 18.1° and 19.9 to 20.0° and the peak height at the peak of 19.9 to 20.0° is maximum among the three peaks, and also has peaks at diffraction angles 2Θ at least in the ranges of 9.9 to 10.0°, 20.6 to 20.7°, 26.9 to 27.0° and 30.1 to 30.3°.

In another embodiment, the powder X-ray diffraction spectrum of the crystal of pyroxasulfone of the present invention has characteristic peaks at diffraction angles 2Θ at least in the ranges of 17.7 to 17.8°, 18.0 to 18.1° and 19.9 to 20.0° and the peak height at the peak of 19.9 to 20.0° is maximum among the three peaks, and also has peaks at diffraction angles 2Θ at least in the ranges of 9.9 to 10.0°, 20.6 to 20.7°, 27.1 to 27.2° and 30.1 to 30.3°.

In another embodiment, the powder X-ray diffraction spectrum of the crystal of pyroxasulfone of the present invention has characteristic peaks at diffraction angles 2Θ at least in the ranges of 17.7 to 17.8°, 18.0 to 18.1° and 19.9 to 20.0° and the peak height at the peak of 19.9 to 20.0° is maximum among the three peaks, and also has peaks at diffraction angles 2Θ at least in the ranges of 9.9 to 10.0°, 20.6 to 20.7°, 30.1 to 30.3° and 35.5 to 35.6°.

In any of the above embodiments, the powder X-ray diffraction spectrum of the crystal of pyroxasulfone of the present invention may further have peaks at one or two or more of diffraction angles 2Θ of 11.2 to 11.4°, 14.3 to 14.4°, 14.9 to 15.1°, 17.1 to 17.3°, 19.6 to 19.7°, 20.1 to 20.2°, 20.8 to 20.9°, 22.0 to 22.1°, 22.5 to 22.6°, 22.7 to 22.8°, 23.1 to 23.3°, 24.3 to 24.5°, 25.1 to 25.2°, 26.2 to 26.3°, 26.9 to 27.0°, 27.1 to 27.2°, 27.3 to 27.4°, 27.7 to 27.9°, 28.3 to 28.4°, 28.5 to 28.6°, 29.7 to 29.8°, 30.4 to 30.5°, 31.5° to 31.6°, 32.1 to 32.3°, 32.4 to 32.5°, 32.8 to 33.0°, 33.3 to 33.4°, 34.5 to 34.7°, 34.8 to 34.9°, 35.3 to 35.4°, 36.1 to 36.2°, 36.3 to 36.4°, 36.6 to 36.7°, 37.2 to 37.3°, 38.0 to 38.1° and 38.6 to 38.7°.

In another preferred embodiment, the powder X-ray diffraction spectrum of the crystal of pyroxasulfone of the present invention has characteristic peaks at diffraction angles 2θ of 17.8°, 18.0° and 19.9° and the peak height at the peak of 19.9° is maximum among the three peaks, and also has peaks at diffraction angles 2Θ of 5.0°, 9.9°, 11.3°, 14.4°, 15.0°, 19.7°, 20.2°, 20.4°, 20.6°, 20.8°, 21.8°, 22.1°, 22.3°, 22.8°, 23.2°, 24.4°, 25.1°, 25.4°, 26.3°, 26.6°, 27.0°, 27.1°, 28.3°, 28.5°, 29.7°, 30.1°, 30.3°, 30.6°, 31.6°, 32.5°, 32.9°, 34.6°, 35.4°, 35.5°, 36.1°, 36.7°, 37.2°, 38.0° and 38.7°.

In another preferred embodiment, the powder X-ray diffraction spectrum of the crystal of pyroxasulfone of the present invention has characteristic peaks at diffraction angles 2θ of 17.7°, 18.0° and 19.9° and the peak height at the peak of 19.9° is maximum among the three peaks, and also has peaks at diffraction angles 2Θ of 9.9°, 14.4°, 20.4°, 20.6°, 20.8°, 21.8°, 22.4°, 22.5°, 25.5°, 26.3°, 26.6°, 27.0°, 27.1°, 28.4°, 30.1°, 30.3°, 32.4°, 34.7°, 35.3°, 36.1°, 36.4°, 38.0° and 38.7°.

In another preferred embodiment, the powder X-ray diffraction spectrum of the crystal of pyroxasulfone of the present invention has characteristic peaks at diffraction angles 2θ of 17.8°, 18.1° and 19.9° and the peak height at the peak of 19.9° is maximum among the three peaks, and also has peaks at diffraction angles 2Θ of 5.0°, 9.9°, 17.2°, 20.6°, 21.8°, 22.4°, 23.2°, 25.2°, 25.5°, 26.3°, 26.7°, 26.9°, 27.1°, 29.8°, 30.1°, 30.2°, 34.7°, 34.8° and 36.2°.

In another preferred embodiment, the powder X-ray diffraction spectrum of the crystal of pyroxasulfone of the present invention has characteristic peaks at diffraction angles 2θ of 17.7°, 18.0° and 19.9° and the peak height at the peak of 19.9° is maximum among the three peaks, and also has peaks at diffraction angles 2Θ of 9.9°, 20.3°, 20.6°, 20.7°, 21.8°, 22.4°, 25.4°, 27.0°, 27.1°, 30.1°, 30.4°, 31.5°, 32.4°, 33.3°, 35.5°, 36.1°, 36.2° and 36.3°.

In another preferred embodiment, the powder X-ray diffraction spectrum of the crystal of pyroxasulfone of the present invention has characteristic peaks at diffraction angles 2θ of 17.8°, 18.0° and 19.9° and the peak height at the peak of 19.9° is maximum among the three peaks, and also has peaks at diffraction angles 2Θ of 5.0°, 9.9°, 20.4°, 20.6°, 20.8, 21.8°, 22.4°, 22.8°, 25.4°, 26.6°, 27.0°, 27.1°, 28.5°, 29.8°, 30.3°, 31.6°, 34.7° and 35.6°.

In another preferred embodiment, the powder X-ray diffraction spectrum of the crystal of pyroxasulfone of the present invention has characteristic peaks at diffraction angles 2θ of 17.7°, 18.0° and 19.9° and the peak height at the peak of 19.9° is maximum among the three peaks, and also has peaks at diffraction angles 2Θ of 4.9°, 5.0°, 9.9°, 19.6°, 20.2°, 20.4°, 20.6°, 21.8°, 22.4°, 22.7°, 23.2°, 25.1°, 25.4°, 26.6°, 27.0°, 27.1°, 28.5°, 30.1°, 31.5°, 34.6°, 34.8°, 35.5° and 36.1°.

In another preferred embodiment, the powder X-ray diffraction spectrum of the crystal of pyroxasulfone of the present invention has characteristic peaks at diffraction angles 2θ of 17.7°, 18.0° and 19.9° and the peak height at the peak of 19.9° is maximum among the three peaks, and also has peaks at diffraction angles 2Θ of 4.9°, 9.9°, 14.4°, 20.4°, 20.6°, 21.8°, 22.3°, 22.8°, 24.4°, 25.1°, 25.4°, 26.3°, 26.6°, 27.1°, 28.5°, 29.7°, 30.2°, 31.6°, 32.4°, 33.3°, 34.6°, 34.7°, 35.6°, 36.1°, 36.3°, 36.4° and 36.7°.

In another preferred embodiment, the powder X-ray diffraction spectrum of the crystal of pyroxasulfone of the present invention has characteristic peaks at diffraction angles 2θ of 17.7°, 18.0° and 19.9° and the peak height at the peak of 19.9° is maximum among the three peaks, and also has peaks at diffraction angles 2Θ of 5.0°, 9.9°, 20.6°, 20.7°, 22.3°, 26.3°, 26.6°, 26.7°, 27.0°, 27.1°, 30.1°, 30.2°, 31.5°, 34.7°, 34.8°, 35.3°, 35.4°, 36.1° and 36.2°.

In another preferred embodiment, the powder X-ray diffraction spectrum of the crystal of pyroxasulfone of the present invention has characteristic peaks at diffraction angles 2θ of 17.8°, 18.0° and 20.0° and the peak height at the peak of 20.0° is maximum among the three peaks, and also has peaks at diffraction angles 2Θ of 4.9°, 5.0°, 9.9°, 14.4°, 15.0°, 19.7°, 20.2°, 20.3°,. 20.6°, 20.8°, 21.8°, 22.0°, 22.3°, 22.7°, 23.2°, 24.4°, 25.1°, 25.5°, 26.6°, 27.0°, 27.3°, 28.5°, 29.7°, 30.2°, 30.3°, 31.5°, 32.4°, 32.9°, 33.4°, 34.7°, 35.6°, 36.1°, 36.7°, 38.1° and 38.7°.

In another preferred embodiment, the powder X-ray diffraction spectrum of the crystal of pyroxasulfone of the present invention has characteristic peaks at diffraction angles 2θ of 17.7°, 18.0° and 19.9° and the peak height at the peak of 19.9° is maximum among the three peaks, and also has peaks at diffraction angles 2Θ of 5.0°, 9.9°, 14.4°, 19.6°, 20.4°, 20.6°, 20.7°, 21.8°, 22.3°, 22.4°, 22.8°, 23.2°, 25.1°, 25.4°, 26.6°, 27.0°, 28.5°, 30.1°, 30.3°, 31.5°, 32.2°, 34.6° and 35.6°.

In another preferred embodiment, the powder X-ray diffraction spectrum of the crystal of pyroxasulfone of the present invention has characteristic peaks at diffraction angles 2θ of 17.7°, 18.0° and 19.9° and the peak height at the peak of 19.9° is maximum among the three peaks, and also has peaks at diffraction angles 2Θ of 5.0°, 9.9°, 14.4°, 20.4°, 20.6°, 20.8°, 21.8°, 22.3°, 22.8°, 23.2°, 25.1°, 25.5°, 26.3°, 26.6°, 27.0°, 27.1°, 28.5°, 29.8°, 30.1°, 32.4°, 34.6°, 34.7°, 35.5°, 36.1°, 38.1° and 38.6°.

In another preferred embodiment, the powder X-ray diffraction spectrum of the crystal of pyroxasulfone of the present invention has characteristic peaks at diffraction angles 2θ of 17.7°, 18.0° and 19.9° and the peak height at the peak of 19.9° is maximum among the three peaks, and also has peaks at diffraction angles 2Θ of 5.0°, 9.9°, 14.4°, 15.0°, 20.3°, 20.6°, 20.8°, 21.8°, 22.0°, 22.4°, 22.8°, 23.2°, 24.4°, 25.1°, 25.5°, 26.2°, 26.6°, 27.1°, 27.8°, 28.5°, 29.8°, 30.3°, 31.5°, 32.4°, 34.5°, 35.6°, 37.2° and 38.1°.

As one embodiment, the process for obtaining a crystal of pyroxasulfone of the present invention includes a known crystallization technique such as a concentration method, an antisolvent addition method, a vapor diffusion method (including a sitting drop method, a hanging drop method and a sandwich drop method), a batch method (including an oil batch method), a dialysis method, a liquid-liquid diffusion method (a counter diffusion method), a cooling method, a pressure method, a melt quenching method, a temperature cycling method, a slurry stirring method, and an ultrasonic method. As one preferred embodiment, the process for obtaining a crystal of pyroxasulfone of the present invention includes a concentration method, i.e., a method in which an organic solvent is distilled off from a solution of pyroxasulfone comprising a solvent mainly composed of the organic solvent and pyroxasulfone as a solute to precipitate the pyroxasulfone. As another preferred embodiment, the process for obtaining a crystal of pyroxasulfone of the present invention includes an antisolvent addition method, i.e., a method in which an antisolvent for pyroxasulfone is added to a solution of pyroxasulfone comprising a solvent mainly composed of an organic solvent and pyroxasulfone as a solute to precipitate the pyroxasulfone.

Distillation refers to removal of a part or all of the organic solvent constituting the solvent from the solution by evaporating it by volatilizing or boiling it. When the organic solvent constituting the solution of pyroxasulfone is distilled off, the solution is concentrated, resulting in a supersaturated state, so that the pyroxasulfone excess with respect to the medium precipitates as a crystal. The distillation may be performed under normal pressure, or may be performed under reduced pressure or increased pressure as desired. The distillation may be performed at room temperature, or may be performed by heating or cooling the system as desired.

The antisolvent refers to a solvent having a low ability to dissolve a solute. When an antisolvent is added to the medium constituting the solution of pyroxasulfone, the solubility of the pyroxasulfone decreases as the amount of the antisolvent increases, so that the solution results in a supersaturated state and pyroxasulfone excess with respect to the medium precipitates as a crystal. The addition of the antisolvent may be performed at room temperature, or may be performed by heating or cooling the system as desired.

In any of the above embodiments, any organic solvent cannot necessarily be used in the process for obtaining a crystal of pyroxasulfone of the present invention, and selection of an organic solvent is extremely important. When the selection of an organic solvent is wrong, crystals of pyroxasulfone having the characteristic pattern observed in the desired powder X-ray diffraction spectrum cannot be obtained.

In an embodiment in which the organic solvent is distilled off to precipitate the pyroxasulfone of the present invention, organic solvents that can be used include at least the following: aromatic hydrocarbon derivatives (e.g., benzene, toluene, xylenes, chlorobenzene, dichlorobenzenes, trichlorobenzenes and nitrobenzene), halogenated aliphatic hydrocarbons (e.g., dichloromethane and tetrachloroethylene), alcohols (e.g., methanol, ethanol, isopropanol, butanol and tert-butanol), nitriles (e.g., acetonitrile and propionitrile), carboxylic acids (e.g., formic acid, acetic acid, propionic acid, and butyric acid), carboxylic acid esters (e.g., methyl acetate, ethyl acetate, propyl acetate, isopropyl acetate, butyl acetate and isomers thereof and pentyl acetate and isomers thereof), ethers (e.g., tetrahydrofuran, 2-methyltetrahydrofuran, 1,4-dioxane, diisopropyl ether, dibutyl ether, di-tert-butyl ether, cyclopentyl methyl ether, methyl tert-butyl ether, 1,2-dimethoxyethane and diglyme), ketones (e.g., methyl isopropyl ketone and methyl isobutyl ketone), amides (e.g., N,N-dimethylformamide and N,N-dimethylacetamide), ureas (e.g., N,N'-dimethylimidazolidinone and tetramethylurea), sulfoxides (e.g., diethyl sulfoxide), sulfones (e.g., sulfolane), and any combination thereof in any ratio. In particular, nitriles, carboxylic acids, carboxylic acid esters, ketones, amides and dihalogenated aliphatic hydrocarbons are included.

Among the above, preferable organic solvents include the following: C2-C5 alkanenitriles, C1-C4 carboxylic acids, C1-C4 alkyl C1-C4 carboxylates, C1-C4 alkyl C1-C4 alkyl ketones, N,N-di(C1-C4 alkyl)C1-C4 alkanamides, C1-C4 dihaloalkanes. In particular, acetonitrile, acetic acid, ethyl acetate, methyl isobutyl ketone, N,N-dimethylformamide, N,N-dimethylacetamide and dichloromethane are included.

In the above embodiment, the medium constituting the solution of pyroxasulfone may be a hydrous medium further comprising water. It is noted that from the viewpoint of making the solubility of pyroxasulfone in the hydrous medium sufficiently high, the medium preferably comprises an organic solvent as a main component. Herein, "to comprise a certain component as a main component" means that the volume of the component occupies 1/3 or more of the total volume of the components constituting the composition to be discussed.

Among the above, preferable medium include the following: C1-C4 alcohol/C2-C5 alkanenitrile mixtures, hydrous C2-C5 alkanenitriles, C1-C4 carboxylic acids, C1-C4 alkyl C1-C4 carboxylates, N,N-di(C1-C4 alkyl)C1-C4 alkaneamides and C1-C4 dihaloalkane/C1-C4 alcohol mixtures. In particular, acetonitrile/methanol mixtures, hydrous acetonitrile, acetic acid, ethyl acetate, methyl isobutyl ketone, N,N-dimethylformamide, N,N-dimethylacetamide and dichloromethane/ethanol mixtures are included.

On the other hand, among the above, organic solvents that should be avoided from being used singly include the following: chloroform, dimethyl sulfoxide, 1,4-dioxane, 2-methyltetrahydrofuran, N-methylpyrrolidone, tetrahydrofuran, trifluoroethanol and carbon disulfide. It is noted that an embodiment in which these organic solvents are used in combination with other organic solvents and an embodiment in which the solvent is a hydrous medium comprising such an organic solvent and water are not excluded.

In an embodiment in which an antisolvent is added to precipitate the pyroxasulfone of the present invention, organic solvents that can be used include at least the following: aromatic hydrocarbon derivatives (e.g., benzene, toluene, xylenes, chlorobenzene, dichlorobenzenes, trichlorobenzenes and nitrobenzene), halogenated aliphatic hydrocarbons (e.g., dichloromethane and tetrachloroethylene), alcohols (e.g., methanol, ethanol, isopropanol, butanol and tert-butanol), nitriles (e.g., acetonitrile and propionitrile), carboxylic acids (e.g., formic acid, acetic acid, propionic acid, and butyric acid), carboxylic acid esters (e.g., methyl acetate, ethyl acetate, propyl acetate, isopropyl acetate, butyl acetate and isomers thereof and pentyl acetate and isomers thereof), ethers (e.g., tetrahydrofuran, 1,4-dioxane, diisopropyl ether, dibutyl ether, di-tert-butyl ether, cyclopentyl methyl ether, methyl tert-butyl ether, 1,2-dimethoxyethane and diglyme), ketones (e.g., acetone, methyl ethyl ketone, methyl isopropyl ketone and methyl isobutyl ketone), amides (e.g., N,N-dimethylformamide, N,N-dimethylacetamide and N-methylpyrrolidone), ureas (e.g., N,N'-dimethylimidazolidinone and tetramethylurea), sulfoxides (e.g., dimethyl sulfoxide and diethyl sulfoxide), sulfones (e.g., sulfolane), and any combination thereof in any ratio. In particular, nitriles, ketones, and carboxylic acid esters are included.

Among the above, preferable organic solvents include the following: C2-C5 alkanenitriles and C1-C4 alkyl C1-C4 carboxylates. In particular, acetonitrile, acetone and ethyl acetate are included.

In the above embodiment, the medium constituting the solution of pyroxasulfone may be a hydrous medium further comprising water. It is noted that from the viewpoint of making the solubility of pyroxasulfone in the hydrous medium sufficiently high, the solvent preferably comprises an organic solvent as a main component.

In the above embodiment, antisolvents to be used, which refer to solvents in which the solubility of pyroxasulfone at 20°C is 50 g/L or less, include at least the following: ethers (e.g., diethyl ether, methyl tert-butyl ether, anisole, 2-methyltetrahydrofuran), carboxylic acid esters (e.g., isopropyl acetate), ketones (e.g., methyl isobutyl ketone), aliphatic hydrocarbons (e.g., cyclohexane and heptane), alcohols (e.g., methanol, ethanol, propanol, isopropanol, butanol, isobutanol and tert-butanol), aromatic hydrocarbon derivatives (e.g., toluene and xylene) and water. In particular, alcohols are included

As the antisolvent mentioned above, one that is compatible with the medium constituting the solution of pyroxasulfone is preferably used. In addition, C1 to C4 alcohols are preferable, and ethanol or isopropanol is more preferable, and ethanol is particularly preferable.

Among the combinations of a medium and an antisolvent constituting the solution of pyroxasulfone, particularly preferred combinations include the following: acetonitrile and ethanol, acetone and ethanol, and ethyl acetate and ethanol.

In any of the above cases, a seed crystal may be used in obtaining the crystal of pyroxasulfone of the present invention.

In one embodiment, the solution of pyroxasulfone may be a reaction solution used in a reaction for synthesizing pyroxasulfone. The process for synthesizing pyroxasulfone is not particularly limited, and pyroxasulfone can be synthesized according to a known process. The process for synthesizing pyroxasulfone is preferably a process comprising the step (iii) described above.

In the crystal of pyroxasulfone of the present invention, the shape of the crystal is usually columnar or short columnar. Herein, "the shape of a crystal is columnar or short columnar" means that the crystal has a shape characterized in that in a virtual rectangle inscribed in the orthographic projection of the crystal to be observed, the ratio of the length of the short side to that of the long side of the rectangle is 1 : 1 to 1 : 10, preferably 1 : 1 to 1 : 5. In addition, "the shape of a crystal is acicular" means that the crystal has a shape characterized in that the length of the long side of the aforementioned rectangle exceeds 10 times the length of the short side. The shape of the crystal of pyroxasulfone can be observed using a means such as an optical microscope or an electron microscope, and the observation method is not particularly limited. Preferably, when ten crystals of pyroxasulfone of the present invention are randomly observed, the shape of eight or more of the crystals is columnar or short columnar.

In one embodiment, the crystal of pyroxasulfone of the present invention is a columnar crystal having a bulk specific gravity of about 1.0 g/mL. On the other hand, the bulk specific gravity of acicular crystals produced by the processes disclosed in Patent Documents 2 and 10 is about 0.5 g/mL.

Columnar crystals have a large bulk specific gravity, and are more advantageous than acicular crystals from the viewpoint of storage space during transportation or at a producing site, and the capacity and handling of various devices to be used. Further, when acicular crystals are formed, scaling is observed, so that their recovery rate tends to be low.

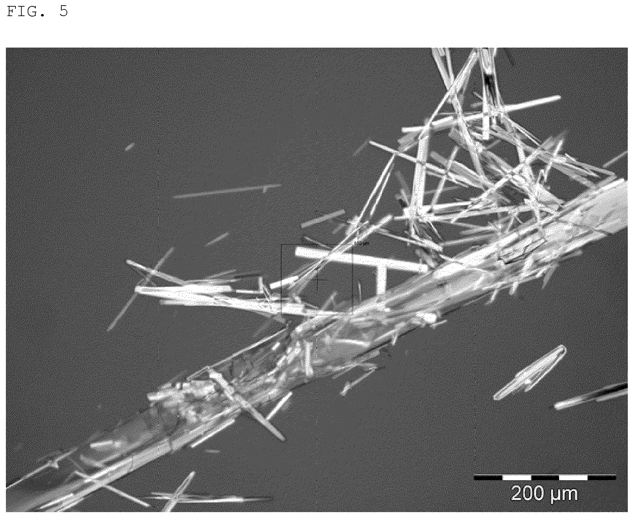

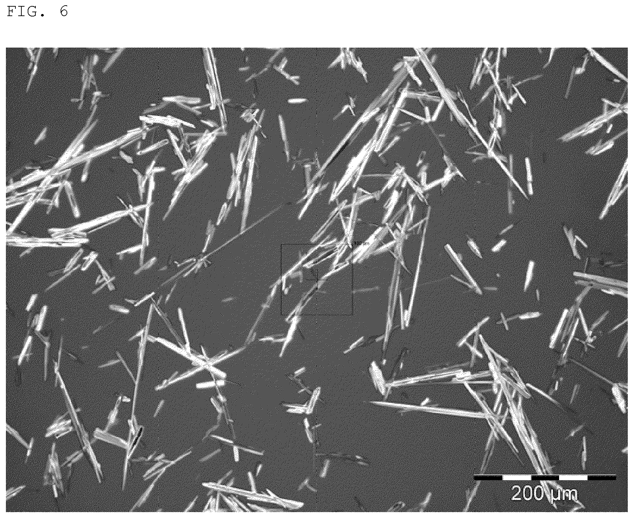

A microscopic photograph of the crystal of pyroxasulfone of the present invention is shown in FIG. 4. Further, microscopic photographs of the crystals of pyroxasulfone produced by the processes disclosed in Patent Documents 2 and 10 are shown in FIGs. 5 and 6.

The crystal of pyroxasulfone of the present invention can be used alone as a herbicidally active ingredient, but from the viewpoint of safety, convenience, etc. of an agricultural worker as a consumer, it is preferable to use the crystal after processing it into an agrochemical formulation, that is, an agrochemical composition in which the crystal is mixed with various agrochemical auxiliaries.

The crystal of pyroxasulfone of the present invention can be processed into agrochemical formulations in various dosage forms by known conventional formulation techniques, and such agrochemical formulations (hereinafter, sometimes referred to as agrochemical formulations of the present invention) are also encompassed by the present invention.

Examples of the dosage form of the agrochemical formulation of the present invention include, but are not limited to, the following: an embodiment of a formulation in which the formulation is sprinkled as it is on a farmland or the like, such as a dust and a granule; an embodiment of a formulation in which the formulation is dispersed in sprinkling water to form a suspension and the suspension is sprinkled on a farmland or the like, such as a wettable powder, a water-dispersible granule, an aqueous suspension concentrate and an oil dispersion; an embodiment of a formulation in which the formulation is dispersed in sprinkling water to prepare an emulsion and the emulsion is sprinkled on a farmland or the like, such as an emulsifiable concentrate and an emulsion in water.

Preferred examples of the dosage form include an embodiment of a formulation in which the formulation is dispersed in sprinkling water to prepare a suspension and the suspension is sprinkled on a farmland of the like, such as a wettable powder, a water-dispersible granule, an aqueous suspension concentrate or an oil dispersion.

In one embodiment, more preferred specific examples of the dosage form include solid formulations such as a wettable powder and a water-dispersible granule.

More preferred specific examples of the solid formulation include a wettable powder.

In another embodiment, more preferred specific examples of the dosage form include liquid formulations such as an aqueous suspension concentrate or an oil dispersion.

Further preferred specific examples of the liquid formulation include an aqueous suspension concentrate.

The wettable powder is a powdery solid formulation comprising an agrochemically active ingredient (a crystal of pyroxasulfone in the present invention) and a surfactant and a solid carrier as agrochemical auxiliaries. The process for producing the wettable powder is not particularly limited.

The water-dispersible granule is a granular solid formulation comprising an agrochemically active ingredient (a crystal of pyroxasulfone in the present invention) and a surfactant and a solid carrier as agrochemical auxiliaries. The process for producing the water-dispersible granule is not particularly limited.

The aqueous suspension concentrate is an aqueous liquid formulation comprising an agrochemically active ingredient (a crystal of pyroxasulfone in the present invention) and a surfactant and water as agrochemical auxiliaries. The process for producing the aqueous suspension concentrate is not particularly limited.

The oil dispersion is an oily liquid formulation comprising an agrochemically active ingredient (a crystal of pyroxasulfone in the present invention) and a surfactant and an oil-based dispersion medium as agrochemical auxiliaries. As the oil-based dispersion medium, an antisolvent for an agrochemically active ingredient is preferably used. The process for producing the oil dispersion is not particularly limited.

The blending amount and the blending ratio of the surfactant may be appropriately set by a person skilled in the art. Such surfactant may be used singly or any two or more of them may be used in combination. Examples of the surfactant include, but are not limited to, nonionic surfactants such as polyoxyalkylene fatty acid esters, polyoxyalkylene sorbitan fatty acid esters, polyoxyalkylene sorbitol fatty acid esters, polyoxyalkylene castor oils, polyoxyalkylene hydrogenated castor oils, polyglycerol fatty acid esters, polyoxyalkylene alkyl ethers, polyoxyalkylene alkylaryl ethers, polyoxyalkylene aryl phenyl ethers, sorbitan monoalkylate, acetylene alcohols, acetylene diols and alkylene oxide adducts thereof; cationic surfactants such as tetraalkylammonium salts, alkylamines and alkylpyrimidinium salts; anionic surfactants such as alkylaryl sulfonates and condensates thereof, dialkyl sulfonates, dialkyl succinates, aryl sulfonates and condensates thereof, alkyl sulfate ester salts, alkyl phosphate ester salts, alkylaryl sulfate ester salts, alkylaryl phosphate ester salts, lignin sulfonates, polycarboxylic acid salts, polyoxyalkylene alkyl ether sulfates, polyoxyalkylene alkyl ether phosphates, polyoxyalkylene aryl ether sulfates, polyoxyalkylene aryl ether phosphates, polyoxyalkylene alkylaryl ether sulfates and polyoxyalkylene alkylaryl ether phosphates; amphoteric surfactants such as alkyl betaines, alkyl amine oxides, alkylimidazolinium betaines, amino acids and lecithin; silicone-based surfactants such as polyether-modified silicones; fluorine-based surfactants such as perfluoroalkyl sulfonic acids, perfluoroalkyl carboxylic acids and fluorotelomer alcohols.

The blending amount and the blending ratio of the solid carrier may be appropriately set by a person skilled in the art. Such solid carriers may be used singly or any two or more of them may be used in combination. Examples of the solid carrier include, but are not limited to, the following: mineral powders such as bentonite, talc, clay, kaolin, diatomaceous earth, amorphous silicon dioxide, calcium carbonate and magnesium carbonate; organic matters such as saccharides (e.g., glucose, sugar and lactose), carboxymethyl cellulose and salts thereof, starch, dextrin and derivatives thereof, microcrystalline cellulose and urea, water-soluble inorganic salts such as sodium sulfate, ammonium sulfate and potassium chloride.

The blending amount and the blending ratio of the oil-based dispersion medium may be appropriately set by a person skilled in the art. Such oil-based dispersion medium may be used singly or any two or more of them may be used in combination. Examples of the oil-based dispersion medium include, but are not limited to, the following: animal oils such as whale oil, cod liver oil, musk oil and mink oil; vegetable oils such as soybean oil, rapeseed oil, corn oil, sunflower oil, cottonseed oil, linseed oil, coconut oil, palm oil, thistle oil, walnut oil, arachis oil, olive oil, papaya oil, tea seed oil, sesame oil, rice bran oil, peanut oil and castor oil; fatty acid esters such as methyl oleate, methyl rapeseed oil, or ethyl rapeseed oil; mineral oils such as paraffin, olefin, alkylbenzenes (e.g., toluene, xylene, mesitylene and ethylbenzene), alkylnaphthalenes (e.g., methylnaphthalene, dimethylnaphthalene and ethylnaphthalene), kerosene and phenylxylylethane.

In addition to the above, the agrochemical formulation of the present invention may contain, as desired, agrochemical auxiliaries, for example, binders such as starch, alginic acid, glycerin, polyvinylpyrrolidone, polyurethane, polyethylene glycol, polypropylene glycol, polybutene, polyvinyl alcohol, liquid paraffin, ethyl cellulose, polyvinyl acetate and thickening polysaccharides (e.g., xanthan gum, gum arabic and guar gum); lubricants such as calcium stearate, talc and silica; antifreezers such as relatively low molecular weight water-soluble substances (e.g., urea and sodium chloride) and water-soluble polyhydric alcohols (e.g., propylene glycol, ethylene glycol, diethylene glycol and glycerin); colorants such as Brilliant Blue FCF, Cyanine Green G and Erio Green G; preservatives such as sorbic acid, potassium sorbate, p-chloro-m-xylenol, butyl p-oxybenzoate, sodium dehydroacetate, 5-chloro-2-methyl-4-isothiazolin-3-one, 2-bromo-2-propane-1,3-diol and 1,2-benzoisothiazolin-3-one; pH regulators such as inorganic acids (e.g., hydrochloric acid, sulfuric acid and phosphoric acid), organic acids (e.g., citric acid, phthalic acid and succinic acid), inorganic metal salts (e.g., disodium hydrogen phosphate, sodium dihydrogen phosphate, dipotassium hydrogen phosphate, potassium dihydrogen phosphate, sodium carbonate, potassium carbonate and sodium borate), hydroxides (e.g., sodium hydroxide and potassium hydroxide), organic amines (e.g., triethanolamine); defoamers such as silicone-based defoamers (e.g., dimethylpolysiloxane and polyphenylsiloxane), fatty acids (e.g., myristic acid) and fatty acid metal salts (e.g., sodium stearate). The agrochemical formulation of the present invention that is a liquid formulation may contain a thickener, if desired. The thickener is not particularly limited, and for example, the materials described above as a solid carrier and a binder can be used. When these agrochemical auxiliaries are used for the agrochemical formulation of the present invention, the blending amount and the blending ratio thereof may be appropriately set by a person skilled in the art.

Further, the agrochemical formulation of the present invention may contain a safener, if desired. When a safener is contained, the blending amount and the blending ratio thereof may be appropriately set by a person skilled in the art. Such safeners may be used singly or any two or more of them may be used in combination. Examples of the safener include, but are not limited to, the following: benoxacor, furilazole, dichlormid, dicyclonone, DKA-24 (N1,N2-diallyl-N2-dichloroacetylglycinamide), AD-67 (4-dichloroacetyl-1-oxa-4-azaspiro[4.5]decane), PPG-1292 (2,2-dichloro-N-(1,3-dioxan-2-ylmethyl)-N-(2-propenyl)acetamide), R-29148 (3-dichloroacetyl-2,2,5-trimethyl-1,3-oxazolidine), cloquintcet-mexyl, 1,8-naphthalic anhydride, mefenpyr-diethyl, mefenpyr, mefenpyr-ethyl, fenchlorazole-O-ethyl, fenclorim, MG-191 (2-dichloromethyl-2-methyl-1,3-dioxane), cyometrinil, flurazole, fluxofenim, isoxadifen, isoxadifen-ethyl, MON4660 (Code Number), oxabetrinil, cyprosulfamide, lower alkyl-substituted benzoic acid, TI-35 (Code Number) and N-(2-methoxybenzoyl)-4-[(methylaminocarbonyl)amino]benzenesulfonamide (chemical name, CAS Registry Number: 129531-12-0).

Further, the agrochemical formulation of the present invention may optionally contain an additional herbicidally active ingredient in addition to the crystal of pyroxasulfone of the present invention, if desired. When an additional herbicidally active ingredient is contained, the blending amount and the blending ratio thereof may be appropriately set by a person skilled in the art. Such additional herbicidally active ingredients may be used singly or any two or more of them may be used in combination. Examples of the additional herbicidally active ingredient include, but are not limited to, the following: ioxynil, aclonifen, acrolein, azafenidin, acifluorfen (including its salts with sodium, etc.), azimsulfuron, asulam, acetochlor, atrazine, anilofos, amicarbazone, amidosulfuron, amitrole, aminocyclopyrachlor, aminopyralid, amiprofos-methyl, ametryn, alachlor, alloxydim, isouron, isoxachlortole, isoxaflutole, isoxaben, isoproturon, ipfencarbazone, imazaquin, imazapic (including its salts with amines, etc.), imazapyr (including its salts with isopropylamine, etc.), imazamethabenz-methyl, imazamox, imazethapyr, imazosulfuron, indaziflam, indanofan, eglinazine-ethyl, esprocarb, ethametsulfuron-methyl, ethalfluralin, ethidimuron, ethoxysulfuron, ethoxyfen-ethyl, ethofumesate, etobenzanid, endothal-disodium, oxadiazon, oxadiargyl, oxaziclomefone, oxasulfuron, oxyfluorfen, oryzalin, orthosulfamuron, orbencarb, oleic acid, cafenstrole, carfentrazone-ethyl, karbutilate, carbetamide, quizalofop-ethyl, quizalofop-P-ethyl, quizalofop-P-tefuryl, quinoclamine, quinclorac, quinmerac, cumyluron, clacyfos, glyphosate (including its salts with sodium, potassium, ammonium, amines, propylamine, isopropylamine, dimethylamine, trimesium, etc.), glufosinate (including its salts with amines, sodium, etc.), glufosinate-P-sodium, clethodim, clodinafop-propargyl, clopyralid, clomazone, chlomethoxyfen, clomeprop, cloransulam-methyl, chloramben, chloridazon, chlorimuron-ethyl, chlorsulfuron, chlorthal-dimethyl, chlorthiamid, chlorphthalim, chlorflurenol-methyl, chlorpropham, chlorbromuron, chloroxuron, chlorotoluron, ketospiradox (including its salts with sodium, calcium, ammonium, etc.), saflufenacil, sarmentine, cyanazine, cyanamide, diuron, diethatyl-ethyl, dicamba ([including its salts with amines, diethylamine. isopropylamine, diglycolamine, sodium, lithium, etc.), cycloate, cycloxydim, diclosulam, cyclosulfamuron, cyclopyranil, cyclopyrimorate, dichlobenil, diclofop-P-methyl, diclofop-methyl, dichlorprop, dichlorprop-P, diquat, dithiopyr, siduron, dinitramine, cinidon-ethyl, cinosulfuron, dinoseb, dinoterb, cyhalofop-butyl, diphenamid, difenzoquat, diflufenican, diflufenzopyr, simazine, dimethachlor, dimethametryn, dimethenamid, dimethenamid-P, simetryn, dimepiperate, dimefuron, cinmethylin, swep, sulcotrione, sulfentrazone, sulfosate, sulfosulfuron, sulfometuron-methyl, sethoxydim, terbacil, daimuron, thaxtomin A, dalapon, thiazopyr, tiafenacil, thiencarbazone (including its sodium salt, methyl ester, etc.), tiocarbazil, thiobencarb, thidiazimin, thifensulfuron-methyl, desmedipham, desmetryne, tetflupyrolimet, thenylchlor, tebutam, tebuthiuron, tepraloxydim, tefuryltrione, tembotrione, terbuthylazine, terbutryn, terbumeton, topramezone, tralkoxydim, triaziflam, triasulfuron, triafamone, tri-allate, trietazine, triclopyr, triclopyr-butotyl, trifludimoxazin, tritosulfuron, triflusulfuron-methyl, trifluralin, trifloxysulfuron-sodium, tribenuron-methyl, tolpyralate, naptalam (including its salts with sodium, etc.), naproanilide, napropamide, napropamide-M, nicosulfuron, neburon, norflurazon, vernolate, paraquat, halauxifen-benzyl, halauxifen-methyl, haloxyfop, haloxyfop-P, haloxyfop-etotyl, halosafen, halosulfuron-methyl, bixlozone, picloram, picolinafen, bicyclopyrone, bispyribac-sodium, pinoxaden, bifenox, piperophos, pyraclonil, pyrasulfotole, pyrazoxyfen, pyrazosulfuron-ethyl, pyrazolynate, bilanafos, pyraflufen-ethyl, pyridafol, pyrithiobac-sodium, pyridate, pyriftalid, pyributicarb, pyribenzoxim, pyrimisulfan, pyriminobac-methyl, pyroxsulam, phenisopham, fenuron, fenoxasulfone, fenoxaprop (including its methyl, ethyl, and isopropyl esters), fenoxaprop-P (including its methyl, ethyl, and isopropyl esters), fenquinotrione, fenthiaprop-ethyl, fentrazamide, phenmedipham, butachlor, butafenacil, butamifos, butylate, butenachlor, butralin, butroxydim, flazasulfuron, flamprop (including its methyl, ethyl, and isopropyl esters), flamprop-M (including its methyl, ethyl, and isopropyl esters), primisulfuron-methyl, fluazifop-butyl, fluazifop-P-butyl, fluazolate, fluometuron, fluoroglycofen-ethyl, flucarbazone-sodium, fluchloralin, flucetosulfuron, fluthiacet-methyl, flupyrsulfuron-methyl-sodium, flufenacet, flufenpyr-ethyl, flupropanate, flupoxame, flumioxazin, flumiclorac-pentyl, flumetsulam, fluridone, flurtamone, fluroxypyr, flurochloridone, pretilachlor, procarbazone-sodium, prodiamine, prosulfuron, prosulfocarb, propaquizafop, propachlor, propazine, propanil, propyzamide, propisochlor, propyrisulfuron, propham, profluazol, propoxycarbazone-sodium, profoxydim, bromacil, brompyrazon, prometryn, prometon, bromoxynil (including its esters with butylic acid, octanoic acid, heptanoic acid, etc.), bromofenoxim, bromobutide, florasulam, florpyrauxifen, hexazinone, pethoxamid, benazolin, penoxsulam, heptamaloxyloglucan, beflubutamid, beflubutamid-M, pebulate, pelargonic-acid, bencarbazone, pendimethalin, benzfendizone, bensulide, bensulfuron-methyl, benzobicyclon, benzofenap, bentazone, pentanochlor, pentoxazone, benfluralin, benfuresate, fosamine, fomesafen, foramsulfuron, mecoprop (including its salts with sodium, potassium, isopropylamine, triethanolamine, dimethylamine, etc.), mecoprop-P-potassium, mesosulfuron-methyl, mesotrione, metazachlor, metazosulfuron, methabenzthiazuron, metamitron, metamifop, DSMA (disodium methanearsonate), methiozolin, methyldymuron, metoxuron, metosulam, metsulfuron-methyl, metobromuron, metobenzuron, metolachlor, metribuzin, mefenacet, monosulfuron (including its methyl, ethyl, and isopropyl esters), monolinuron, molinate, iodosulfuron, iodosulfulon-methyl-sodium, iofensulfuron, iofensulfuron-sodium, lactofen, lancotrione, linuron, rimsulfuron, lenacil, TCA (2,2,2-trichloroacetic acid) (including its salts with sodium, calcium, ammonium, etc.), 2,3,6-TBA (2,3,6-trichlorobenzoic acid), 2,4,5-T (2,4,5-trichlorophenoxyacetic acid), 2,4-D ((2,4-dichlorophenoxyacetic acid) (including its salts with amines, diethylamine, triethanolamine, isopropylamine, sodium, lithium, etc.), ACN (2-amino-3-chloro-1,4-naphthoquinone), MCPA (2-methyl-4-chlorophenoxyacetic acid), MCPB (2-methyl-4-chlorophenoxybutylic acid) (including its sodium salt, ethyl ester, etc.), 2,4-DB (4-(2,4-dichlorophenoxy)butylic acid), DNOC (4,6-dinitro-ortho-cresol) (including its salts with amines, sodium, etc.), AE-F-150944 (code number), HW-02 (code number), IR-6396 (code number), MCPA-thioethyl, SYP-298 (code number), SYP-300 (code number), EPTC (S-ethyldipropylthiocarbamate), S-metolachlor, S-9750 (code number) and MSMA.

Further, the agrochemical formulation of the present invention may contain an insecticidally active ingredient in addition to the crystal of pyroxasulfone of the present invention, if desired. When an insecticidally active ingredient is contained, the blending amount and the blending ratio thereof may be appropriately set by a person skilled in the art. Such insecticidally active ingredients may be used singly or any two or more of them may be used in combination. Examples of the insecticidally active ingredient include, but are not limited to, the following: acrinathrin, azadirachtin, azamethiphos, azinphos-ethyl, azinphos-methyl, acequinocyl, acetamiprid, acetoprole, acephate, azocyclotin, abamectin, afidopyropen, afoxolaner, amidoflumet, amitraz, alanycarb, aldicarb, aldoxycarb, allethrin) [including d-cis-trans-form and d-trans-form], isazophos, isamidofos, isocarbophos, isoxathion, isocycloseram, isofenphos-methyl, isoprocarb, ivermectin, imicyafos, imidacloprid, imiprothrin, indoxacarb, esfenvalerate, ethiofencarb, ethion, ethiprole, ethylene dibromide, etoxazole, etofenprox, ethoprophos, etrimfos, emamectin benzoate, endosulfan, empenthrin, oxazosulfyl, oxamyl, oxydemeton-methyl, oxydeprofos, omethoate, cadusafos, kappa-tefluthrin, kappa-bifenthrin, kadethrin, karanjin, cartap, carbaryl, carbosulfan, carbofuran, gamma-BHC, xylylcarb, quinalphos, kinoprene, chinomethionat, coumaphos, cryolite, clothianidin, clofentezine, chromafenozide, chlorantraniliprole, chlorethoxyfos, chlordane, chloropicrin, chlorpyrifos, chlorpyrifos-methyl, chlorfenapyr, chlorfenvinphos, chlorfluazuron, chlormephos, chloroprallethrin, cyanophos, diafenthiuron, diamidafos, cyantraniliprole, dienochlor, cyenopyrafen, dioxabenzofos, diofenolan, cyclaniliprole, dicrotophos, dichlofenthion, cycloprothrin, dichlorvos, dicloromezotiaz, 1,3-dichloropropene, dicofol, dicyclanil, disulfoton, dinotefuran, dinobuton, cyhalodiamide, cyhalothrin) [including gamma-form and lambda-form], cyphenothrin) [including (1R)-trans-form], cyfluthrin) [including beta-form], diflubenzuron, cyflumetofen, diflovidazin, cyhexatin, cypermethrin) [including alpha-form, beta-form, theta-form and zeta-form], dimpropyridaz, dimethylvinphos, dimefluthrin, dimethoate, silafluofen, cyromazine, spinetoram, spinosad, spirodiclofen, spirotetramat, spiropidion, spiromesifen, sulcofuron-sodium, sulfluramid, sulfoxaflor, sulfotep, diazinon, thiacloprid, thiamethoxam, tioxazafen, thiodicarb, thiocyclam, thiosultap, thionazin, thiofanox, thiometon, tyclopyrazoflor, tetrachlorantraniliprole, tetrachlorvinphos, tetradifon, tetraniliprole, tetramethylfluthrin, tetramethrin, tebupirimfos, tebufenozide, tebufenpyrad, tefluthrin, teflubenzuron, demeton-S-methyl, temephos, deltamethrin, terbufos, tralomethrin, transfluthrin, triazamate, triazophos, trichlorfon, triflumuron, triflumezopyrim, trimethacarb, tolfenpyrad, naled, nitenpyram, novaluron, noviflumuron, Verticillium lecanii, hydroprene, Pasteuriapenetrans, vamidothion, parathion, parathion-methyl, halfenprox, halofenozide, bioallethrin, bioallethrin S-cyclopentenyl, bioresmethrin, bistrifluron, hydramethylnon, bifenazate, bifenthrin, pyflubumide, piperonyl butoxide, pymetrozine, pyraclofos, pyrafluprole, pyridaphenthion, pyridaben, pyridalyl, pyrifluquinazon, pyriprole, pyriproxyfen, pirimicarb, pyrimidifen, pyriminostrobin, pirimiphos-methyl, pyrethrine, famphur, fipronil, fenazaquin, fenamiphos, fenitrothion, fenoxycarb, fenothiocarb, phenothrin) [including (1R)-trans-form], fenobucarb, fenthion, phenthoate, fenvalerate, fenpyroximate, fenbutatin oxide, fenpropathrin, fonofos, sulfuryl fluoride, butocarboxim, butoxycarboxim, buprofezin, furathiocarb, prallethrin, fluacrypyrim, fluazaindolizine, fluazuron, fluensulfone, sodium fluoroacetate, fluxametamide, flucycloxuron, flucythrinate, flusulfamide, fluvalinate) [including tau-form], flupyradifurone, flupyrazofos, flupyrimin, flufiprole, flufenerim, flufenoxystrobin, flufenoxuron, fluhexafon, flubendiamide, flumethrin, fluralaner, prothiofos, protrifenbute, flonicamid, propaphos, propargite, profenofos, broflanilide, brofluthrinate, profluthrin, propetamphos, propoxur, flometoquin, bromopropylate, hexythiazox, hexaflumuron, Paecilomyces tenuipes, Paecilomyces fumosoroceus, heptafluthrin, heptenophos, permethrin, benclothiaz, benzpyrimoxan, bensultap, benzoximate, bendiocarb, benfuracarb, Beauveria tenella, Beauveria bassiana, Beauveria brongniartii, phoxim, phosalone, fosthiazate, fosthietan, phosphamidon, phosmet, polynactins, formetanate, phorate, malathion, milbemectin, mecarbam, mesulfenfos, methoprene, methomyl, metaflumizone, methamidophos, metham, methiocarb, methidathion, methyl isothiocyanate, methyl bromide, methoxychlor, methoxyfenozide, methothrin, metofluthrin, epsilon-metofluthrin, metolcarb, mevinphos, meperfluthrin, Monacrosporium phymatophagum, monocrotophos, momfluorothrin, epsilon-momfluorothrin, litlure-A, litlure-B, aluminum phosphide, zinc phosphide, phosphine, lufenuron, rescalure, resmethrin, lepimectin, rotenone, fenbutatin oxide, calcium cyanide, nicotine sulfate, (Z)-11-tetradecenyl=acetate, (Z)-11-hexadecenal, (Z)-11-hexadecenyl=acetate, (Z)-9,12-tetradecadienyl=acetate, (Z)-9-tetradecen-1-ol, (Z,E)-9,11-tetradecadienyl=acetate, (Z,E)-9,12-tetradecadienyl=acetate, Bacillus popilliae, Bacillus subtillis, Bacillus sphaericus, Bacillus thuringiensis subsp. Aizawai, Bacillus thuringiensis subsp. Israelensis, Bacillus thuringiensis subsp. Kurstaki, Bacillus thuringiensis subsp. Tenebrionis, Bt protein (Cry1Ab, Cry1Ac, Cry1Fa, Cry2Ab, mCry3A, Cry3Ab, Cry3Bb, Cry34/35Ab1), CL900167 (Code Numbers), DCIP ((bis-(2-chloro-1-methylethyl) ether), DDT (1,1,1-trichloro-2,2-bis(4-chlorophenyl)ethane), DEP (dimethyl-2,2,2-trichloro-1-hydroxyethylphosphonate), DNOC (4,6-dinitro-o-cresol), DSP (O,O-diethyl-O-[4-(dimethylsulfamoyl)phenyl]-phosphorothionate), EPN (O-ethyl-O-4-(nitrophenyl)phenyl phosphonothioate), nuclear polyhedrosis virus occlusion body, NA-85 (Code Number), NA-89 (Code Number), NC-515 (Code Number), RU15525 (Code Number), XMC, Z-13-icosen-10-one, ZXI8901 (Code Number), 2-chloro-4-fluoro-5-[(5-trifluoromethylthio)pentyloxy]phenyl-2,2,2-trifluoroethyl sulfoxide (chemical name, CAS Registry Number: 1472050-04-6), 2,4-dichloro-5-{2-[4-(trifluoromethyl)phenyl]ethoxy}phenyl-2,2,2-trifluoroethyl sulfoxide (chemical name, CAS Registry Number: 1472052-11-1), 2,4-dimethyl-5-[6-(trifluoromethylthio)hexyloxy]phenyl-2,2,2-trifluoroethyl sulfoxide (chemical name, CAS Registry Number: 1472050-34-2), 2-{2-fluoro-4-methyl-5-[(2,2,2-trifluoroethyl)sulfinyl]phenoxy}-5-(trifluoromethyl)pyridine (chemical name, CAS Registry Number: 1448758-62-0), 3-chloro-2-{2-fluoro-4-methyl-5-[(2,2,2-trifluoroethyl)sulfinyl]phenoxy}-5-(trifluoromethyl)pyridine (chemical name, CAS Registry Number: 1448761-28-1), 4-fluoro-2-methyl-5-(5,5-dimethylhexyloxy)phenyl-2,2,2-trifluoroethyl sulfoxide (chemical name, CAS Registry Number: 1472047-71-4) and NI-30 (Code Number).

Further, the agrochemical formulation of the present invention may contain a fungicidally active ingredient in addition to the crystal of pyroxasulfone of the present invention, if desired. When a fungicidally active ingredient is contained, the blending amount and the blending ratio thereof may be appropriately set by a person skilled in the art. Such fungicidally active ingredients may be used singly or any two or more of them may be used in combination. Examples of the fungicidally active ingredient include, but are not limited to, the following: azaconazole, acibenzolar-S-methyl, azoxystrobin, anilazine, amisulbrom, aminopyrifen, ametoctradin, aldimorph, isotianil, isopyrazam, isofetamid, isoflucypram, isoprothiolane, ipconazole, ipflufenoquin, ipfentrifluconazole, iprodione, iprovalicarb, iprobenfos, imazalil, iminoctadine-trialbesilate, iminoctadine-triacetate, imibenconazole, inpyrfluxam, imprimatin A, imprimatin B, edifenphos, etaconazole, ethaboxam, ethirimol, ethoxyquin, etridiazole, enestroburin, enoxastrobin, epoxiconazole, organic oils, oxadixyl, oxazinylazole, oxathiapiprolin, oxycarboxin, oxine-copper, oxytetracycline, oxpoconazole-fumarate, oxolinic acid, copper dioctanoate, octhilinone, ofurace, orysastrobin, o-phenylphenol, kasugamycin, captafol, carpropamid, carbendazim, carboxin, carvone, quinoxyfen, quinofumelin, chinomethionat, captan, quinconazole, quintozene, guazatine, cufraneb, coumoxystrobin, kresoxim-methyl, clozylacon, chlozolinate, chlorothalonil, chloroneb, cyazofamid, diethofencarb, diclocymet, dichlofluanid, dichlobenthiazox, diclomezine, dicloran, dichlorophen, dithianon, diniconazole, diniconazole-M, zineb, dinocap, dipymetitrone, diphenylamine, difenoconazole, cyflufenamid, diflumetorim, cyproconazole, cyprodinil, simeconazole, dimethirimol, dimethyl disulfide, dimethomorph, cymoxanil, dimoxystrobin, ziram, silthiofam, streptomycin, spiroxamine, sedaxane, zoxamide, dazomet, tiadinil, thiabendazole, thiram, thiophanate, thiophanate-methyl, thifluzamide, tecnazene, tecloftalam, tetraconazole, debacarb, tebuconazole, tebufloquin, terbinafine, dodine, dodemorph, triadimenol, triadimefon, triazoxide, trichlamide, triclopyricarb, tricyclazole, triticonazole, tridemorph, triflumizole, trifloxystrobin, triforine, tolylfluanid, tolclofos-methyl, tolnifanide, tolprocarb, nabam, natamycin, naftifine, nitrapyrin, nitrothal-isopropyl, nuarimol, copper nonyl phenol sulphonate, Bacillus subtilis (strain:QST 713), validamycin, valifenalate, picarbutrazox, bixafen, picoxystrobin, pydiflumetofen, bitertanol, binapacryl, biphenyl, piperalin, hymexazol, pyraoxystrobin, pyraclostrobin, pyraziflumid, pyrazophos, pyrapropoyne, pyrametostrobin, pyriofenone, pyrisoxazole, pyridachlometyl, pyrifenox, pyributicarb, pyribencarb, pyrimethanil, pyroquilon, vinclozolin, ferbam, famoxadone, phenazine oxide, fenamidone, fenaminstrobin, fenarimol, fenoxanil, ferimzone, fenpiclonil, fenpicoxamid, fenpyrazamine, fenbuconazole, fenfuram, fenpropidin, fenpropimorph, fenhexamid, folpet, phthalide, bupirimate, fuberidazole, blasticidin-S, furametpyr, furalaxyl, furancarboxylic acid, fluazinam, fluindapyr, fluoxastrobin, fluoxapiprolin, fluopicolide, fluopimomide, fluopyram, fluoroimide, fluxapyroxad, fluquinconazole, furconazole, furconazole-cis, fludioxonil, flusilazole, flusulfamide, flutianil, flutolanil, flutriafol, flufenoxystrobin, flumetover, flumorph, proquinazid, prochloraz, procymidone, prothiocarb, prothioconazole, bronopol, propamocarb-hydrochloride, propiconazole, propineb, probenazole, bromuconazole, flometoquin, florylpicoxamid, hexaconazole, benalaxyl, benalaxyl-M, benodanil, benomyl, pefurazoate, penconazole, pencycuron, benzovindiflupyr, benthiazole, benthiavalicarb-isopropyl, penthiopyrad, penflufen, boscalid, fosetyl (alminum, calcium, sodium), polyoxin, polycarbamate, Bordeaux mixture, mancozeb, mandipropamid, mandestrobin, maneb, myclobutanil, mineral oils, mildiomycin, methasulfocarb, metam, metalaxyl, metalaxyl-M, metiram, metyltetraprole, metconazole, metominostrobin, metrafenone, mepanipyrim, mefentrifluconazole, meptyldinocap, mepronil, iodocarb, laminarin, phosphorous acid and salts, copper oxychloride, silver, cuprous oxide, copper hydroxide, potassium bicarbonate, sodium bicarbonate, sulfur, oxyquinoline sulfate, copper sulfate, (3,4-dichloroisothiazol-5-yl)methyl 4-(tert-butyl)benzoate (chemical name, CAS Registry Number: 1231214-23-5), BAF-045 (Code Number), BAG-010 (Code Number), UK-2A (Code Number), DBEDC (dodecylbenzenesulfonic acid bisethylenediamine copper complex salt [II]), MIF-1002 (Code Number), NF-180 (Code Number), TPTA (triphenyltin acetate), TPTC (triphenyltin chloride), TPTH (triphenyltin hydroxide) and nonpathogenic Erwinia carotovora.

Further, the agrochemical formulation of the present invention may contain a plant growth-regulating active ingredient in addition to the crystal of pyroxasulfone of the present invention, if desired. When a plant growth-regulating active ingredient is contained, the blending amount and the blending ratio thereof may be appropriately set by a person skilled in the art. Such plant growth-regulating active ingredients may be used singly or any two or more of them may be used in combination. Examples of the plant growth-regulating active ingredient include, but are not limited to, the following: 1-methylcyclopropene, 1-naphthylacetamide, 2,6-diisopropylnaphthalene, 4-CPA (4-chlorophenoxyacetic acid), benzylaminopurine, ancymidol, aviglycine, carvone, chlormequat, cloprop, cloxyfonac, cloxyfonac-potassium, cyclanilide, cytokinins, daminozide, dikegulac, dimethipin, ethephon, epocholeone, ethychlozate, flumetralin, flurenol, flurprimidol, pronitridine, forchlorfenuron, gibberellins, inabenfide, indole acetic acid, indole butyric acid, maleic hydrazide, mefluidide, mepiquat chloride, n-decanol, paclobutrazol, prohexadione-calcium, prohydrojasmon, sintofen, thidiazuron, triacontanol, trinexapac-ethyl, uniconazole, uniconazole-P, 4-oxo-4-(2-phenylethyl)aminobutyric acid (chemical name, CAS Registry Number: 1083-55-2) and calcium peroxide.

A preferred embodiment of the agrochemical formulation of the present invention wherein the dosage form is a wettable powder comprises 10 to 90 wt% of the crystal of pyroxasulfone of the present invention, 5 to 20 wt% of a surfactant and 5 to 85 wt% of a solid carrier in the agrochemical formulation. Optionally, 0 to 80 wt% of an additional herbicidally active ingredient, 0 to 5 wt% of a binder, 0 to 1 wt% of a colorant, 0 to 1 wt% of a defoamer and 0 to 80% of a safener may be contained.

One embodiment of producing the wettable powder comprises a step of finely pulverizing a powder comprising the crystal of pyroxasulfone of the present invention and a step of mixing the whole raw material to homogenize the mixture. In any of the steps, known conventional techniques and devices may be used.

A preferred embodiment of the agrochemical formulation of the present invention wherein the dosage form is a water-dispersible granule comprises 10 to 90 wt% of the crystal of pyroxasulfone of the present invention, 5 to 20 wt% of a surfactant and 5 to 85 wt% of a solid carrier in the agrochemical formulation. Optionally, 0 to 80 wt% of an additional herbicidally active ingredient, 0 to 5 wt% of a binder, 0 to 1 wt% of a colorant, 0 to 1 wt% of a defoamer and 0 to 80% of a safener may be contained.

One embodiment of producing the water-dispersible granule comprises a step of finely pulverizing a powder or a slurry comprising the crystal of pyroxasulfone of the present invention, a step of kneading, while homogenizing the whole raw material, further adding a slight amount of water and kneading the mixture, a step of granulating the kneaded product obtained in the preceding step, and a step of drying the granulated product obtained in the preceding step. In any of the steps, known conventional techniques and devices may be used.

A preferred embodiment of the agrochemical formulation of the present invention wherein the dosage form is an aqueous suspension concentrate comprises 5 to 65 wt% of the crystal of pyroxasulfone of the present invention, 5 to 10 wt% of a surfactant and 30 to 90 wt% of water in the agrochemical formulation. Optionally, 0 to 50 wt% of an additional herbicidally active ingredient, 0 to 15 wt% of an antifreezer, 0 to 1 wt% of a colorant, 0 to 3 wt% of a preservative, 0 to 5% of a pH regulator, 0 to 1 wt% of a defoamer, 0 to 5 wt% of a thickener and 0 to 50% of a safener may be contained. Further, 0 to 20 wt% of an oil-based dispersion medium may be contained for the purpose of improving efficacy, adjusting specific gravity, and so forth.

One embodiment of producing the aqueous suspension concentrate comprises a step of finely pulverizing a slurry comprising the crystal of pyroxasulfone of the present invention and a step of mixing the whole raw material to homogenize the mixture. In another embodiment, the process comprises a step of finely pulverizing a powder comprising the crystal of pyroxasulfone of the present invention and a step of mixing the whole raw material to homogenize the mixture. In any of the steps, known conventional techniques and devices may be used.

A preferred embodiment of the agrochemical formulation of the present invention wherein the dosage form is an oil dispersion comprises 5 to 65 wt% of the crystal of pyroxasulfone of the present invention, 5 to 10 wt% of a surfactant and 30 to 90 wt% of an oil-based dispersion medium in the agrochemical formulation. Optionally, 0 to 50 wt% of an additional herbicidally active ingredient, 0 to 15 wt% of an antifreezer, 0 to 1 wt% of a colorant, 0 to 3 wt% of a preservative, 0 to 5% of a pH regulator, 0 to 1 wt% of a defoamer, 0 to 5 wt% of a thickener and 0 to 50% of a safener may be contained.

One embodiment of producing the oil dispersion comprises a step of finely pulverizing a slurry comprising the crystal of pyroxasulfone of the present invention and a step of mixing the whole raw material to homogenize the mixture. In another embodiment, the process comprises a step of finely pulverizing a powder comprising the crystal of pyroxasulfone of the present invention and a step of mixing the whole raw material to homogenize the mixture. In any of the steps, known conventional techniques and devices may be used.

The agrochemical formulation of the present invention exhibit several superior physicochemical properties.

One example of the superior physicochemical properties of the agrochemical formulation of the present invention is that a solid formulation (e.g., a wettable powder or a water-dispersible granule), which is to be dispersed in sprinkling water to prepare a suspension, which is then sprinkled on a farmland or the like, is highly compatible with water when added to water. As for a wettable powder or a water-dispersible granule produced using the known crystal of pyroxasulfone disclosed in Patent Documents 2 and 10 as a raw material, when it is added to water, lumps may be formed, so that the sprinkling liquid needs to be continuously stirred until the lumps are loosened. In contrast to this, since the agrochemical formulation of the present invention does not form lumps, a homogeneous suspension can be quickly prepared at the time of use.

As an index for quantitatively evaluating whether or not a solid formulation such as a wettable powder is compatible with water, wettability has been proposed. This testing method is described in Notifications No. 71 (February 3, 1960) and No.750(July 25, 1975) of the Ministry of Agriculture, Forestry and Fisheries of Japan.

The 3 degree hard water required for measuring the wettability is prepared as follows. Calcium carbonate (0.3077 g) and magnesium oxide (0.092 g) are dissolved in a small amount of dilute hydrochloric acid, then the solution is evaporated to dryness on a sand bath to remove hydrochloric acid, and the residue is diluted to 1 L with water. This water is further diluted 10 times and the diluted water is used for the test as 3 degree hard water. The hardness of the 3 degree hard water prepared as described above is 3.004° dH in German hardness notation, and is equivalent to 53.47 ppm in terms of American hardness.

The method of measuring wettability is outlined below. In a 500 mL beaker is put 200 mL of 3 degrees hard water at 20°C, and 5 g of a sample that has passed through a #40 mesh standard sieve (test sieve) in advance is gently dropped into the beaker from a position of about 10 cm above the water surface so as to spread thinly. The time T between the end of the dropping of the sample and the sinking of the whole sample under the water surface is measured. Further, the contents in the beaker are stirred with a glass rod and the homogeneity of the suspended state is observed.

Usually, when a sample with good compatibility with water is measured, the time T is short, whereas when a sample with poor compatibility with water is measured, the time T is long. However, in the case of a sample whose poor conformability with water is exhibited as a tendency of agglomeration and which forms a lump, the lump is wetted with water only on its surface and sinks under the water surface, and thus the time T does not reflect the degree of compatibility between the measurement sample and water, and a small value may be measured. In the case where lumps floating in water are observed when the contents in the beaker are stirred with a glass rod, it should be determined that the measurement sample is poor in compatibility with water regardless of the value of the time T.

Another example of the superior physicochemical properties of the agrochemical formulation of the present invention is that the agrochemical formulation of the present invention that is a formulation which is to be dispersed in sprinkling water to yield a suspension which is then sprinkled on a farmland or the like (e.g., a wettable powder, a water-dispersible granule, an aqueous suspension concentrate or an oil dispersion) prevents the solid matter contained in a sprinkling liquid prepared by dispersing the formulation in water at the time of use from forming a hard cake. The operation of preparing a sprinkling liquid by dispersing the agrochemical formulation in water is a preparatory action for sprinkling an agrochemical, and it is not usual to leave the prepared sprinkling liquid for a long time without subjecting it to sprinkling of the agrochemical. However, there may be a situation in which the sprinkling of the agrochemical has to be interrupted or postponed due to circumstances such as an urgent need for the agriculture worker during the work or a sudden change in the weather during the work. Even in a case where the field to be sprinkled is wide and a long time is required for sprinkling the agrochemical, the sprinkling liquid to be sprinkled in the latter half of the entire work goes through a certain period of time until it is sprinkled after the preparation. Since the composition of the diluted liquid is a diluted suspension composed mostly of water, the liquid is low in viscosity and does not have structural viscosity, so that if stirring is stopped, solid matter starts to settle immediately. In a case where in the diluted liquid of an agrochemical formulation produced by using the known crystal of pyroxasulfone disclosed in Patent Documents 2 and 10 as a raw material is formed a deposit layer of solid matter, the solid matter is not redispersed even if stirring is resumed, and a homogeneous suspended state may not be recovered. In order to prevent such a situation, it is necessary to continue stirring the prepared sprinkling liquid until the sprinkling is completed. This is a waste of energy and the worker is forced to bear the cost of the energy. In contrast to this, even if in the diluted liquid of the agrochemical formulation of the present invention is formed a deposition layer of solid matter, the deposited layer of solid matter can be easily wound up in the vortex flow and redispersed when stirring is resumed. Therefore, stirring may be stopped after a sprinkling liquid is prepared, so that the sprinkling of the agrochemical can be interrupted or postponed at a lower cost or the sprinkling of the agrochemical can be continued over a long time at a lower cost.

As for the reason why the agrochemical formulation of the present invention exhibits the superior physicochemical properties as described above, the present inventors do not have firm knowledge and do not desire to be bound by theory. However, as one hypothesis, it is considered that conventionally known crystals of pyroxasulfone are likely to be entangled with each other and tend to aggregate as shown in FIGS. 5 and 6, whereas crystals of pyroxasulone of the present invention do not have such a tendency, so that hard caking is less likely to occur, and a result that a wet-spreading and dispersing action of a surfactant is likely to be exhibited.

Hereinafter, the present invention will be described in more detail by Examples, but the present invention is not limited in any way by these Examples.

In the present description, the following instruments and conditions were used for the determination of physical properties and yields in Examples, Comparative Examples and Reference Examples. In addition, the products obtained in the present invention are known compounds, and were identified in the usual manner known to a person skilled in the art.
(HPLC Analysis: High Performance Liquid Chromatography Analysis)
(HPLC Analysis Conditions)
Instrument: LC 2010 Series manufactured by Shimadzu Corporation or any equivalent thereto
Column: YMC-Pack, ODS-A, A-312 (150 mm × 6.0 mm ID, S-5 µm, 120A)

### Eluent:

**[Table 1]**

| Time (min) | Acetonitrile (%) | 0.05% Phosphoric acid aqueous solution (%) |
|---|---|---|
| 0 | 45 | 55 |
| 10 | 45 | 55 |
| 15 | 80 | 20 |
| 20 | 80 | 20 |

Flow rate: 1.0 ml/min
Detection: UV 230 nm
Column temperature: 40°C
Injection volume: 5 µL

The following documents can be referred to for the HPLC analysis method, as desired.

Literature (a): "Shin Jikkenkagaku Koza 9 (A New Course in Experimental Chemistry Course 9) Bunsekikagaku II (Analytical Chemistry II)", pages 86 to 112 (1977), edited by the Chemical Society of Japan, published by Shingo Iizumi, Maruzen Co., Ltd.

Literature (b): "Jikkenkagaku Koza 20-1 (A Course in Experimental Chemistry 20-1), Bunseki Kagaku (Analytical Chemistry)", 5th edition, pages 130 to 151 (2007), edited by the Chemical Society of Japan, published by Seishiro Murata, Maruzen Co., Ltd.
(GC analysis: gas chromatography analysis)
(GC analysis conditions)
Instrument: GC-1700 (manufactured by Shimadzu Corporation)
Column: DB-5MS (25 m × 0.2 mmϕ × 0.33 µm)
Temperature raising condition: 80°C (3 minutes)→ 10°C/min → 250°C (0 minutes)
Injection temperature: 280°C
Detector temperature: 300°C
Detection method: FID
Column flow rate: 1 mL/min
Internal standard substance: ditolyl ether

The following documents can be referred to for the GC analysis method, as desired.

Document (c): "Shin Jikkenkagaku Koza 9 (A New Course in Experimental Chemistry 9) Bunsekikagaku II (Analytical Chemistry II)", pages 60 to 86 (1977), edited by the Chemical Society of Japan, published by Shingo Iizumi, Maruzen Co., Ltd.

Document (d): "Jikkenkagaku Koza 20-1 (A Course in Experimental Chemistry 20-1), Bunseki Kagaku (Analytical Chemistry)", 5th edition, pages 121 to 129 (2007), edited by the Chemical Society of Japan, published by Seishiro Murata, Maruzen Co., Ltd.
(GC-MS Analysis: Gas Chromatography Mass Spectrometric Analysis)
Analyzer: 6890N Network GC System (manufactured by Agilent Technologies)
Mass detector: 5973N MSD (manufactured by Agilent Technologies)

(¹H-NMR: ¹H nuclear magnetic resonance spectrum)
Instrument: JEOL JMN-ECS-300 or JEOL JMN-Lambda-400 (manufactured by JEOL RESONANCE)
Solvent: CDCl₃ and/or DMSO-d₆
Internal standard substance: tetramethylsilane (TMS) and others known to a person skilled in the art.

### (Powder X-ray diffraction pattern measurement)

Instrument: X'Pert PRO (manufactured by PANalytical)
X-ray: Cu-Kα
Measurement format: transmission mode
Voltage: 45 kV
Current: 40 mA
Measurement range (2θ): 3 to 40°
Measurement interval (2θ): 0.013°

### (Yield and Purity)

Unless otherwise specified, the yield in the present invention can be calculated from the number of moles of the obtained target compound with respect to the number of moles of the raw material compound (starting compound).

That is, the term "yield" means "molar yield".

Thus, the yield is represented by the following equation: Yield (%) = (the number of moles of the target compound obtained)/(the number of moles of the starting compound) × 100.

However, for example, in the evaluation of the reaction yield of the target product, the yield of impurities, the purity of the product, etc., HPLC area percentage analysis or GC area percentage analysis may be employed.

Herein, room temperature and ordinary temperature are from 10°C to 30°C.

Herein, the term "overnight" means from 8 hours to 16 hours.

Herein, the procedure of "age/aged/aging" includes stirring a mixture by the usual manner known to a person skilled in the art.

### Examples

### [Example 1]

Production of 3-[(5-difluoromethoxy-1-methyl-3-trifluoromethylpyrazol-4-yl)methylthio]-4,5-dihydro-5,5-dimethylisoxazole (Compound 4-a)

### Example 1-1

### (Step i)

### Production of 4-chloromethyl-5-difluoromethoxy-1-methyl-3-trifluoromethylpyrazole (Compound 2-a)

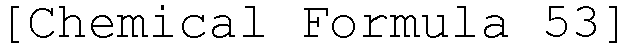

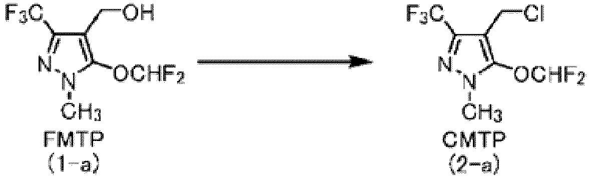

To 5-difluoromethoxy-4-hydroxymethyl-1-methyl-3-trifluoromethylpyrazole (1-a) (46.7 g, purity: 68.6%, containing acetonitrile, 0.13 mol, 100 mol%) was added thionyl chloride (17.0 g, 0.14 mol, 110 mol%) dropwise at an internal temperature of 20°C to 30°C over 1 hour. After the dropwise addition, the mixture was aged at an internal temperature of 20°C to 30°C for 1 hour. After the completion of the reaction, nitrogen was blown into the reaction mixture for 30 minutes to remove the excess thionyl chloride, and ethyl acetate (78 mL, 0.6 L/mol) was added thereto. The obtained solution of the title compound (2-a) in ethyl acetate weighed 134 g.

### (Step ii)

### Production of 3-[(5-difluoromethoxy-1-methyl-3-trifluoromethylpyrazol-4-yl)methylthio]-4,5-dihydro-5,5-dimethylisoxazole (Compound 4-a)

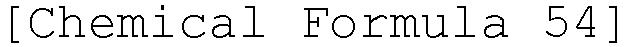

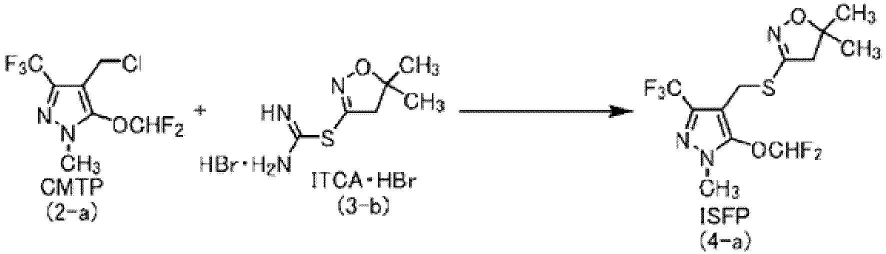

The solution (134 g, corresponding to 0.13 mol scale) of 4-chloromethyl-5-difluoromethoxy-1-methyl-3-trifluoromethyl-pyrazole (2-a) in ethyl acetate produced in the step i was cooled to an internal temperature of 10°C or lower with ice-cooling and stirring. To this was added an aqueous solution (134.6 g, purity: 27%, equivalent to 0.14 mol) of [5,5-dimethyl(4,5-dihydroisoxazolo-3-yl)]thiocarboxamidine hydrobromide (3-b), and then a 48% aqueous sodium hydroxide solution (54.2 g, 0.65 mol, 500 mol%) was added dropwise over 30 minutes such that the internal temperature did not exceed 10°C. After the dropwise addition, the mixture was aged at an internal temperature of 10°C or lower for 30 minutes, then warmed to an internal temperature of 25°C and aged for 4 hours. After the completion of the reaction, the reaction mixture was separated into an organic layer and an aqueous layer. The obtained organic layer was analyzed by the HPLC absolute calibration curve method. As a result, the yield of the target product (4-a) was 91.6% (127.8 g, through 2 steps).

### [Example 1-2] and [Example 1-3]

The reaction and the analysis were performed in the same manner as in Example 1-1, except that isopropyl acetate or butyl acetate were used as a solvent instead of ethyl acetate. The results are shown in the following Table 2. The results of Example 1-1 are also summarized in the table.

**[Table 2]**

| | Step ii | | |
|---|---|---|---|
| | Solvent | L/mol | Yield |
| Example 1-1 | Ethyl acetate | 0.6 | 91.6 |
| Example 1-2 | Isopropyl acetate | 0.6 | 90.4 |
| Example 1-3 | Butyl acetate | 0.6 | 92.5 |

In the above table, the yield means the yield through two steps.

### [Example 1-4]

### (Step i)

### Production of 4-chloromethyl-5-difluoromethoxy-1-methyl-3-trifluoromethylpyrazole (Compound 2-a)

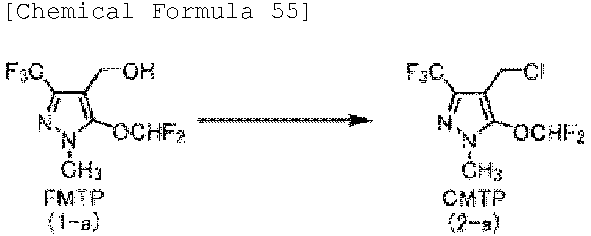

To 5-difluoromethoxy-4-hydroxymethyl-1-methyl-3-trifluoromethylpyrazole (1-a) (123.2 g, purity: 69.9%, containing acetonitrile, 0.35 mol, 100 mol%) was added thionyl chloride (45.8 g, 0.39 mol, 110 mol%) dropwise at an internal temperature of 20°C to 30°C over 1 hour. After the dropwise addition, the mixture was aged at an internal temperature of 20°C to 30°C for 1 hour. After the completion of the reaction, nitrogen was blown into the reaction mixture for 30 minutes to remove the excess thionyl chloride, and butyl acetate (280 mL, 0.9 L/mol) was added. The obtained solution was used in the next step.

### (Step ii)

### Production of 3-[(5-difluoromethoxy-1-methyl-3-trifluoromethylpyrazol-4-yl)methylthio]-4,5-dihydro-5,5-dimethylisoxazole (Compound 4-a)

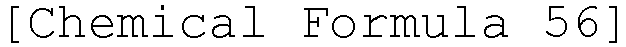

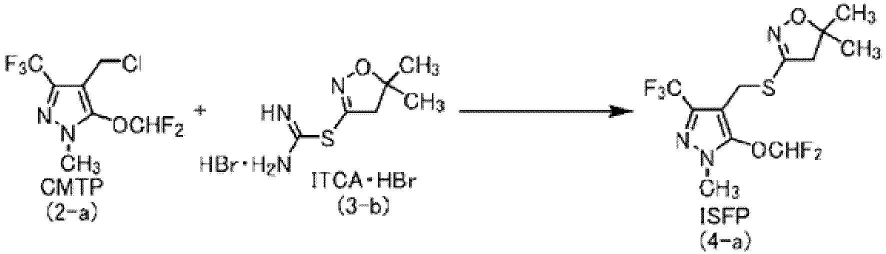

The solution of 4-chloromethyl-5-difluoromethoxy-1-methyl-3-trifluoromethyl-pyrazole (2-a) (corresponding to 0.35 mol scale) in butyl acetate produced in the step i was cooled to an internal temperature of 10°C or lower with ice-cooling and stirring. To this was added an aqueous solution of [5,5-dimethyl(4,5-dihydroisoxazolo-3-yl)]thiocarboxamidine hydrobromide (3-b) (362.4 g, purity: 27%, equivalent to 0.39 mol), and then a 48% aqueous sodium hydroxide solution (145.8 g, 1.8 mol, 500 mol%) was added dropwise over 2 hours such that the internal temperature did not exceed 10°C. After the dropwise addition, the mixture was aged at an internal temperature of 10°C or lower for 30 minutes, then warmed to an internal temperature of 25°C and aged for 4 hours. After the completion of the reaction, the reaction mixture was separated into an organic layer and an aqueous layer. The obtained organic layer was analyzed by the HPLC absolute calibration curve method. As a result, the yield of the target product (4-a) was 87.4% (287.8 g, through 2 steps).

### [Example 1-5]

### (Step i)

### Production of 4-chloromethyl-5-difluoromethoxy-1-methyl-3-trifluoromethylpyrazole (Compound 2-a)

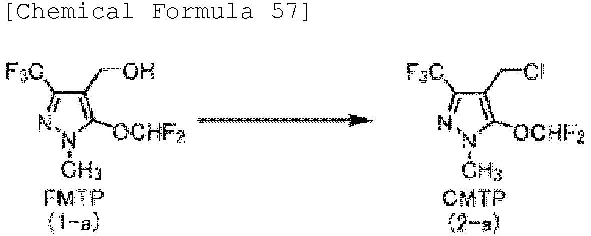

To 5-difluoromethoxy-4-hydroxymethyl-1-methyl-3-trifluoromethylpyrazole (1-a) (45.8 g, purity: 69.9%, containing acetonitrile, 0.13 mol, 100 mol%) was added thionyl chloride (17.0 g, 0.14 mol, 110 mol%) dropwise at an internal temperature of 20°C to 30°C over 1 hour. After the dropwise addition, the mixture was aged at an internal temperature of 20°C to 30°C for 1 hour. After the completion of the reaction, nitrogen was blown into the reaction mixture for 30 minutes to remove the excess thionyl chloride, and butanol (130 mL, 1.0 L/mol) was added. The obtained solution of the title compound (2-a) in butanol weighed 168 g.

### (Step ii)

### Production of 3-[(5-difluoromethoxy-1-methyl-3-trifluoromethylpyrazol-4-yl)methylthio]-4,5-dihydro-5,5-dimethylisoxazole

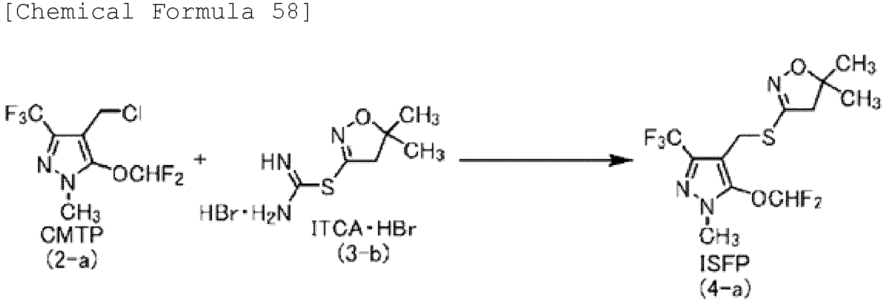

The solution of 4-chloromethyl-5-difluoromethoxy-1-methyl-3-trifluoromethyl-pyrazole (2-a) (corresponding to 0.13 mol scale) in butanol produced in the step i was adjusted to an internal temperature of 20°C to 30°C. To this was added an aqueous solution of [5,5-dimethyl(4,5-dihydroisoxazolo-3-yl)]thiocarboxamidine hydrobromide (3-b) (134.6 g, purity: 27%, equivalent to 0.14 mol), and then a 48% aqueous sodium hydroxide solution (54.2 g, 0.65 mol, 500 mol%) was added dropwise over 30 minutes at an internal temperature of 20°C to 30°C. After the dropwise addition, the mixture was aged at an internal temperature of 20°C to 30°C for 3 hours. After the completion of the reaction, the reaction mixture was separated into an organic layer and an aqueous layer. The obtained organic layer was analyzed by the HPLC absolute calibration curve method. As a result, the yield of the target product (4-a) was 91.9% (185.7 g, through 2 steps).

### [Example 1-6]

### (Step i)

### Production of 4-chloromethyl-5-difluoromethoxy-1-methyl-3-trifluoromethylpyrazole (Compound 2-a)

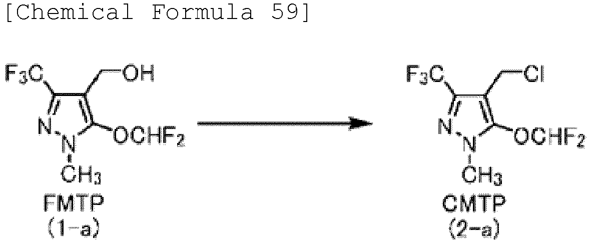

To 5-difluoromethoxy-4 hydroxymethyl-1-methyl-3-trifluoromethylpyrazole (1-a) (35.5 g, purity: 69.4%, containing acetonitrile, 0.10 mol, 100 mol%) was added thionyl chloride (13.1 g, 0.11 mol, 110 mol%) dropwise at an internal temperature of 15°C to 20°C over 1 hour. After the dropwise addition, the mixture was aged at an internal temperature of 15°C to 20°C for 1 hour. After the completion of the reaction, nitrogen was blown into the reaction mixture for 30 minutes to remove the excess thionyl chloride, and 80 mL (0.8 L/mol) of acetonitrile was added. The obtained solution was analyzed by a GC internal standard method. As a result, the yield of the title compound (2-a) was 96.4%.

### (Step ii)

### Production of 3-[(5-difluoromethoxy-1-methyl-3-trifluoromethylpyrazol-4-yl)methylthio]-4,5-dihydro-5,5-dimethylisoxazole (Compound 4-a)

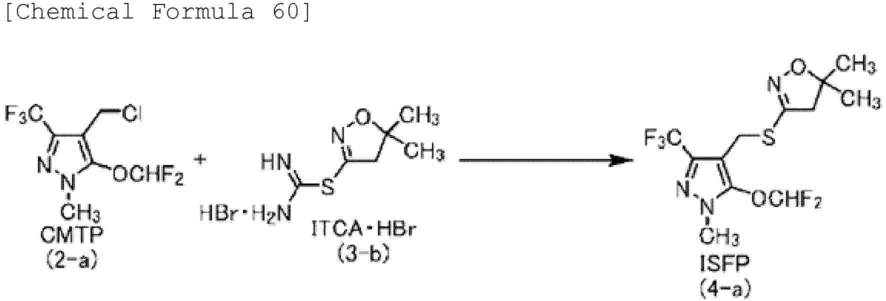

A 48% aqueous sodium hydroxide solution (50.0 g, 0.6 mol, 600 mol%) was added to a reaction flask, an aqueous solution of [5,5-dimethyl(4,5-dihydroisoxazolo-3-yl)]thiocarboxamidine hydrobromide (3-b) (111.8 g, purity: 25%, 0.11 mol, 110 mol%) was added dropwise at an internal temperature of 15°C to 25°C over 1 hour. After the dropwise addition, the mixture was aged at an internal temperature of 15°C to 20°C for 30 minutes. To the reaction mixture, a solution of 4-chloromethyl-5-difluoromethoxy-1-methyl-3-trifluoromethyl-pyrazole (2-a) in acetonitrile produced in the step i was added dropwise at an internal temperature of 15°C to 25°C over 1 hour, and the resulting mixture was aged at room temperature overnight. The obtained reaction mixture was separated into an organic layer and an aqueous layer. The obtained organic layer was analyzed by the HPLC absolute calibration curve method. As a result, the yield of the target product (4-a) was 94.6% (101.5 g, through 2 steps).

### [Example 1-7]

### (Step i)

### Production of 4-chloromethyl-5-difluoromethoxy-1-methyl-3-trifluoromethylpyrazole (Compound 2-a)

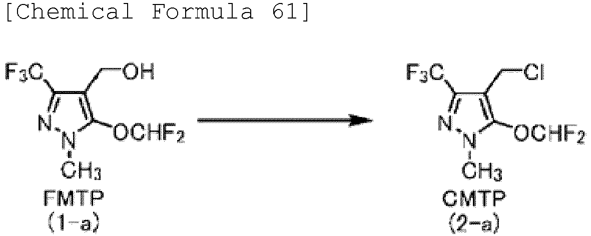

To 5-difluoromethoxy-4-hydroxymethyl-1-methyl-3-trifluoromethylpyrazole (1-a) (35.5 g, purity: 69.4%, containing acetonitrile, 0.10 mol, 100 mol%) was added 80 mL (0.8 L/mol) of acetonitrile. Thionyl chloride (13.1 g, 0.11 mol, 110 mol%) was added dropwise at an internal temperature of 15°C to 20°C over 1 hour. After the dropwise addition, the mixture was aged at an internal temperature of 15°C to 20°C for 1 hour. After the completion of the reaction, nitrogen was blown into the reaction mixture for 30 minutes to remove the excess thionyl chloride. The obtained solution was analyzed by a GC internal standard method. As a result, the yield of the title compound (2-a) was 97.3%.

### (Step ii)

### Production of 3-[(5-difluoromethoxy-1-methyl-3-trifluoromethylpyrazol-4-yl)methylthio]-4,5-dihydro-5,5-dimethylisoxazole (Compound 4-a)

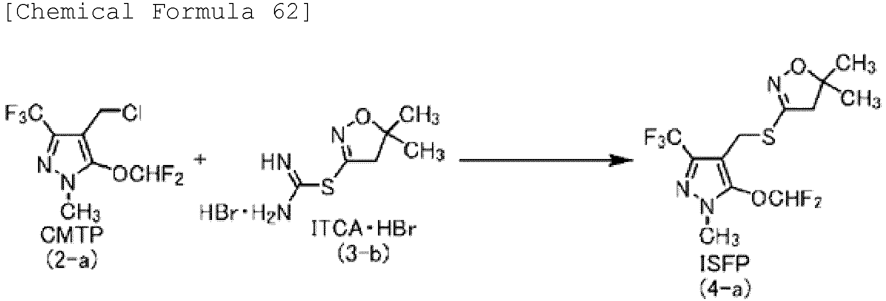

A 48% aqueous sodium hydroxide solution (50.0 g, 0.6 mol, 600 mol%) was added to a reaction flask, an aqueous solution of [5,5-dimethyl(4,5-dihydroisoxazolo-3-yl)]thiocarboxamidine hydrobromide (3-b) (111.8 g, purity: 25%, 0.11 mol, 110 mol%) was added dropwise at an internal temperature of 15°C to 25°C over 1 hour. To the reaction mixture, a solution of 4-chloromethyl-5-difluoromethoxy-1-methyl-3-trifluoromethyl-pyrazole (2-a) in acetonitrile produced in the step i was added dropwise at an internal temperature of 15°C to 25°C over 1 hour, and the resulting mixture was aged at room temperature overnight. The obtained reaction mixture was separated into an organic layer and an aqueous layer. The obtained organic layer was analyzed by the HPLC absolute calibration curve method. As a result, the yield of the target product (4-a) was 94.6% (101.5 g, through 2 steps).

### [Example 1-8]

### (Step i)

### Production of 4-chloromethyl-5-difluoromethoxy-1-methyl-3-trifluoromethylpyrazole (Compound 2-a)

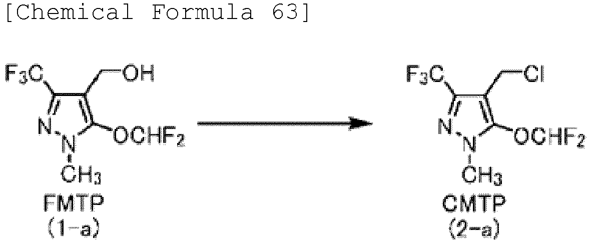

Acetonitrile (320 ml, 0.8 L/mol) was added as a solvent to 5-difluoromethoxy-4 hydroxymethyl-1-methyl-3-trifluoromethylpyrazole (1-a) (116.6 g, purity: 84.4%, containing acetonitrile, 0.40 mol, 100 mol%) to dilute. With water-cooling and stirring, thionyl chloride (52.4 g, 0.44 mol, 110 mol%) was added dropwise over 1 hour such that the internal temperature did not exceed 20°C. After the dropwise addition, the mixture was aged at an internal temperature of 25°C or lower for 30 minutes. After the completion of the reaction, nitrogen was blown into the reaction mixture for 30 minutes to remove the excess thionyl chloride. As a result, a reddish brown solution (366 g) of the title compound (2-a) in acetonitrile was obtained.

### (Step ii)

### Production of 3-[(5-difluoromethoxy-1-methyl-3-trifluoromethylpyrazol-4-yl)methylthio]-4,5-dihydro-5,5-dimethylisoxazole (Compound 4-a)

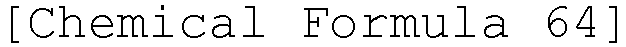

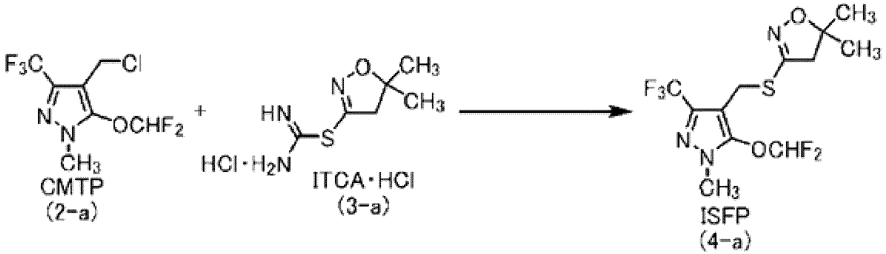

The solution of 4-chloromethyl-5-difluoromethoxy-1-methyl-3-trifluoromethyl-pyrazole (2-a) (188 g, corresponding to 0.2 mol scale) in acetonitrile produced in the step i was cooled to an internal temperature of 10°C or lower with ice-cooling and stirring. To this was added an aqueous solution (183.3 g, purity: 30%, equivalent to 0.22 mol) of [5,5-dimethyl(4,5-dihydroisoxazolo-3-yl)]thiocarboxamidine hydrochloride (3-a), and then a 48% aqueous sodium hydroxide solution (83.3 g, 1.0 mol, 500 mol%) was added dropwise over 30 minutes such that the internal temperature did not exceed 10°C. After the dropwise addition, the mixture was aged at an internal temperature of 10°C or lower for 30 minutes, then warmed to an internal temperature of 25°C and aged for 4 hours. The obtained reaction mixture was separated into an organic layer and an aqueous layer. The obtained organic layer was analyzed by the HPLC absolute calibration curve method. As a result, the yield of the target product (4-a) was 94.1% (181.8 g, through 2 steps).

### [Example 1-9]

### (Step ii)

### Production of 3-[(5-difluoromethoxy-1-methyl-3-trifluoromethylpyrazol-4-yl)methylthio]-4,5-dihydro-5,5-dimethylisoxazole (Compound 4-a)

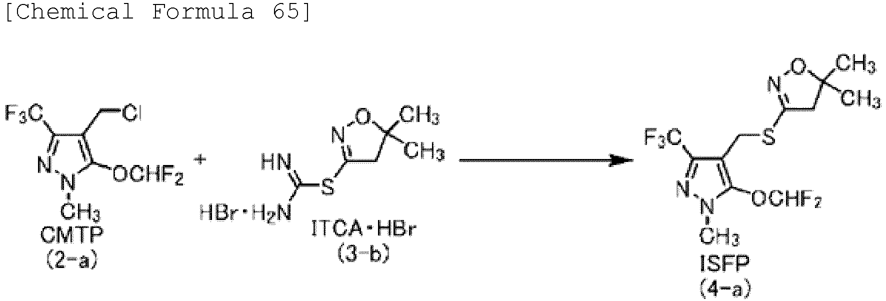

To 4-chloromethyl-5-difluoromethoxy-1-methyl-3-trifluoromethylpyrazole (2-a) (29.1 g, 0.11 mol, 100 mol%) was added acetonitrile (88 mL, 0.8 L/mol) to prepare the acetonitrile solution. To this was added a 48% aqueous sodium hydroxide solution (54.2 g, 0.66 mol, 600 mol%), and the internal temperature was adjusted to 5°C to 10°C. To this was added an aqueous solution (103.7 g, 0.12 mol, 110 mol%) of [5,5-dimethyl(4,5-dihydroisoxazolo-3-yl)]thiocarboxamidine hydrobromide (3-b) dropwise at an internal temperature of 5°C to 10°C over 1 hour. Further, a 48% aqueous sodium hydroxide solution (3.0 g, 0.04 mol, 30 mol%) was added, and the mixture was aged at room temperature overnight. After the completion of the reaction, the reaction mixture was separated into an organic layer and an aqueous layer. The obtained organic layer was analyzed by the HPLC absolute calibration curve method. As a result, the yield of the target product (4-a) was 91.9% (88.1 g).

### [Example 1-10]

### (Step ii)

### Production of 3-[(5-difluoromethoxy-1-methyl-3-trifluoromethylpyrazol-4-yl)methylthio]-4,5-dihydro-5,5-dimethylisoxazole (Compound 4-a)

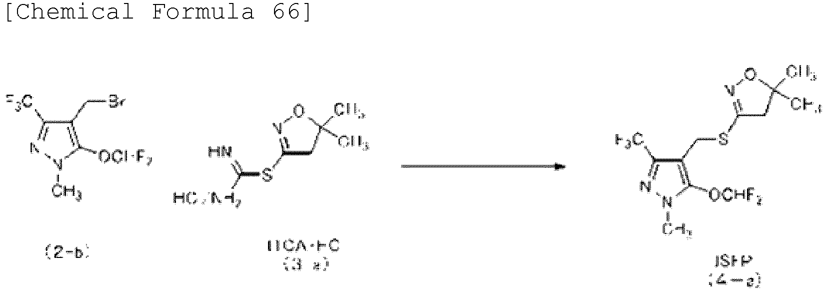

Diethyl ether (20 mL, 2.3 L/mol) was added to an aqueous solution (2.5 g, 0.012 mol, 100 mol%) of [5,5-dimethyl(4,5-dihydroisoxazolo-3-yl)]thiocarboxamidine hydrochloride (3-a) to prepare the solution in diethyl ether. To this was added a 10% aqueous sodium hydroxide solution (16.7g, 0.042 mol, 350 mol%), and the temperature was adjusted to room temperature. To this was added 4-bromomethyl-5-difluoromethoxy-1-methyl-3-trifluoromethyl-pyrazole (2-b) (2.5 g, 0.012 mol, 100 mol%), and the mixture was aged at room temperature overnight. After concentration in vacuo, the obtained crystals were analyzed. As a result, the yield of the target product (4-a) was 94% (4.05 g).

### [Example 2]

### Production of 3-[(5-difluoromethoxy-1-methyl-3-trifluoromethylpyrazol-4-yl)methylsulfonyl]-4,5-dihydro-5,5-dimethylisoxazole (compound 5-a)

### [Example 2-1]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

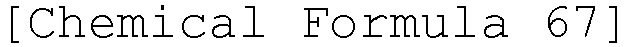

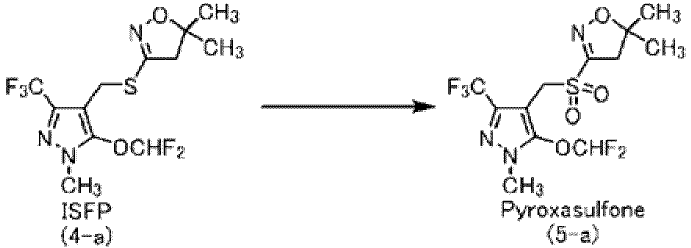

Under a nitrogen stream, a solution of the compound (4-a) produced in Example 1-3 in butyl acetate (87.2 g, purity: 41.2%, 100 mmol, 100 mol%, containing 51.3 g (0.6 L/mol) of butyl acetate), 10 ml (0.1 L/mol) of water and sodium tungstate dihydrate (1.6 g, 5 mmol, 5 mol%) were added to a reaction flask. The mixture was heated to an internal temperature of 70°C to 80°C. A 35% aqueous hydrogen peroxide solution (27.2 g, 280 mmol, 280 mol%, containing 17.7 g (0.2 L/mol) of water) was added dropwise thereto at an internal temperature of 70°C to 80°C over 1 hour, and the mixture was aged for 7 hours while the internal temperature was maintained at 75°C to 80°C.

At this point of time, 3-[(5-difluoromethoxy-1-methyl-3-trifluoromethylpyrazol-4-yl)methylsulfinyl]-4,5-dihydro-5,5-dimethylisoxazole (Compound 6-a; SO derivative), which is a reaction intermediate, was 0.6% (HPLC area percentage; 230 nm).

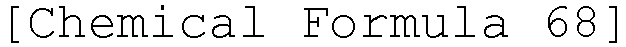

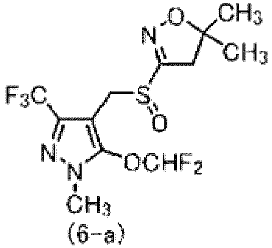

A 20% aqueous sodium sulfite solution (31.5 g, 50 mmol, 50 mol%) was added to the reaction mixture, and the resulting mixture was stirred at an internal temperature of 60°C to 70°C for 30 minutes. The obtained mixture was separated into an organic layer and an aqueous layer. To the obtained organic layer was added 50 ml (0.5 L/mol) of water, and the mixture was concentrated under reduced pressure. To the obtained crude product was added 133.5 ml (1.7 L/mol) of isopropanol, and crystals were separated by filtration at room temperature. The obtained crystals were washed successively with 12.4 ml (0.2 L/mol) of isopropanol and 10 ml (0.1 L/mol) of water. As a result, crystals of the target product (Compound 5-a) were obtained with a yield of 93.0%.

¹H-NMR value (CDCl₃/TMS δ (ppm)): 6.83 (1H, t, J = 71.9 Hz), 4.60 (2H, s), 3.88 (3H, s), 3.11 (2H, s), 1.52 (6H, s)

### [Example 2-2]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

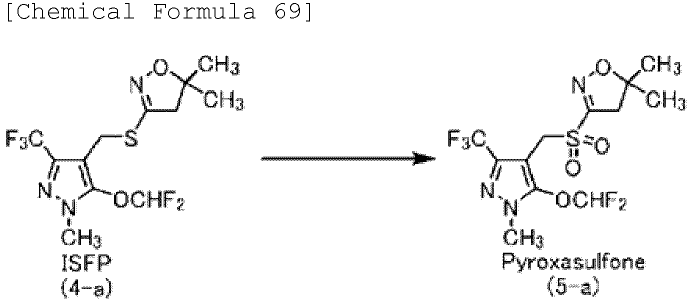

Under a nitrogen stream, a solution of the compound (4-a) in butyl acetate (94.1 g, purity: 38.2%, 100 mmol, 100 mol%, 58.2 g (0.7 L/mol) of butyl acetate), 10 ml (0.1 L/mol) of water and sodium tungstate dihydrate (1.6 g, 5 mmol, 5 mol%) were added to a reaction flask. The mixture was heated to an internal temperature of 70°C to 80°C. A 30% aqueous hydrogen peroxide solution (28.4 g, 250 mmol, 250 mol%, containing 19.9 g (0.2 L/mol) of water) was added dropwise thereto at an internal temperature of 70°C to 80°C over 1 hour, and the mixture was aged for 5 hours while the internal temperature was maintained at 75°C to 80°C.

At this point of time, 3-[(5-difluoromethoxy-1-methyl-3-trifluoromethylpyrazol-4-yl)methylsulfinyl]-4,5-dihydro-5,5-dimethylisoxazole (Compound 6-a; SO derivative), which is a reaction intermediate, was 0.2% (HPLC area percentage; 230 nm).

A 20% aqueous sodium sulfite solution (31.5 g, 50 mmol, 50 mol%) was added to the reaction mixture, and the resulting mixture was stirred at an internal temperature of 60°C to 70°C for 30 minutes. The obtained mixture was separated into an organic layer and an aqueous layer. To the obtained organic layer was added 50 ml (0.5 L/mol) of water, and the mixture was concentrated under reduced pressure. To the obtained crude product was added 133.5 ml (1.7 L/mol) of isopropanol, and crystals were separated by filtration at room temperature. The obtained crystals were washed with 12.4 ml (0.2 L/mol) of isopropanol and 10 ml (0.1 L/mol) of water. As a result, crystals of the target product (Compound 5-a) were obtained with a yield of 89.6%.

### [Example 2-3]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

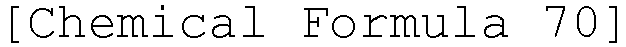

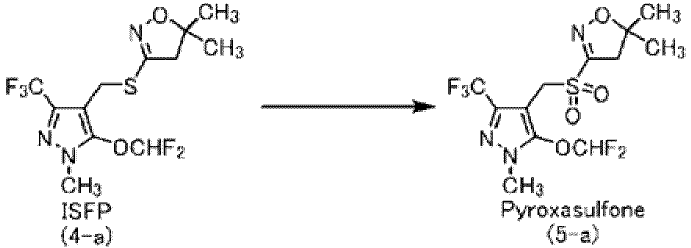

Under a nitrogen stream, a solution of the compound (4-a) in isopropyl acetate (100.6 g, purity: 35.7%, 100 mmol, 100 mol%, containing 64.7 g (0.7 L/mol) of isopropyl acetate), 10 ml (0.1 L/mol) of water and sodium tungstate dihydrate (1.6 g, 5 mmol, 5 mol%) were added to a reaction flask. The mixture was heated to an internal temperature of 70°C to 80°C. A 35% aqueous hydrogen peroxide solution (27.2 g, 280 mmol, 280 mol%, containing 17.7 g (0.2 L/mol) of water) was added dropwise thereto at an internal temperature of 70°C to 80°C over 1 hour, and the mixture was aged for 7 hours while the internal temperature was maintained at 75°C to 80°C.

At this point of time, 3-[(5-difluoromethoxy-1-methyl-3-trifluoromethylpyrazol-4-yl)methylsulfinyl]-4,5-dihydro-5,5-dimethylisoxazole (Compound 6-a; SO derivative), which is a reaction intermediate, was 2.5% (HPLC area percentage; 230 nm).

A 20% aqueous sodium sulfite solution (31.5 g, 50 mmol, 50 mol%) was added to the reaction mixture, and the resulting mixture was stirred at an internal temperature of 60°C to 70°C for 30 minutes. The obtained mixture was separated into an organic layer and an aqueous layer. To the obtained organic layer was added 50 ml (0.5 L/mol) of water, and the mixture was concentrated under reduced pressure. To the obtained crude product was added 133.5 ml (1.7 L/mol) of isopropanol, and crystals were separated by filtration at room temperature. The obtained crystals were washed successively with 12.4 ml (0.2 L/mol) of isopropanol and 10 ml (0.1 L/mol) of water. As a result, crystals of the target product (Compound 5-a) were obtained with a yield of 91.8%.

### [Example 2-4]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

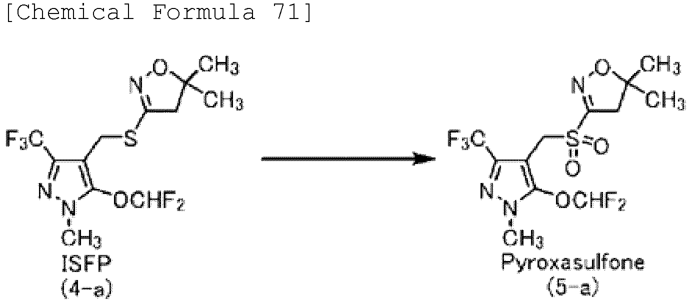

Under a nitrogen stream, a solution of the compound (4-a) in ethyl acetate (107.3 g, purity: 33.5%, 100 mmol, 100 mol%, containing 71.4 g (0.8 L/mol) of ethyl acetate), 10 ml (0.1 L/mol) of water and sodium tungstate dihydrate (1.6 g, 5 mmol, 5 mol%) were added to a reaction flask. The mixture was heated to an internal temperature of 70°C to 80°C. A 35% aqueous hydrogen peroxide solution (27.2 g, 280 mmol, 280 mol%, containing 17.7 g (0.2 L/mol) of water) was added dropwise thereto at an internal temperature of 70°C to 80°C over 1 hour, and the mixture was aged for 7 hours while the internal temperature was maintained at 75°C to 80°C.

At this point of time, 3-[(5-difluoromethoxy-1-methyl-3-trifluoromethylpyrazol-4-yl)methylsulfinyl]-4,5-dihydro-5,5-dimethylisoxazole (Compound 6-a; SO derivative), which is a reaction intermediate, was 1.8% (HPLC area percentage; 230 nm).

A 20% aqueous sodium sulfite solution (31.5 g, 50 mmol, 50 mol%) was added to the reaction mixture, and the resulting mixture was stirred at an internal temperature of 60°C to 70°C for 30 minutes. The obtained mixture was separated into an organic layer and an aqueous layer. To the obtained organic layer was added 50 ml (0.5 L/mol) of water, and the mixture was concentrated under reduced pressure. To the obtained crude product was added 133.5 ml (1.7 L/mol) of isopropanol, and crystals were separated by filtration at room temperature. The obtained crystals were washed successively with 12.4 ml (0.2 L/mol) of isopropanol and 10 ml (0.1 L/mol) of water. As a result, crystals of the target product (Compound 5-a) were obtained with a yield of 89.1%.

### [Example 2-5]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

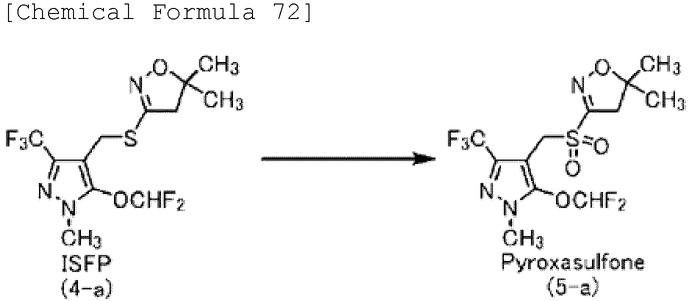

Under a nitrogen stream, a solution of the compound (4-a) in butyl acetate (94.1 g, purity: 38.2%, 100 mmol, 100 mol%, 58.2 g (0.7 L/mol) of butyl acetate), 5.0 g (0.06 L/mol) of butanol, 10 ml (0.1 L/mol) of water and sodium tungstate dihydrate (1.6 g, 5 mmol, 5 mol%) were added to a reaction flask. The mixture was heated to an internal temperature of 70°C to 80°C. A 30% aqueous hydrogen peroxide solution (28.4 g, 250 mmol, 250 mol%, containing 19.9 g (0.2 L/mol) of water) was added dropwise thereto at an internal temperature of 70°C to 80°C over 1 hour, and the mixture was aged for 5 hours while the internal temperature was maintained at 75°C to 80°C.

At this point of time, 3-[(5-difluoromethoxy-1-methyl-3-trifluoromethylpyrazol-4-yl)methylsulfinyl]-4,5-dihydro-5,5-dimethylisoxazole (Compound 6-a; SO derivative), which is a reaction intermediate, was 0.2% (HPLC area percentage; 230 nm).

A 20% aqueous sodium sulfite solution (31.5 g, 50 mmol, 50 mol%) was added to the reaction mixture, and the resulting mixture was stirred at an internal temperature of 60°C to 70°C for 30 minutes. The obtained mixture was separated into an organic layer and an aqueous layer. To the obtained organic layer was added 50 ml (0.5 L/mol) of water, and the mixture was concentrated under reduced pressure. To the obtained crude product was added 117.8 ml (1.5 L/mol) of isopropanol, and crystals were separated by filtration at room temperature. The obtained crystals were washed with 12.4 ml (0.2 L/mol) of isopropanol and 10 ml (0.1 L/mol) of water. As a result, crystals of the target product (Compound 5-a) were obtained with a yield of 92%.

### [Example 2-6]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

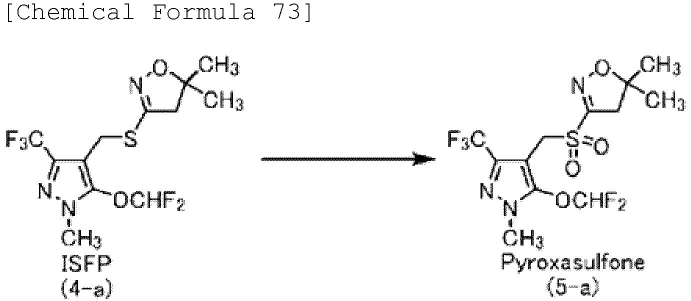

Under a nitrogen stream, a solution of the compound (4-a) in butanol (99.3 g, purity: 36.2%, 100 mmol, 100 mol%, containing 63.4 g (0.8 L/mol) of butanol), 10 ml (0.1 L/mol) of water and sodium tungstate dihydrate (1.0 g, 3 mmol, 3 mol%) were added to a reaction flask. The mixture was heated to an internal temperature of 70°C to 80°C. A 30% aqueous hydrogen peroxide solution (28.4 g, 250 mmol, 250 mol%, containing 19.9 g (0.2 L/mol) of water) was added dropwise thereto at an internal temperature of 90°C to 95°C over 1 hour, and the mixture was aged for 5 hours while the internal temperature was maintained at 90°C to 95°C. Further, a 30% aqueous hydrogen peroxide solution (9.0 g, 80 mmol, 80 mol%, containing 6.3 g (0.1 L/mol) of water) was added dropwise thereto, and the mixture was aged for 2 hours while the internal temperature was maintained at 90°C to 95°C.

At this point of time, 3-[(5-difluoromethoxy-1-methyl-3-trifluoromethylpyrazol-4-yl)methylsulfinyl]-4,5-dihydro-5,5-dimethylisoxazole (Compound 6-a; SO derivative), which is a reaction intermediate, was 2.2% (HPLC area percentage; 230 nm).

A 20% aqueous sodium sulfite solution (31.5 g, 50 mmol, 50 mol%) was added to the reaction mixture, and crystals were separated by filtration at room temperature. As a result, crystals of the target product (Compound 5-a) were obtained with a yield of 91.2%.

### [Example 2-7]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

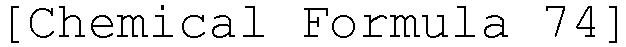

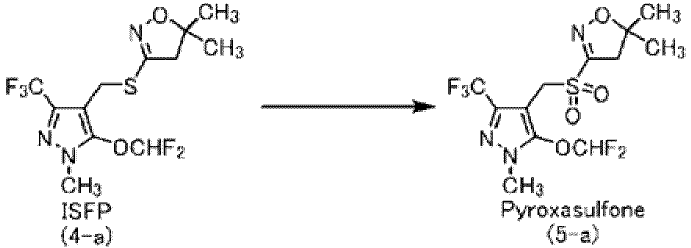

Under a nitrogen stream, a solution of the compound (4-a) in butanol (89.7 g, purity: 20.0%, 50 mmol, 50 mol%, 71.8 g (1.8 L/mol) of butanol), 5 ml (0.1 L/mol) of water, sodium tungstate dihydrate (0.5 g, 1.5 mmol, 3 mol%), tetrabutylammonium hydrogen sulfate (0.2 g, 0.5 mmol, 1 mol%), and phenyl phosphate (0.1 g, 0.5 mmol, 1 mol%) were added to a reaction flask. The mixture was heated to an internal temperature of 75°C to 85°C. A 35% aqueous hydrogen peroxide solution (14.6 g, 150 mmol, 300 mol%, containing 9.5 g (0.2 L/mol) of water) was added dropwise thereto at an internal temperature of 75°C to 85°C over 1 hour, and the mixture was aged for 5 hours while the internal temperature was maintained at 75°C to 85°C. Further, a 35% aqueous hydrogen peroxide solution (4.9 g, 50 mmol, 100 mol%, containing 3.2 g (0.1 L/mol) of water) was added dropwise thereto, and the mixture was aged for 3 hours while the internal temperature was maintained at 75°C to 85°C.

At this point of time, 3-[(5-difluoromethoxy-1-methyl-3-trifluoromethylpyrazol-4-yl)methylsulfinyl]-4,5-dihydro-5,5-dimethylisoxazole (Compound 6-a; SO derivative), which is a reaction intermediate, was 0.7% (HPLC area percentage; 230 nm).

A 10% aqueous sodium sulfite solution (12.6 g, 10 mmol, 20 mol%) was added to the reaction mixture, and crystals were separated by filtration at room temperature. The obtained crystals were washed successively with 10 ml (0.2 L/mol) of butanol and 10 ml (0.2 L/mol) of water. As a result, crystals of the target product (Compound 5-a) were obtained with a yield of 90.0%.

### [Example 2-8]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

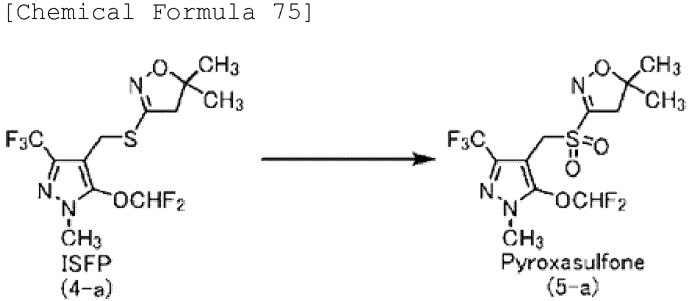

Under a nitrogen stream, the compound (4-a) (3.05 g, purity: 100%, 8.5 mmol, 100 mol%, 6.7 g (1.0 L/mol)) of ethanol, 0.9 ml (0.1 L/mol) of water, sodium tungstate dihydrate (0.084 g, 0.3 mmol, 3 mol%), tetrabutylammonium hydrogen sulfate (0.087 g, 0.3 mmol, 3 mol%), and phenyl phosphate (0.083 g, 0.3 mmol, 3 mol%) were added to a reaction flask. The mixture was heated to an internal temperature of 75°C to 80°C. A 35% aqueous hydrogen peroxide solution (4.13 g, 42.5 mmol, 500 mol%, containing 2.7 g (0.3 L/mol) of water) was added dropwise thereto at an internal temperature of 75°C to 80°C over 1 hour, and the mixture was aged for 3 hours while the internal temperature was maintained at 75°C to 80°C.

At this point of time, 3-[(5-difluoromethoxy-1-methyl-3-trifluoromethylpyrazol-4-yl)methylsulfinyl]-4,5-dihydro-5,5-dimethylisoxazole (Compound 6-a; SO derivative), which is a reaction intermediate, was 0.6% (HPLC area percentage; 230 nm).

Acetonitrile was added to the reaction mixture to make it homogeneous. As a result of analysis by the HPLC external standard method, the target product (5-a) was obtained with a yield of 92.3%.

### [Example 2-9]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

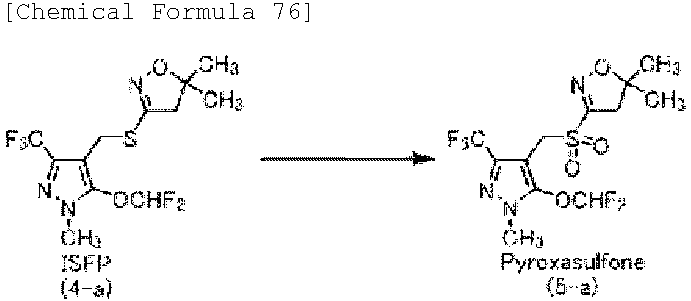

Under a nitrogen stream, the compound (4-a) (3.05 g, purity: 100%, 8.5 mmol, 100 mol%, 6.6 g (1.0 L/mol) of tert-butanol), 0.9 ml (0.1 L/mol) of water, sodium tungstate dihydrate (0.084 g, 0.3 mmol, 3 mol%), tetrabutylammonium hydrogen sulfate (0.087 g, 0.3 mmol, 3 mol%), and phenyl phosphate (0.083 g, 0.3 mmol, 3 mol%) were added to a reaction flask. The mixture was heated to an internal temperature of 75°C to 80°C. A 35% aqueous hydrogen peroxide solution (4.13 g, 42.5 mmol, 500 mol%, containing 2.7 g (0.3 L/mol) of water) was added dropwise thereto at an internal temperature of 75°C to 80°C over 1 hour, and the mixture was aged for 3 hours while the internal temperature was maintained at 75°C to 80°C.

At this point of time, 3-[(5-difluoromethoxy-1-methyl-3-trifluoromethylpyrazol-4-yl)methylsulfinyl]-4,5-dihydro-5,5-dimethylisoxazole (Compound 6-a; SO derivative), which is a reaction intermediate, was 0.6% (HPLC area percentage; 230 nm).

Acetonitrile was added to the reaction mixture to make it homogeneous. As a result of analysis by the HPLC external standard method, the target product (5-a) was obtained with a yield of 95.0%.

### [Example 2-10]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

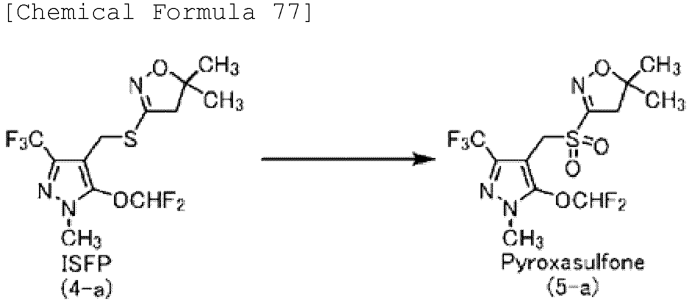

Under a nitrogen stream, the compound (4-a) (3.05 g, purity: 100%, 8.5 mmol, 100 mol%, 6.7 g (1.0 L/mol) of acetonitrile), 0.9 ml (0.1 L/mol) of water, sodium tungstate dihydrate (0.084 g, 0.3 mmol, 3 mol%), tetrabutylammonium hydrogen sulfate (0.087 g, 0.3 mmol, 3 molt), and phenyl phosphate (0.083 g, 0.3 mmol, 3 mol%) were added to a reaction flask. The mixture was heated to an internal temperature of 75°C to 80°C. A 35% aqueous hydrogen peroxide solution (2.48 g, 25.5 mmol, 300 molt, containing 1.6 g (0.2 L/mol) of water) was added dropwise thereto at an internal temperature of 75°C to 80°C over 1 hour, and the mixture was aged for 3 hours while the internal temperature was maintained at 75°C to 80°C.

At this point of time, 3-[(5-difluoromethoxy-1-methyl-3-trifluoromethylpyrazol-4-yl)methylsulfinyl]-4,5-dihydro-5,5-dimethylisoxazole (Compound 6-a; SO derivative), which is a reaction intermediate, was 0.4% (HPLC area percentage; 230 nm).

Acetonitrile was added to the reaction mixture to make it homogeneous. As a result of analysis by the HPLC external standard method, the target product (5-a) was obtained with a yield of 95.7%.

### [Example 2-11]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

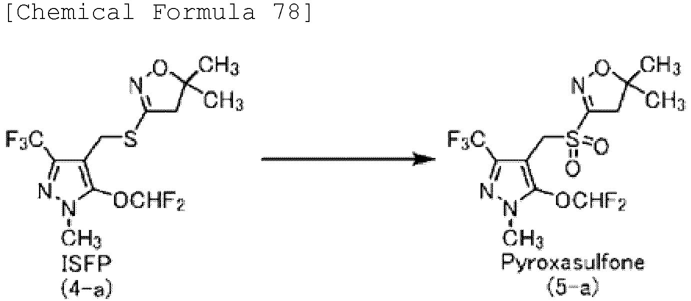

Under a nitrogen stream, the compound (4-a) (3.05 g, purity: 100%, 8.5 mmol, 100 mol%), 6.7 g (1.0 L/mol) of methanol), 0.9 ml (0.1 L/mol) of water, sodium tungstate dihydrate (0.084 g, 0.3 mmol, 3 mol%), tetrabutylammonium hydrogen sulfate (0.087 g, 0.3 mmol, 3 mol%), and phenyl phosphate (0.083 g, 0.3 mmol, 3 mol%) were added to a reaction flask. The mixture was heated to an internal temperature of 65°C to 70°C. A 35% aqueous hydrogen peroxide solution (4.13 g, 42.5 mmol, 500 mol%, containing 2.7 g (0.3 L/mol) of water) was added dropwise thereto at an internal temperature of 65°C to 70°C over 1 hour, and the mixture was aged for 6 hours while the internal temperature was maintained at 65°C to 70°C.

At this point of time, 3-[(5-difluoromethoxy-1-methyl-3-trifluoromethylpyrazol-4-yl)methylsulfinyl]-4,5-dihydro-5,5-dimethylisoxazole (Compound 6-a; SO derivative), which is a reaction intermediate, was 1.8% (HPLC area percentage; 230 nm) .

Acetonitrile was added to the reaction mixture to make it homogeneous. As a result of analysis by the HPLC external standard method, the target product (5-a) was obtained with a yield of 96%.

### [Example 2-12]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

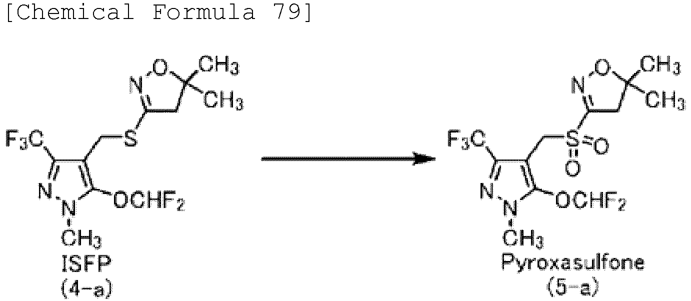

Under a nitrogen stream, the compound (4-a) (3.05 g, purity: 100%, 8.5 mmol, 100 mol%), 6.7 g (1.0 L/mol) of methanol, 0.9 ml (0.1 L/mol) of water, and sodium tungstate dihydrate (0.084 g, 0.3 mmol, 3 mol%), and sulfuric acid (0.087 g, 0.85 mmol, 10 mol%) were added to a reaction flask. The mixture was heated to an internal temperature of 65°C to 70°C. A 35% aqueous hydrogen peroxide solution (4.13 g, 42.5 mmol, 500 mol%, containing 2.7 g (0.3 L/mol) of water) was added dropwise thereto at an internal temperature of 65°C to 70°C over 1 hour, and the mixture was aged for 6 hours while the internal temperature was maintained at 65°C to 70°C.

At this point of time, 3-[(5-difluoromethoxy-1-methyl-3-trifluoromethylpyrazol-4-yl)methylsulfinyl]-4,5-dihydro-5,5-dimethylisoxazole (Compound 6-a; SO derivative), which is a reaction intermediate, was 1.4% (HPLC area percentage; 230 nm).

Acetonitrile was added to the reaction mixture to make it homogeneous. As a result of the analysis by the HPLC external standard method, the target product (5-a) was obtained with a yield of 96%.

### [Example 2-13]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

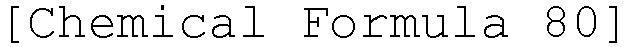

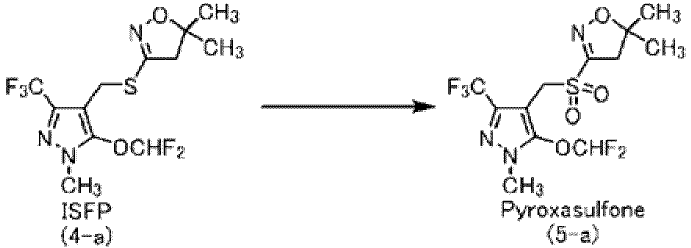

Under a nitrogen stream, a solution of the compound (4-a) in acetonitrile (86.2 g, purity: 41.7%, 100 mmol, 100 mol%, containing 50.3 g (0.6 L/mol) of acetonitrile), 10 ml

(0.1 L/mol) of water and sodium tungstate dihydrate (1.6 g, 5 mmol, 5 mol%) were added to a reaction flask. The mixture was heated to an internal temperature of 70°C to 80°C. A 35% aqueous hydrogen peroxide solution (24.3 g, 250 mmol, 250 mol%, containing 15.8 g (0.2 L/mol) of water) was added dropwise thereto at an internal temperature of 70°C to 80°C over 1 hour, and the mixture was aged for 5 hours while the internal temperature was maintained at 75°C to 80°C.

At this point of time, 3-[(5-difluoromethoxy-1-methyl-3-trifluoromethylpyrazol-4-yl)methylsulfinyl]-4,5-dihydro-5,5-dimethylisoxazole (Compound 6-a; SO derivative), which is a reaction intermediate, was 0.5% (HPLC area percentage; 230 nm).

A 20% aqueous sodium sulfite solution (31.5 g, 50 mmol, 50 mol%) was added to the reaction mixture, and the resulting mixture was stirred at an internal temperature of 60°C to 70°C for 30 minutes. The obtained mixture was separated into an organic layer and an aqueous layer. To the obtained organic layer was added 50 ml (0.5 L/mol) of water, and the mixture was concentrated under reduced pressure. To the obtained crude product was added 117.8 ml (1.5 L/mol) of isopropanol, and crystals were separated by filtration at room temperature. The obtained crystals were washed successively with 12.4 ml (0.2 L/mol) of isopropanol and 10 ml (0.1 L/mol) of water. As a result, crystals of the target product (Compound 5-a) were obtained with a yield of 95.0%.

### [Example 2-14]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

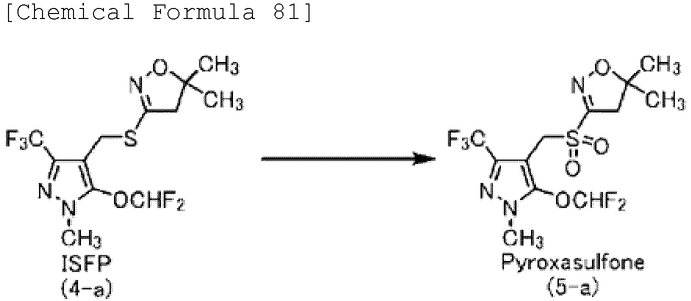

Under a nitrogen stream, a solution of the compound (4-a) in acetonitrile (88.9 g, purity: 40.4%, 100 mmol, 100 mol%, containing 65 g (0.8 L/mol) of acetonitrile), 10 ml

(0.1 L/mol) of water and sodium tungstate dihydrate (1.0 g, 3 mmol, 3 mol%) were added to a reaction flask. The mixture was heated to an internal temperature of 75°C to 80°C. A 35% aqueous hydrogen peroxide solution (22.4 g, 230 mmol, 230 mol%, containing 15 g (0.15 L/mol) of water) was added dropwise thereto at an internal temperature of 75°C to 80°C over 1 hour, and the mixture was aged for 5 hours while the internal temperature was maintained at 75°C to 80°C.

At this point of time, 3-[(5-difluoromethoxy-1-methyl-3-trifluoromethylpyrazol-4-yl)methylsulfinyl]-4,5-dihydro-5,5-dimethylisoxazole (Compound 6-a; SO derivative), which is a reaction intermediate, was 0.5% (HPLC area percentage; 230 nm).

A 17% aqueous sodium sulfite solution (22.2 g, 30 mmol, 30 mol%) was added to the reaction mixture, and the resulting mixture was stirred at an internal temperature of 55°C to 65°C for 30 minutes. The obtained mixture was separated into an organic layer and an aqueous layer. To the obtained organic layer was added 50 ml (0.5 L/mol) of water, and the mixture was concentrated under reduced pressure. To the obtained crude product was added 80 ml (0.8 L/mol) of isopropanol, and crystals were separated by filtration at room temperature. The obtained crystals were washed successively with 10 ml (0.1 L/mol) of isopropanol and 10 ml (0.1 L/mol) of water. As a result, crystals of the target product (5-a) were obtained with a yield of 95.9%.

### [Example 2-15]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

The reaction and the analysis were performed in the same manner as Example 2-14, except that the amount of the acetonitrile solvent used was changed to 1.3 L/mol, the amount of the water solvent (pre-added water) added first was changed to 0.3 L/mol, the amount of the sodium tungstate dihydrate used was changed to 5 mol%, the amount of the 35% aqueous hydrogen peroxide solution used was changed to 300 mmol%, the dropwise addition temperature and the aging temperature were changed to 55°C to 65°C, and the aging time was changed to 3 hours.

At the point when the aging had been performed for 3 hours, 3-[(5-difluoromethoxy-1-methyl-3-trifluoromethylpyrazol-4-yl)methylsulfinyl]-4,5-dihydro-5,5-dimethylisoxazole (Compound 6-a; SO derivative) was 0.1% (HPLC area percentage; 230 nm).

Working-up was performed in the same manner as in Example 2-14. As a result, crystals of the target product (5-a) were obtained with a yield of 92.2%.

### [Example 2-16]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

The reaction and the analysis were performed in the same manner as Example 2-14, except that the amount of the acetonitrile solvent used was changed to 0.45 L/mol, the amount of the water solvent (pre-added water) added first was changed to 0.1 L/mol, the amount of the sodium tungstate dihydrate used was changed to 5 mol%, the amount of the 35% aqueous hydrogen peroxide solution used was changed to 300 mmol%, the dropwise addition temperature and the aging temperature were changed to 60°C to 65°C, and the aging time was changed to 5 hours.

At the point when the aging had been performed for 5 hours, 3-[(5-difluoromethoxy-1-methyl-3-trifluoromethylpyrazol-4-yl)methylsulfinyl]-4,5-dihydro-5,5-dimethylisoxazole (Compound 6-a; SO derivative) was 0.3% (HPLC area percentage; 230 nm). Yield: 90% or more

### [Example 2-17]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

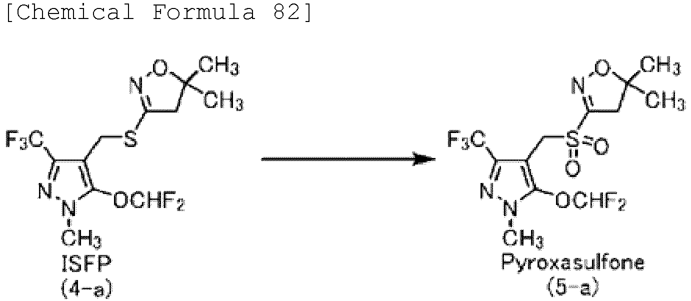

Under a nitrogen stream, a solution of the compound (4-a) in acetonitrile (102.6 g, purity: 35.0%, 100 mmol, 100 mol%, containing 65 g (0.8 L/mol) of acetonitrile), 10 ml (0.1 L/mol) of water and ammonium molybdate tetrahydrate (1.23 g, 1 mmol, 1 mol%) were added to a reaction flask. The mixture was heated to an internal temperature of 75°C to 80°C. A 35% aqueous hydrogen peroxide solution (29.2 g, 300 mmol, 300 mol%, containing 19 g (0.19 L/mol) of water) was added dropwise thereto at an internal temperature of 75°C to 80°C over 1 hour, and the mixture was aged for 2 hours while the internal temperature was maintained at 75°C to 80°C.

At this point of time, 3-[(5-difluoromethoxy-1-methyl-3-trifluoromethylpyrazol-4-yl)methylsulfinyl]-4,5-dihydro-5,5-dimethylisoxazole (Compound 6-a; SO derivative), which is a reaction intermediate, was 0% (HPLC area percentage; 230 nm).

A 20% aqueous sodium sulfite solution (22.2 g, 35 mmol, 35 mol%) was added to the reaction mixture, and the resulting mixture was stirred at an internal temperature of 55°C to 65°C for 30 minutes. The obtained mixture was separated into an organic layer and an aqueous layer. To the obtained organic layer was added 50 ml (0.5 L/mol) of water, and the mixture was concentrated under reduced pressure. To the obtained crude product was added 80 ml (0.8 L/mol) of isopropanol, and crystals were separated by filtration at room temperature. The obtained crystals were washed successively with 10 ml (0.1 L/mol) of isopropanol and 10 ml (0.1 L/mol) of water. As a result, crystals of the target product (5-a) were obtained with a yield of 94.7%.

### [Example 2-18]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

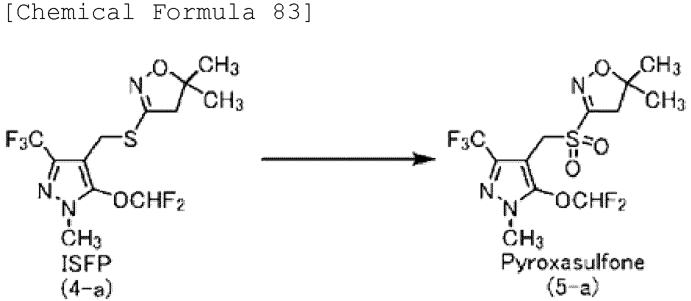

Under a nitrogen stream, the compound (4-a) (100.1 g, purity: 35.0%, 100 mmol, 100 mol%, containing 62.5 g (0.8 L/mol) of 2-propanol), 10 ml (0.1 L/mol) of water and ammonium molybdate tetrahydrate (1.23 g, 1 mmol, 1 mol%) were added to a reaction flask. The mixture was heated to an internal temperature of 75°C to 80°C. A 35% aqueous hydrogen peroxide solution (29.2 g, 300 mmol, 300 mol%, containing 12.8 g (0.12 L/mol) of water) was added dropwise thereto at an internal temperature of 75°C to 80°C over 1 hour, and the mixture was aged for 5 hours while the internal temperature was maintained at 75°C to 80°C.

At this point of time, there remained a reaction intermediate, 3-[(5-difluoromethoxy-1-methyl-3-trifluoromethylpyrazol-4-yl)methylsulfinyl]-4,5-dihydro-5,5-dimethylisoxazole (Compound 6-a; SO derivative). Because of this reason, a 35% aqueous hydrogen peroxide solution (19.5 g, 200 mmol, 200 mol%, containing 19 g (0.19 L/mol) of water) was added dropwise, and the mixture was aged for 5 hours while the internal temperature was maintained at 75°C to 80°C.

At this point of time, 3-[(5-difluoromethoxy-1-methyl-3-trifluoromethylpyrazol-4-yl)methylsulfinyl]-4,5-dihydro-5,5-dimethylisoxazole (Compound 6-a; SO derivative), which is a reaction intermediate, was 2.0% (HPLC area percentage; 230 nm).

The reaction mixture was stirred and cooled to room temperature and the crystals were separated by filtration at room temperature. The obtained crystals were washed successively with 10 ml (0.1 L/mol) of isopropanol and 10 ml (0.1 L/mol) of water. As a result, crystals of the target product (5-a) were obtained with a yield of 93.2%.

### [Example 2-19]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

The reaction and the analysis were performed in the same manner as in Example 2-17, except that the solvent was changed to dimethylformamide, the amount of the 35% aqueous hydrogen peroxide solution used was changed to 400 mmol%, and the aging time was changed to 8 hours.

At the point when the aging had been performed for 8 hours, 3-[(5-difluoromethoxy-1-methyl-3-trifluoromethylpyrazol-4-yl)methylsulfinyl]-4,5-dihydro-5,5-dimethylisoxazole (Compound 6-a; SO derivative) was 1.9% (HPLC area percentage; 230 nm).

The reaction mixture was stirred and cooled to room temperature, and dimethylformamide was added thereto to form a homogeneous solvent. As a result of analysis by the HPLC external standard method, crystals of the target product (5-a) were obtained with a yield of 95.1%.

### [Example 2-20]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

The reaction and the analysis were performed in the same manner as in Example 2-17, except that the solvent was changed to diethylene glycol dimethyl ether (diglyme) and the aging time after the dropwise addition of the 35% aqueous hydrogen peroxide solution was changed to 1 hour.

At the point when the aging had been performed for 1 hour, 3-[(5-difluoromethoxy-1-methyl-3-trifluoromethylpyrazol-4-yl)methylsulfinyl]-4,5-dihydro-5,5-dimethylisoxazole (Compound 6-a; SO derivative) was 0% (HPLC area percentage; 230 nm).

Dimethylformamide was added to the reaction mixture to make it homogeneous. As a result of analysis by the HPLC external standard method, the target product (5-a) was obtained with a yield of 96.9%.

### [Example 2-21]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

The compound (4-a) (0.9 g, purity: 100%, 100 mmol, 100 mol%), 2 ml (0.8 L/mol) of acetonitrile and ammonium molybdate tetrahydrate (0.03 g, 0.025 mmol, 1 mol%) were added to a reaction vessel. The mixture was heated to 80°C. A 35% aqueous hydrogen peroxide solution (0.85 g, 350 mmol, 350 mol%, containing 0.55 g (0.2 L/mol) of water) was added thereto, and the mixture was aged for 6 hours.

At this point of time, 3-[(5-difluoromethoxy-1-methyl-3-trifluoromethylpyrazol-4-yl)methylsulfinyl]-4,5-dihydro-5,5-dimethylisoxazole (Compound 6-a; SO derivative), which is a reaction intermediate, was 0% (HPLC area percentage; 230 nm) .

In addition, 3-[(5-difluoromethoxy-1-methyl-3-trifluoromethylpyrazol-4-yl)methylsulfonyl]-4,5-dihydro-5,5-dimethylisoxazole (Compound 5-a; SO2 derivative), which is the target product, was 99.4% (HPLC area percentage; 230 nm), and
3-[(5-difluoromethoxy-1-methyl-3-trifluoromethylpyrazol-4-yl)methylthio]-4,5-dihydro-5,5-dimethylisoxazole (Compound 4-a; S derivative) was 0% (HPLC area percentage; 230 nm).

### [Example 2-22]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

The compound (4-a) (0.9 g, purity: 100%, 100 mmol, 100 mol%), 2 ml (0.8 L/mol) of ethanol and ammonium molybdate tetrahydrate (0.03 g, 0.025 mmol, 1 mol%) were added to a reaction vessel. The mixture was heated to 80°C. A 35% aqueous hydrogen peroxide solution (0.85 g, 350 mmol, 350 mol%, containing 0.55 g (0.2 L/mol) of water) was added thereto, and the mixture was aged for 6 hours.

At this point of time, 3-[(5-difluoromethoxy-1-methyl-3-trifluoromethylpyrazol-4-yl)methylsulfinyl]-4,5-dihydro-5,5-dimethylisoxazole (Compound 6-a; SO derivative), which is a reaction intermediate, was 0% (HPLC area percentage; 230 nm).

In addition, 3-[(5-difluoromethoxy-1-methyl-3-trifluoromethylpyrazol-4-yl)methylsulfonyl]-4,5-dihydro-5,5-dimethylisoxazole (Compound 5-a; SO2 derivative), which is the target product, was 99.4% (HPLC area percentage; 230 nm), and
3-[(5-difluoromethoxy-1-methyl-3-trifluoromethylpyrazol-4-yl)methylthio]-4,5-dihydro-5,5-dimethylisoxazole (Compound 4-a; S derivative) was 0% (HPLC area percentage; 230 nm).

### [Example 2-23]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

The compound (4-a) (0.9 g, purity: 100%, 100 mmol, 100 mol%), 2 ml (0.8 L/mol) of N-methylpyrrolidone and ammonium molybdate tetrahydrate (0.03 g, 0.025 mmol, 1 mol%) were added to a reaction vessel. The mixture was heated to 80°C. A 35% aqueous hydrogen peroxide solution (0.85 g, 350 mmol, 350 mol%, containing 0.55 g (0.2 L/mol) of water) was added thereto, and the mixture was aged for 6 hours.

At this point of time, 3-[(5-difluoromethoxy-1-methyl-3-trifluoromethylpyrazol-4-yl)methylsulfinyl]-4,5-dihydro-5,5-dimethylisoxazole (Compound 6-a; SO derivative), a reaction intermediate, was 0% (HPLC area percentage; 230 nm) .

In addition, 3-[(5-difluoromethoxy-1-methyl-3-trifluoromethylpyrazol-4-yl)methylsulfonyl]-4,5-dihydro-5,5-dimethylisoxazole (Compound 5-a; SO2 derivative), which is the target product, was 98.0% (HPLC area percentage; 230 nm), and

3-[(5-difluoromethoxy-1-methyl-3-trifluoromethylpyrazol-4-yl)methylthio]-4,5-dihydro-5,5-dimethylisoxazole (Compound 4-a; S derivative) was 0% (HPLC area percentage; 230 nm).

### [Example 2-24]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

The compound (4-a) (0.9 g, purity: 100%, 100 mmol, 100 mol%), 2 ml (0.8 L/mol) of sulfolane and ammonium molybdate tetrahydrate (0.03 g, 0.025 mmol, 1 mol%) were added to a reaction vessel. The mixture was heated to 80°C. A 35% aqueous hydrogen peroxide solution (0.85 g, 350 mmol, 350 mol%, containing 0.55 g (0.2 L/mol) of water) was added thereto, and the mixture was aged for 6 hours.

At this point of time, 3-[(5-difluoromethoxy-1-methyl-3-trifluoromethylpyrazol-4-yl)methylsulfinyl]-4,5-dihydro-5,5-dimethylisoxazole (Compound 6-a; SO derivative), which is a reaction intermediate, was 0% (HPLC area percentage; 230 nm).

In addition, 3-[(5-difluoromethoxy-1-methyl-3-trifluoromethylpyrazol-4-yl)methylsulfonyl]-4,5-dihydro-5,5-dimethylisoxazole (Compound 5-a; SO2 derivative), which is the target product, was 98.7% (HPLC area percentage; 230 nm), and
3-[(5-difluoromethoxy-1-methyl-3-trifluoromethylpyrazol-4-yl)methylthio]-4,5-dihydro-5,5-dimethylisoxazole (Compound 4-a; S derivative) was 0% (HPLC area percentage; 230 nm).

### [Example 2-25]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

The compound (4-a) (0.9 g, purity: 100%, 100 mmol, 100 mol%), 2 ml (0.8 L/mol) of dioxane and ammonium molybdate tetrahydrate (0.03 g, 0.025 mmol, 1 mol%) were added to a reaction vessel. The mixture was heated to 80°C. A 35% aqueous hydrogen peroxide solution (0.85 g, 350 mmol, 350 mol%, containing 0.55 g (0.2 L/mol) of water) was added thereto, and the mixture was aged for 6 hours.

At this point of time, 3-[(5-difluoromethoxy-1-methyl-3-trifluoromethylpyrazol-4-yl)methylsulfinyl]-4,5-dihydro-5,5-dimethylisoxazole (Compound 6-a; SO derivative), which is a reaction intermediate, was 0% (HPLC area percentage; 230 nm).

In addition, 3-[(5-difluoromethoxy-1-methyl-3-trifluoromethylpyrazol-4-yl)methylsulfonyl]-4,5-dihydro-5,5-dimethylisoxazole (Compound 5-a; SO2 derivative), which is the target product, was 99.4% (HPLC area percentage; 230 nm), and
3-[(5-difluoromethoxy-1-methyl-3-trifluoromethylpyrazol-4-yl)methylthio]-4,5-dihydro-5,5-dimethylisoxazole (Compound 4-a; S derivative) was 0% (HPLC area percentage; 230 nm).

### [Example 2-26]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

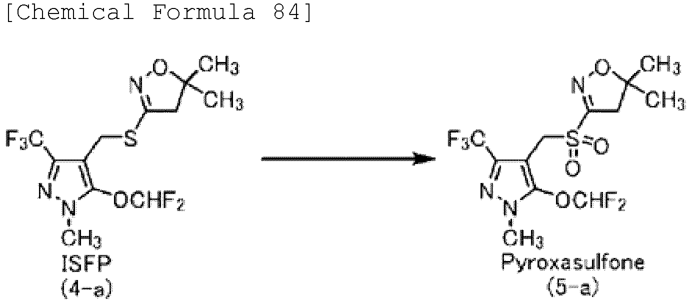

Under a nitrogen stream, the compound (4-a) (0.9 g, purity: 100%, 100 mmol, 100 mol%), 2 mL (0.8 L/mol) of dimethylformamide, sodium niobate (20 mg, 0.125 mmol, 5 mol%), and sulfuric acid (29 mg, 0.3 mmol, 12 mol%) were added to a reaction flask. The mixture was heated to an internal temperature of 75°C to 80°C. A 35% aqueous hydrogen peroxide solution (0.73 g, 7.5 mmol, 300 mol%, containing 0.47 g (0.2 L/mol) of water) was added dropwise thereto at an internal temperature of 75°C to 80°C over 1 hour, and the mixture was aged for 4 hours while the internal temperature was maintained at 75°C to 80°C. A 35% aqueous hydrogen peroxide solution (0.36 g, 3.8 mmol, 150 mol%, containing 0.2 g (0.1 L/mol) of water) was added, and the mixture was aged for 3 hours.

At this point of time, 3-[(5-difluoromethoxy-1-methyl-3-trifluoromethylpyrazol-4-yl)methylsulfinyl]-4,5-dihydro-5,5-dimethylisoxazole (Compound 6-a; SO derivative), which is a reaction intermediate, was 2.6% (HPLC area percentage; 230 nm) .

Acetonitrile was added to the reaction mixture to make it homogeneous. As a result of analysis by the HPLC external standard method, the target product (5-a) was obtained with a yield of 93.9%.

### [Example 2-27]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

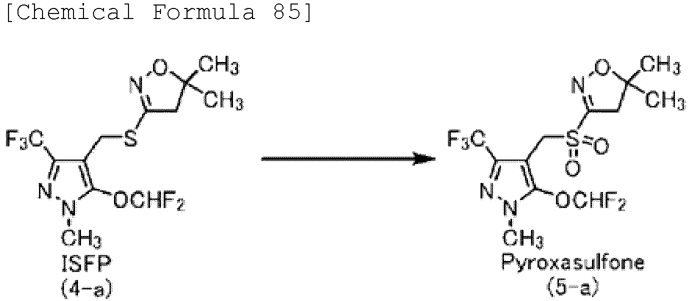

Under a nitrogen stream, the compound (4-a) (0.9 g, purity: 100%, 2.5 mmol, 100 mol%), 2 mL (0.8 L/mol) of dimethylformamide, and sodium tungstate dihydrate (41 mg, 0.125 mmol, 5 mol%) were added to a reaction flask. The mixture was heated to an internal temperature of 75°C to 80°C. A 35% aqueous hydrogen peroxide solution (0.85 g, 8.75 mmol, 300 mol%, containing 0.55 g (0.2 L/mol) of water) was added thereto, and the mixture was aged for 6 hours.

At this point of time, the mixture was analyzed in HPLC area percentage (230 nm). As a result,
3-[(5-difluoromethoxy-1-methyl-3-trifluoromethylpyrazol-4-yl)methylsulfinyl]-4,5-dihydro-5,5-dimethylisoxazole (Compound 6-a; SO derivative) was 0.5%,
3-[(5-difluoromethoxy-1-methyl-3-trifluoromethylpyrazol-4-yl)methylsulfonyl]-4,5-dihydro-5,5-dimethylisoxazole (Compound 5-a; SO2 derivative) was 97.2%, and
3-[(5-difluoromethoxy-1-methyl-3-trifluoromethylpyrazol-4-yl)methylthio]-4,5-dihydro-5,5-dimethylisoxazole (Compound 4-a; S derivative) was 0%.

### [Example 2-28]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

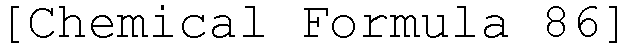

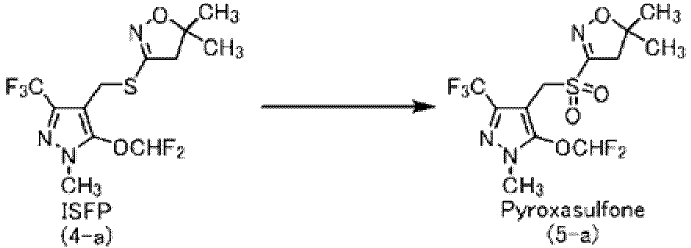

Under a nitrogen stream, the compound (4-a) (0.9 g, purity: 100%, 2.5 mmol, 100 mol%), 2 mL (0.8 L/mol) of 2-propanol, and titanium acetylacetonate (33 mg, 0.125 mmol, 5 mol%) were added to a reaction flask. The mixture was heated to an internal temperature of 75°C to 80°C. A 35% aqueous hydrogen peroxide solution (0.85 g, 8.75 mmol, 300 mol%, containing 0.55 g (0.2 L/mol) of water) was added thereto, and the mixture was aged for 6 hours.

At this point of time, the mixture was analyzed in HPLC area percentage (230 nm). As a result,
3-[(5-difluoromethoxy-1-methyl-3-trifluoromethylpyrazol-4-yl)methylsulfinyl]-4,5-dihydro-5,5-dimethylisoxazole (Compound 6-a; SO derivative) was 71.7%,
3-[(5-difluoromethoxy-1-methyl-3-trifluoromethylpyrazol-4-yl)methylsulfonyl]-4,5-dihydro-5,5-dimethylisoxazole (Compound 5-a) (SO2 derivative) was 6.9%, and 3-[(5-difluoromethoxy-1-methyl-3-trifluoromethylpyrazol-4-yl)methylthio]-4,5-dihydro-5,5-dimethylisoxazole (Compound 4-a; S derivative) was 19.4%.

### [Example 2-29]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

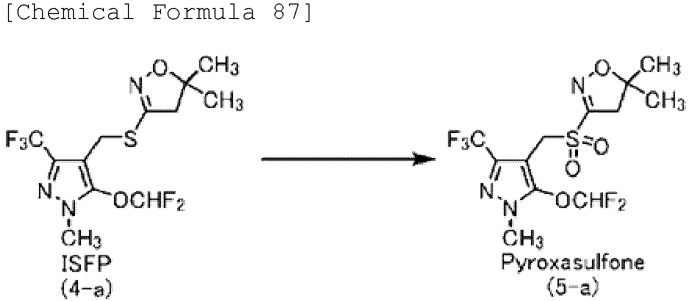

Under a nitrogen stream, the compound (4-a) (0.9 g, purity: 100%, 2.5 mmol, 100 mol%), 2 mL (0.8 L/mol) of acetonitrile, and zirconium chloride oxide octahydrate (40 mg, 0.125 mmol, 5 mol%) were added to a reaction flask. The mixture was heated to an internal temperature of 75°C to 80°C. A 35% aqueous hydrogen peroxide solution (0.85 g, 8.75 mmol, 300 mol%, containing 0.55 g (0.2 L/mol) of water) was added thereto, and the mixture was aged for 6 hours.

At this point of time, the mixture was analyzed in HPLC area percentage (230 nm). As a result,
3-[(5-difluoromethoxy-1-methyl-3-trifluoromethylpyrazol-4-yl)methylsulfinyl]-4,5-dihydro-5,5-dimethylisoxazole (Compound 6-a; SO derivative) was 56.5%,
3-[(5-difluoromethoxy-1-methyl-3-trifluoromethylpyrazol-4-yl)methylsulfonyl]-4,5-dihydro-5,5-dimethylisoxazole (Compound 5-a; SO2 derivative) was 11.0%, and
3-[(5-difluoromethoxy-1-methyl-3-trifluoromethylpyrazol-4-yl)methylthio]-4,5-dihydro-5,5-dimethylisoxazole (Compound 4-a; S derivative) was 0%.

### [Example 2-30]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

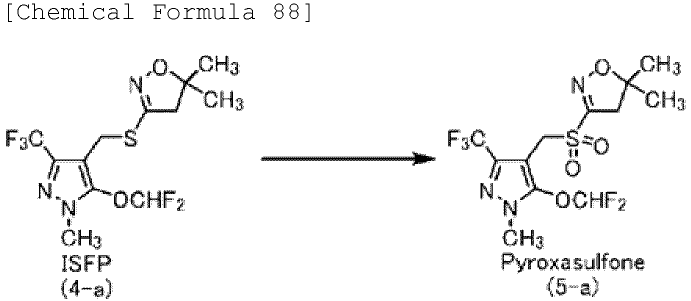

Under a nitrogen stream, the compound (4-a) (0.9 g, purity: 100%, 2.5 mmol, 100 mol%), 2 mL (0.8 L/mol) of 2-propanol, and zirconium chloride oxide octahydrate (40 mg, 0.125 mmol, 5 mol%) were added to a reaction flask. The mixture was heated to an internal temperature of 75°C to 80°C. A 35% aqueous hydrogen peroxide solution (0.85 g, 8.75 mmol, 300 mol%, containing 0.55 g (0.2 L/mol) of water) was added thereto, and the mixture was aged for 6 hours.

At this point of time, the mixture was analyzed in HPLC area percentage (230 nm). As a result, 3-[(5-difluoromethoxy-1-methyl-3-trifluoromethylpyrazol-4-yl)methylsulfinyl]-4,5-dihydro-5,5-dimethylisoxazole (Compound 6-a; SO derivative) was 71.6%,
3-[(5-difluoromethoxy-1-methyl-3-trifluoromethylpyrazol-4-yl)methylsulfonyl]-4,5-dihydro-5,5-dimethylisoxazole (compound 5-a; SO2 derivative) was 12.5%, and
3-[(5-difluoromethoxy-1-methyl-3-trifluoromethylpyrazol-4-yl)methylthio]-4,5-dihydro-5,5-dimethylisoxazole (Compound 4-a; S derivative) was 1.4%.

### [Example 2-31]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

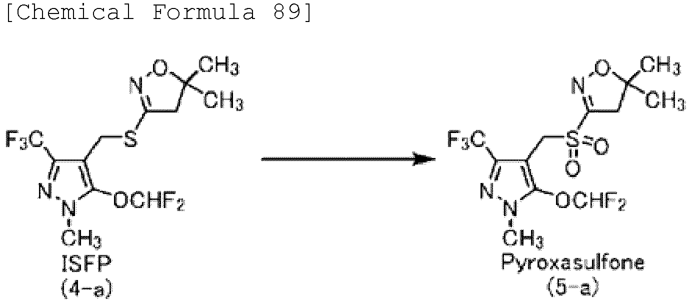

Under a nitrogen stream, the compound (4-a) (0.9 g, purity: 100%, 2.5 mmol, 100 mol%), 2 mL (0.8 L/mol) of dimethylformamide, and lithium tungstate (29 mg, 0.125 mmol, 5 mol%) were added to a reaction flask. The mixture was heated to an internal temperature of 75°C to 80°C. A 35% aqueous hydrogen peroxide solution (1.42 g, 14.6 mmol, 500 mol%, containing 0.92 g (0.37 L/mol) of water) was added thereto, and the mixture was aged for 6 hours.

At this point of time, the mixture was analyzed in HPLC area percentage (230 nm). As a result, 3-[(5-difluoromethoxy-1-methyl-3-trifluoromethylpyrazol-4-yl)methylsulfinyl]-4,5-dihydro-5,5-dimethylisoxazole (Compound 6-a; SO derivative) was 29.3%,
3-[(5-difluoromethoxy-1-methyl-3-trifluoromethylpyrazol-4-yl)methylsulfonyl]-4,5-dihydro-5,5-dimethylisoxazole (Compound 5-a; SO2 derivative) was 67.1%, and
3-[(5-difluoromethoxy-1-methyl-3-trifluoromethylpyrazol-4-yl)methylthio]-4,5-dihydro-5,5-dimethylisoxazole (Compound 4-a; S derivative) was 0%.

### [Example 2-32]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

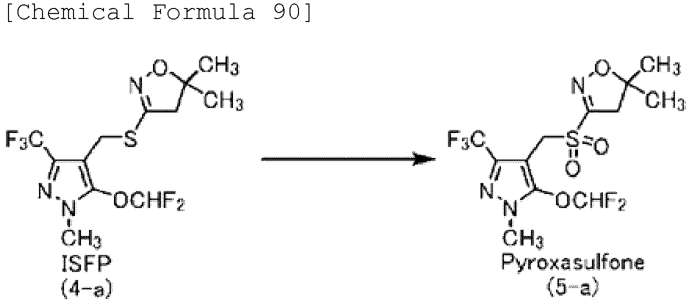

Under a nitrogen stream, the compound (4-a) (0.9 g, purity: 100%, 2.5 mmol, 100 mol%), 2 mL (0.8 L/mol) of dimethylformamide, and lithium tungstate (290 mg, 1.25 mmol, 50 mol%) were added to a reaction flask. The mixture was heated to an internal temperature of 75°C to 80°C. A 35% aqueous hydrogen peroxide solution (0.99 g, 10.2 mmol, 350 mol%, containing 0.64 g (0.36 L/mol) of water) was added thereto, and the mixture was aged for 6 hours.

At this point of time, the mixture was analyzed in HPLC area percentage (230 nm). As a result, 3-[(5-difluoromethoxy-1-methyl-3-trifluoromethylpyrazol-4-yl)methylsulfinyl]-4,5-dihydro-5,5-dimethylisoxazole (Compound 6-a; SO derivative) was 17.9%,
3-[(5-difluoromethoxy-1-methyl-3-trifluoromethylpyrazol-4-yl)methylsulfonyl]-4,5-dihydro-5,5-dimethylisoxazole (compound 5-a; SO2 derivative) was 80.4%, and
3-[(5-difluoromethoxy-1-methyl-3-trifluoromethylpyrazol-4-yl)methylthio]-4,5-dihydro-5,5-dimethylisoxazole (Compound 4-a; S derivative) was 0%.

### [Example 2-33]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

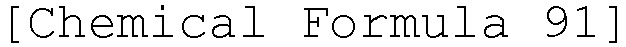

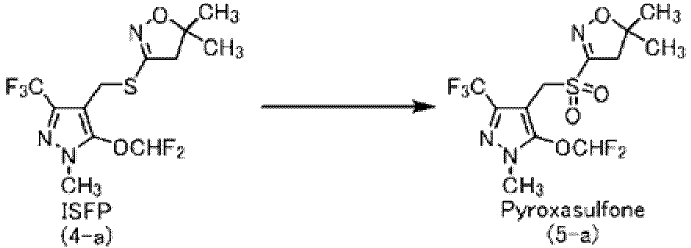

Under a nitrogen stream, the compound (4-a) (8.34 g, purity: 100%, 23.2 mmol, 100 mol%), 24.6 g (0.8 L/mol) of ethanol, 2.3 ml (0.1 L/mol) of water, and sodium tungstate dihydrate (0.38 g, 1.16 mmol, 5 mol%) were added to a reaction flask. The mixture was heated to an internal temperature of 75°C to 78°C. A 35% aqueous hydrogen peroxide solution (11.3 g, 116 mmol, 500 mol%, containing 17.2 g (0.3 L/mol) of water) was added dropwise thereto at an internal temperature of 75°C to 78°C, and the mixture was aged for 5 hours while the internal temperature was maintained at 75°C to 78°C.

At this point of time, 3-[(5-difluoromethoxy-1-methyl-3-trifluoromethylpyrazol-4-yl)methylsulfinyl]-4,5-dihydro-5,5-dimethylisoxazole (Compound 6-a; SO derivative), which is a reaction intermediate, was 2.9% (HPLC area percentage; 230 nm).

Further, a 35% aqueous hydrogen peroxide solution (2.26 g, 23.2 mmol, 100 mol%, containing 6.4 g (0.06 L/mol) of water) was added dropwise at an internal temperature of 75°C to 78°C, and the mixture was aged for 1 hour while the internal temperature was maintained at 75°C to 78°C.

At this point of time, 3-[(5-difluoromethoxy-1-methyl-3-trifluoromethylpyrazol-4-yl)methylsulfinyl]-4,5-dihydro-5,5-dimethylisoxazole (Compound 6-a; SO derivative), which is a reaction intermediate, was 0.6% (HPLC area percentage; 230 nm).

Dimethylformamide was added to the reaction mixture to make it homogeneous. As a result of analysis by the HPLC external standard method, the target product (5-a) was obtained with a yield of 94.2%.

### [Example 2-34]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

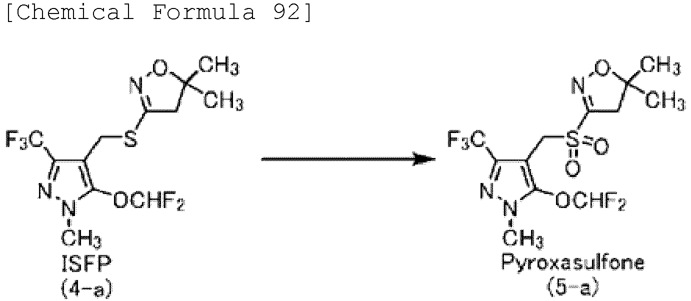

Under a nitrogen stream, the compound (4-a) (138 g, purity: 78.1%, 300 mmol, 100 mol%), 237 g (1.0 L/mol) of ethanol, 90 ml (0.3 L/mol) of water, and sodium tungstate dihydrate (5.0 g, 15 mmol, 5 mol%) were added to a reaction flask. The mixture was heated to an internal temperature of 50°C to 60°C. A 35% aqueous hydrogen peroxide solution (87.5 g, 900 mmol, 300 mol%, containing 56.9 g (0.2 L/mol) of water) was added dropwise thereto at an internal temperature of 50°C to 60°C over 1 hour, and the mixture was aged for 7 hours while the internal temperature was maintained at 50°C to 60°C.

At this point of time, 3-[(5-difluoromethoxy-1-methyl-3-trifluoromethylpyrazol-4-yl)methylsulfinyl]-4,5-dihydro-5,5-dimethylisoxazole (Compound 6-a; SO derivative), which is a reaction intermediate, was 3.2% (HPLC area percentage; 230 nm).

### [Example 2-35]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

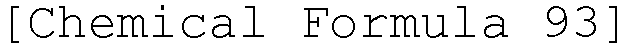

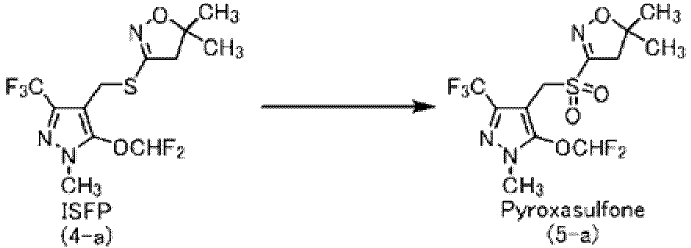

Under a nitrogen stream, the compound (4-a) (3.1 g, purity: 100%, 8.5 mmol, 100 mol%), 2.7 g (0.4 L/mol) of acetonitrile, 0.9 ml (0.1 L/mol) of water, sodium tungstate dihydrate (0.084 g, 0.26 mmol, 3 mol%), and sulfuric acid (0.087 g, 0.85 mmol, 10 mol%) were added to a reaction flask. The mixture was heated to an internal temperature of 75°C to 80°C. A 35% aqueous hydrogen peroxide solution (2.48 g, 25.5 mmol, 300 mol%, containing 1.6 g (0.2 L/mol) of water) was added dropwise thereto at an internal temperature of 75°C to 80°C over 1 hour, and the mixture was aged for 6 hours while the internal temperature was maintained at 75°C to 80°C.

At this point of time, 3-[(5-difluoromethoxy-1-methyl-3-trifluoromethylpyrazol-4-yl)methylsulfinyl]-4,5-dihydro-5,5-dimethylisoxazole (Compound 6-a; SO derivative) was 0.3% (HPLC area percentage; 230 nm).

Acetonitrile was added to the reaction mixture to make it homogeneous. As a result of analysis by the HPLC external standard method, the target product (5-a) was obtained with a yield of 96.4%.

### [Example 2-36]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

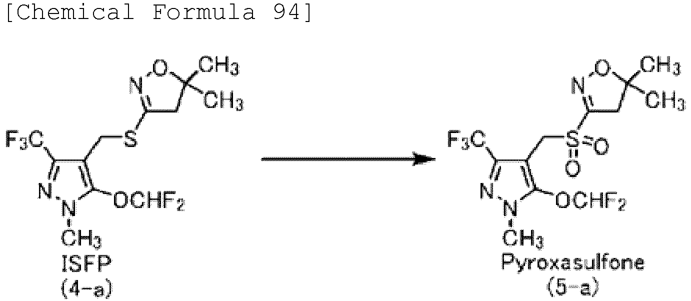

Under a nitrogen stream, the compound (4-a) (0.9 g, purity: 100%, 2.5 mmol, 100 mol%), 0.8 g (0.4 L/mol) of acetonitrile, 0.3 ml (0.1 L/mol) of water and ammonium molybdate tetrahydrate (0.031 g, 0.03 mmol, 1 mol%) were added to a reaction flask. The mixture was heated to 80°C. A 35% aqueous hydrogen peroxide solution (0.9 g, 8.8 mmol, 350 mol%, containing 0.6 g (0.2 L/mol) of water) was added thereto, and the mixture was aged for 6 hours.

At this point of time, 3-[(5-difluoromethoxy-1-methyl-3-trifluoromethylpyrazol-4-yl)methylsulfinyl]-4,5-dihydro-5,5-dimethylisoxazole (Compound 6-a; SO derivative), which is a reaction intermediate, was 0.67% (HPLC area percentage; 230 nm). At this point of time, 3-[(5-difluoromethoxy-1-methyl-3-trifluoromethylpyrazol-4-yl)methylsulfonyl]-4,5-dihydro-5,5-dimethylisoxazole (Compound 5-a; SO2 derivative), which is the target product, was 97.4% (HPLC area percentage; 230 nm).

### [Comparative Example 2-1]

### (Step iii)

### Production of 3-[(5-hydroxy-1-methyl-3-trifluoromethylpyrazol-4-yl)methylsulfonyl]-4,5-dihydro-5,5-dimethylisoxazole

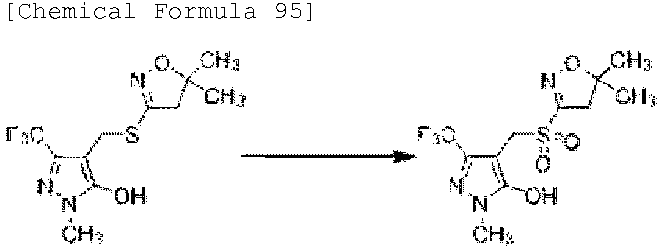

Under a nitrogen stream, 3-[(5-hydroxy-1-methyl-3-trifluoromethylpyrazol-4-yl)methylthio]-4,5-dihydro-5,5-dimethylisoxazole (2.6 g, purity: 100%, 8.5 mmol, 100 mol%), 2.7 g (0.4 L/mol) of acetonitrile, 0.9 ml (0.1 L/mol) of water, sodium tungstate dihydrate (0.084 g, 0.26 mmol, 3 mol%), and sulfuric acid (0.087 g, 0.85 mmol, 10 mol%) were added to a reaction flask. The mixture was heated to an internal temperature of 75°C to 80°C. A 35% aqueous hydrogen peroxide solution (2.48 g, 25.5 mmol, 300 mol%, containing 1.6 g (0.2 L/mol) of water) was added dropwise thereto at an internal temperature of 75°C to 80°C over 1 hour, and the mixture was aged for 6 hours while the internal temperature was maintained at 75°C to 80°C.

At this point of time, 3-[(5-hydroxy-1-methyl-3-trifluoromethylpyrazol-4-yl)methylsulfinyl]-4,5-dihydro-5,5-dimethylisoxazole, which is a reaction intermediate, was 4.55% (HPLC area percentage; 230 nm).

Further, at this point of time, 3-[(5-hydroxy-1-methyl-3-trifluoromethylpyrazol-4-yl)methylsulfonyl]-4,5-dihydro-5,5-dimethylisoxazole, which is the target product, was 0.53% (HPLC area percentage; 230 nm).

### [Comparative Example 2-2]

### (Step iii)

### Production of 3-[(1,3,5-trimethylpyrazol-4-yl)methylsulfonyl]-4,5-dihydro-5,5-dimethylisoxazole

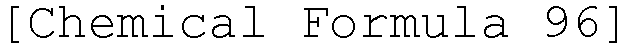

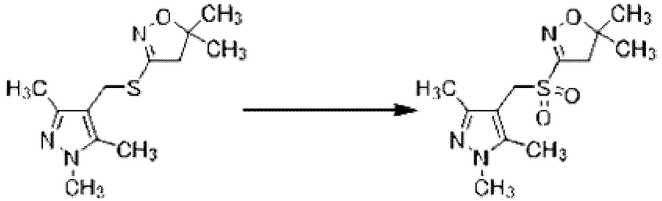

Under a nitrogen stream, 3-[(1,3,5-trimethylpyrazol-4-yl)methylthio]-4,5-dihydro-5,5-dimethylisoxazole (2.2 g, purity: 100%, 8.5 mmol, 100 mol%), 2.7 g (0.4 L/mol) of acetonitrile, 0.9 ml (0.1 L/mol) of water, sodium tungstate dihydrate (0.084 g, 0.26 mmol, 3 mol%), and sulfuric acid (0.087 g, 0.85 mmol, 10 mol%) were added to a reaction flask. The mixture was heated to an internal temperature of 75°C to 80°C. A 35% aqueous hydrogen peroxide solution (2.48 g, 25.5 mmol, 300 mol%, containing 1.6 g (0.2 L/mol) of water) was added dropwise thereto at an internal temperature of 75°C to 80°C over 1 hour, and the mixture was aged for 6 hours while the internal temperature was maintained at 75°C to 80°C.

At this point of time, 3-[(1,3,5-trimethylpyrazol-4-yl)methylsulfinyl]-4,5-dihydro-5,5-dimethylisoxazole, which is a reaction intermediate, was 0% (HPLC area percentage; 230 nm).

Further, at this point of time, 3-[(1,3,5-trimethylpyrazol-4-yl)methylsulfonyl]-4,5-dihydro-5,5-dimethylisoxazole, which is the target product, was 0.4% (HPLC area percentage; 230 nm).

### [Comparative Example 2-3]

### (Step iii)

### Production of 3-[(5-methoxy-1-methyl-3-trifluoromethylpyrazol-4-yl)methylsulfonyl]-4,5-dihydro-5,5-dimethylisoxazole

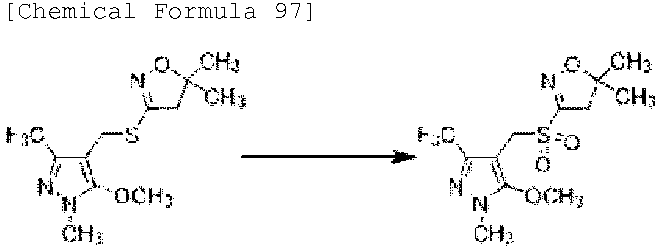

Under a nitrogen stream, 3-[(5-methoxy-1-methyl-3-trifluoromethylpyrazol-4-yl)methylthio]-4,5-dihydro-5,5-dimethylisoxazole (2.8 g, purity: 100%, 8.5 mmol, 100 mol%), 2.7 g (0.4 L/mol) of acetonitrile, 0.9 ml (0.1 L/mol) of water, sodium tungstate dihydrate (0.084 g, 0.26 mmol, 3 mol%), and sulfuric acid (0.087 g, 0.85 mmol, 10 mol%) were added to a reaction flask. The mixture was heated to an internal temperature of 75°C to 80°C. A 35% aqueous hydrogen peroxide solution (2.48 g, 25.5 mmol, 300 mol%, containing 1.6 g (0.2 L/mol) of water) was added dropwise thereto at an internal temperature of 75°C to 80°C over 1 hour, and the mixture was aged for 6 hours while the internal temperature was maintained at 75°C to 80°C.

At this point of time, 3-[(5-methoxy-1-methyl-3-trifluoromethylpyrazol-4-yl)methylsulfinyl]-4,5-dihydro-5,5-dimethylisoxazole, which is a reaction intermediate, was 0% (HPLC area percentage; 230 nm).

Further, at this point of time, 3-[(5-methoxy-1-methyl-3-trifluoromethylpyrazol-4-yl)methylsulfonyl]-4,5-dihydro-5,5-dimethylisoxazole, which is the target product, was 3.3% (HPLC area percentage; 230 nm).

### [Comparative example 2-4]

### (Step iii)

### Production of 3-[(5-hydroxy-1-methyl-3-trifluoromethylpyrazol-4-yl)methylsulfonyl]-4,5-dihydro-5,5-dimethylisoxazole

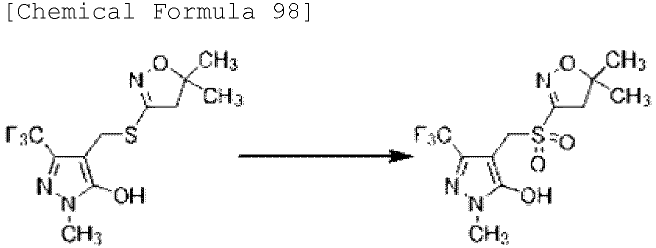

Under a nitrogen stream, 3-[(5-hydroxy-1-methyl-3-trifluoromethylpyrazol-4-yl)methylthio]-4,5-dihydro-5,5-dimethylisoxazole (0.8 g, purity: 100%, 2.5 mmol, 100 mol%), 0.8 g (0.4 L/mol) of acetonitrile, 0.3 ml (0.1 L/mol) of water, and ammonium molybdate tetrahydrate (0.031 g, 0.03 mmol, 1 mol%) were added to a reaction flask. The mixture was heated to 80°C. A 35% aqueous hydrogen peroxide solution (0.9 g, 8.8 mmol, 350 mol%, containing 0.6 g (0.2 L/mol) of water) was added thereto, and the mixture was aged for 6 hours.

At this point of time, 3-[(5-hydroxy-1-methyl-3-trifluoromethylpyrazol-4-yl)methylsulfinyl]-4,5-dihydro-5,5-dimethylisoxazole, which is a reaction intermediate, was 0% (HPLC area percentage; 230 nm).

Further, at this point of time, 3-[(5-hydroxy-1-methyl-3-trifluoromethylpyrazol-4-yl)methylsulfonyl]-4,5-dihydro-5,5-dimethylisoxazole, which is the target product, was 0% (HPLC area percentage; 230 nm).

### [Comparative example 2-5]

### (Step iii)

### Production of 3-[(1,3,5-trimethylpyrazol-4-yl)methylsulfonyl]-4,5-dihydro-5,5-dimethylisoxazole

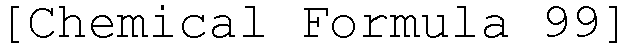

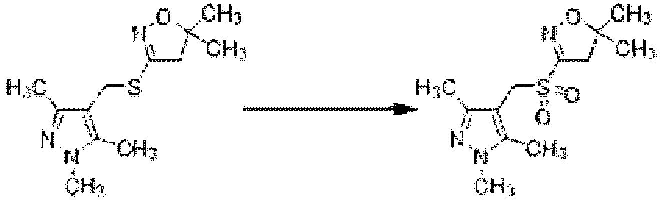

Under a nitrogen stream, 3-[(1,3,5-trimethylpyrazol-4-yl)methylthio]-4,5-dihydro-5,5-dimethylisoxazole (0.6 g, purity: 100%, 2.5 mmol, 100 mol%), 0.8 g (0.4 L/mol) of acetonitrile, 0.3 ml (0.1 L/mol) of water, and ammonium molybdate tetrahydrate (0.031 g, 0.03 mmol, 1 mol%) were added to a reaction flask. The mixture was heated to 80°C. A 35% aqueous hydrogen peroxide solution (0.9 g, 8.8 mmol, 350 mol%, containing 0.6 g (0.2 L/mol) of water) was added thereto, and the mixture was aged for 6 hours.

At this point of time, 3-[(1,3,5-trimethylpyrazol-4-yl)methylsulfinyl]-4,5-dihydro-5,5-dimethylisoxazole, which is a reaction intermediate, was 0.7% (HPLC area percentage; 230 nm).

Further, at this point of time, 3-[(1,3,5-trimethylpyrazol-4-yl)methylsulfonyl]-4,5-dihydro-5,5-dimethylisoxazole, which is the target product, was 14.6% (HPLC area percentage; 230 nm).

### [Comparative Example 2-6]

### (Step iii)

### Production of 3-[(5-methoxy-1-methyl-3-trifluoromethylpyrazol-4-yl)methylsulfonyl]-4,5-dihydro-5,5-dimethylisoxazole

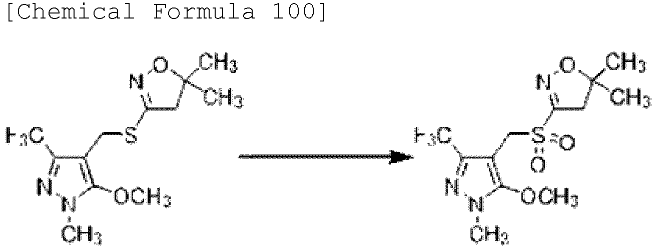

Under a nitrogen stream, 3-[(5-methoxy-1-methyl-3-trifluoromethylpyrazol-4-yl)methylthio]-4,5-dihydro-5,5-dimethylisoxazole (0.8 g, purity: 100%, 2.5 mmol, 100 mol%), 0.8 g (0.4 L/mol) of acetonitrile, 0.3 ml (0.1 L/mol) of water, and ammonium molybdate tetrahydrate (0.031 g, 0.03 mmol, 1 mol%) were added to a reaction flask. The mixture was heated to 80°C. A 35% aqueous hydrogen peroxide solution (0.9 g, 8.8 mmol, 350 mol%, containing 0.6 g (0.2 L/mol) of water) was added thereto, and the mixture was aged for 6 hours.

At this point of time, 3-[(5-methoxy-1-methyl-3-trifluoromethylpyrazol-4-yl)methylsulfinyl]-4,5-dihydro-5,5-dimethylisoxazole, which is a reaction intermediate, was 0% (HPLC area percentage; 230 nm).

Further, at this point of time, 3-[(5-methoxy-1-methyl-3-trifluoromethylpyrazol-4-yl)methylsulfonyl]-4,5-dihydro-5,5-dimethylisoxazole, which is the target product, was 82.7% (HPLC area percentage; 230 nm).

In the above Comparative Examples 2-1 to 2-6, the process of the present invention was applied to compounds similar to the compounds of the formula (4). As a result, for the similar compounds other than the compounds of the formula (4), the reaction was not completed, a large amount of the SO derivative as a reaction intermediate remained, decomposition occurred, and/or the yield was low. Surprisingly, the process of the present invention was specifically effective only for the compounds of the formula (4). In addition, a particularly high effect was unexpectedly obtained with the compound (4-a).

### [Comparative Example 2-7]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

In the production of the compound (5-a) from the compound (4-a) using sodium tungstate dihydrate and an aqueous hydrogen peroxide solution, methyl isobutyl ketone (MIBK) was used as an organic solvent. The mixture was heated to an internal temperature of 70°C to 80°C, and then an aqueous hydrogen peroxide solution was added dropwise. At the point when the aging had been performed for 7 hours, 3-[(5-difluoromethoxy-1-methyl-3-trifluoromethylpyrazol-4-yl)methylsulfinyl]-4,5-dihydro-5,5-dimethylisoxazole (Compound 6-a; SO derivative) remained 10% or more (HPLC area percentage; 230 nm).

### [Reference Example]

### (Step iii)

### Production of 3-[(5-difluoromethoxy-1-methyl-3-trifluoromethylpyrazol-4-yl)methylsulfonyl]-4,5-dihydro-5,5-dimethylisoxazole (compound 5-a)

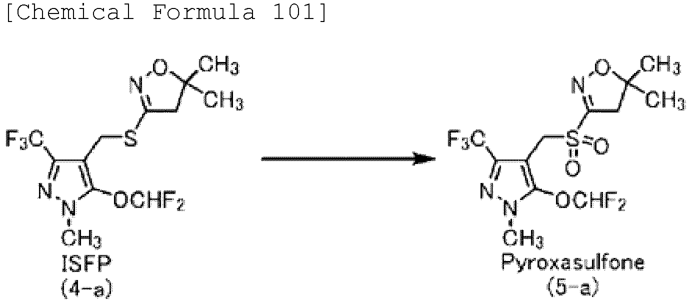

Under a nitrogen stream, 2.8 g (100 mmol, 100 mol%) of the compound (4-a), 8.4 g (1.0 L/mol) of acetic acid and 80 mg (3 mmol, 3 mol%) of sodium tungstate dihydrate were added to a reaction flask. To the mixture was added 2.2 g of a 30% aqueous hydrogen peroxide solution (250 mmol, 250 mol%) dropwise at an internal temperature of 26°C to 35°C over 20 minutes, and the resulting mixture was aged for 16 hours while the internal temperature was maintained at 26°C to 35°C.

At the point when the aging had been performed for 16 hours, 3-[(5-difluoromethoxy-1-methyl-3-trifluoromethylpyrazol-4-yl)methylsulfinyl]-4,5-dihydro-5,5-dimethylisoxazole (Compound 6-a; SO derivative), which is a reaction intermediate, remained 5.0% (HPLC area percentage).

To the reaction mixture was added 4 g of water, and the resulting mixture was aged at 10°C for 1 hour, and then the precipitated crystals were separated by filtration.

The obtained crystals were successively washed with 20 ml of petroleum ether and 20 ml of water. The obtained crystals were analyzed by HPLC (area percentage; 230 nm). As a result, 3-[(5-difluoromethoxy-1-methyl-3-trifluoromethylpyrazol-4-yl)methylsulfinyl]-4,5-dihydro-5,5-dimethylisoxazole (Compound 6-a; SO derivative), which is a reaction intermediate, had an HPLC area percentage of 5.5%.

Reference Example is a reproduction experiment of Example 9C in JP 2013-512201 A (Patent Document 7). In the production process described in JP2013-512201A (Patent Document 7), even after adding an excess amount of hydrogen peroxide and aging for 16 hours, the compound (6-a), which is a reaction intermediate, remained no less than 5.0%. In addition, even after purification, the ratio of the compound (6-a) did not decrease. It has been confirmed that it is difficult to purify the compound of the formula (5) by separating the compound of the formula (5) from the compound of the formula (6). The production process described in JP 2013-512201 A (Patent Document 7) also has the problem that a dangerous organic peracid (peracetic acid) is generated in the reaction system. Due to this risk, heating should be avoided in this process.

### [Referential Production Example 1]

### Production of [5,5-dimethyl(4,5-dihydroisoxazolo-3-yl)]thiocarboxamidine hydrobromide aqueous solution

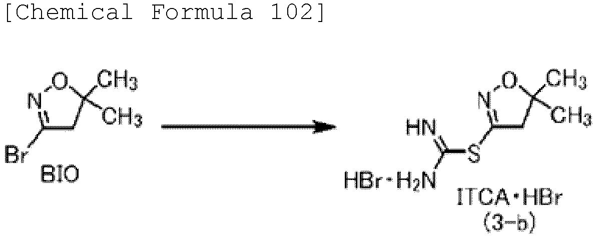

Thiourea (20 g, 0.26 mol, 105 mol%) was added to a solution of 3-bromo-5,5-dimethyl-4,5-dihydroisoxazole (BIO) obtained by a process described in WO 2006/038657A in butyl acetate (251.5 g, purity: 18%, 0.25 mol), and the internal temperature was adjusted to 15°C to 25°C. To this was added 35% hydrochloric acid (26 g, 0.25 mol, 100 mol%) dropwise at an internal temperature of 15°C to 25°C over 30 minutes. After the dropwise addition, the mixture was aged at an internal temperature of 15°C to 25°C for 6 hours. After the completion of the reaction, water (88 g, 0.35 L/mol) was added, the mixture was stirred for 15 minutes, and the reaction mixture was separated into an organic layer and an aqueous layer. Water (25 g, 0.1 L/mol) was added to the obtained organic layer, the mixture was stirred for 15 minutes, and the reaction mixture was separated into an organic layer and an aqueous layer. The obtained aqueous layers were combined to afford 208.6 g of an aqueous solution containing the target product corresponding to a yield of 90%. The obtained target product contained a hydrobromide derived from the raw material BIO and hydrochloride derived from hydrochloric acid.

### [Example 3]

Crystallization of 3-[(5-difluoromethoxy-1-methyl-3-trifluoromethylpyrazol-4-yl)methylsulfonyl]-4,5-dihydro-5,5-dimethylisoxazole (Compound 5-a, Pyroxasulfone)

### [Example 3-1]

A crystal of pyroxasulfone was prepared by a process according to Example 2-14. In powder X-ray diffraction measurement by a transmission method using Cu-Kα rays, this crystal exhibited a powder X-ray diffraction spectrum in which there are characteristic peaks at diffraction angles 2θ of 17.8°, 18.0° and 19.9° and the peak height at the peak of 19.9° is maximum among the three peaks. The diffraction angle 2Θ and the peak height at all peaks of the powder X-ray diffraction spectrum are shown in Table 3. The shape of this crystal was columnar or short columnar. The columnar crystal of pyroxasulfone produced in the same manner as Example 2-14 in the present production process had a bulk specific gravity of 1.04 g/mL.

In Examples 3-2 to 3-12 and Comparative Examples 3-3 to 3-12, pyroxasulfone obtained by a process according to Example 2-14 was used.

### [Example 3-2]

Pyroxasulfone was dissolved in an 87 vol% acetonitrile hydrous medium. Acetonitrile and water were evaporated from the obtained solution under normal pressure to precipitate crystals of pyroxasulfone. The obtained crystals were collected to afford crystals of pyroxasulfone of the present invention. In powder X-ray diffraction measurement by a transmission method using Cu-Kα rays, this crystal exhibited a powder X-ray diffraction spectrum in which there are characteristic peaks at diffraction angles 2θ of 17.7°, 18.0° and 19.9° and the peak height at the peak of 19.9° is maximum among the three peaks. The diffraction angle 2Θ and the peak height at all peaks of the powder X-ray diffraction spectrum are shown in Table 3. The shape of this crystal was columnar or short columnar.

### [Example 3-3]

Pyroxasulfone was dissolved in N,N-dimethylacetamide. N,N-dimethylacetamide was evaporated from the obtained solution under normal pressure to precipitate crystals of pyroxasulfone. The obtained crystals were collected to afford crystals of pyroxasulfone of the present invention. In powder X-ray diffraction measurement by a transmission method using Cu-Kα rays, this crystal exhibited a powder X-ray diffraction spectrum in which there are characteristic peaks at diffraction angles 2Θ of 17.8°, 18.1° and 19.9° and the peak height at the peak of 19.9° is maximum among the three peaks. The diffraction angle 2Θ and the peak height at all peaks of the powder X-ray diffraction spectrum are shown in Table 3. The shape of this crystal was columnar or short columnar.

### [Example 3-4]

Pyroxasulfone was dissolved in N,N-dimethylformamide. N,N-dimethylformamide was evaporated from the obtained solution under normal pressure to precipitate crystals of pyroxasulfone. The obtained crystals were collected to afford crystals of pyroxasulfone of the present invention. In powder X-ray diffraction measurement by a transmission method using Cu-Kα rays, this crystal exhibited a powder X-ray diffraction spectrum in which there are characteristic peaks at diffraction angles 2Θ of 17.7°, 18.0° and 19.9° and the peak height at the peak of 19.9° is maximum among the three peaks. The diffraction angle 2Θ and the peak height at all peaks of the powder X-ray diffraction spectrum are shown in Table 3. The shape of this crystal was columnar or short columnar.

### [Example 3-5]

Pyroxasulfone was dissolved in ethyl acetate. Ethyl acetate was evaporated from the obtained solution under normal pressure to precipitate crystals of pyroxasulfone. The obtained crystals were collected to afford crystals of pyroxasulfone of the present invention. In powder X-ray diffraction measurement by a transmission method using Cu-Kα rays, this crystal exhibited a powder X-ray diffraction spectrum in which there are characteristic peaks at diffraction angles 2Θ of 17.8°, 18.0° and 19.9° and the peak height at the peak of 19.9° is maximum among the three peaks. The diffraction angle 2Θ and the peak height at all peaks of the powder X-ray diffraction spectrum are shown in Table 3. The shape of this crystal was columnar or short columnar.

### [Example 3-6]

Pyroxasulfone was dissolved in methyl isobutyl ketone. Methyl isobutyl ketone was evaporated from the obtained solution under normal pressure to precipitate crystals of pyroxasulfone. The obtained crystals were collected to afford crystals of pyroxasulfone of the present invention. In powder X-ray diffraction measurement by a transmission method using Cu-Kα rays, this crystal exhibited a powder X-ray diffraction spectrum in which there are characteristic peaks at diffraction angles 2Θ of 17.7°, 18.0° and 19.9° and the peak height at the peak of 19.9° is maximum among the three peaks. The diffraction angle 2Θ and the peak height at all peaks of the powder X-ray diffraction spectrum are shown in Table 3. The shape of this crystal was columnar or short columnar.

### [Example 3-7]

Pyroxasulfone was dissolved in acetic acid. Acetic acid was evaporated from the obtained solution under normal pressure to precipitate crystals of pyroxasulfone. The obtained crystals were collected and dried to afford crystals of pyroxasulfone of the present invention. In powder X-ray diffraction measurement by a transmission method using Cu-Kα rays, this crystal exhibited a powder X-ray diffraction spectrum in which there are characteristic peaks at diffraction angles 2Θ of 17.7°, 18.0° and 19.9° and the peak height at the peak of 19.9° is maximum among the three peaks. The diffraction angle 2Θ and the peak height at all peaks of the powder X-ray diffraction spectrum are shown in Table 3. The shape of this crystal was columnar or short columnar.

### [Example 3-8]

Pyroxasulfone was dissolved in a mixed medium composed of 1 part by volume of acetonitrile and 2 parts by volume of methanol. Acetonitrile and methanol were evaporated from the obtained solution under normal pressure to precipitate crystals of pyroxasulfone. The obtained crystals were collected and dried to afford crystals of pyroxasulfone of the present invention. In powder X-ray diffraction measurement by a transmission method using Cu-Kα rays, this crystal exhibited a powder X-ray diffraction spectrum in which there are characteristic peaks at diffraction angles 2Θ of 17.7°, 18.0° and 19.9° and the peak height at the peak of 19.9° is maximum among the three peaks. The diffraction angle 2Θ and the peak height at all peaks of the powder X-ray diffraction spectrum are shown in Table 3. The shape of this crystal was columnar or short columnar.

### [Example 3-9]

Pyroxasulfone was dissolved in a mixed medium composed of 1 part by volume of dichloromethane and 1 part by volume of ethanol. Dichloromethane and ethanol were evaporated from the obtained solution under normal pressure to precipitate crystals of pyroxasulfone. The obtained crystals were collected and dried to afford crystals of pyroxasulfone of the present invention. In powder X-ray diffraction measurement by a transmission method using Cu-Kα rays, this crystal exhibited a powder X-ray diffraction spectrum in which there are characteristic peaks at diffraction angles 2Θ of 17.8°, 18.0° and 20.0° and the peak height at the peak of 20.0° is maximum among the three peaks. The diffraction angle 2Θ and the peak height at all peaks of the powder X-ray diffraction spectrum are shown in Table 3. The shape of this crystal was columnar or short columnar.

### [Example 3-10]

Pyroxasulfone was dissolved in acetone. Double amount of ethanol was added to the obtained solution to precipitate crystals of pyroxasulfone. The obtained crystals were dried to afford crystals of pyroxasulfone of the present invention. In powder X-ray diffraction measurement by a transmission method using Cu-Kα rays, this crystal exhibited a powder X-ray diffraction spectrum in which there are characteristic peaks at diffraction angles 2θ of 17.7°, 18.0° and 19.9° and the peak height at the peak of 19.9° is maximum among the three peaks. The diffraction angle 2Θ and the peak height at all peaks of the powder X-ray diffraction spectrum are shown in Table 3. The shape of this crystal was columnar or short columnar.

### [Example 3-11]

Pyroxasulfone was dissolved in acetonitrile. Double amount of ethanol was added to the obtained solution to precipitate crystals of pyroxasulfone. The obtained crystals were dried to afford crystals of pyroxasulfone of the present invention. In powder X-ray diffraction measurement by a transmission method using Cu-Kα rays, this crystal exhibited a powder X-ray diffraction spectrum in which there are characteristic peaks at diffraction angles 2θ of 17.7°, 18.0° and 19.9° and the peak height at the peak of 19.9° is maximum among the three peaks. The diffraction angle 2Θ and the peak height at all peaks of the powder X-ray diffraction spectrum are shown in Table 3. The shape of this crystal was columnar or short columnar.

### [Example 3-12]

Pyroxasulfone was dissolved in ethyl acetate. Double amount of ethanol was added to the obtained solution to precipitate crystals of pyroxasulfone. The obtained crystals were dried to afford crystals of pyroxasulfone of the present invention. In powder X-ray diffraction measurement by a transmission method using Cu-Kα rays, this crystal exhibited a powder X-ray diffraction spectrum in which there are characteristic peaks at diffraction angles 2θ of 17.7°, 18.0° and 19.9° and the peak height at the peak of 19.9° is maximum among the three peaks. The diffraction angle 2Θ and the peak height at all peaks of the powder X-ray diffraction spectrum are shown in Table 3. The shape of this crystal was columnar or short columnar.

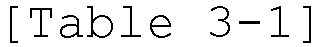

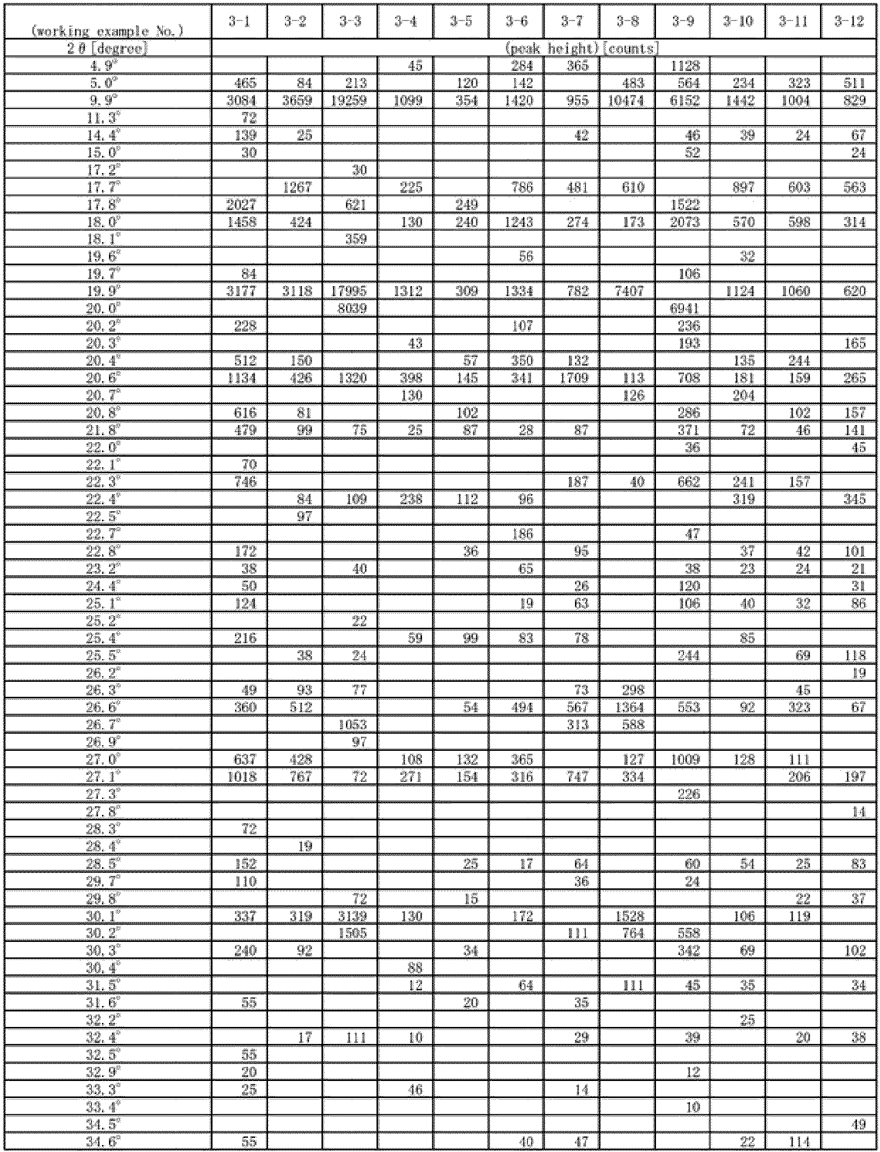

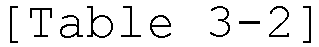

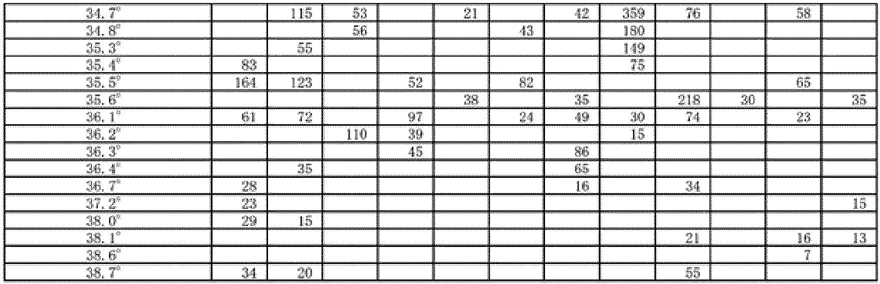

### [Example 3-A]

50 parts by mass of the crystals of pyroxasulfone of Example 3-1, 8 parts by mass of polycarboxylic acid salt, 5 parts of polyoxyethylene distyrylphenyl ether sulfate, 1 part of alkylbenzene sulfonate and the balance of clay were added to adjust the whole to 100 parts by mass, and the mixture was mixed and pulverized using an impact pulverizer to afford a wettable powder.

### [Example 3-B]

40 parts by mass of the crystals of pyroxasulfone of Example 3-1, 1.67 parts by mass of naphthalene sulfonate formalin condensate, 0.83 parts by mass of dioctyl sulfosuccinate, 2.08 parts by mass of polyoxyethylene tristyrylphenyl ether, 8.75 parts of propylene glycol, 0.05 parts of xanthan gum, 0.2 parts of dimethylpolysiloxane, 0.02 parts by mass of a mixture of methylchloroisothiazolone and bronopol, and the balance of water were added to 85 parts by mass of the whole, and the mixture was mixed and pulverized using a wet pulverizer to afford a slurry. To a mixture prepared by adding 0.08 parts by mass of xanthan gum, 0.08 parts by mass of bentonite and the balance of water to adjust the whole to 15 parts by mass was added 85 parts by mass of the slurry, and the resulting mixture was mixed to afford an aqueous suspension concentrate of the present invention.

### [Comparative Example 3-1]

To 50 mL of a solution containing 7.3 g of the compound (4-a) in chloroform was added 12.5 g of m-chloroperoxybenzoic acid having a purity of 70% under icecooling, and the mixture was stirred for 1 hour. The mixture was stirred at an external temperature of 20°C for 16 hours. After the completion of the reaction was confirmed, the reaction solution was poured into water and extracted with chloroform. The obtained organic layer was washed successively with an aqueous sodium hydrogen sulfite solution, an aqueous sodium hydrogen carbonate solution, water and an aqueous sodium chloride solution, and then dried over anhydrous magnesium sulfate. Chloroform was evaporated under reduced pressure to precipitate crystals of pyroxasulfone, and a residue containing crystals of pyroxasulfone was obtained. The obtained residue was washed with n-hexane to afford crystals of pyroxasulfone. In powder X-ray diffraction measurement by a transmission method using Cu-Kα rays, this crystal exhibited a powder X-ray diffraction spectrum in which there are characteristic peaks at diffraction angles 2Θ of 17.7°, 18.0° and 19.9° and the peak height at the peak of 17.7° is maximum among the three peaks. The diffraction angle 2Θ and the peak height at all peaks of the powder X-ray diffraction spectrum are shown in Table 4. The shape of this crystal was acicular. This example is a reproduction experiment of Reference Example 3 described in Patent Document 2. The acicular crystal of pyroxasulfone produced in the same manner as in the present production process had a bulk specific gravity of 0.53 g/mL.

### [Comparative Example 3-2]

Under a nitrogen stream, 2.8 g of the compound (4-a), 8.4 g of acetic acid and 80 mg of sodium tungstate dihydrate were added to a reaction flask. While the internal temperature of the mixture was maintained at 26 to 35°C, 2.2 g of 30% hydrogen peroxide was added dropwise over 20 minutes. The mixture was stirred for 16 hours while the internal temperature was kept at 26 to 35°C. To the reaction mixture was added 4 g of water, and the resulting mixture was stirred at 10°C for 1 hour to precipitate crystals of pyroxasulfone, and a residue containing crystals of pyroxasulfone was obtained. The obtained residue was washed successively with 20 mL of petroleum ether and 20 mL of water to afford crystals of pyroxasulfone. In powder X-ray diffraction measurement by a transmission method using Cu-Kα rays, this crystal exhibited a powder X-ray diffraction spectrum in which there are characteristic peaks at diffraction angles 2Θ of 17.8°, 18.0° and 19.9° and the peak height at the peak of 17.8° is maximum among the three peaks. The diffraction angle 2Θ and the peak height at all peaks of the powder X-ray diffraction spectrum are shown in Table 4. The shape of this crystal was acicular. This example is a reproduction experiment of Example 9C described in Patent Document 7.

### [Comparative Example 3-3]

Pyroxasulfone was dissolved in chloroform. Chloroform was evaporated from the obtained solution under normal pressure to precipitate crystals of pyroxasulfone. The obtained crystals were collected to afford crystals of pyroxasulfone. In powder X-ray diffraction measurement by a transmission method using Cu-Kα rays, this crystal exhibited a powder X-ray diffraction spectrum in which there are characteristic peaks at diffraction angles 2Θ of 17.7°, 18.0° and 19.9° and the peak height at the peak of 17.7° is maximum among the three peaks. The diffraction angle 2Θ and the peak height at all peaks of the powder X-ray diffraction spectrum are shown in Table 4. The shape of this crystal was acicular.

### [Comparative Example 3-4]

Pyroxasulfone was dissolved in dimethyl sulfoxide. Dimethyl sulfoxide was evaporated from the obtained solution under normal pressure to precipitate crystals of pyroxasulfone. The obtained crystals were recovered to afford crystals of pyroxasulfone. In powder X-ray diffraction measurement by a transmission method using Cu-Kα rays, this crystal exhibited a powder X-ray diffraction spectrum in which there are characteristic peaks at diffraction angles 2Θ of 17.8°, 18.0° and 19.9° and the peak height at the peak of 17.8° is maximum among the three peaks. The diffraction angle 2Θ and the peak height at all peaks of the powder X-ray diffraction spectrum are shown in Table 4. The shape of this crystal was acicular.

### [Comparative Example 3-5]

Pyroxasulfone was dissolved in 1,4-dioxane. 1,4-dioxane was evaporated from the obtained solution under normal pressure to precipitate crystals of pyroxasulfone. The obtained crystals were collected to afford crystals of pyroxasulfone. In powder X-ray diffraction measurement by a transmission method using Cu-Kα rays, this crystal exhibited a powder X-ray diffraction spectrum in which there are characteristic peaks at diffraction angles 2Θ of 17.8°, 18.0° and 20.0° and the peak height at the peak of 18.0° is maximum among the three peaks. The diffraction angle 2Θ and the peak height at all peaks of the powder X-ray diffraction spectrum are shown in Table 4. The shape of this crystal was acicular.

### [Comparative Example 3-6]

Pyroxasulfone was dissolved in 2-methyltetrahydrofuran. 2-methyltetrahydrofuran was evaporated from the obtained solution under normal pressure to precipitate crystals of pyroxasulfone. The obtained crystals were collected to afford crystals of pyroxasulfone. In powder X-ray diffraction measurement by a transmission method using Cu-Kα rays, this crystal exhibited a powder X-ray diffraction spectrum in which there are characteristic peaks at diffraction angles 2Θ of 17.8°, 18.0° and 20.0° and the peak height at the peak of 17.8° is maximum among the three peaks. The diffraction angle 2Θ and the peak height at all peaks of the powder X-ray diffraction spectrum are shown in Table 4. The shape of this crystal was acicular.

### [Comparative Example 3-7]

Pyroxasulfone was dissolved in N-methylpyrrolidone. N-methylpyrrolidone was evaporated from the obtained solution under normal pressure to precipitate crystals of pyroxasulfone. The obtained crystals were collected to afford crystals of pyroxasulfone. In powder X-ray diffraction measurement by a transmission method using Cu-Kα rays, this crystal exhibited a powder X-ray diffraction spectrum in which there are characteristic peaks at diffraction angles 2Θ of 17.8°, 18.0° and 20.0° and the peak height at the peak of 18.0° is maximum among the three peaks. The diffraction angle 2Θ and the peak height at all peaks of the powder X-ray diffraction spectrum are shown in Table 4. The shape of this crystal was acicular.

### [Comparative Example 3-8]

Pyroxasulfone was dissolved in tetrahydrofuran. Tetrahydrofuran was evaporated from the obtained solution under normal pressure to precipitate crystals of pyroxasulfone. The obtained crystals were recovered to afford crystals of pyroxasulfone. In powder X-ray diffraction measurement by a transmission method using Cu-Kα rays, this crystal exhibited a powder X-ray diffraction spectrum in which there are characteristic peaks at diffraction angles 2Θ of 17.8°, 18.0° and 19.9° and the peak height at the peak of 17.8° is maximum among the three peaks. The diffraction angle 2Θ and the peak height at all peaks of the powder X-ray diffraction spectrum are shown in Table 4. The shape of this crystal was acicular.

### [Comparative Example 3-9]

Pyroxasulfone was dissolved in trifluoroethanol. Trifluoroethanol was evaporated from the obtained solution under normal pressure to precipitate crystals of pyroxasulfone. The obtained crystals were recovered to afford crystals of pyroxasulfone. In powder X-ray diffraction measurement by a transmission method using Cu-Kα rays, this crystal exhibited a powder X-ray diffraction spectrum in which there are characteristic peaks at diffraction angles 2Θ of 17.8°, 18.0° and 20.0° and the peak height at the peak of 17.8° is maximum among the three peaks. The diffraction angle 2Θ and the peak height at all peaks of the powder X-ray diffraction spectrum are shown in Table 4. The shape of this crystal was acicular.

### [Comparative Example 3-10]

Pyroxasulfone was dissolved in carbon disulfide. Carbon bisulfide was evaporated from the obtained solution under normal pressure to precipitate crystals of pyroxasulfone. The obtained crystals were recovered to afford crystals of pyroxasulfone. In powder X-ray diffraction measurement by a transmission method using Cu-Kα rays, this crystal exhibited a powder X-ray diffraction spectrum in which there are characteristic peaks at diffraction angles 2Θ of 17.7°, 18.0° and 19.9° and the peak height at the peak of 17.7° is maximum among the three peaks. The diffraction angle 2Θ and the peak height at all peaks of the powder X-ray diffraction spectrum are shown in Table 4. The shape of this crystal was acicular.

### [Comparative Example 3-11]

Pyroxasulfone was dissolved in acetone. Double amount of carbon disulfide was added to the obtained solution to precipitate crystals of pyroxasulfone. The obtained crystals were dried to afford crystals of pyroxasulfone of the present invention. In powder X-ray diffraction measurement by a transmission method using Cu-Kα rays, this crystal exhibited a powder X-ray diffraction spectrum in which there are characteristic peaks at diffraction angles 2θ of 17.7°, 18.0° and 20.0° and the peak height at the peak of 17.7° is maximum among the three peaks. The diffraction angle 2Θ and the peak height at all peaks of the powder X-ray diffraction spectrum are shown in Table 4. The shape of this crystal was acicular.

### [Comparative Example 3-12]

Pyroxasulfone was dissolved in dichloromethane. Double amount of carbon disulfide was added to the obtained solution to precipitate crystals of pyroxasulfone. The obtained crystals were dried to afford crystals of pyroxasulfone of the present invention. In powder X-ray diffraction measurement by a transmission method using Cu-Kα rays, this crystal exhibited a powder X-ray diffraction spectrum in which there are characteristic peaks at diffraction angles 2Θ of 17.8°, 18.0° and 20.0° and the peak height at the peak of 17.8° is maximum among the three peaks. The diffraction angle 2Θ and the peak height at all peaks of the powder X-ray diffraction spectrum are shown in Table 4. The shape of this crystal was acicular.

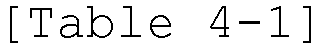

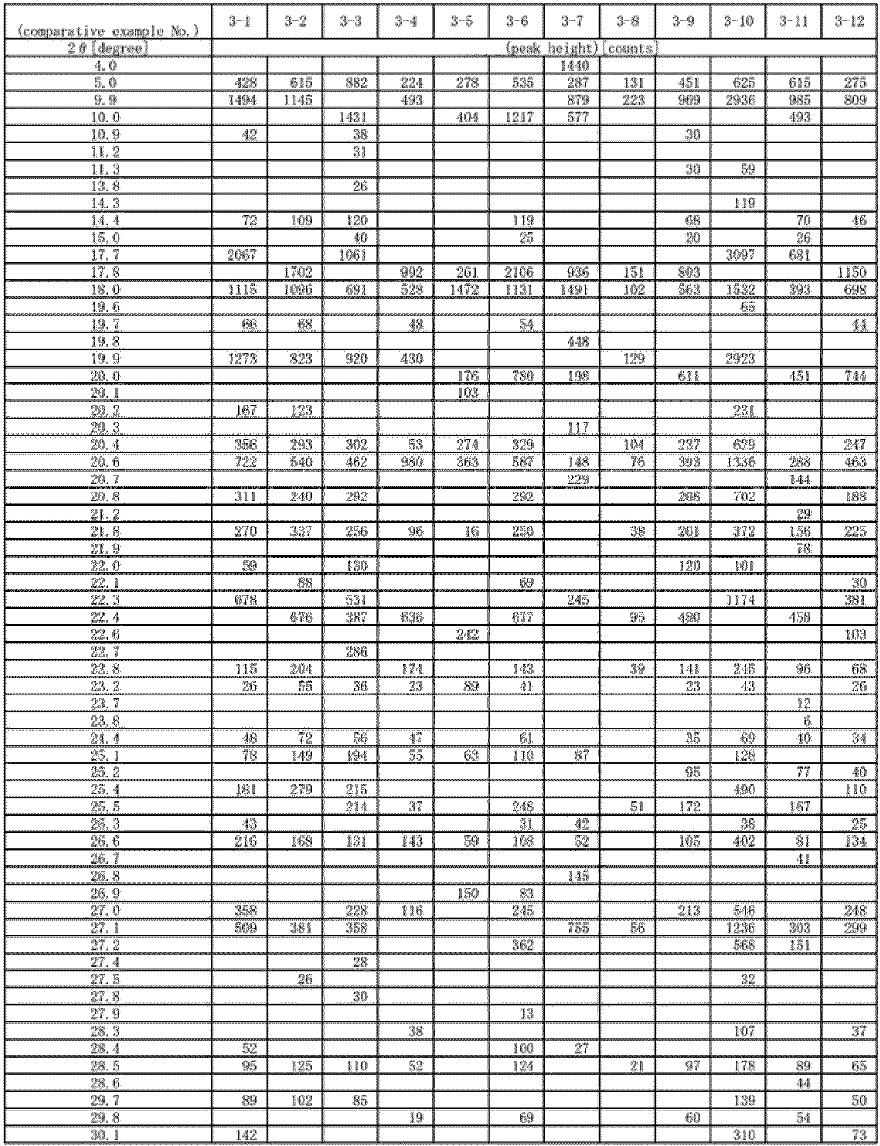

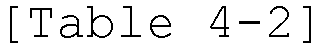

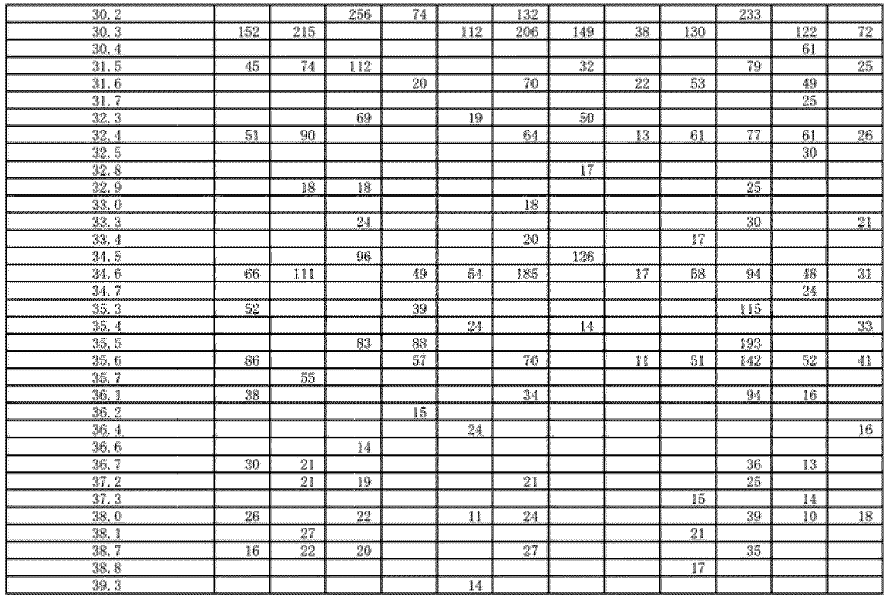

### [Comparative Example 3-X]

50 parts by mass of the crystals of pyroxasulfone of Comparative Example 3-3, 4 parts by mass of polycarboxylic acid salt, 1 part of polyoxyethylene distyrylphenyl ether sulfate and the balance of clay were added to adjust the whole to 100 parts by mass, and the mixture was mixed and pulverized using an impact pulverizer to afford a wettable powder.

### [Comparative Example 3-Y]

40 parts by mass of the crystals of pyroxasulfone of Comparative Example 3-3, 1.67 parts by mass of naphthalene sulfonate formalin condensate, 0.83 parts by mass of dioctyl sulfosuccinate, 2.08 parts by mass of polyoxyethylene tristyrylphenyl ether, 8.75 parts of propylene glycol, 0.05 parts of xanthan gum, 0.2 parts of dimethylpolysiloxane, 0.02 parts by mass of a mixture of methylchloroisothiazolone and bronopol, and the balance of water were added to adjust the whole to 85 parts by mass, and the mixture was mixed and pulverized using a wet pulverizer to afford a slurry. To a mixture prepared by adding 0.08 parts by mass of xanthan gum, 0.08 parts by mass of bentonite and the balance of water and adjusting the whole to 15 parts by mass was added 85 parts by mass of the slurry, and the resulting mixture was mixed to afford an aqueous suspension concentrate of the present invention.

### [Test Example 3-1: Wettability]

In a 500 mL beaker was placed 200 mL of 3 degree hard water at 20°C. Five grams of the wettable powder of Example 3-A or Comparative Example 3-X having passed through a #40 mesh standard sieve in advance was gently dropped into the beaker from a position of about 10 cm above the water surface so as to spread thinly. The time between the end of the dropping of the sample and the sinking of the whole sample under the water surface was measured. Further, the contents in the beaker are stirred with a glass rod and the homogeneity of the suspended state was observe. The results are shown in Table 5.

**[Table 5]**

| Formulation | Wettability | Suspension uniformity |
|---|---|---|
| Working Example 3-A | 4.5min | Good |
| Comparative Example 3-X | 7.2min | Some lumps are observed |

### [Test Example 3-2: Redispersibility of diluted liquid]

0.5 mL of the aqueous suspension concentrates of Example 3-B and of Comparative Example 3-Y were each taken and placed in a 100 mL stoppered graduated cylinder, and 3 degrees hard water was added to adjust the whole volume to 100 mL. Each stoppered graduated cylinder was inverted 30 times at 30 rpm and was allowed to stand at a constant temperature of 20°C for 1 day. Then, the stoppered graduated cylinder was inverted at 30 rpm while the bottom of the stoppered graduated cylinder was observed, and the number of inverted operations required until the solid adhering to the bottom of the stoppered graduated cylinder was no longer observed was recorded. In addition, 50 mL of the aqueous suspension concentrates of Examples 3-B and Comparative Examples 3-Y were each taken and sealed in a 50 mL screw tube, and stored at rest for 2 weeks at a constant temperature of 54°C, and then were subjected to the same test as described above. The results are shown in Table 6.

**[Table 6]**

| Formulation | Required number of Revolution | |
|---|---|---|
| | Initial | After Storage |
| Working Example 3-B | 16 | 28 |
| Comparative Example 3-Y | 39 | 51 |

All publications, patents, and patent applications described herein are hereby fully incorporated by reference in their entirety for the purpose of describing and disclosing the methods described in those publications, patents, and patent applications that may be used in connection with the description herein. To the extent necessary to understand or complete the disclosure of the present invention, all publications, patents, and patent applications described herein are expressly incorporated herein by reference to the same extent as if each were individually incorporated. All publications, patents, and patent applications discussed above and throughout this specification are provided solely for disclosure prior to the filing date of this application.

Any processes and reagents similar or equivalent to those described herein can be employed in the practice of the present invention. Accordingly, the present invention is not to be limited by the foregoing description, but is intended to be defined by the claims and their equivalents. Those equivalents fall within the scope of the present invention as defined by the appended claims.

## Claims

1. A crystal of pyroxasulfone wherein the crystal exhibits a spectrum having peaks at diffraction angles 2Θ at least in the range of 17.7 to 17.8°, 18.0 to 18.1° and 19.9 to 20.0° in powder X-ray diffraction measurement by a transmission method using Cu-Kα ray, and the peak height of 19.9 to 20.0° is maximum among the three peaks.

2. The crystal according to claim 1, wherein the ratio of the peak height at 19.9 to 20.0° to the peak height at 17.7 to 17.8° is 1 : 0.02 to 1 : 0.95 in the powder X-ray diffraction measurement.

3. The crystal according to claim 2, wherein the ratio of the peak height at 19.9 to 20.0° to the peak height at 18.0 to 18.1° is 1 : 0.02 to 1 : 0.95 in the powder X-ray diffraction measurement.

4. The crystal according to any one of claims 1 to 3, wherein the crystal has a short columnar or columnar appearance.

5. An agrochemical composition comprising the crystal of pyroxasulfone according to any one of claims 1 to 4 and a surfactant.

6. A process for producing a crystal of pyroxasulfone according to any one of claims 1 to 4, comprising:
a step of distilling off an organic solvent from a solution of pyroxasulfone comprising the organic solvent to precipitate pyroxasulfone,
wherein the organic solvent is selected from nitriles, carboxylic acids, carboxylic acid esters, ketones, amides and dihalogenated aliphatic hydrocarbons.

7. The process according to claim 6,
wherein the organic solvent is one selected from C2-C5 alkanenitriles, C1-C4 carboxylic acids, C1-C4 alkyl C1-C4 carboxylates, C1-C4 alkyl C1-C4 alkyl ketones, N,N-di(C1-C4 alkyl) C1-C4 alkanamides and C1-C4 dihaloalkanes.

8. The process according to claim 6,
wherein the organic solvent is one selected from acetonitrile, acetic acid, ethyl acetate, methyl isobutyl ketone, N,N-dimethylformamide, N,N-dimethylacetamide and dichloromethane.

9. A process for producing a crystal of pyroxasulfone according to any one of claims 1 to 4, comprising:
a step of adding an antisolvent for pyroxasulfone to a solution of pyroxasulfone to precipitate pyroxasulfone,
wherein the antisolvent for pyroxasulfone is selected from ethers, carboxylic acid esters, ketones, aromatic hydrocarbon derivatives, aliphatic hydrocarbons, alcohols and water,
wherein the solution of pyroxasulfone comprises an organic solvent selected from nitriles, ketones and carboxylic acid esters.

10. The process according to claim 9,
wherein the antisolvent for pyroxasulfone is C1-C4 alcohol.

11. The process according to claim 9 or 10,
wherein the antisolvent for pyroxasulfone is ethanol or isopropanol.

12. A process for producing an agrochemical composition having a dosage form of a wettable powder, comprising:
a step of pulverizing a powder comprising the crystal of pyroxasulfone according to any one of claims 1 to 4, and
a step of mixing the whole raw material comprising the pulverized crystal of pyroxasulfone, a surfactant and a solid carrier to homogenize the mixture.

13. A process for producing an agrochemical composition having a dosage form of a water-dispersible granule, comprising:
a step of pulverizing a powder or a slurry comprising the crystal of pyroxasulfone according to any one of claims 1 to 4,
a step of, while homogenizing the whole raw material comprising the pulverized crystal of pyroxasulfone, a surfactant and a solid carrier, further adding a slight amount of water to the whole raw material mixture and kneading the mixture,
a step of granulating the kneaded product obtained in the preceding step, and
a step of drying the granulated product obtained in the preceding step.

14. A process for producing an agrochemical composition having a dosage form of an aqueous suspension concentrate, comprising:
a step of pulverizing a powder or slurry comprising the crystal of pyroxasulfone according to any one of claims 1 to 4, and
a step of mixing the whole raw material comprising the pulverized crystal of pyroxasulfone, a surfactant and water to homogenize the mixture.

15. A process for producing an agrochemical composition having a dosage form of an oil dispersion, comprising:
a step of pulverizing a powder or slurry comprising the crystal of pyroxasulfone according to any one of claims 1 to 4, and
a step of mixing the whole raw material comprising the pulverized crystal of pyroxasulfone, a surfactant and an oil-based dispersion medium to homogenize the mixture.
